# EUROPÄISCHE PATENTANMELDUNG

(11) **EP 1 164 128 A1**
(43) Veröffentlichungstag der Anmeldung: **19.12.2001**
(21) Anmeldenummer: 01122556.2
(22) Anmeldetag: 05.10.1994
(51) Int. Cl.: C07C 311/08, C07C 255/60

(54) **4-Cyanophenyliminoheterocyclen**

(30) Priorität: 18.10.1993 DE 4335438
(62) Teilanmeldung aus: 94115645.7
(71) Anmelder: BAYER AG, 51368 Leverkusen (DE)
(72) Erfinder: Schallner, Otto, Dr., 40789 Monheim (DE); Andree, Roland, Dr., 40764 Langenfeld (DE); Drewes, Mark Wilhelm, Dr., 40764 Langenfeld (DE); Dollinger, Markus, Dr., 51381 Leverkusen (DE); Santel, Hans-Joachim, Dr., 51371 Leverkusen (DE)

(57) **Zusammenfassung**

Die Erfindung betrifft neue Cyanoarylamine der Formel (Xa) 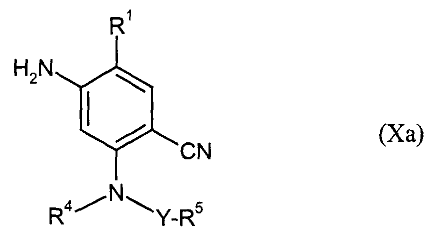 in welcher
- R¹, R⁴, R⁵ und Y: eine in der Beschreibung angegebene Bedeutung haben,
sowie ein Verfahren zu deren Herstellung.

## Beschreibung

Die Erfindung betrifft neue 4-Cyanophenyliminoheterocyclen, Verfahren zu ihrer Herstellung und ihre Verwendung als Herbizide sowie neue Zwischenprodukte für die Synthese.

Es ist bereits bekannt, daß bestimmte Halogenaryliminoheterocyclen herbizide Eigenschaften aufweisen (vgl. EP-A 238711, EP-A 273417, EP-A 312064, EP-A 410265, EP-A 457714, WO-A 92/21684).

Die herbizide Wirksamkeit bzw. die Verträglichkeit dieser bekannten Verbindungen gegenüber Kulturpflanzen ist jedoch nicht immer ganz zufriedenstellend.

Es wurden nun neue 4-Cyanophenyliminoheterocyclen der allgemeinen Formel (I) gefunden, 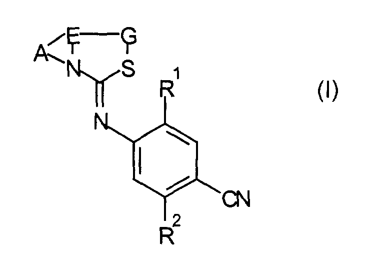 in welcher
- R¹: für Wasserstoff oder Halogen steht,
- R²: für Halogen, Cyano, Hydroxy, Amino oder eine der nachstehenden Gruppierungen -X-R³, -N(R⁴)-CO-R⁵ oder -N(R⁴)-SO₂R⁵ steht, wobei
- X: für Sauerstoff, Schwefel oder eine Einfachbindung steht,

- R³: für jeweils gegebenenfalls substituiertes Alkyl, Alkenyl, Alkinyl, Cycloalkyl, Cycloalkylalkyl, Aryl, Arylalkyl, Heterocyclyl oder Heterocyclylalkyl steht,
- R⁴: für Wasserstoff oder Alkyl steht und
- R⁵: für jeweils gegebenenfalls substituiertes Alkyl, Alkenyl, Alkinyl, Cycloalkyl, Cycloalkylalkyl, Aryl oder Arylalkyl steht,
- A: für jeweils gegebenenfalls substituiertes Alkandiyl oder Alkendiyl steht,
- E: für Stickstoff oder Kohlenstoff steht und
- G: für Stickstoff oder für Kohlenstoff steht, welcher über eine exo-Einfachbindung mit Wasserstoff oder Alkyl oder über eine exo-Doppelbindung mit Sauerstoff oder Schwefel verbunden ist,
wobei für den Fall, daß E für Stickstoff steht, A nicht für gegebenenfalls substituiertes Trimethylen steht.

Man erhält die neuen 4-Cyanophenyliminoheterocyclen der allgemeinen Formel (I), wenn man
a) substituierte Thiocarbonylaminoverbindungen der allgemeinen Formel (II) 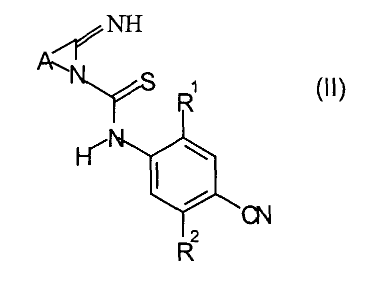 in welcher
   A, R¹ und R² die oben angegebenen Bedeutungen haben,
   mit Oxidationsmitteln bzw. Dehydrierungsmitteln, gegebenenfalls in Gegenwart eines Reaktionshilfsmittels und gegebenenfalls in Gegenwart eines Verdünnungsmittels umsetzt, oder wenn man
b) substituierte Thiocarbonylaminoverbindungen der allgemeinen Formel (III) 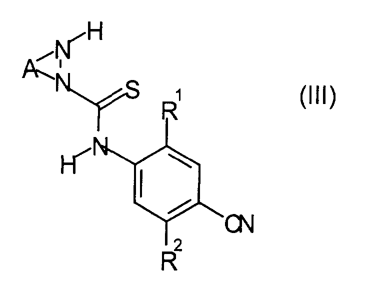 in welcher
   A, R¹ und R² die oben angegebenen Bedeutungen haben,
   mit Phosgen, gegebenenfalls in Gegenwart eines Verdünnungsmittels umsetzt,
   oder wenn man
c) 4-Cyanophenyliminoheterocyclen der allgemeinen Formel (Ia) 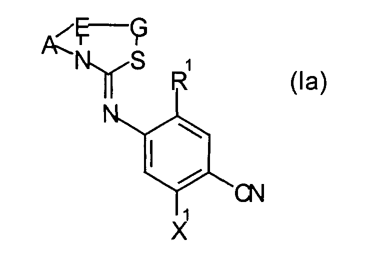 in welcher
   R¹, A, E und G die oben angegebenen Bedeutungen haben und
   X¹ für Halogen steht,
   mit nucleophilen Verbindungen der allgemeinen Formeln (IV) oder (V)

      H-X-R³ (IV)

      H-N(R⁴)-SO₂R⁵ (V)

      in welchen
   X, R³, R⁴ und R⁵ die oben angegebenen Bedeutungen haben,
   - oder mit Alkalimetallsalzen dieser Verbindungen -
   gegebenenfalls in Gegenwart eines Säureakzeptors und gegebenenfalls in Gegenwart eines Verdünnungsmittels umsetzt.

Die neuen Verbindungen der Formel (I) können auch durch Umsetzung von Imino-heterocyclen der allgemeinen Formel (VI) mit 4-Halogen-benzonitrilen der allgemeinen Formel (VII) gemäß folgendem Formelschema erhalten werden (R¹, R², A, E und G wie oben definiert, X²: Halogen):

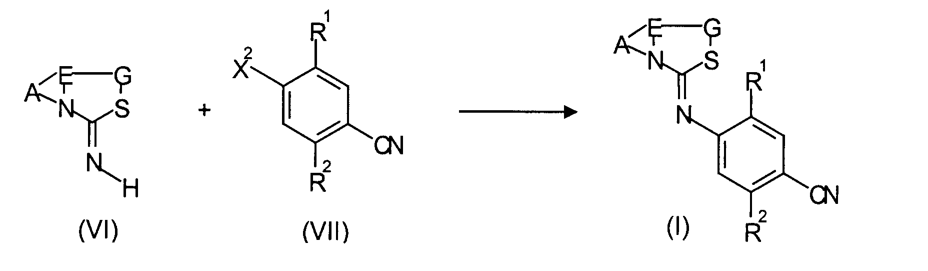

Die neuen 4-Cyanophenyliminoheterocyclen der allgemeinen Formel (I) zeichnen sich durch starke und selektive herbizide Wirksamkeit aus.

In den Definitionen sind die gesättigten oder ungesättigten Kohlenwasserstoffketten, wie Alkyl, Alkandiyl, Alkenyl, Alkendiyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I), in welcher
- R¹: für Wasserstoff, Fluor, Chlor oder Brom steht,
- R²: für Fluor, Chlor, Brom, Cyano, Hydroxy, Amino oder eine der nachstehenden Gruppierungen -X-R³, -N(R⁴)-CO-R⁵ oder -N(R⁴)-SO₂R⁵ steht, wobei
X für Sauerstoff, Schwefel oder eine Einfachbindung steht,
R³ für gegebenenfalls durch Fluor, Chlor, Brom, Cyano, Carboxy, C₁-C₄-Alkoxy, C₁-C₄-Alkoxy-C₁-C₄-alkoxy, C₁-C₄-Alkylthio, C₁-C₄-Alkyl-carbonyl, C₁-C₄-Alkoxy-carbonyl oder C₅-C₆-Cycloalkyloxy-carbonyl substituiertes Alkyl mit 1 bis 8 Kohlenstoffatomen steht,
R³ weiterhin für jeweils gegebenenfalls durch Fluor, Chlor oder Brom substituiertes Alkenyl oder Alkinyl mit jeweils 2 bis 8 Kohlenstoffatomen steht,
R³ weiterhin für jeweils gegebenenfalls durch Fluor, Chlor, Brom, Cyano, Carboxy, C₁-C₄-Alkyl oder C₁-C₄-Alkoxy-carbonyl substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im Alkylteil oder für jeweils gegebenenfalls substituiertes Phenyl, Benzyl, Phenylethyl, Phenylpropyl oder Phenylbutyl, oder für jeweils gegebenenfalls substituiertes Heterocyclyl, Heterocyclylmethyl, Heterocyclylethyl, Heterocyclylpropyl oder Heterocyclylbutyl mit jeweils 3 bis 8 Ringgliedern steht, von denen mindestens eines ein Sauerstoff-, Schwefel- oder Stickstoffatom und gegebenenfalls 1 bis 3 weitere Stickstoffatome sind, wobei die Heterocyclylgruppe gesättigt oder ungesättigt sein kann und die vorgenannten cyclischen Gruppen gegebenenfalls Substituenten aus der folgenden Aufzählung enthalten:
Halogen, Cyano, Nitro, Carboxy, Carbamoyl, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, C₁-C₄-Alkoxy-carbonyl,

R⁴ für Wasserstoff oder für Alkyl mit 1 bis 8 Kohlenstoffatomen steht und
R⁵ für gegebenenfalls durch Fluor, Chlor, Brom, Cyano, Carboxy, C₁-C₄-Alkoxy, C₁-C₄-Alkoxy-C₁-C₄-alkoxy, C₁-C₄-Alkylthio, C₁-C₄-Alkyl-carbonyl oder C₁-C₄-Alkoxy-carbonyl substituiertes Alkyl mit 1 bis 8 Kohlenstoffatomen steht,
R⁵ weiterhin für jeweils gegebenenfalls durch Fluor, Chlor oder Brom substituiertes Alkenyl oder Alkinyl mit jeweils 2 zu 8 Kohlenstoffatomen steht,
R⁵ weiterhin für jeweils gegebenenfalls durch Fluor, Chlor, Brom, Cyano, Carboxy, C₁-C₄-Alkyl oder C₁-C₄-Alkoxy-carbonyl substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im Alkylteil oder für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl, Phenylmethyl, Phenoxy, Phenylthio, Phenylsulfinyl oder Phenylsulfonyl (welche jeweils gegebenenfalls durch Fluor, Chlor, Brom, Cyano, Methyl, Methoxy, Trifluormethyl, Difluormethoxy und/oder Trifluormethoxy substituiert sind) substituiertes Aryl oder Arylalkyl mit 6 oder 10 Kohlenstoffatomen im Arylteil und gegebenenfalls 1 bis 4 Kohlenstoffatomen im Alkylteil steht,
A für jeweils gegebenenfalls durch Fluor, Chlor, Brom oder C₁-C₄-Alkyl (welches gegebenenfalls durch Fluor und/oder Chlor substituiert ist) substituiertes Alkandiyl oder Alkendiyl mit jeweils 2 bis 6 Kohlenstoffatomen steht,
E für Stickstoff oder Kohlenstoff steht und
G für Stickstoff oder Kohlenstoff steht, welcher über eine exo-Einfachbindung mit Wasserstoff oder C₁-C₆-Alkyl oder über eine exo-Doppelbindung mit Sauerstoff oder Schwefel verbunden ist,
wobei für den Fall, daß E für Stickstoff steht, A nicht für gegebenenfalls substituiertes Trimethylen steht.

Die Erfindung betrifft insbesondere Verbindungen der Formel (I), in welcher
- R¹: für Wasserstoff, Fluor oder Chlor steht,
- R²: für Fluor, Chlor, Brom, Cyano, Hydroxy, Amino, oder eine der nachstehenden Gruppierungen -X-R³ -N(R⁴)-CO-R⁵ oder -N(R⁴)-SO₂R⁵ steht, wobei
X für Sauerstoff, Schwefel oder eine Einfachbindung steht,
R³ für jeweils gegebenenfalls durch Fluor, Chlor, Brom, Cyano, Carboxy, Methoxy, Ethoxy, Methoxyethoxy, Ethoxyethoxy, Methylthio, Ethylthio, Acetyl, Propionyl, Methoxycarbonyl oder Ethoxycarbonyl substituiertes Methyl, Ethyl, n- oder i-Propyl, n-, i-, s- oder t-Butyl steht,
R³ weiterhin für jeweils gegebenenfalls durch Fluor oder Chlor substituiertes Allyl, Crotonyl, 1-Methyl-allyl, Propargyl oder 1-Methyl-propargyl steht,
R³ weiterhin für jeweils gegebenenfalls durch Fluor, Chlor, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R⁴ für Wasserstoff, Methyl, Ethyl, n- oder i-Propyl, n-, i- oder s-Butyl steht,
R⁵ für jeweils gegebenenfalls durch Fluor, Chlor, Brom, Cyano, Carboxy, Methoxy, Ethoxy, Methoxyethoxy, Ethoxyethoxy, Methylthio, Ethylthio, Acetyl, Propionyl, Methoxycarbonyl oder Ethoxycarbonyl substituiertes Methyl, Ethyl, n- oder i-Propyl, n-, i-, s- oder t-Butyl steht,
R⁵ weiterhin für jeweils gegebenenfalls durch Fluor oder Chlor substituiertes Allyl, Crotonyl, 1-Methyl-allyl, Propargyl oder 1-Methylpropargyl steht,
R⁵ weiterhin für jeweils gegebenenfalls durch Fluor, Chlor, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R⁵ weiterhin für jeweils gegebenenfalls durch Fluor, Chlor, Brom, Cyano, Methyl, Ethyl, n- oder i-Propyl, n-, i-, s- oder t-Butyl, Methoxy, Ethoxy, Methylthio, Ethylthio, Methylsulfinyl, Ethylsulfinyl, Methylsulfonyl, Ethylsulfonyl, Trifluormethyl, Difluormethoxy, Trifluormethoxy, Trifluormethylthio, Trifluormethylsulfinyl, Trifluormethylsulfonyl, Dimethylaminosulfonyl, Methoxycarbonyl, Ethoxycarbonyl, Phenyl, Phenoxy oder Phenylsulfonyl substituiertes Phenyl, Naphthyl, Benzyl oder Phenylethyl steht,
A für jeweils gegebenenfalls durch Fluor, Chlor, Brom, Methyl, Ethyl, n- oder i-Propyl substituiertes Propan-1,3-diyl (Trimethylen), Butan-1,4-diyl (Tetramethylen), 1-Propen-1,3-diyl, 1-Buten-1,4-diyl oder 2-Buten-1,4-diyl steht,
E für Stickstoff oder Kohlenstoff steht und
G für Stickstoff oder Kohlenstoff steht, welcher über eine exo-Einfachbindung mit Wasserstoff, Methyl, Ethyl, n- oder i-Propyl, n-, i-, s- oder t-Butyl, oder über eine exo-Doppelbindung mit Sauerstoff verbunden ist,
wobei für den Fall, daß E für Stickstoff steht, A nicht für gegebenenfalls substituiertes Trimethylen steht.

Eine besonders bevorzugte Gruppe von Verbindungen der Formel (I) sind die Verbindungen der allgemeinen Formel (IA) 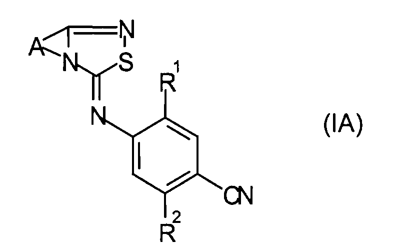 in welcher
R¹, R² und A die oben als insbesondere bevorzugt angegebene Bedeutung haben.
Eine weitere besonders bevorzugte Gruppe von Verbindungen der Formel (I) sind die Verbindungen der allgemeinen Formel (IB) 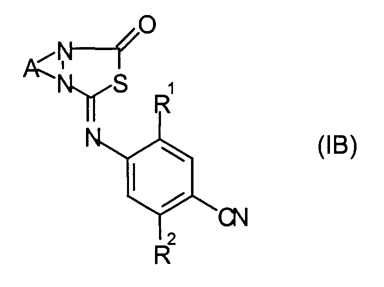
in welcher
R¹, R² und A die oben als insbesondere bevorzugt angegebene Bedeutung haben.
Beispiele für die erfindungsgemäßen Verbindungen der Formel (I) sind in der nachstehenden Tabelle 1 aufgeführt.

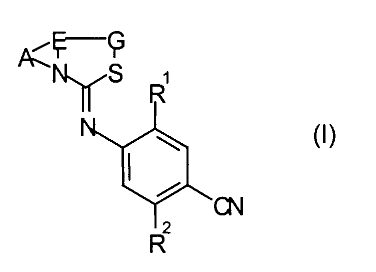

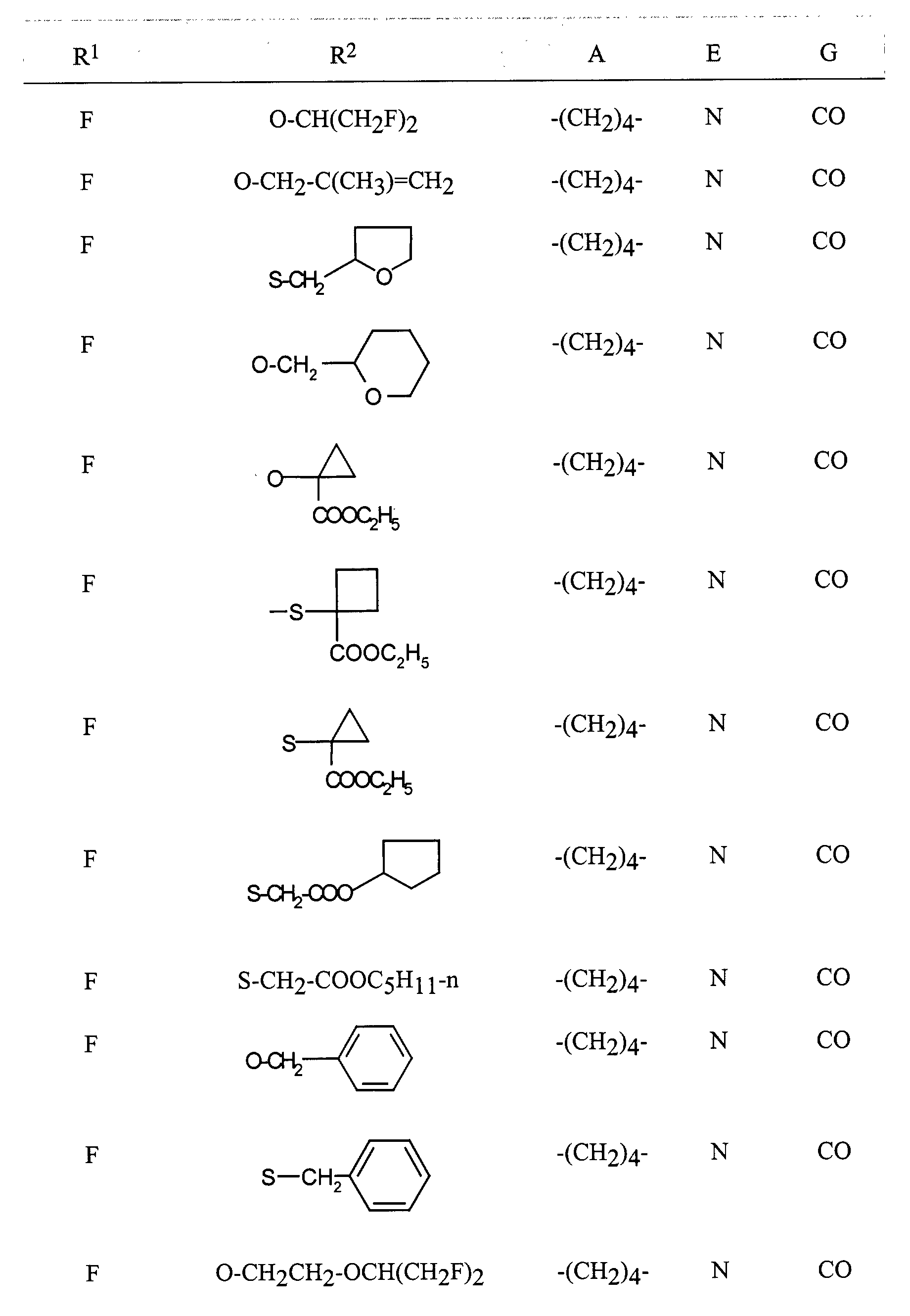

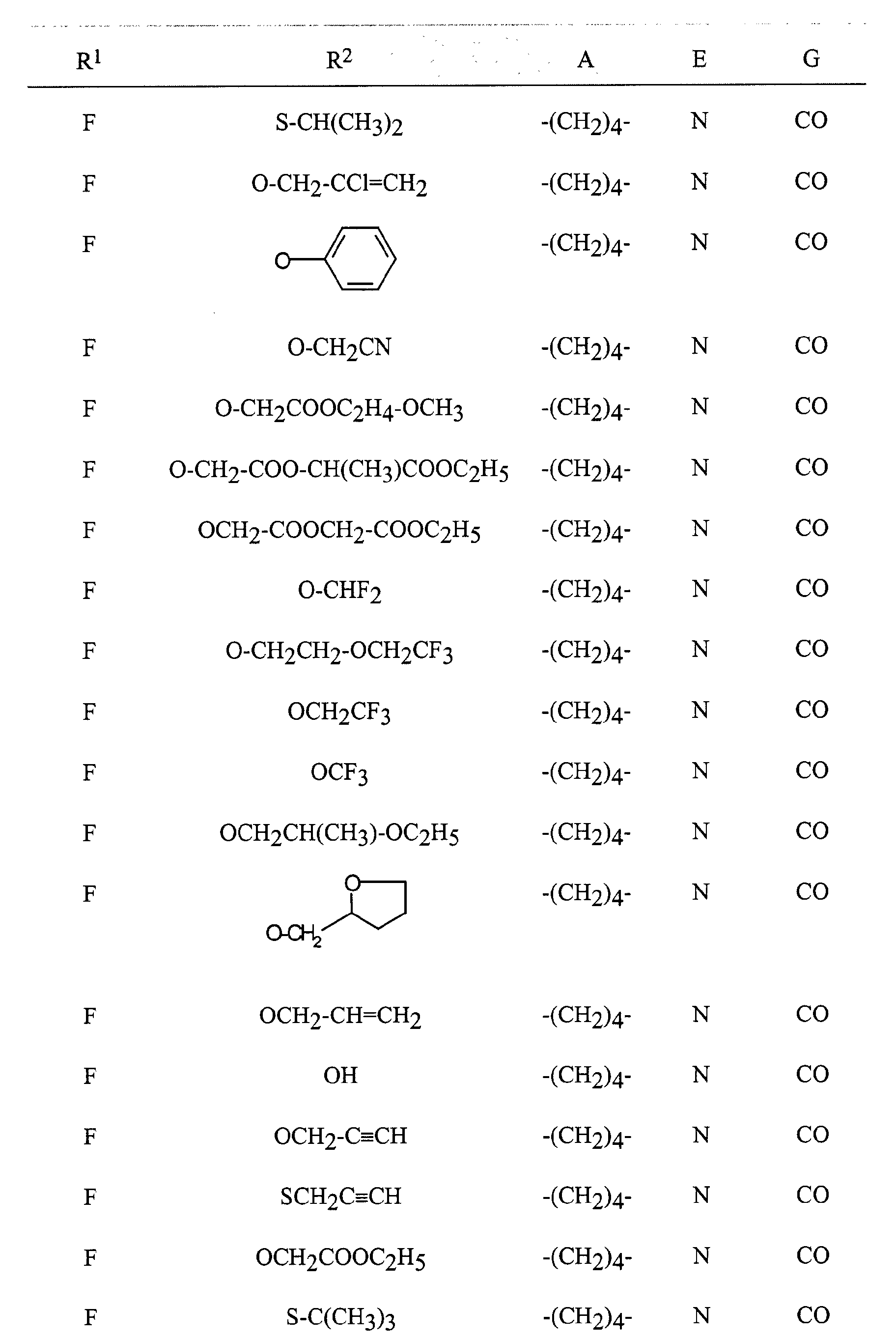

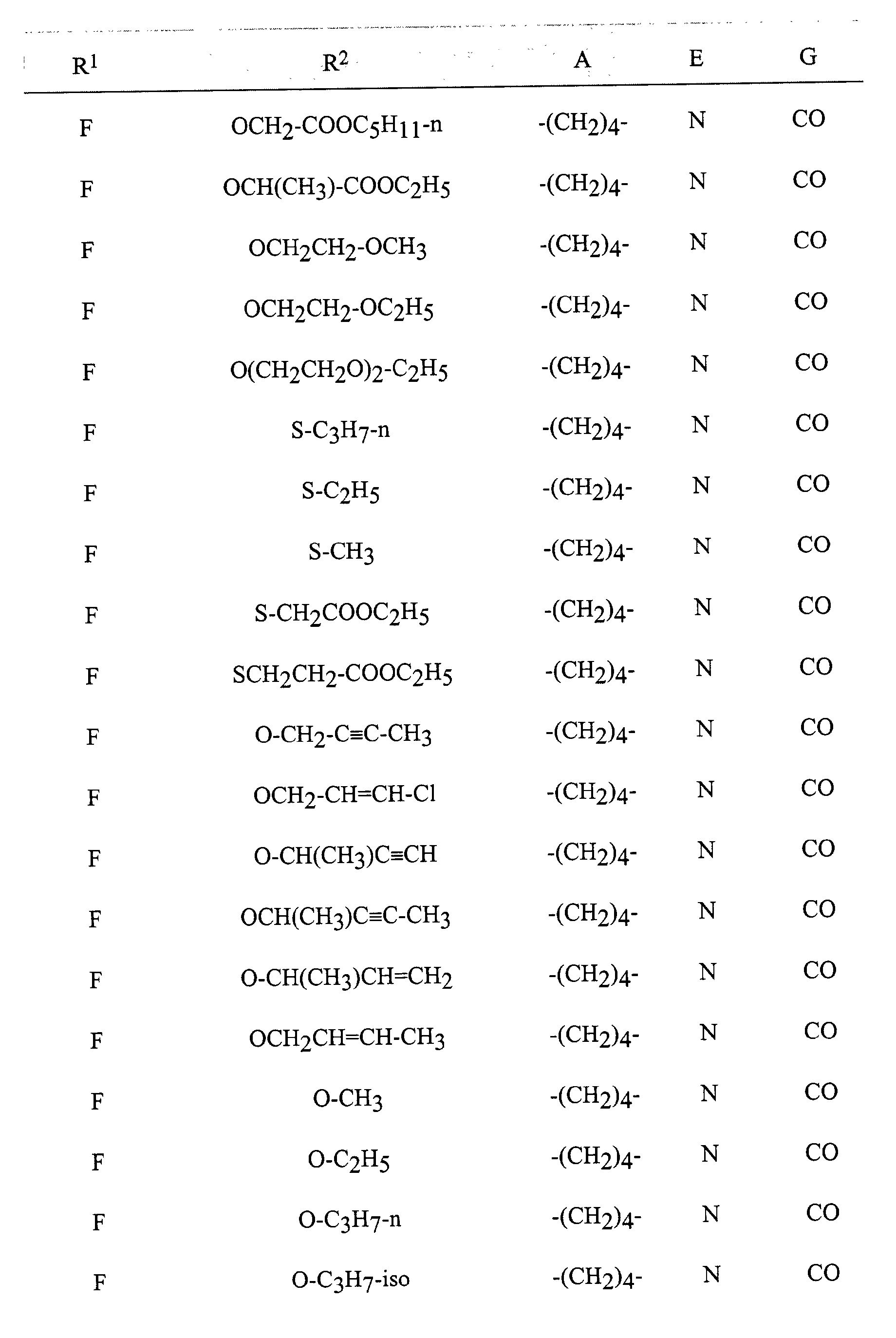

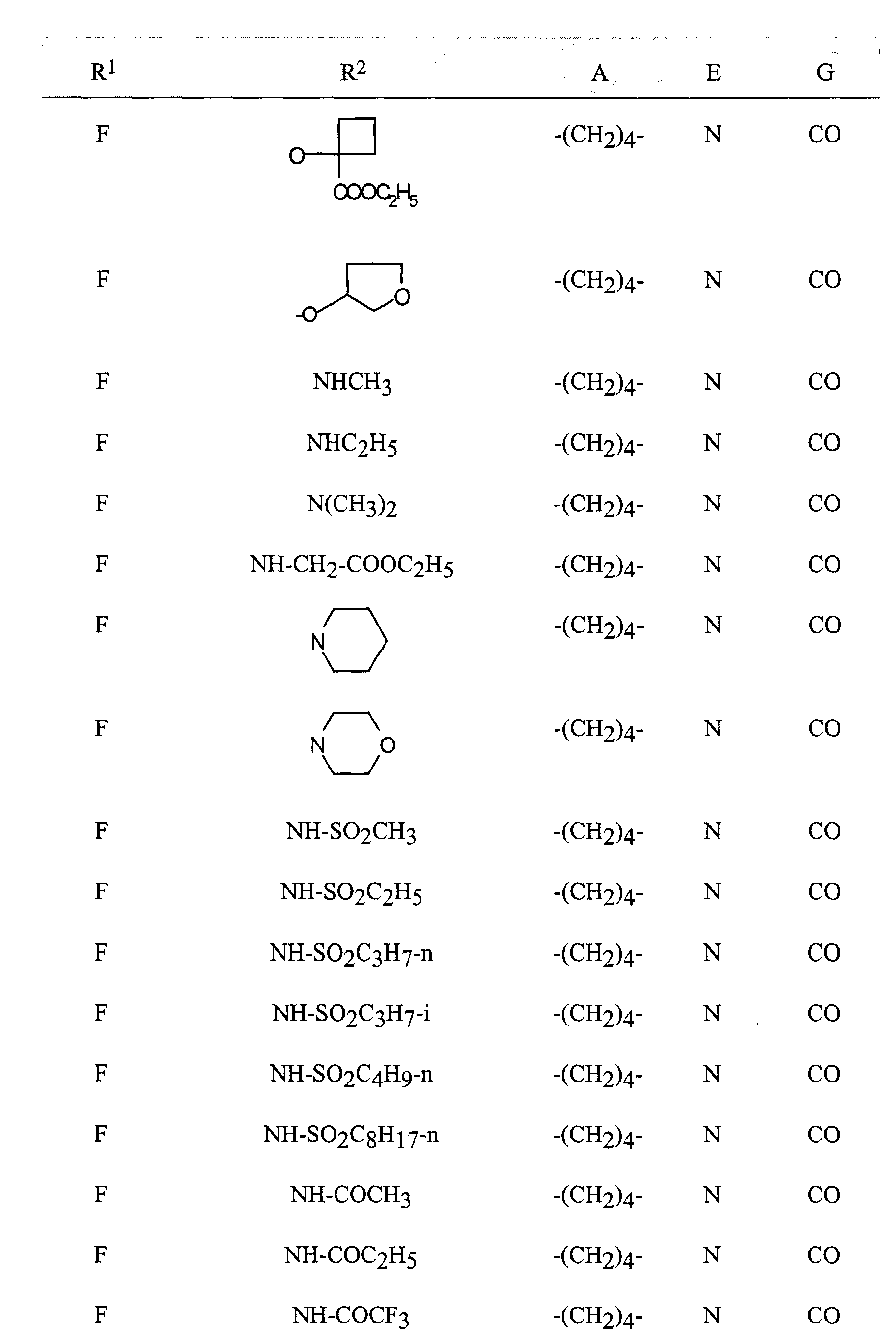

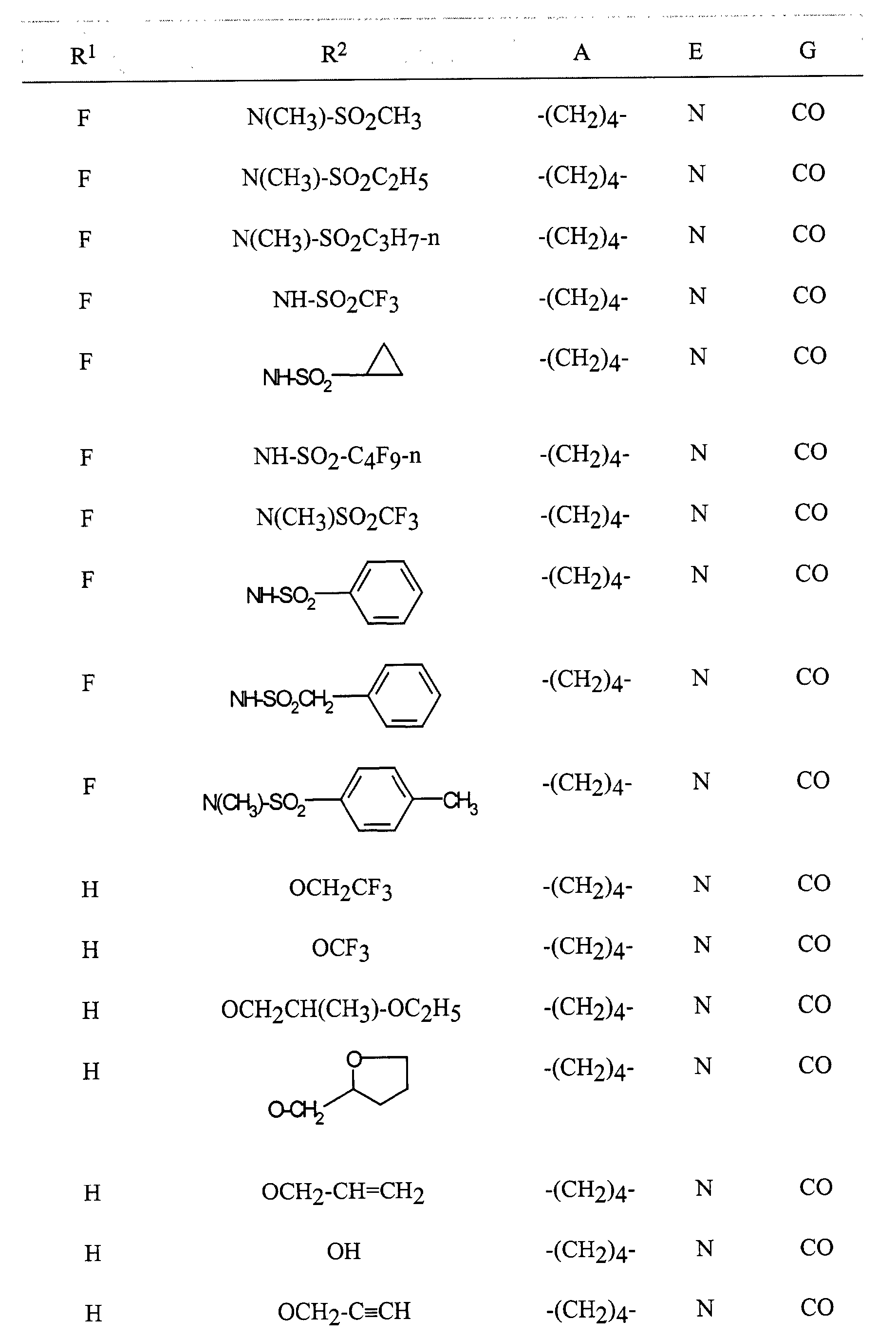

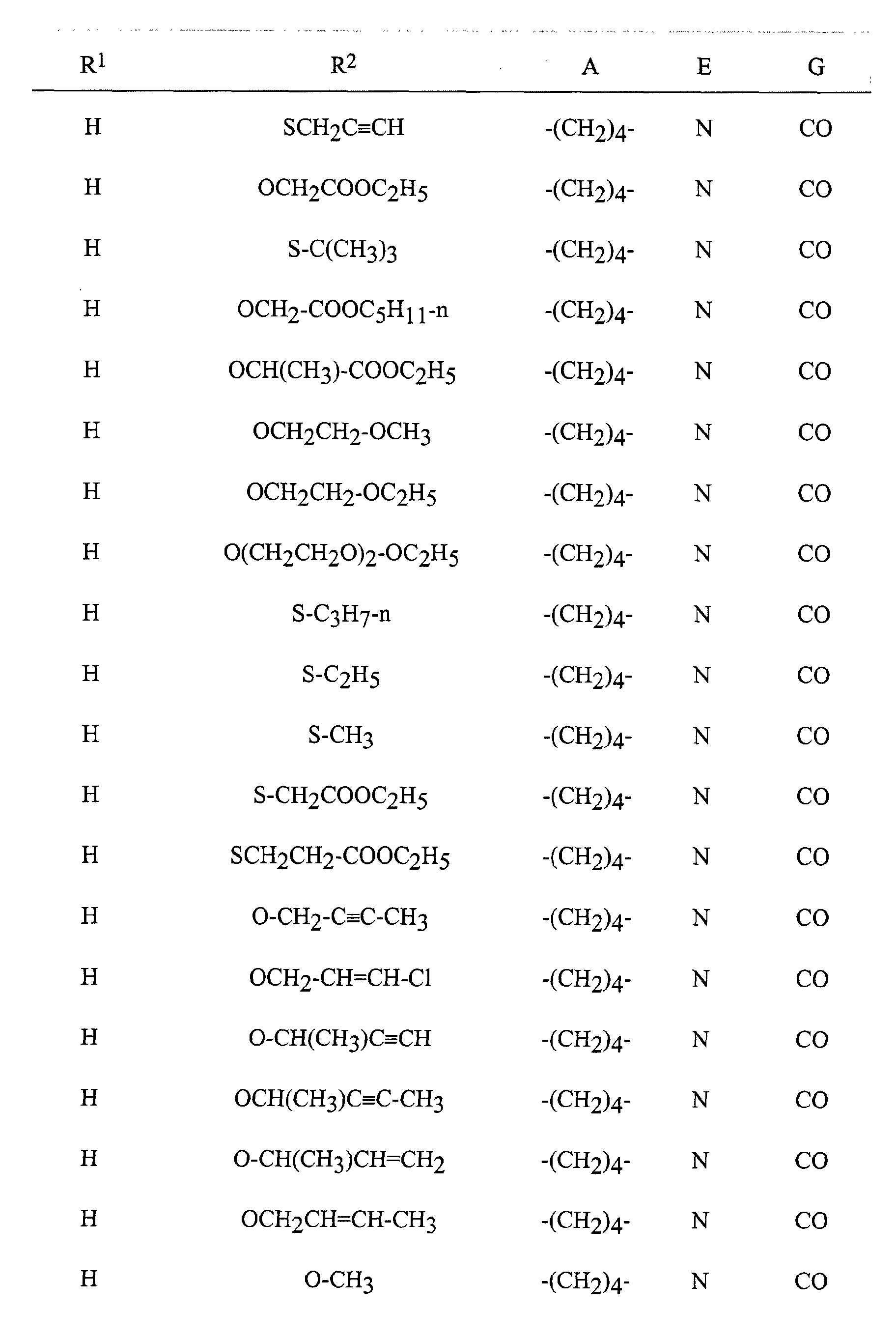

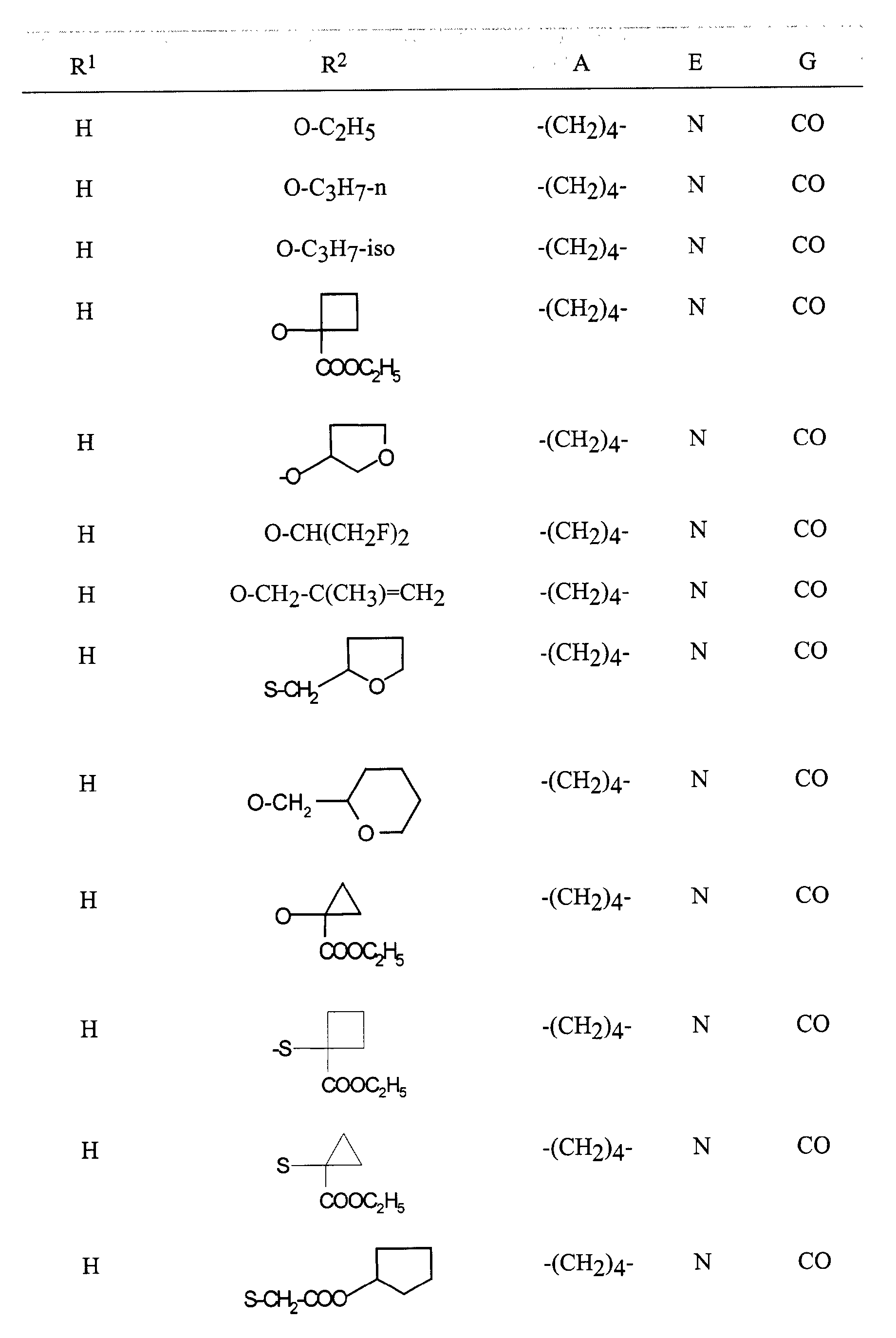

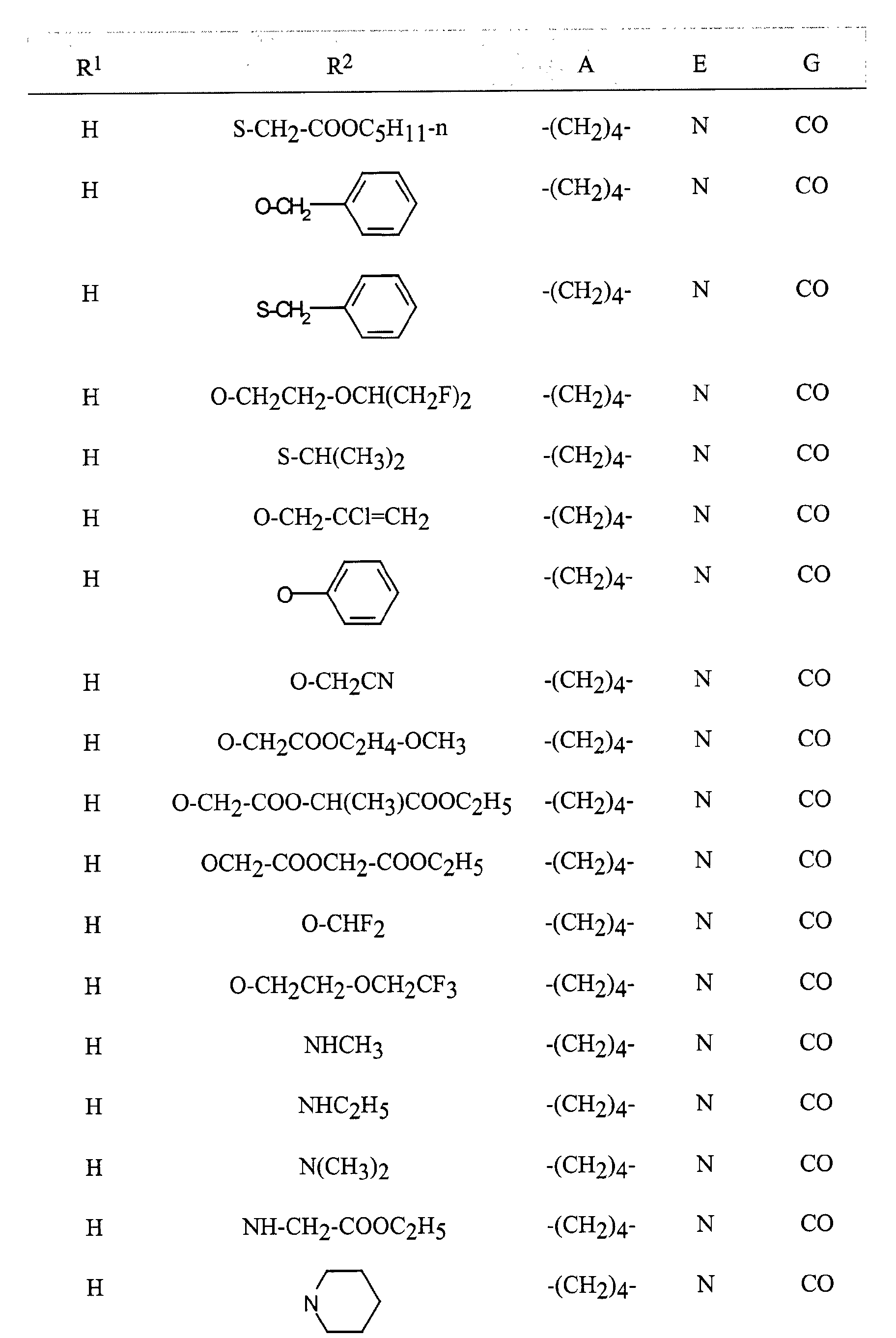

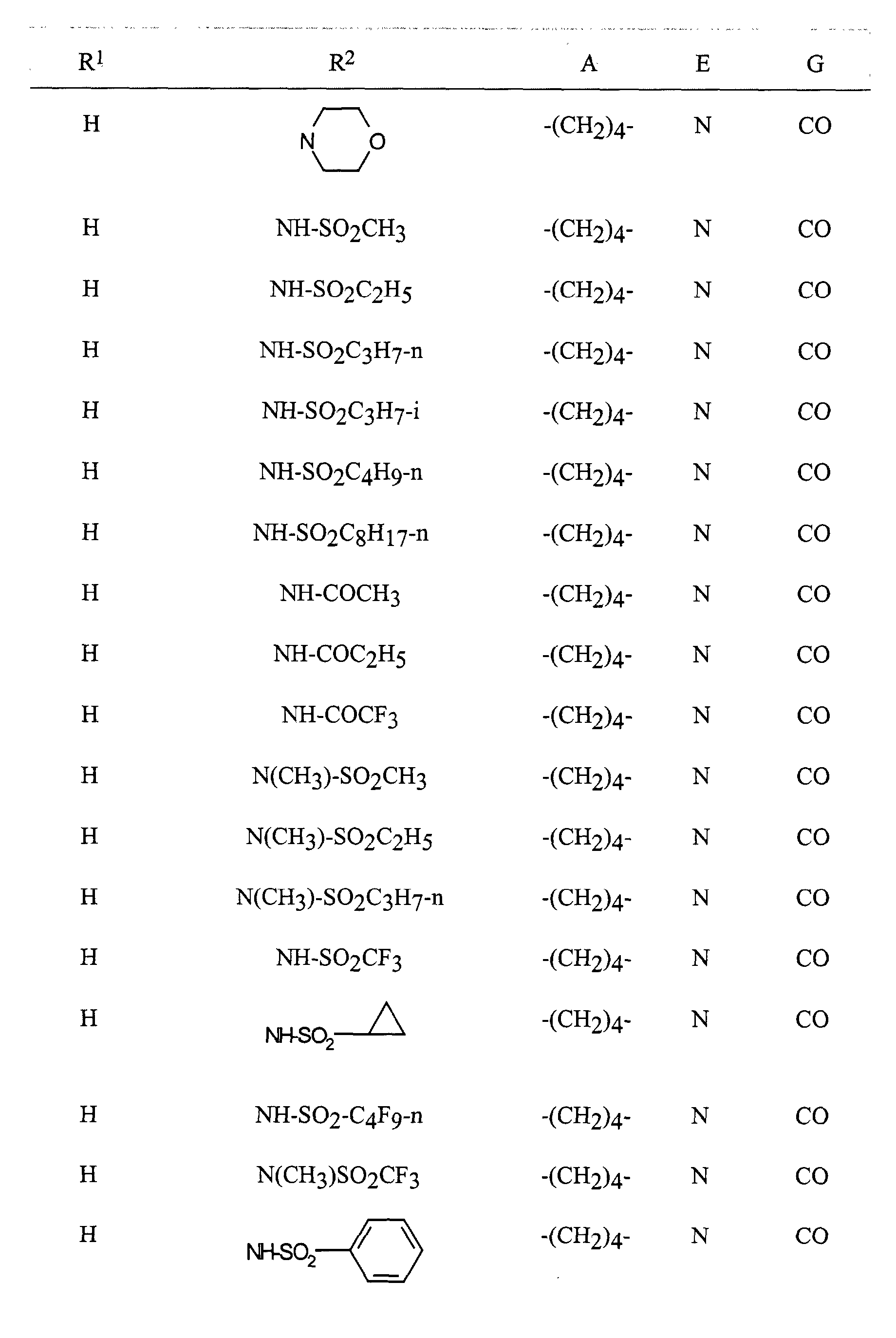

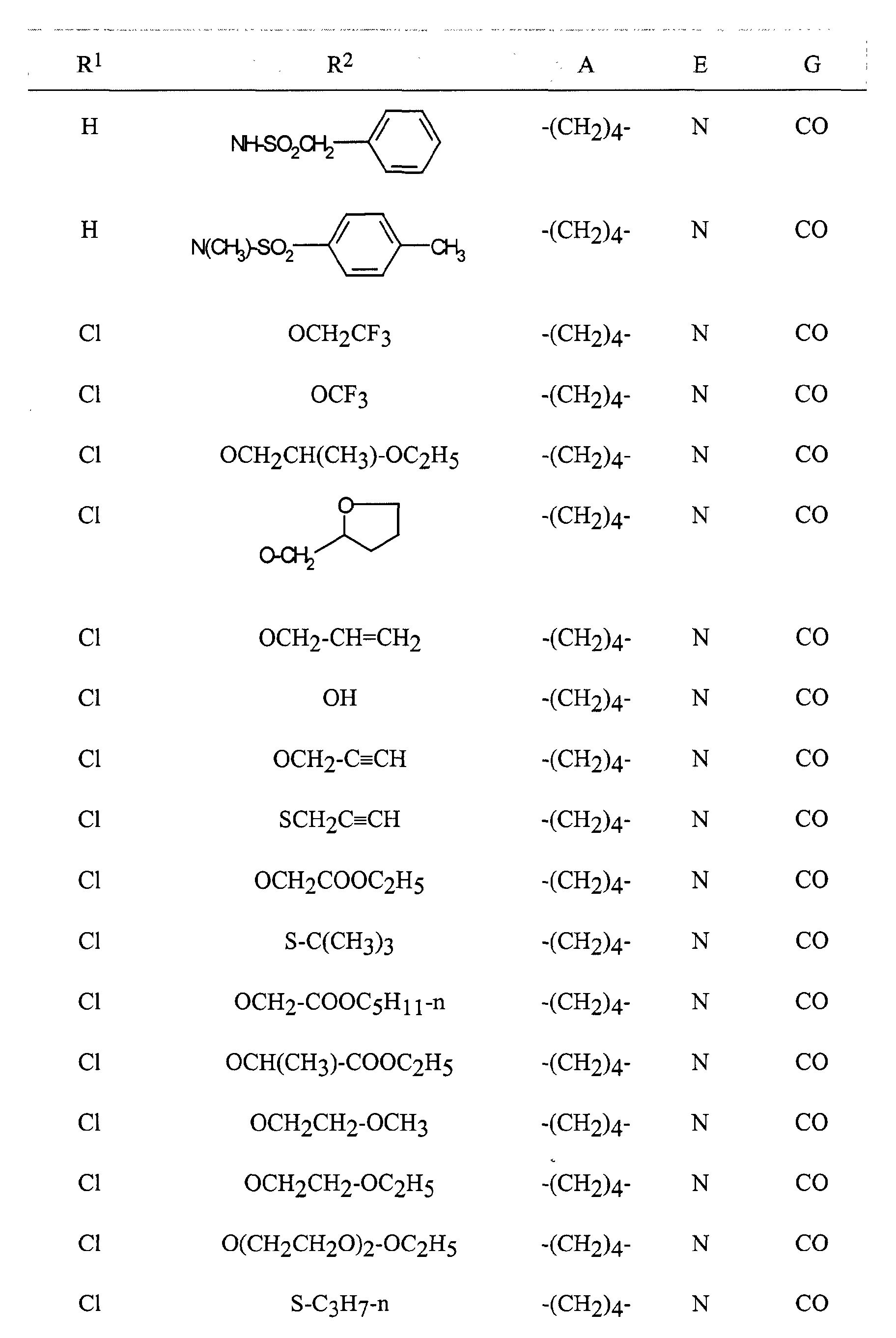

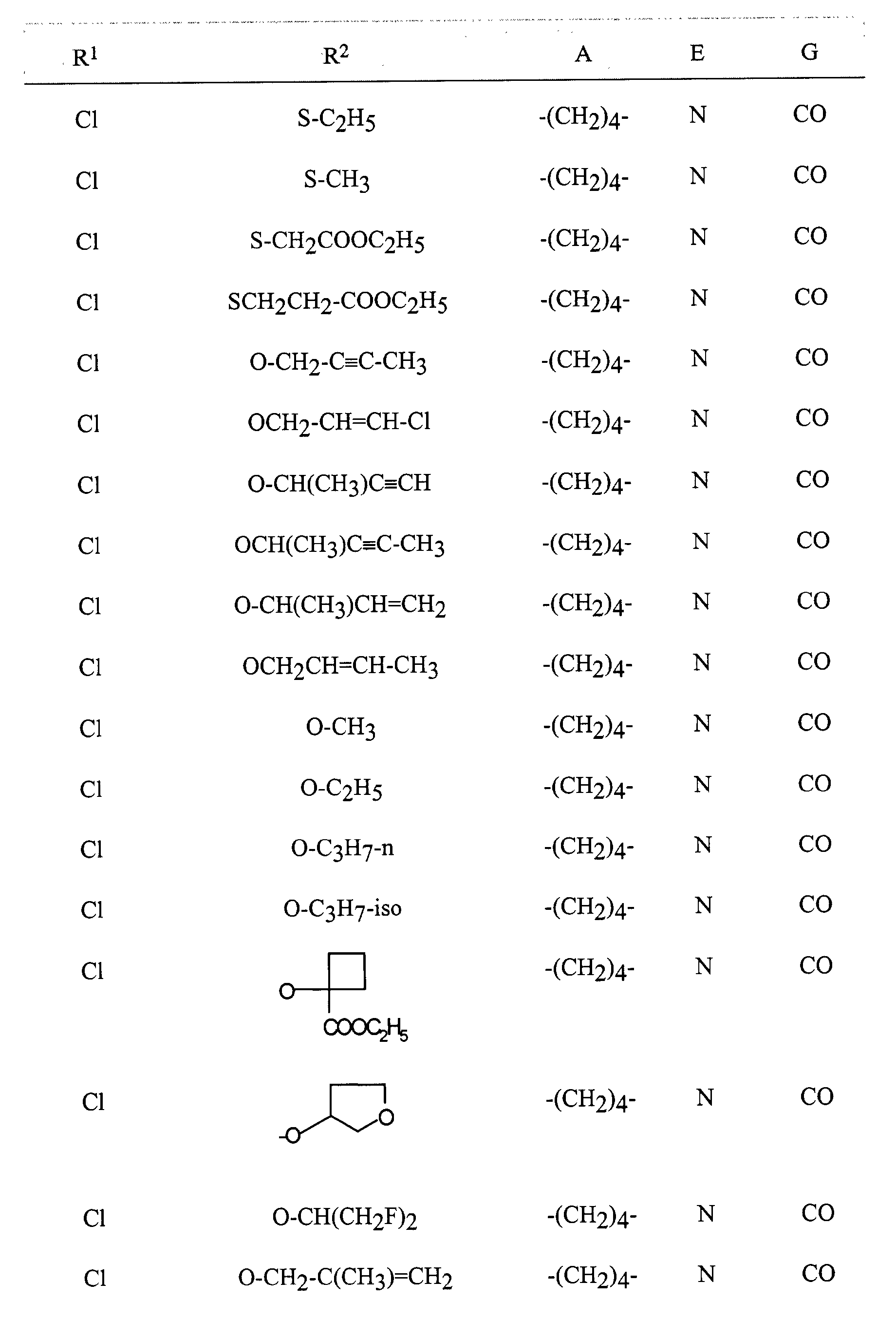

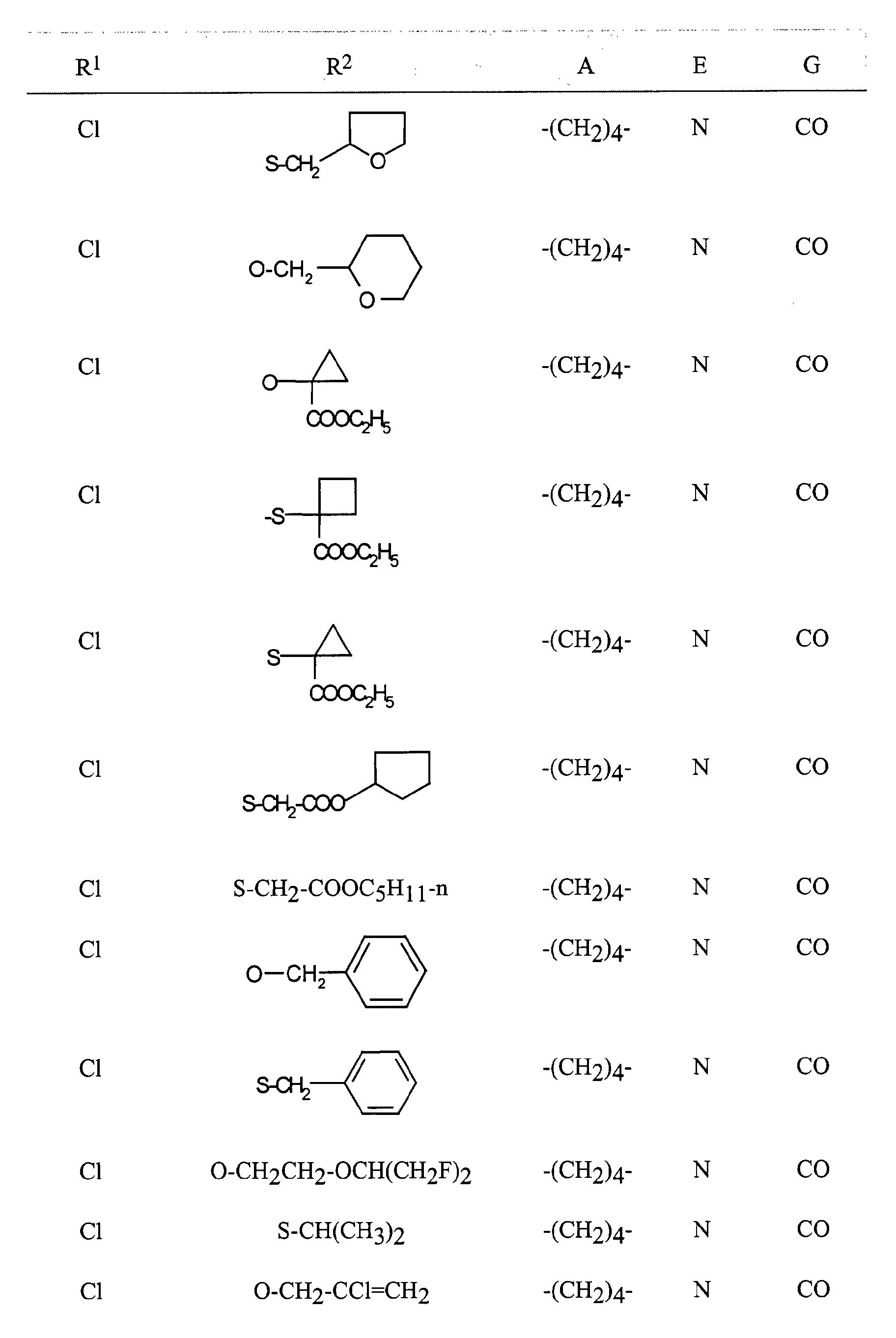

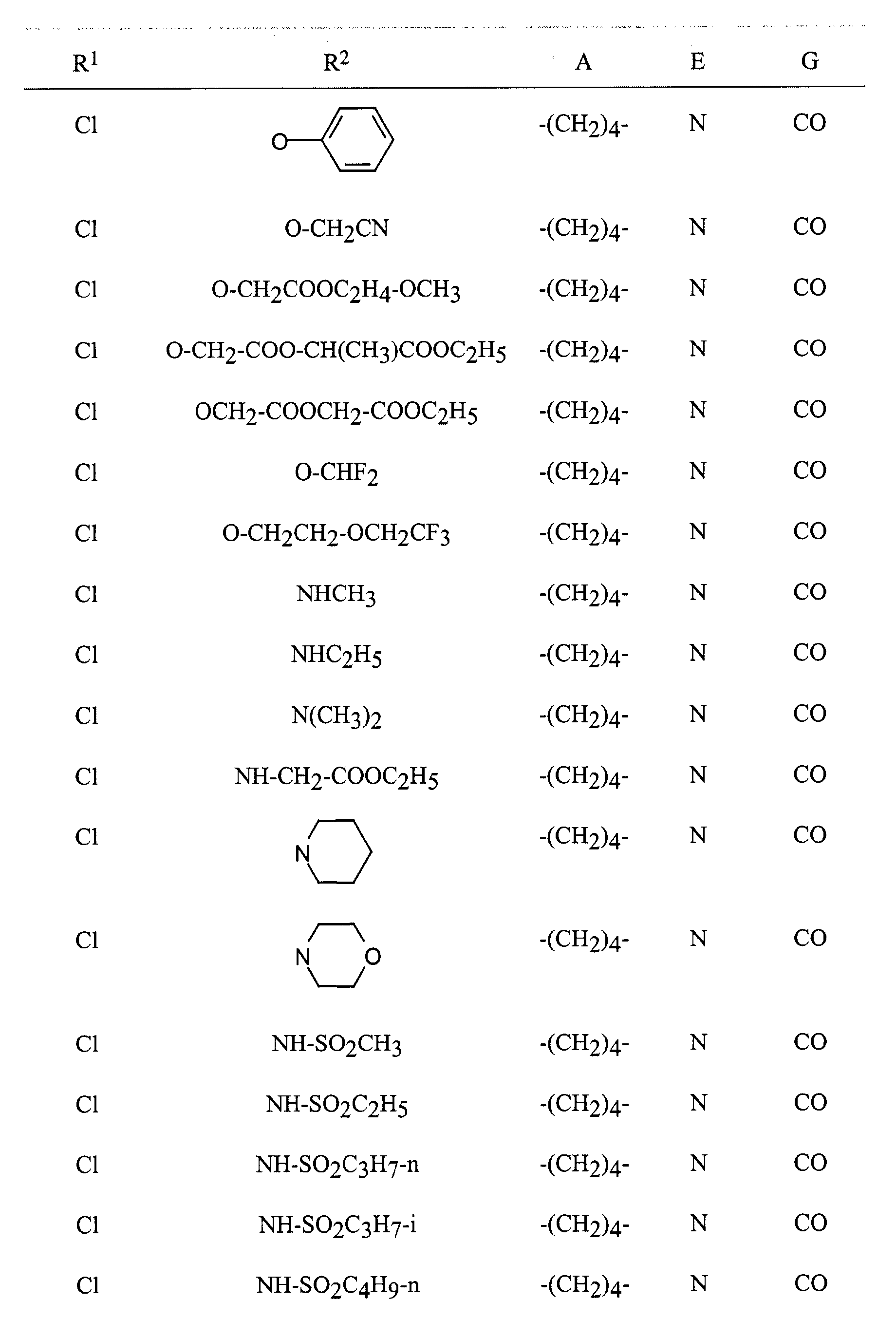

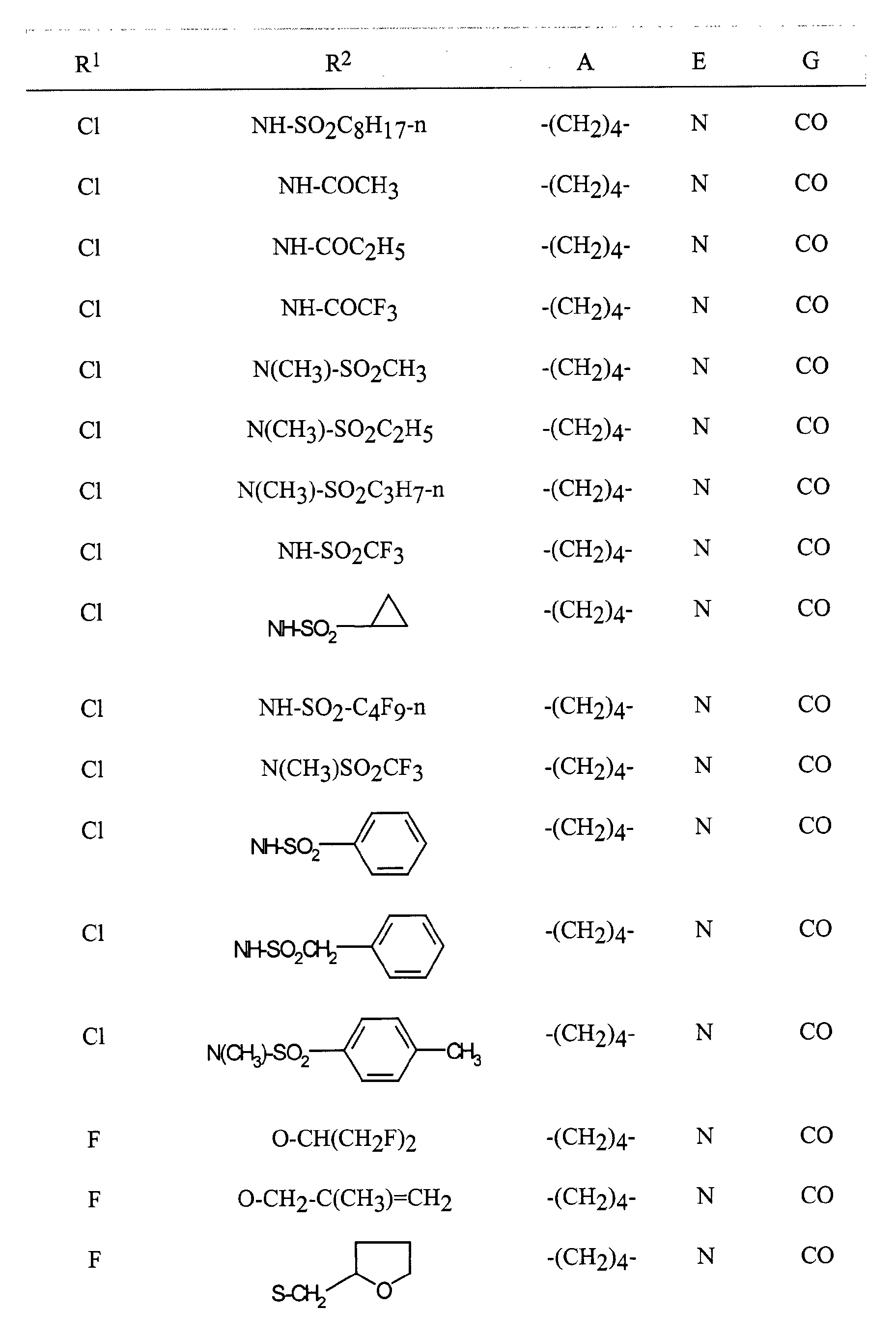

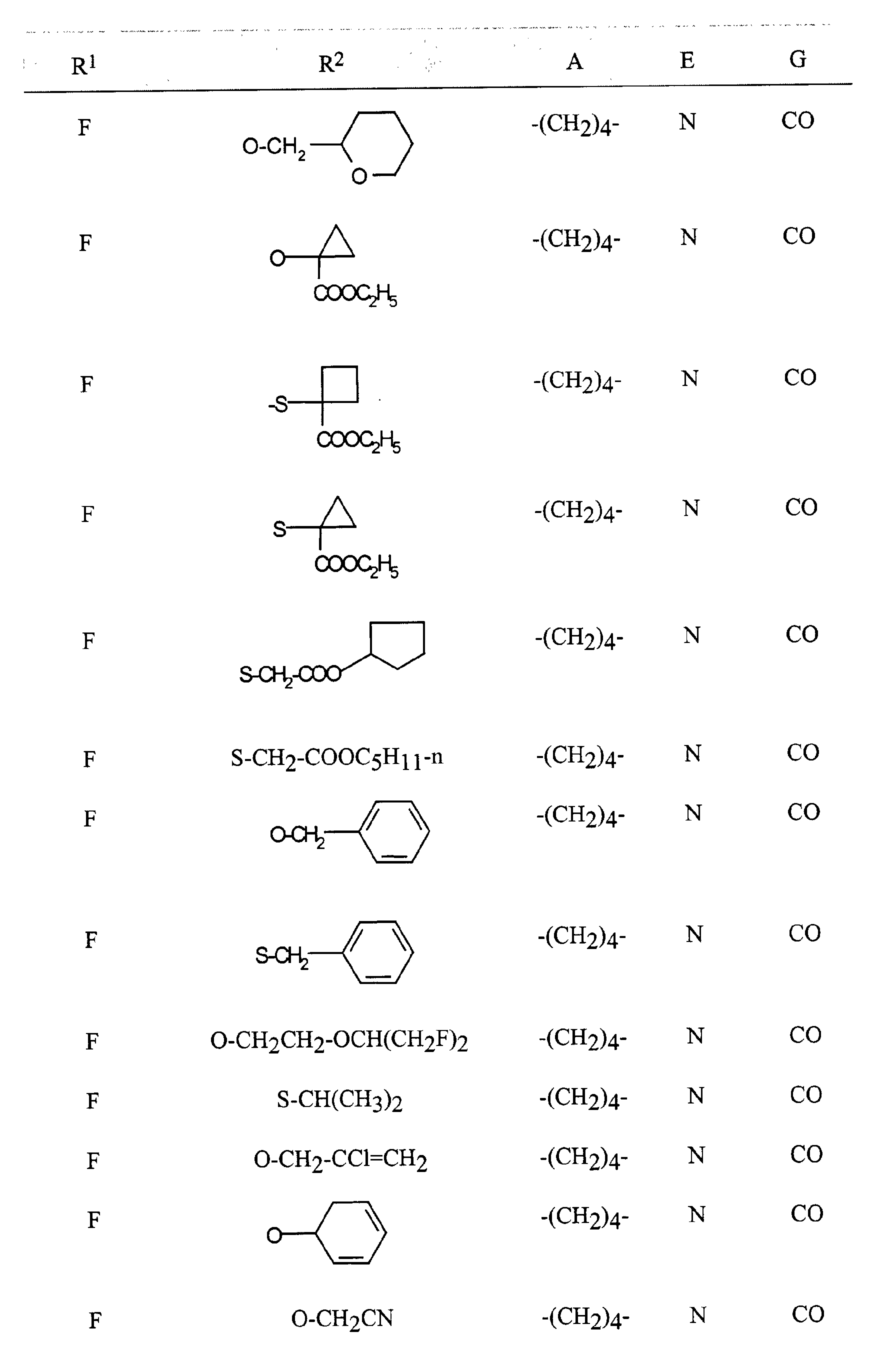

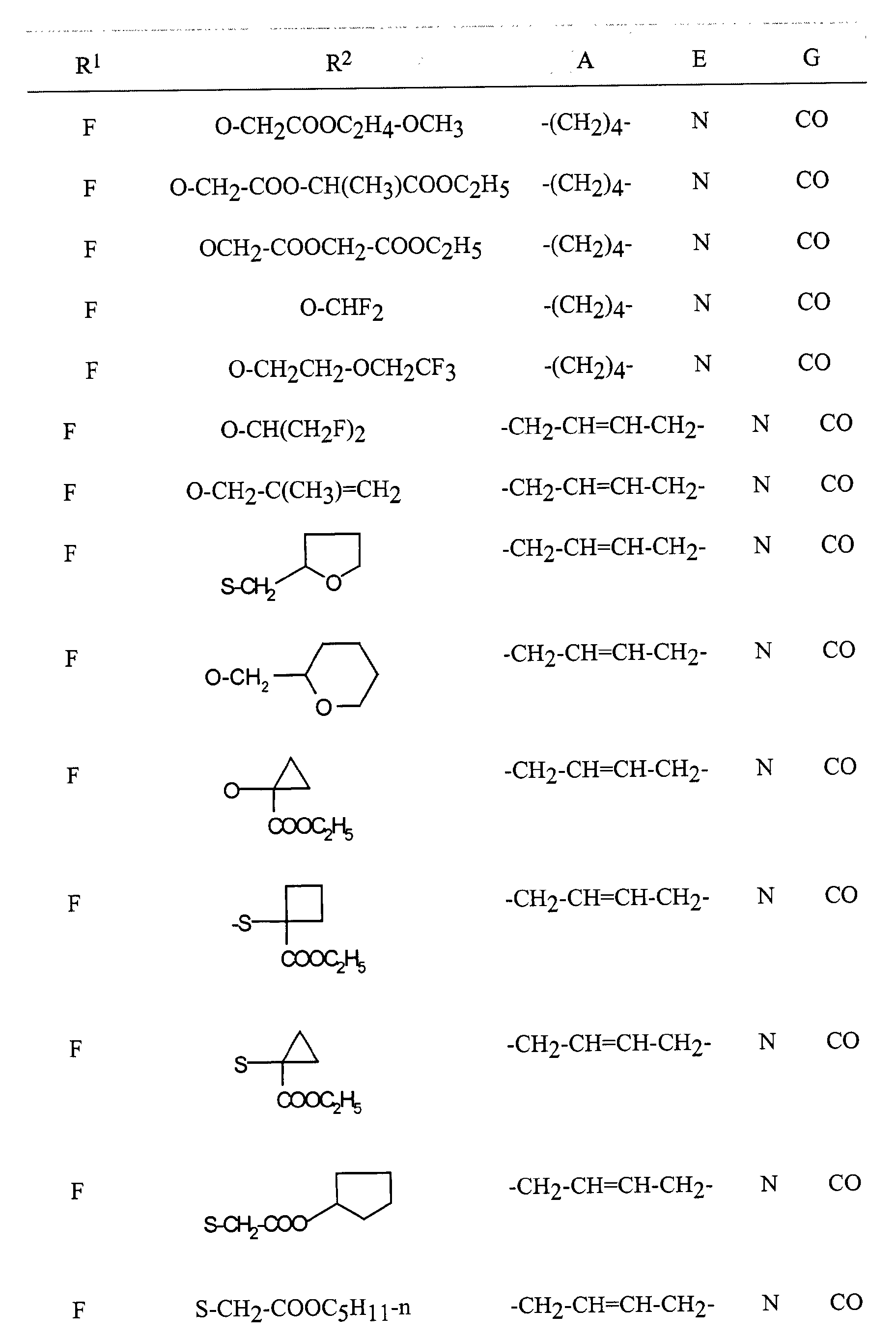

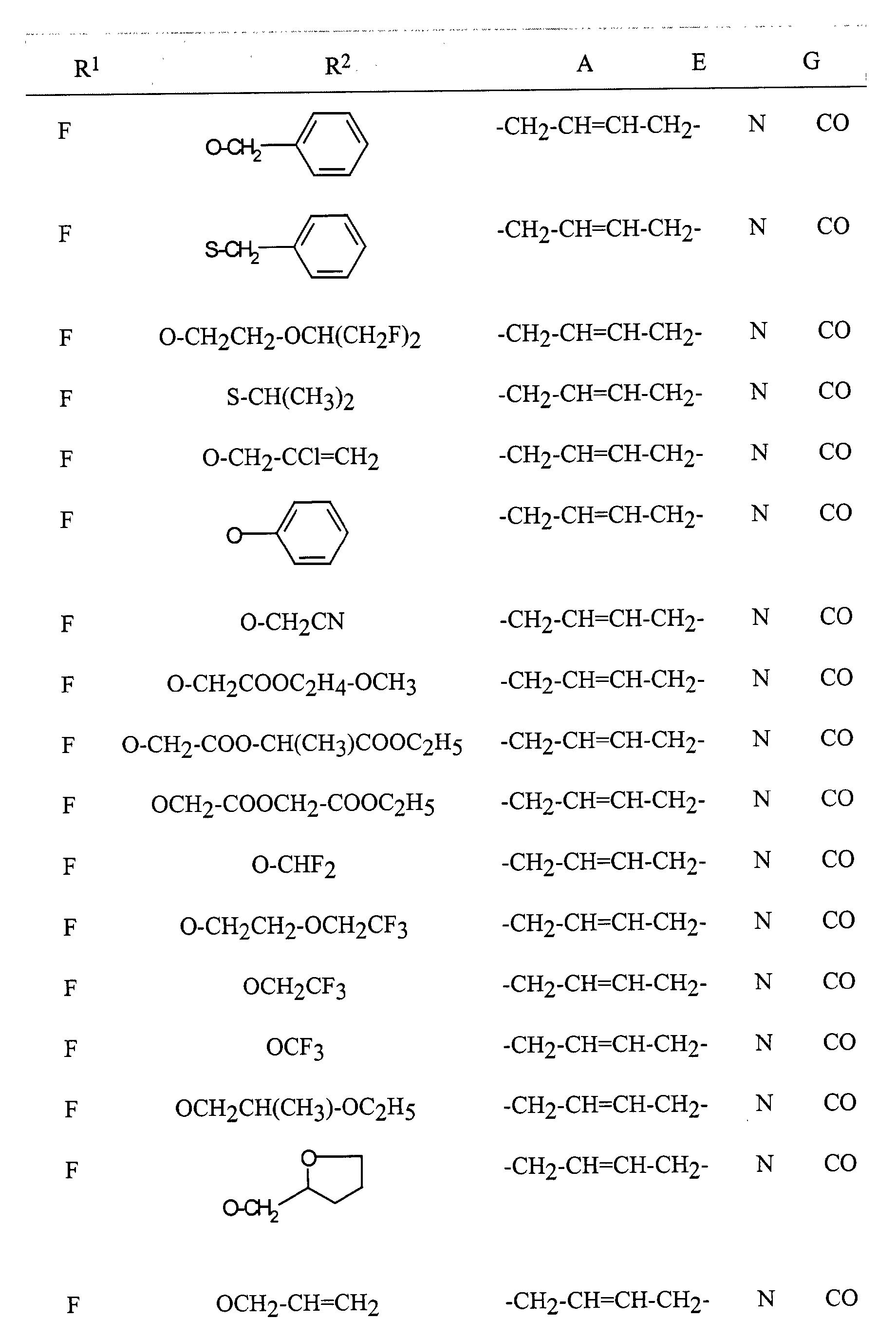

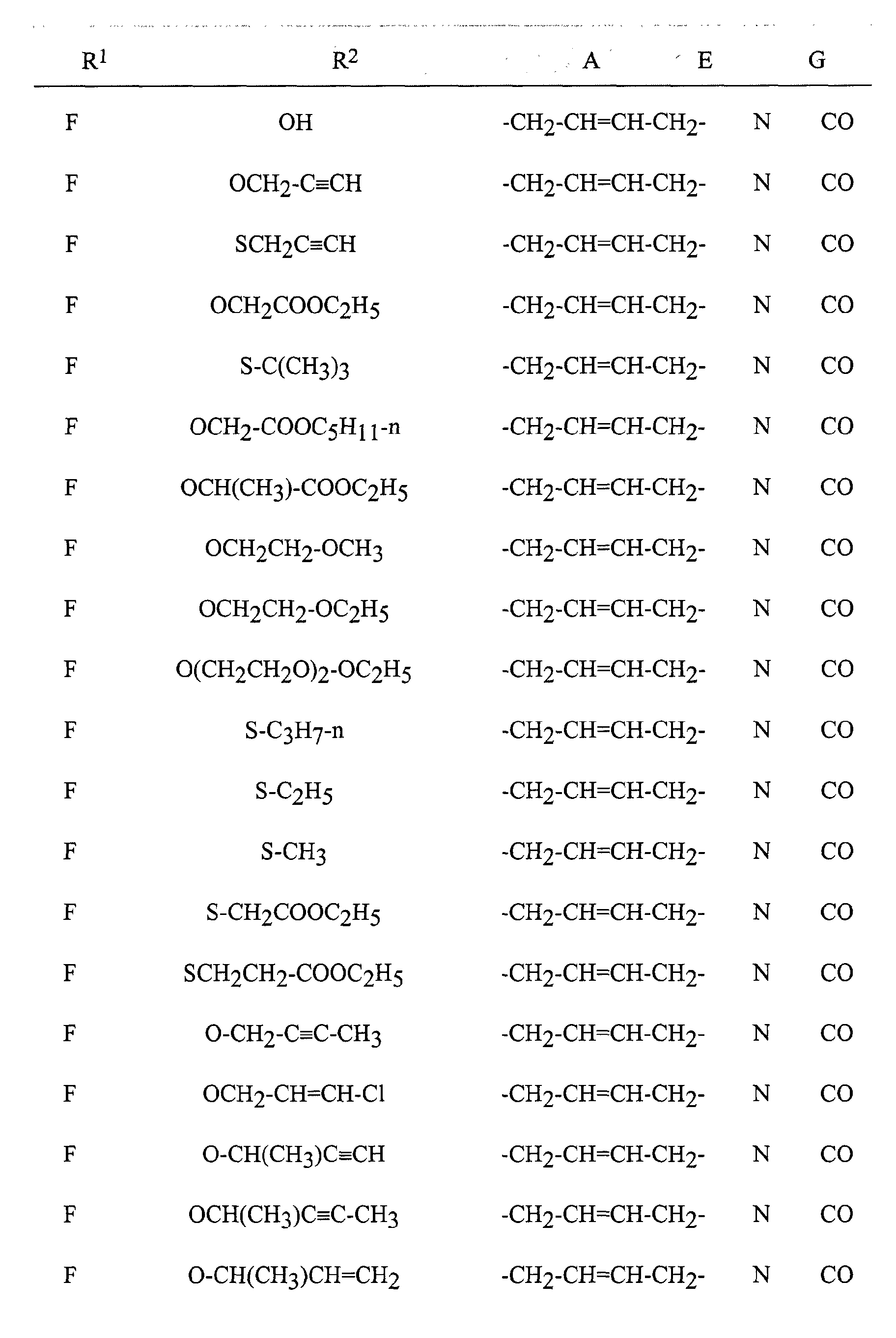

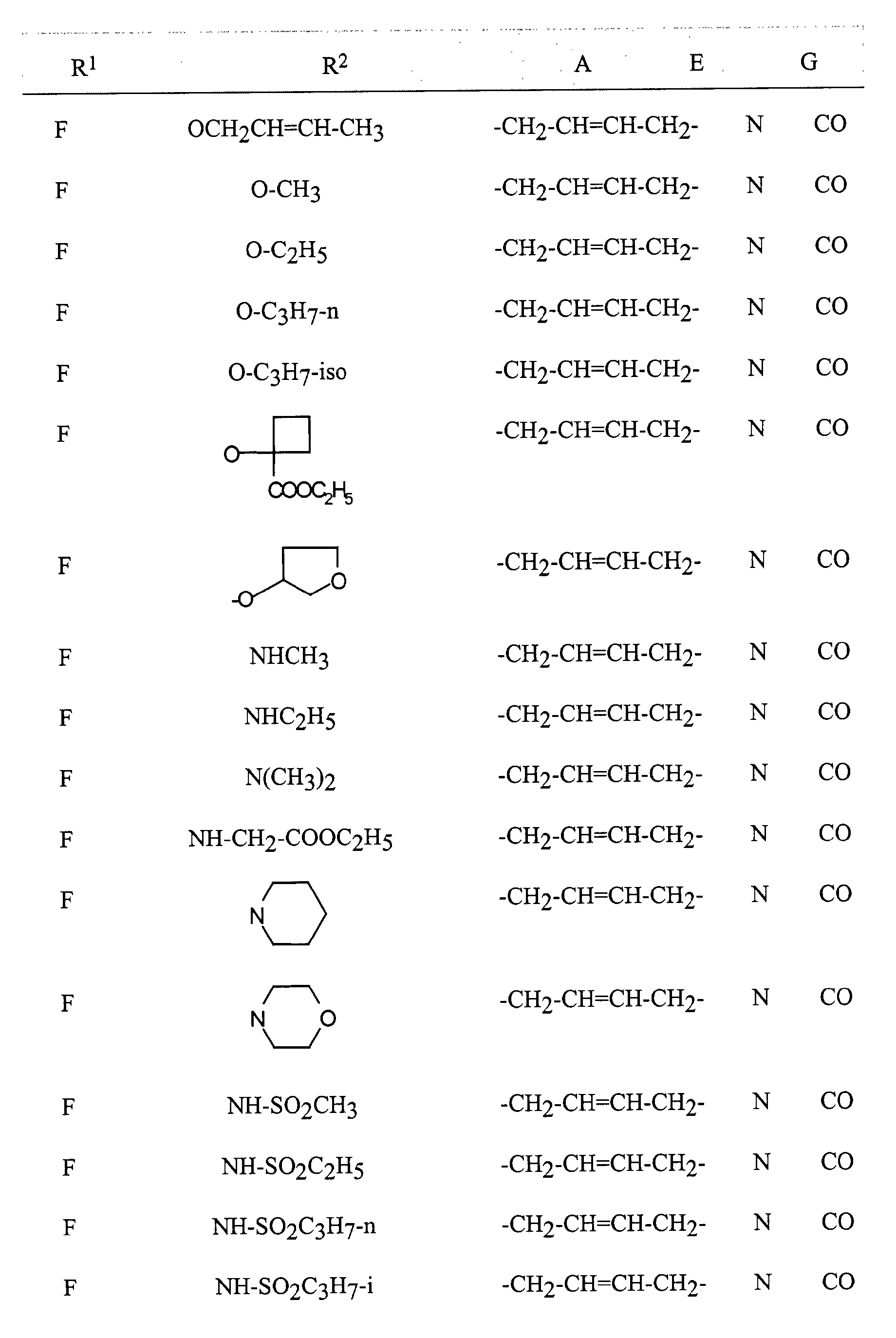

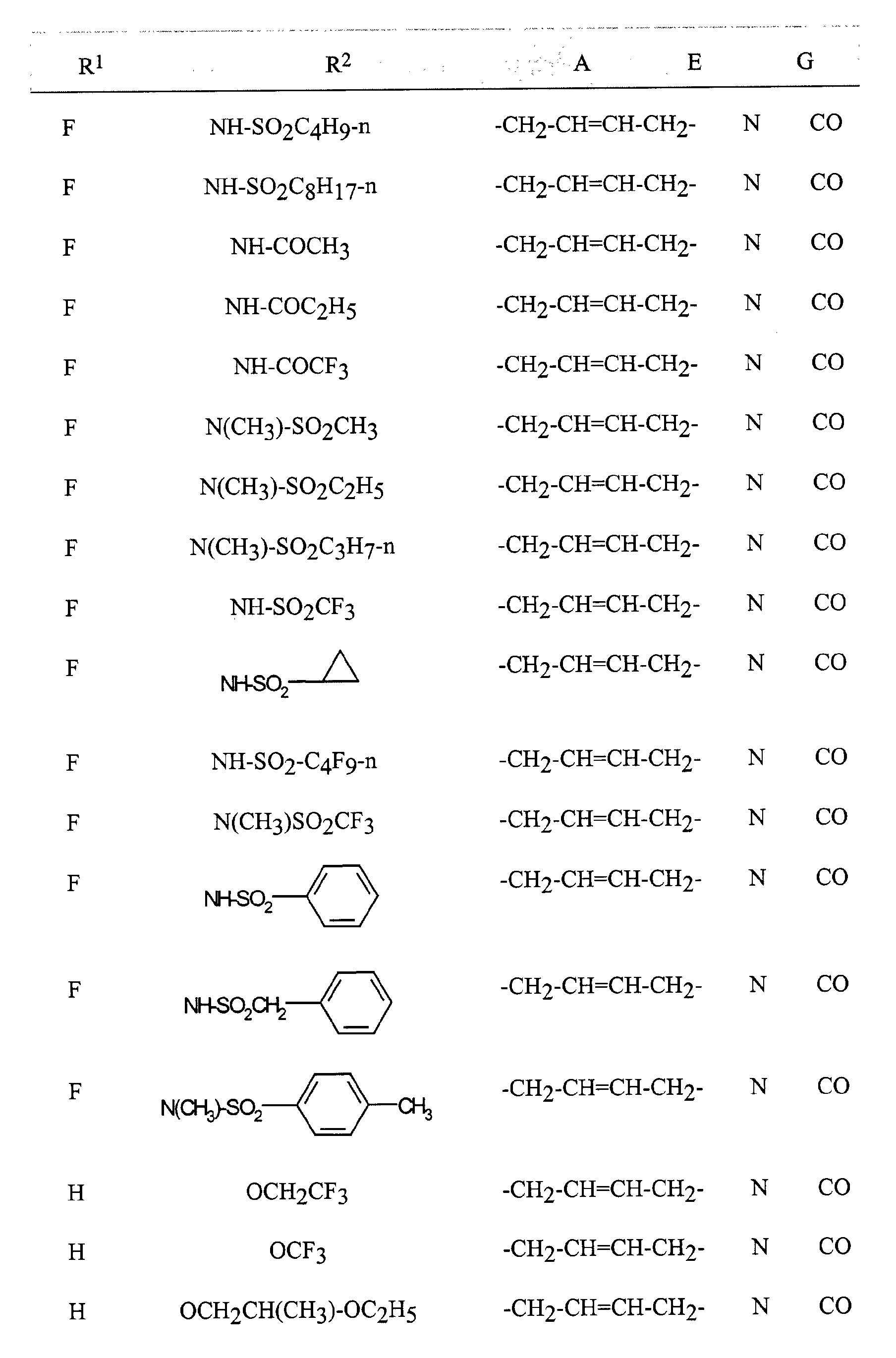

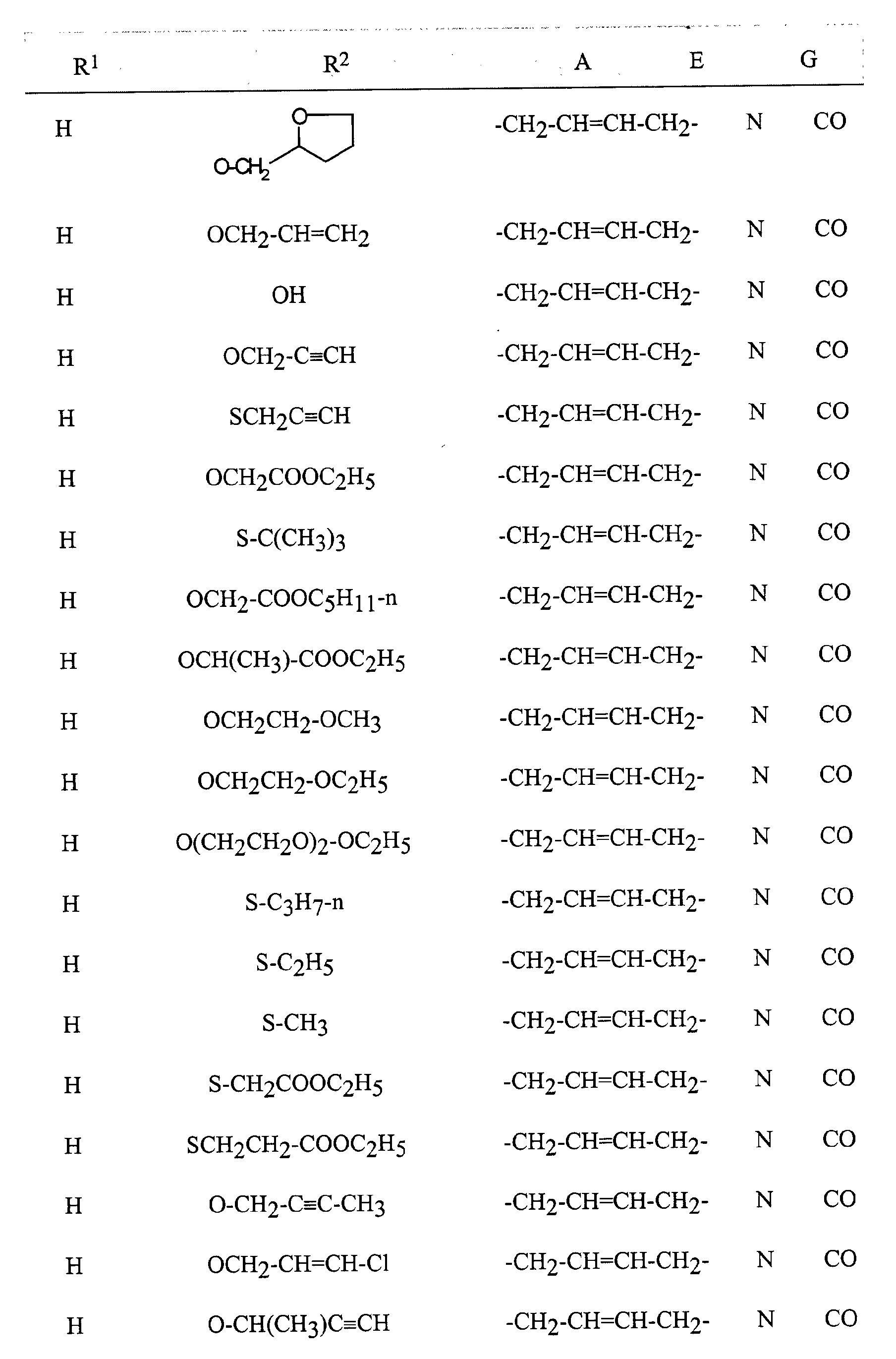

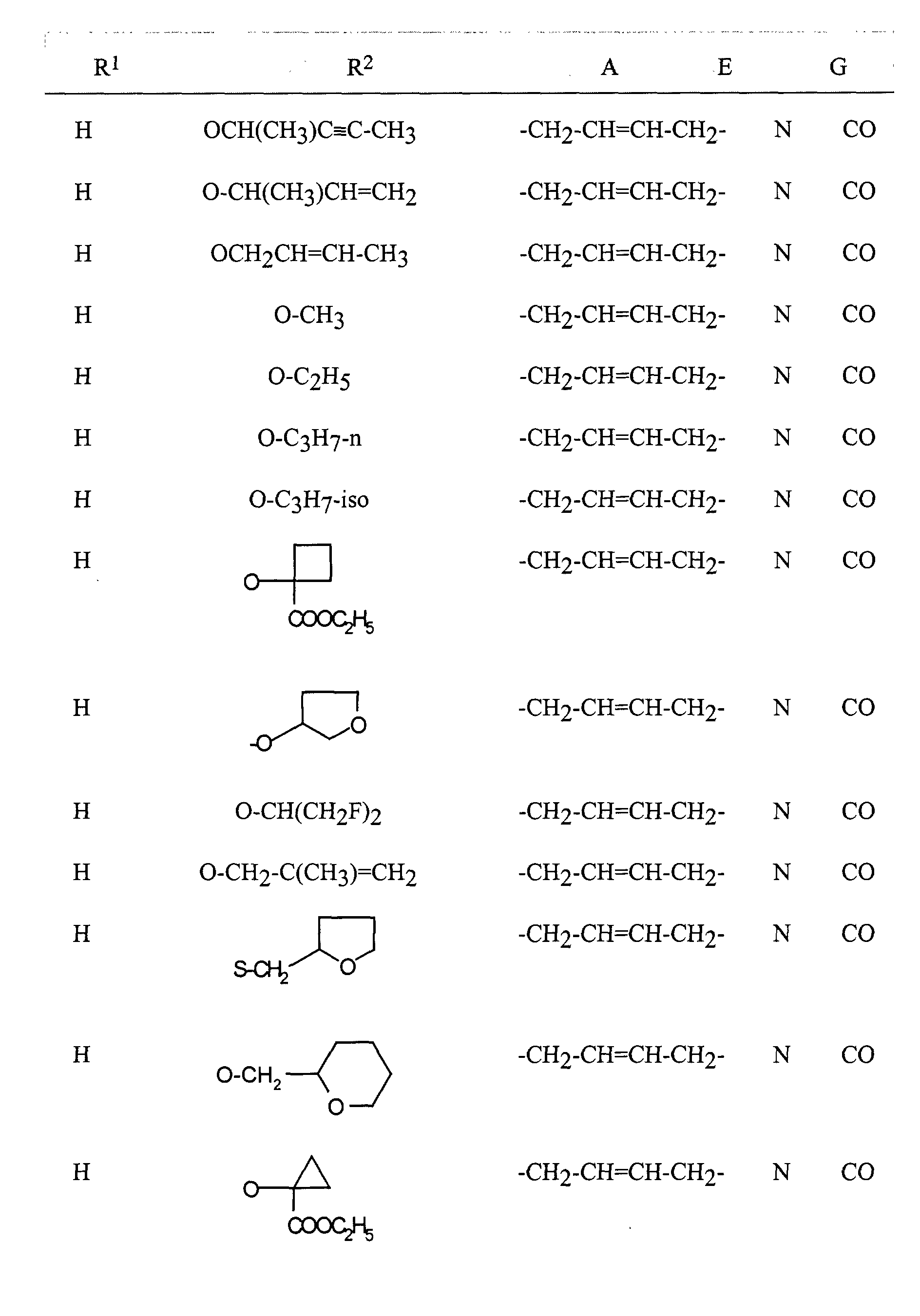

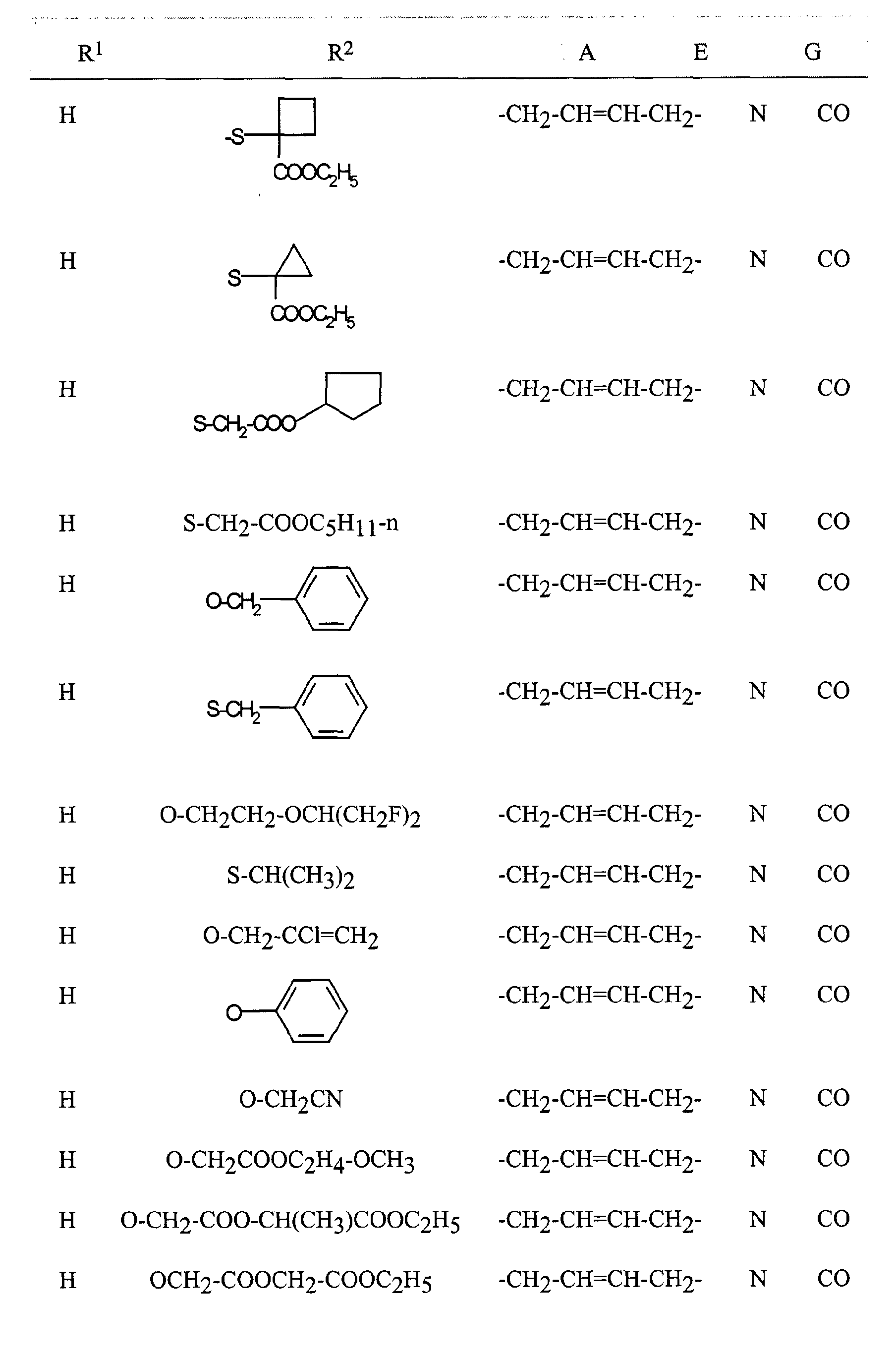

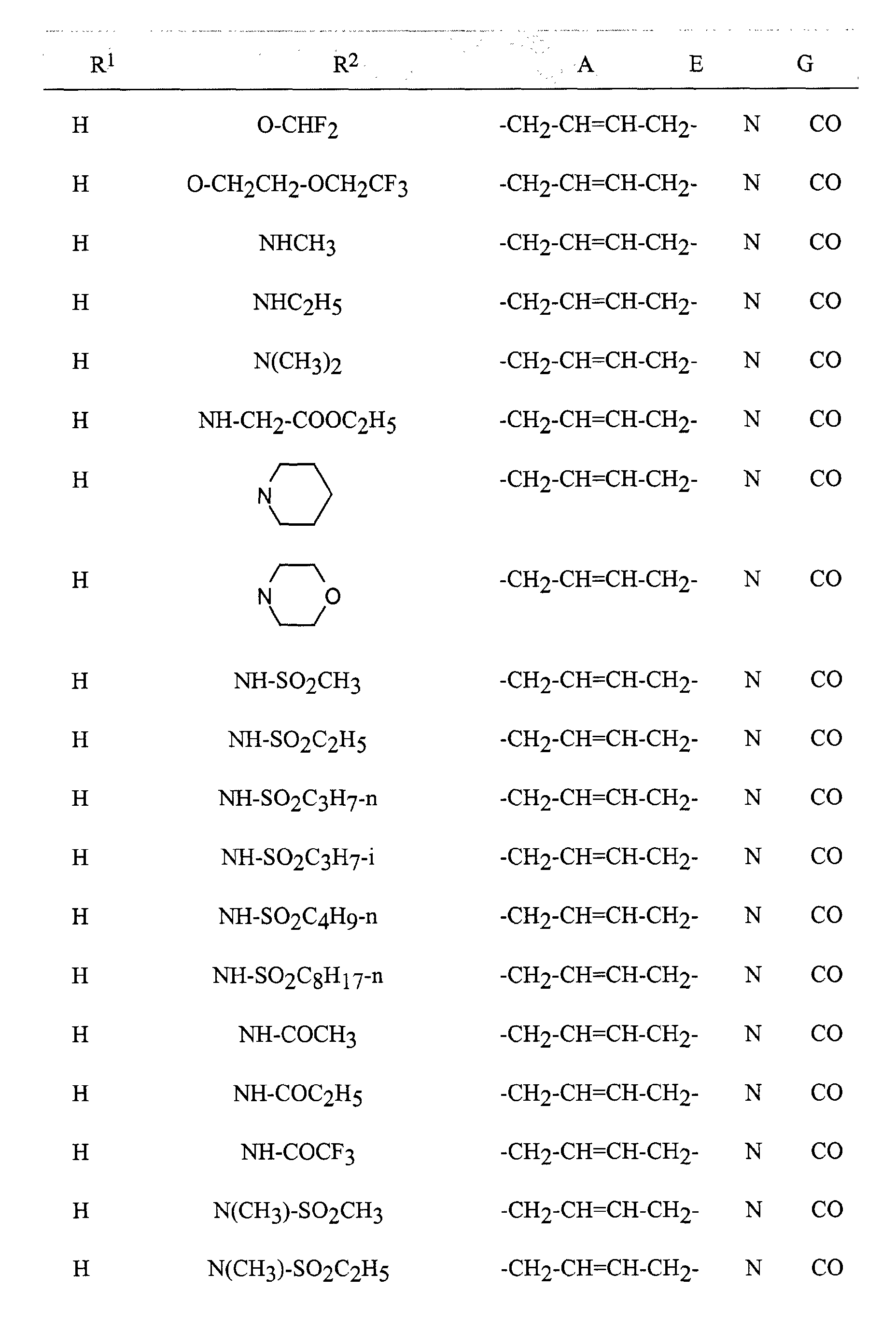

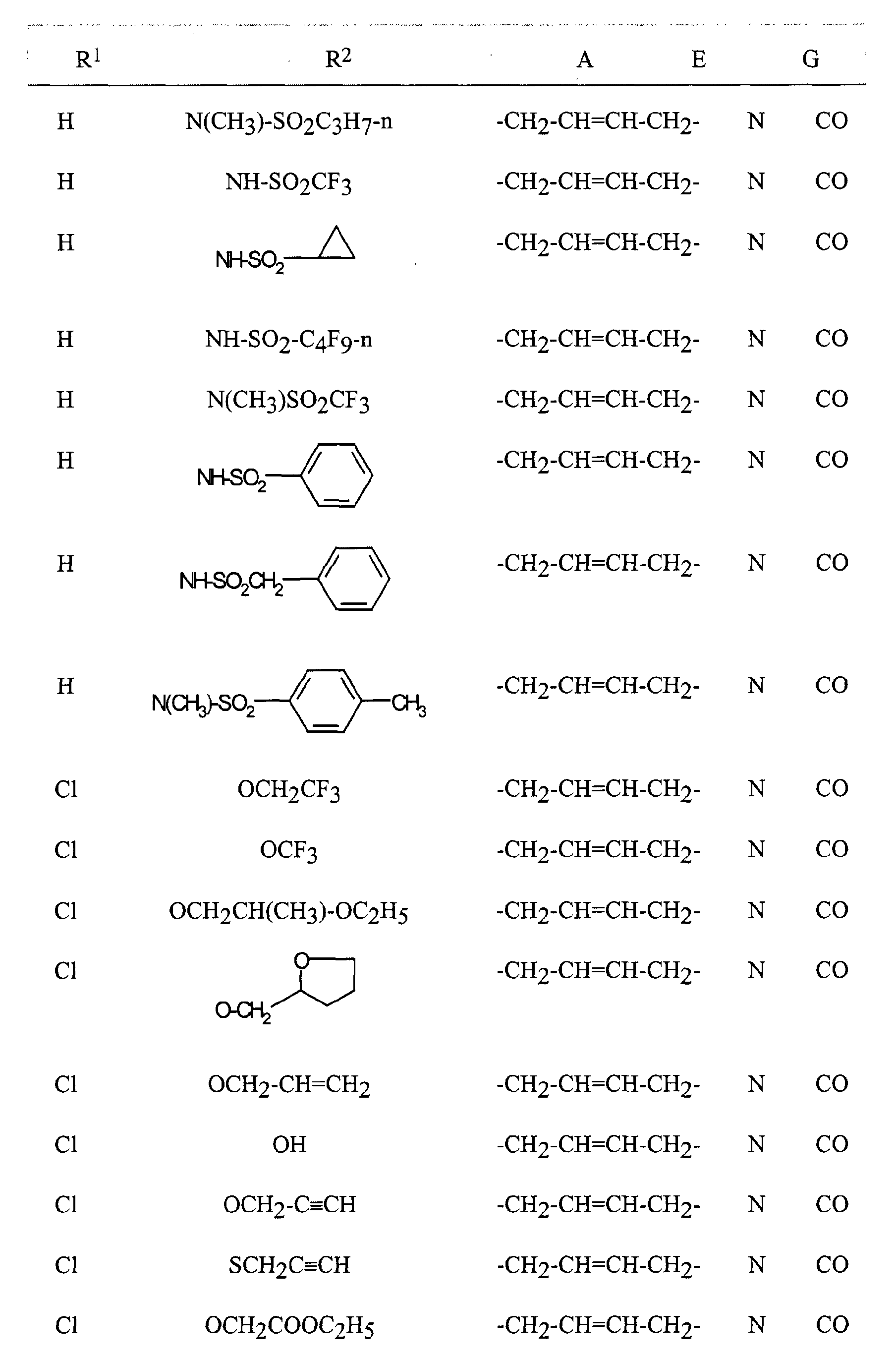

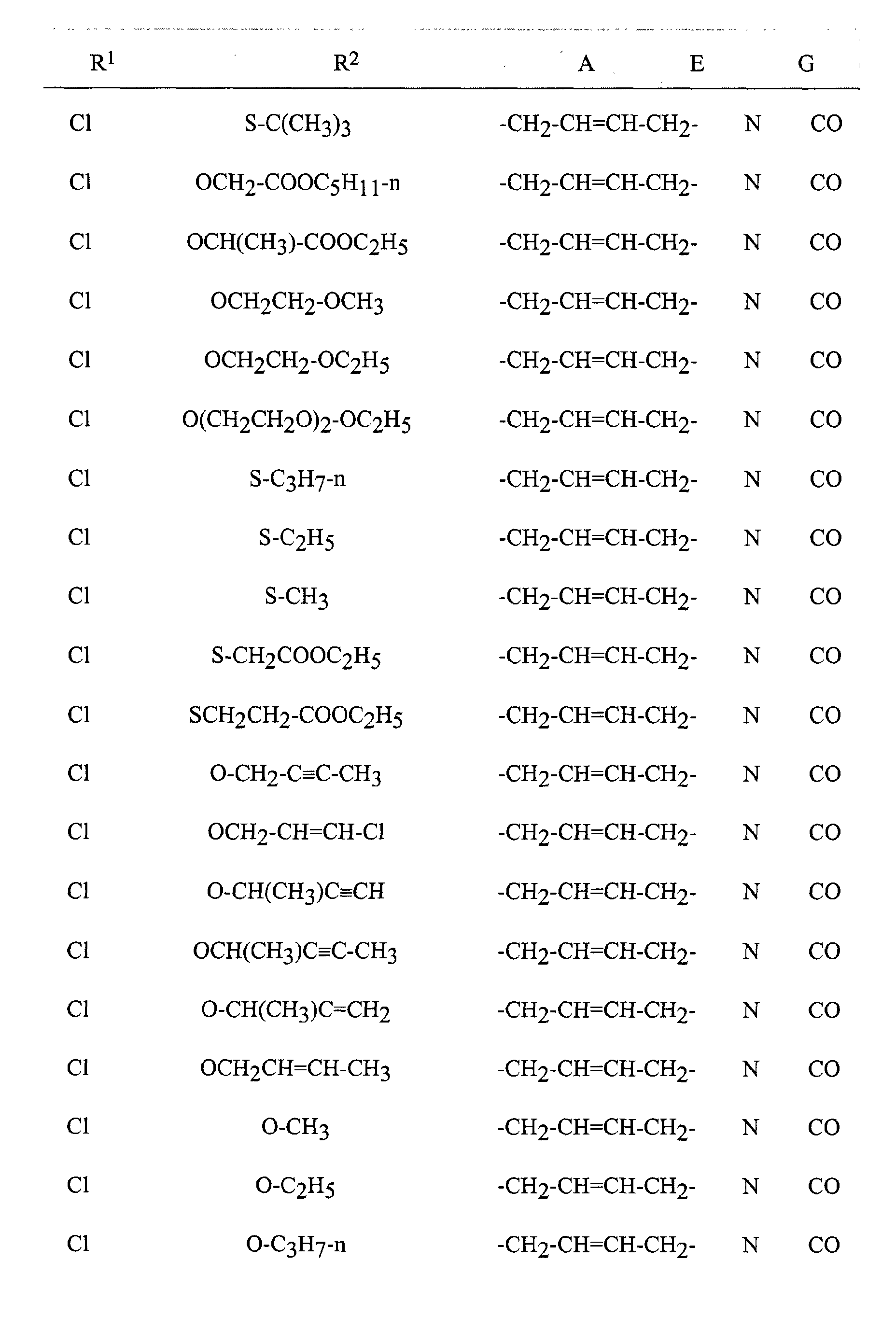

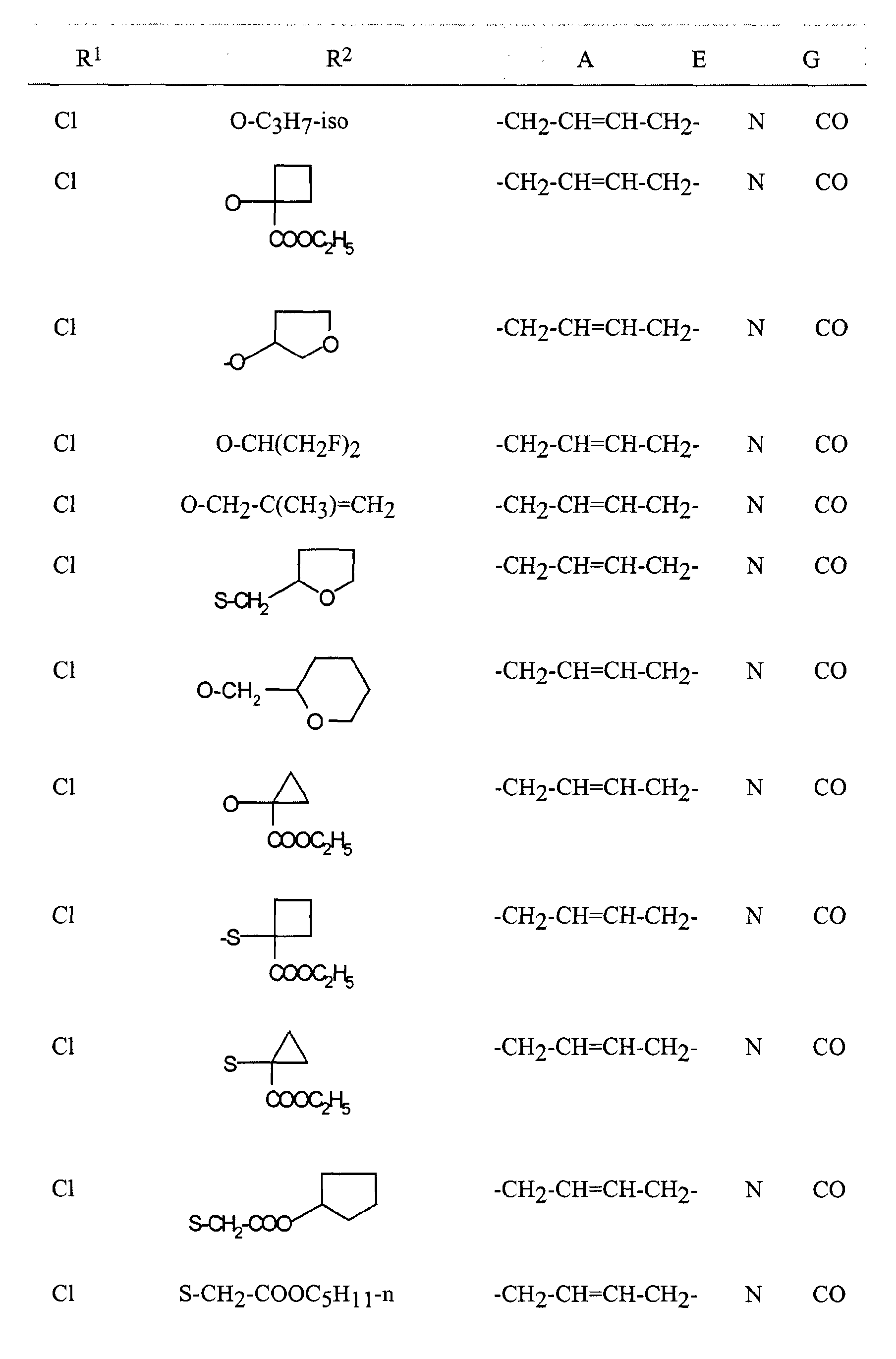

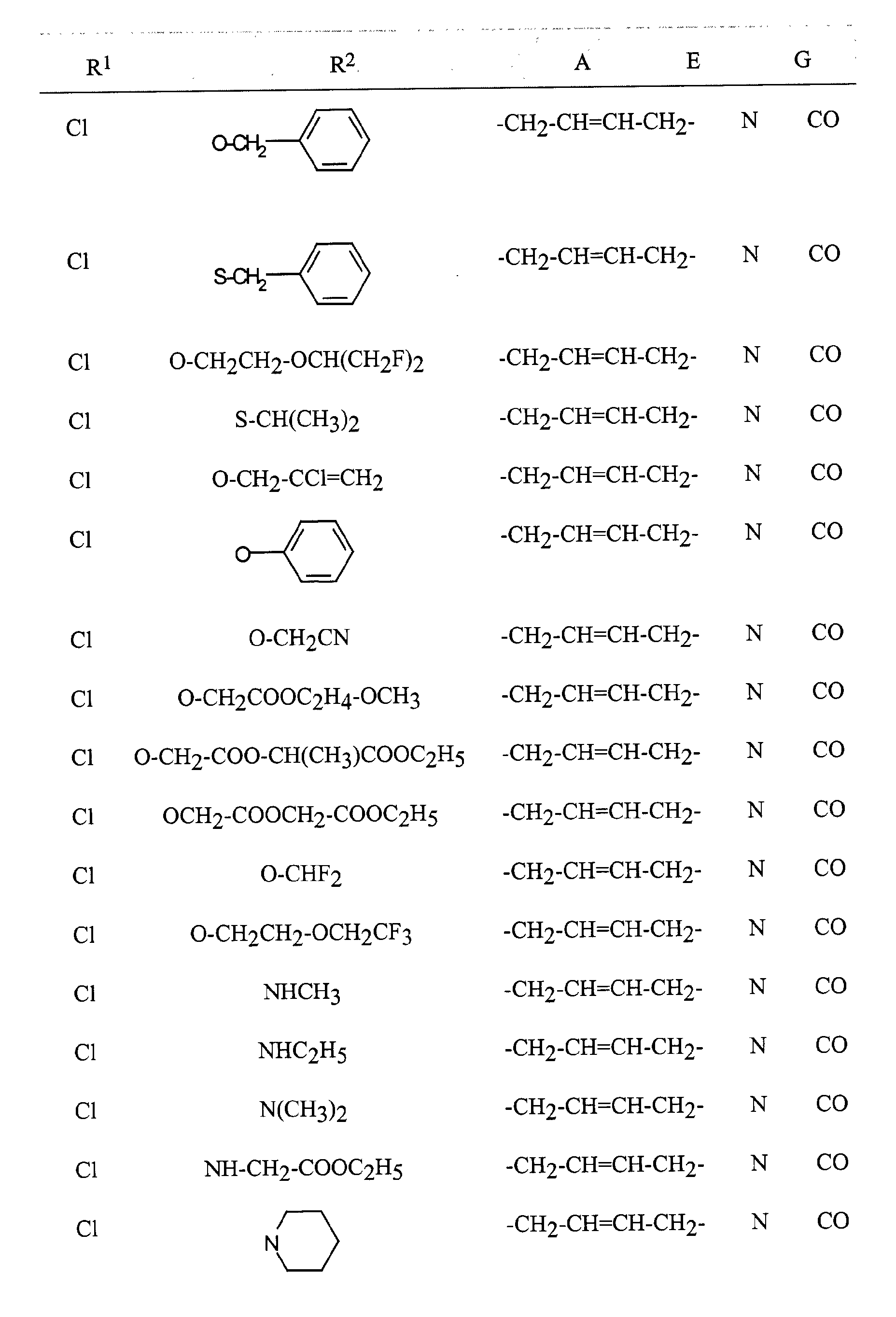

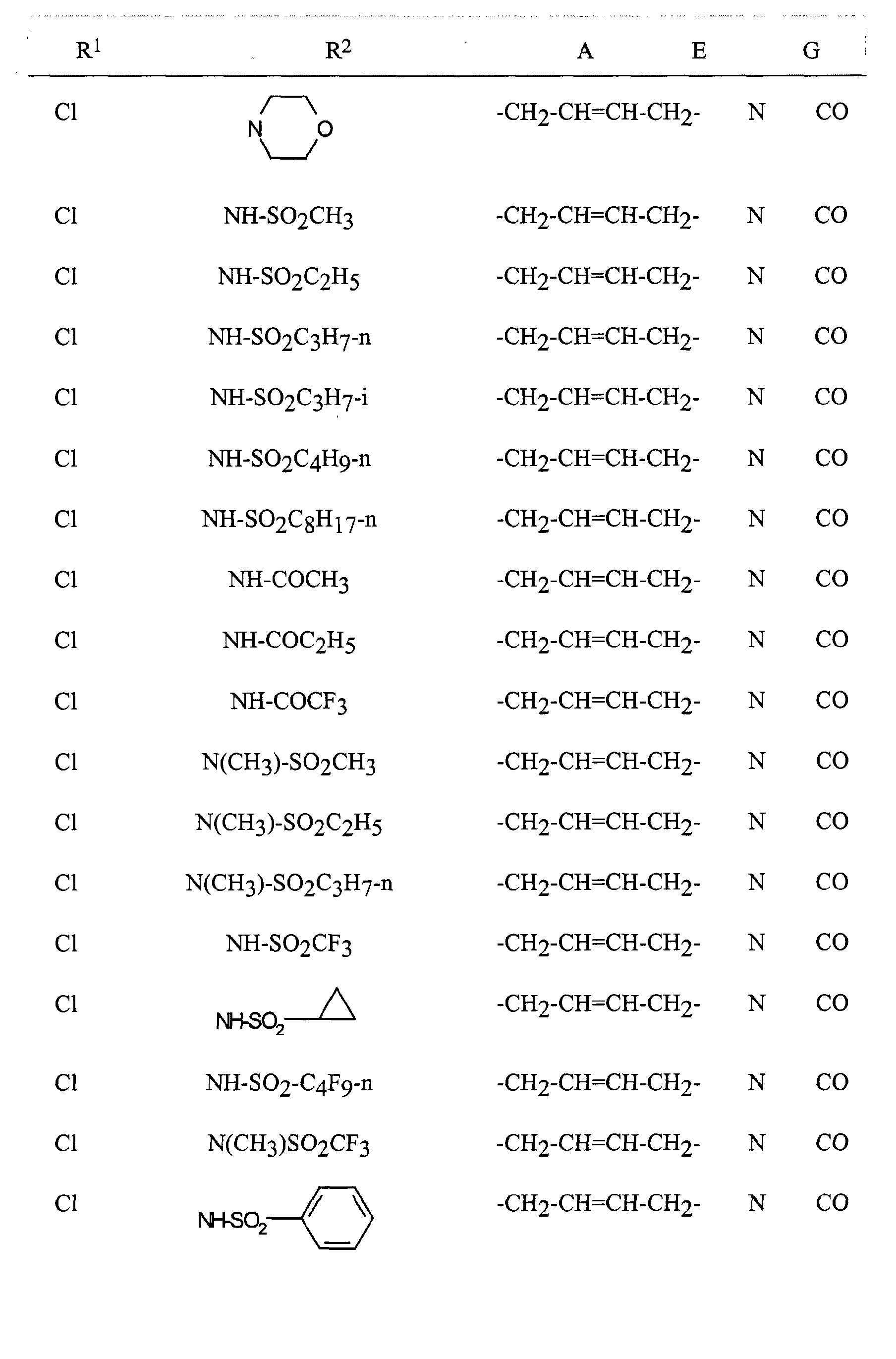

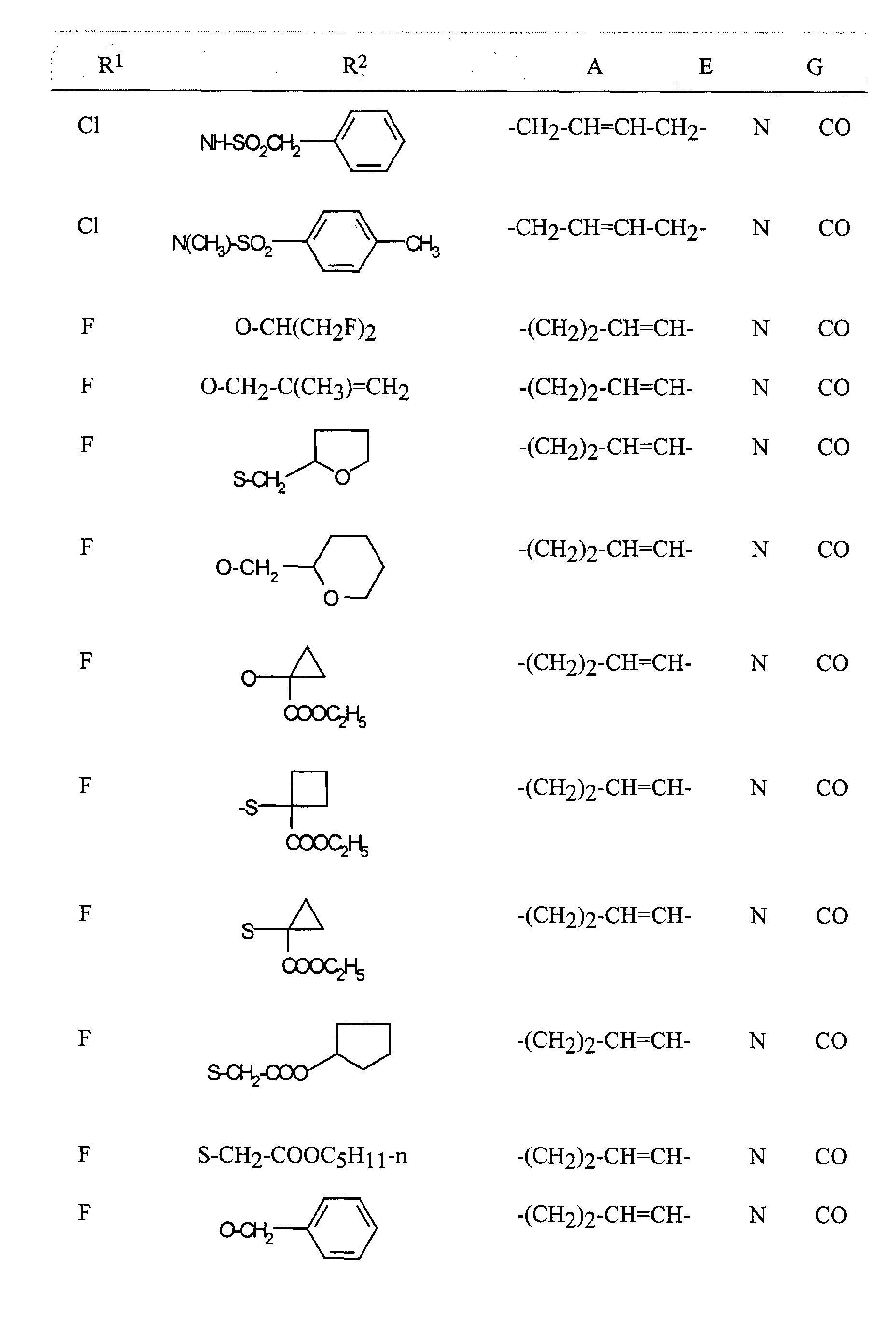

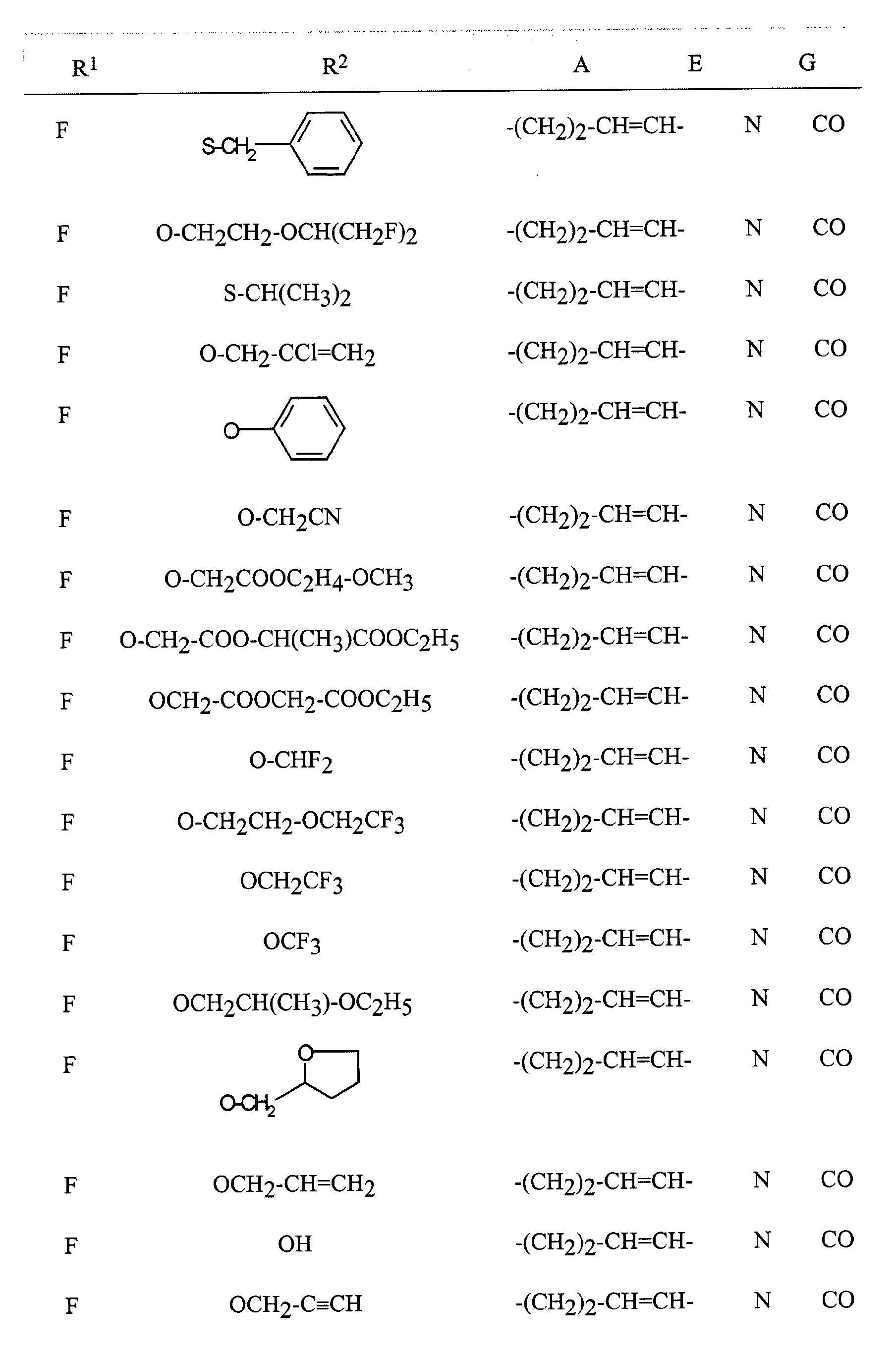

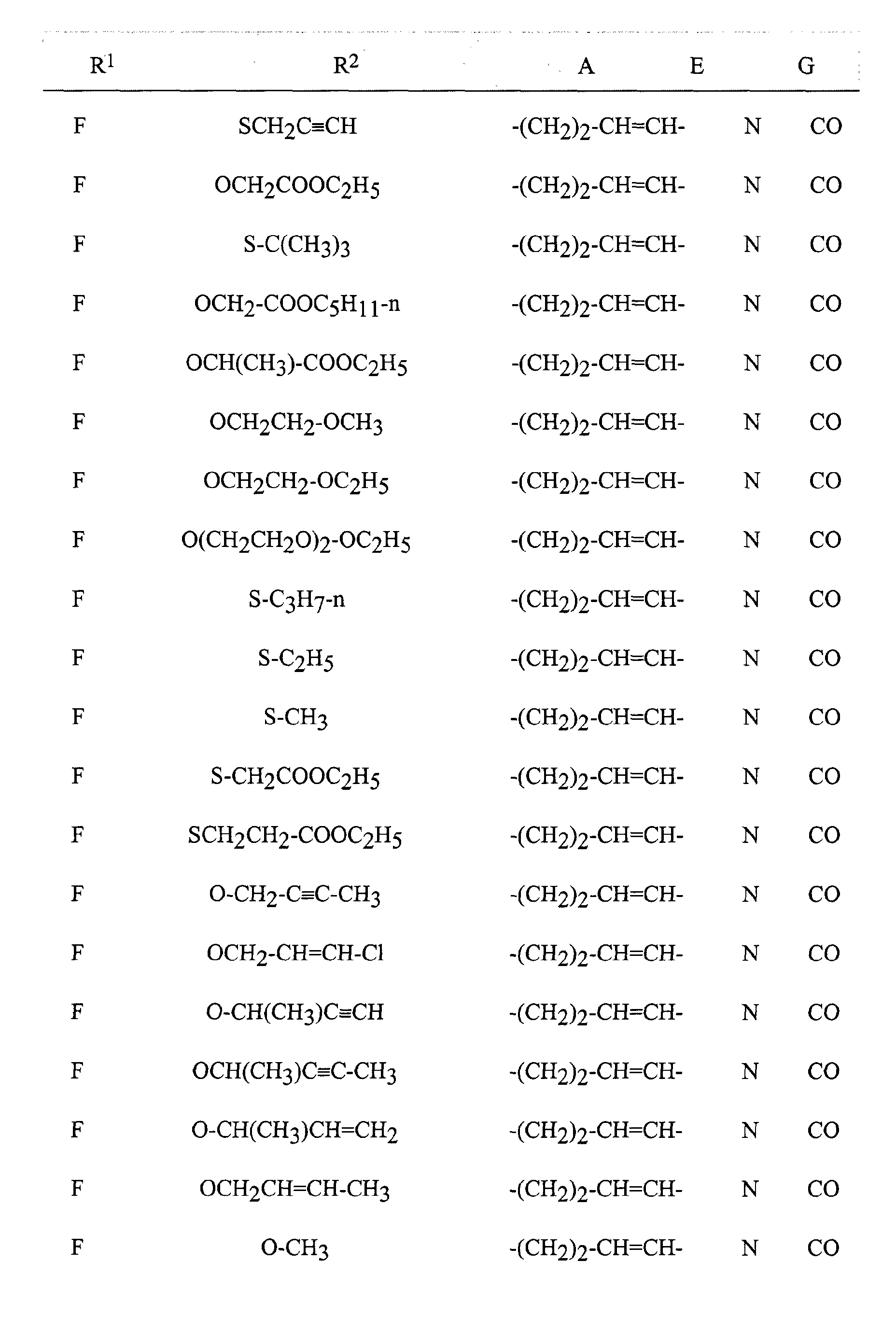

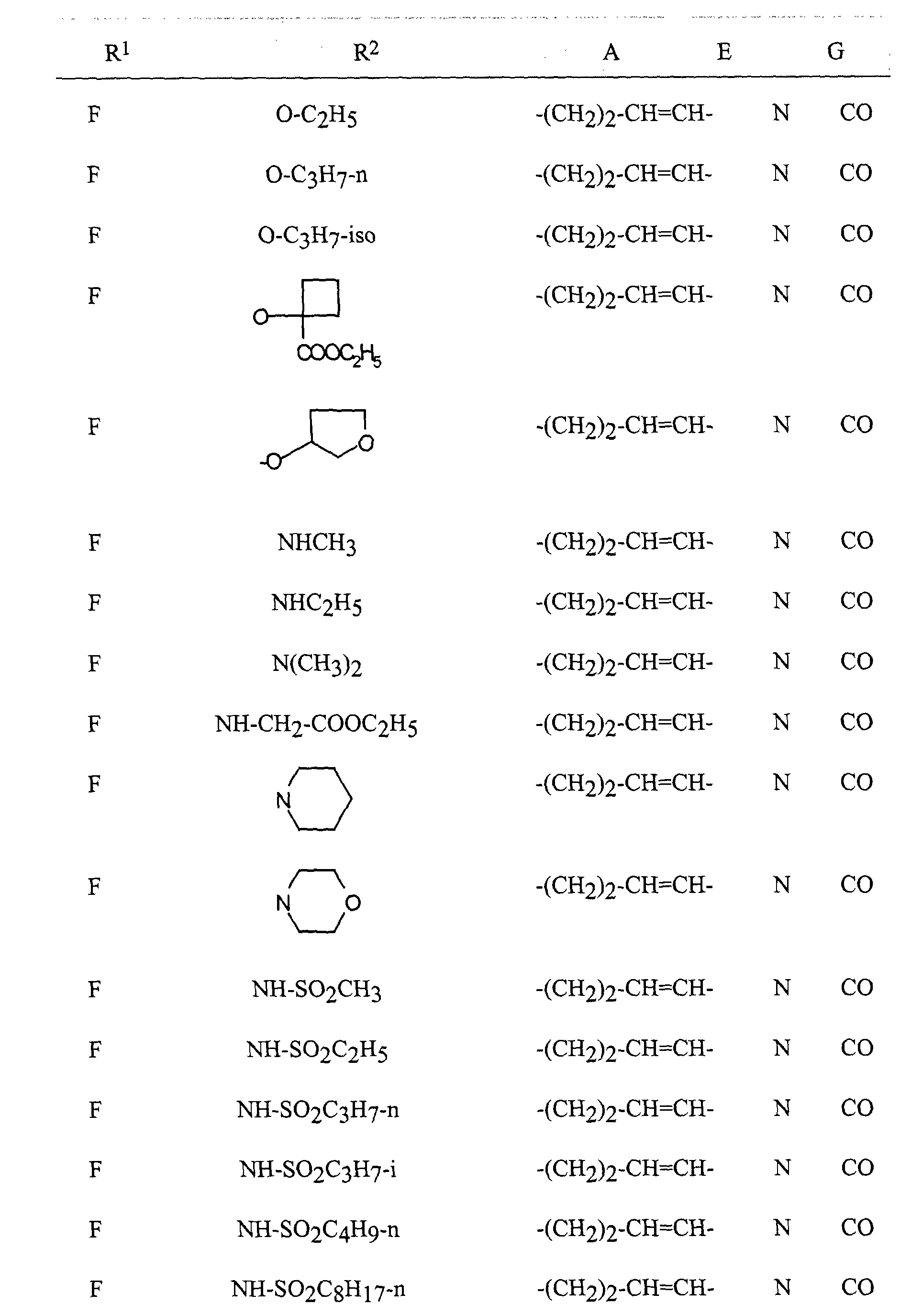

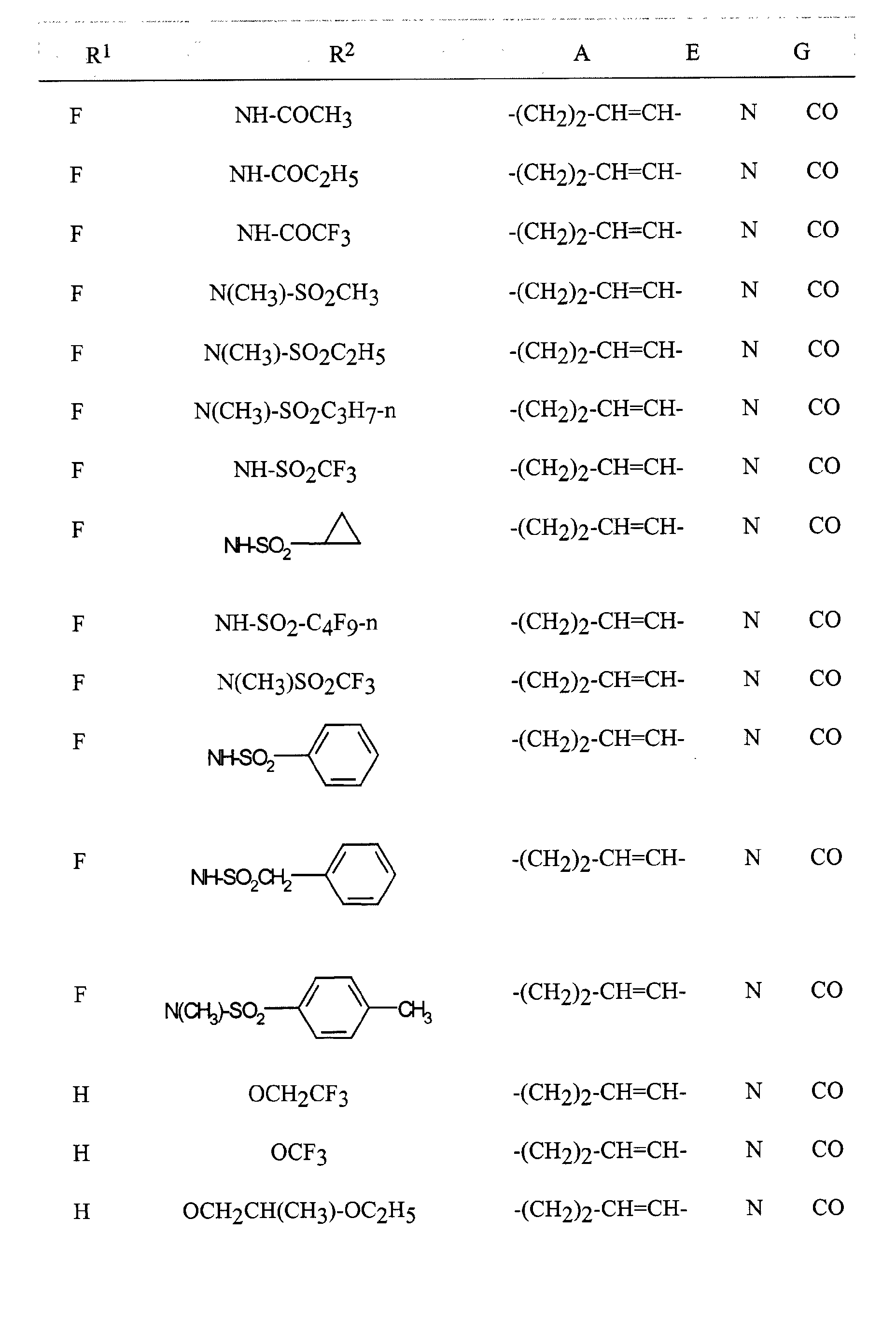

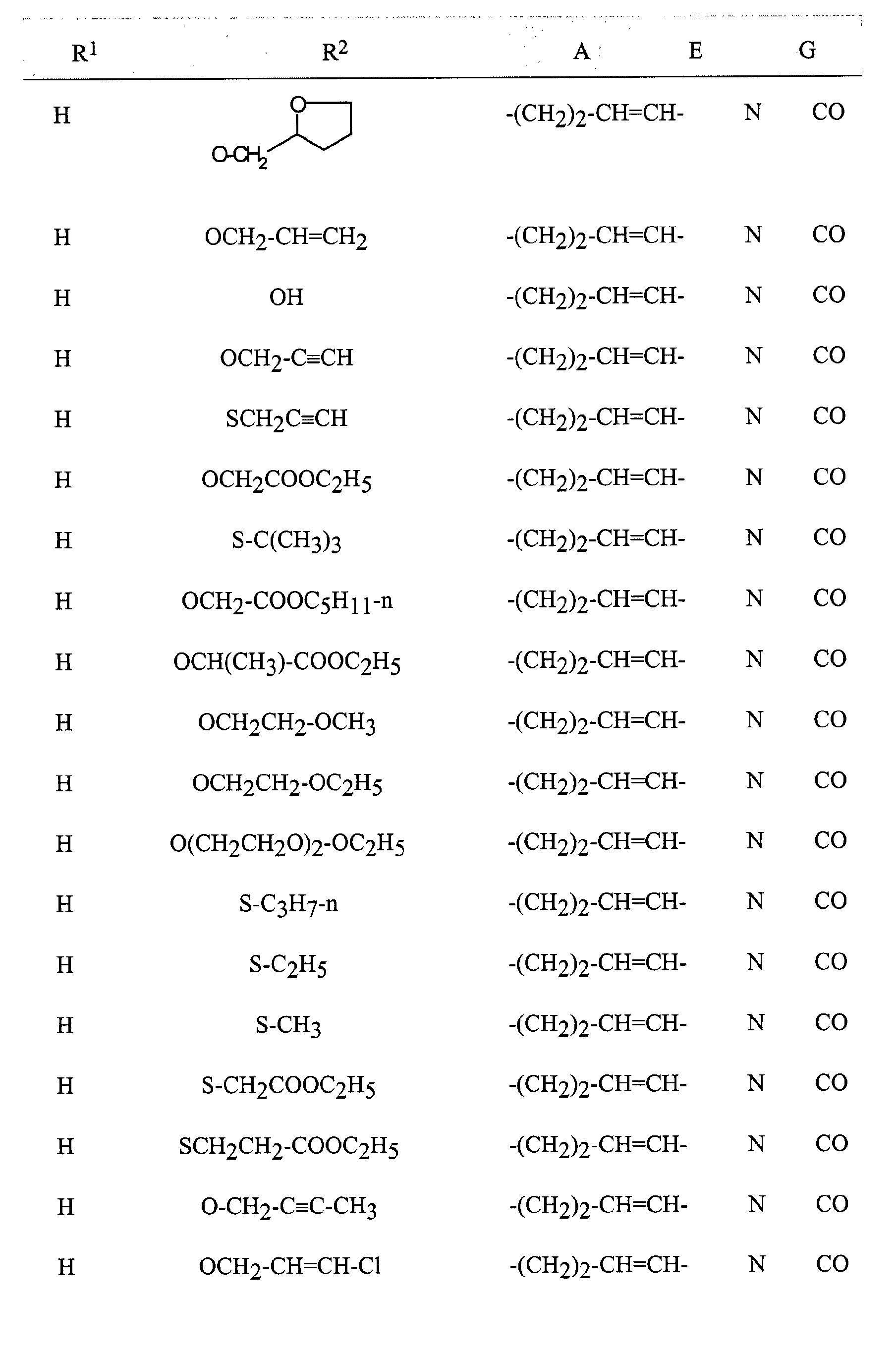

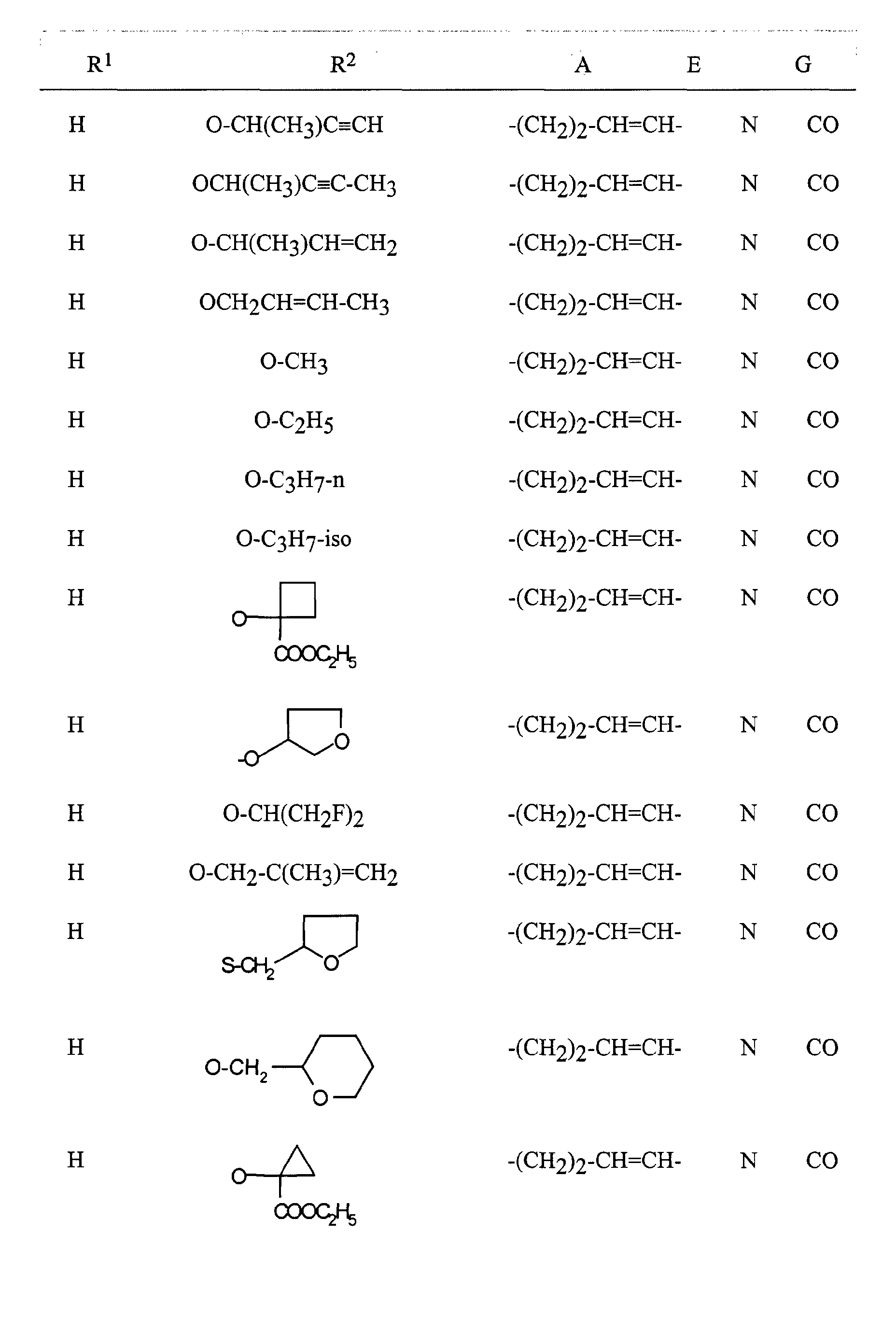

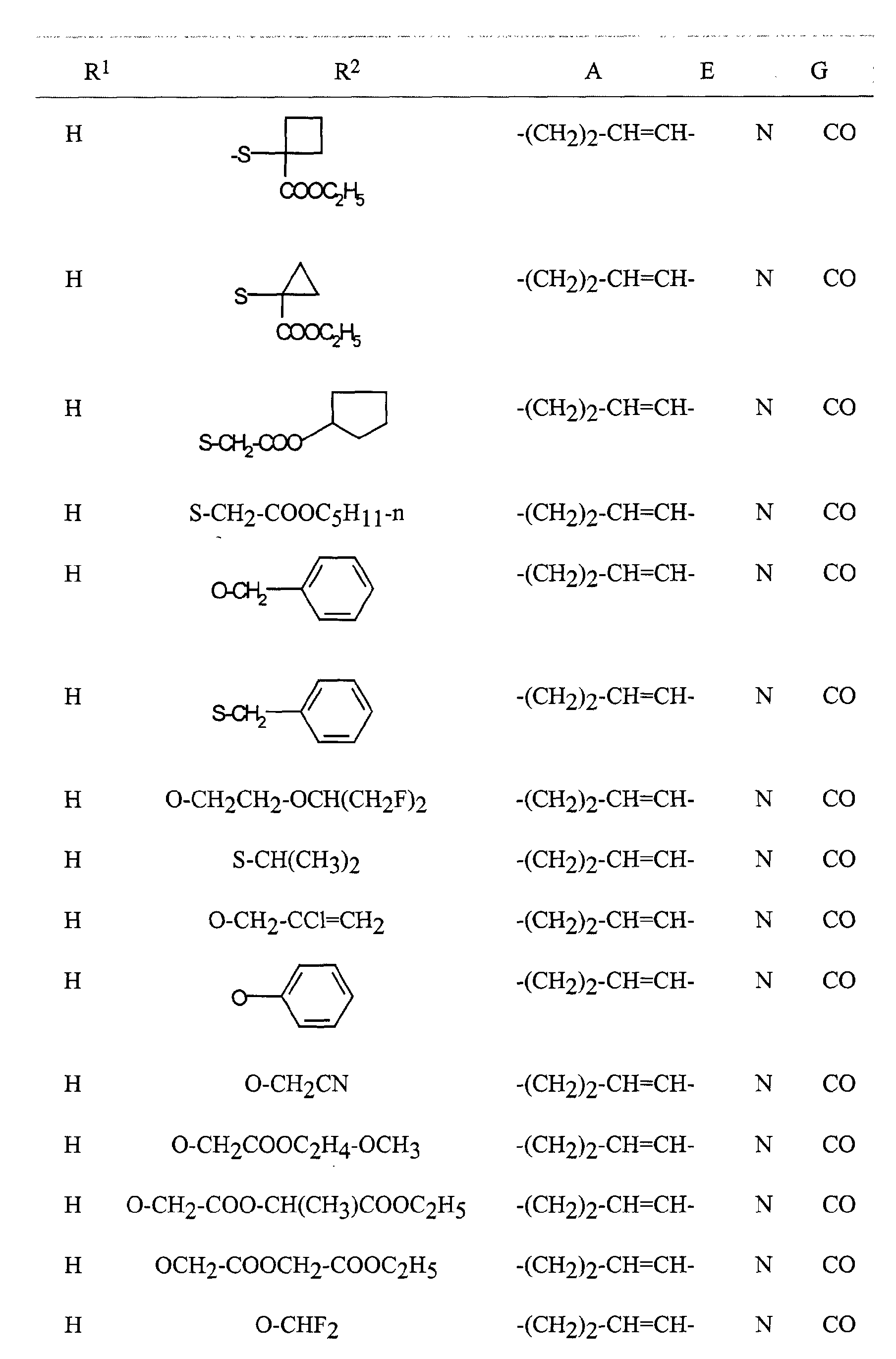

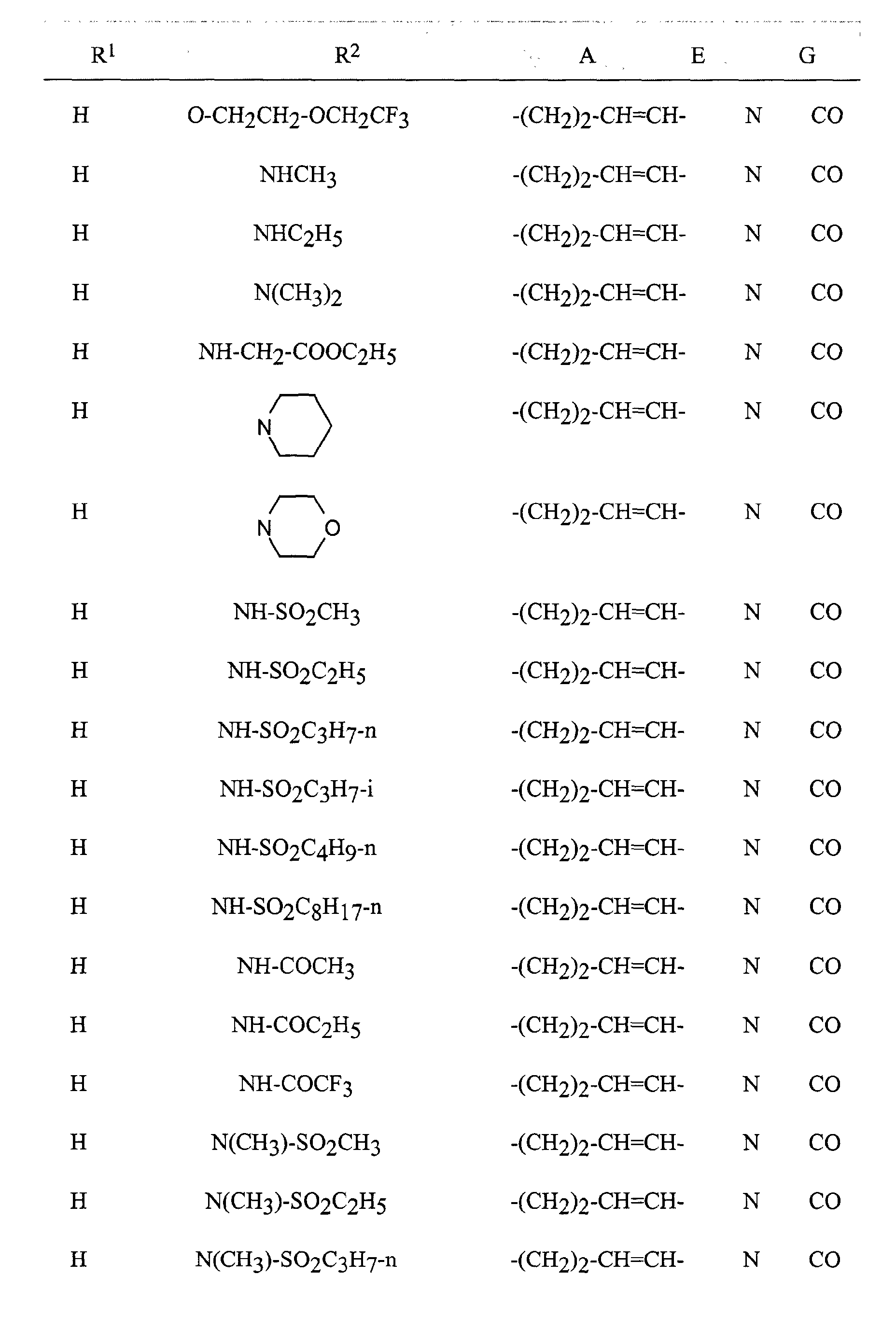

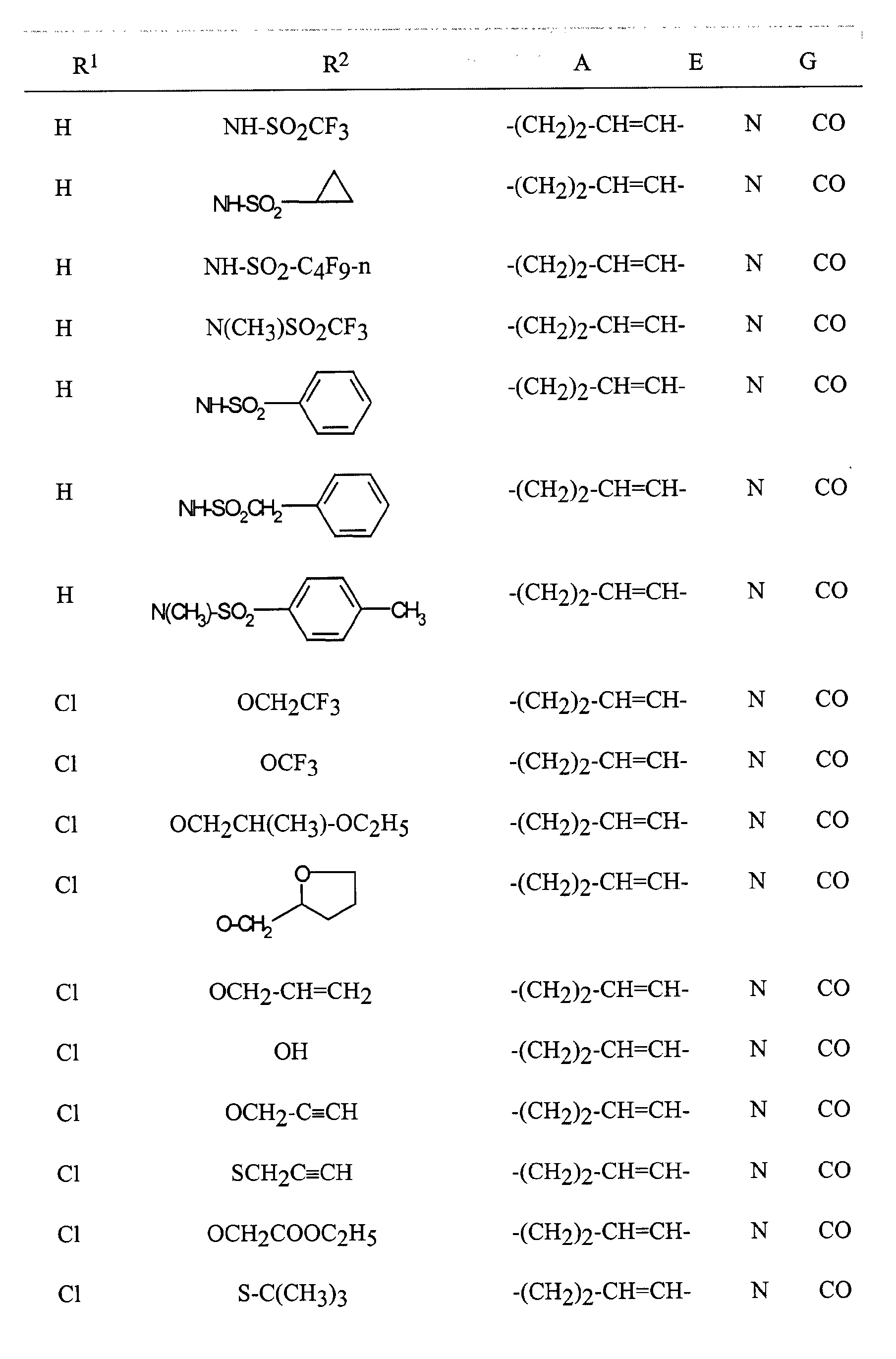

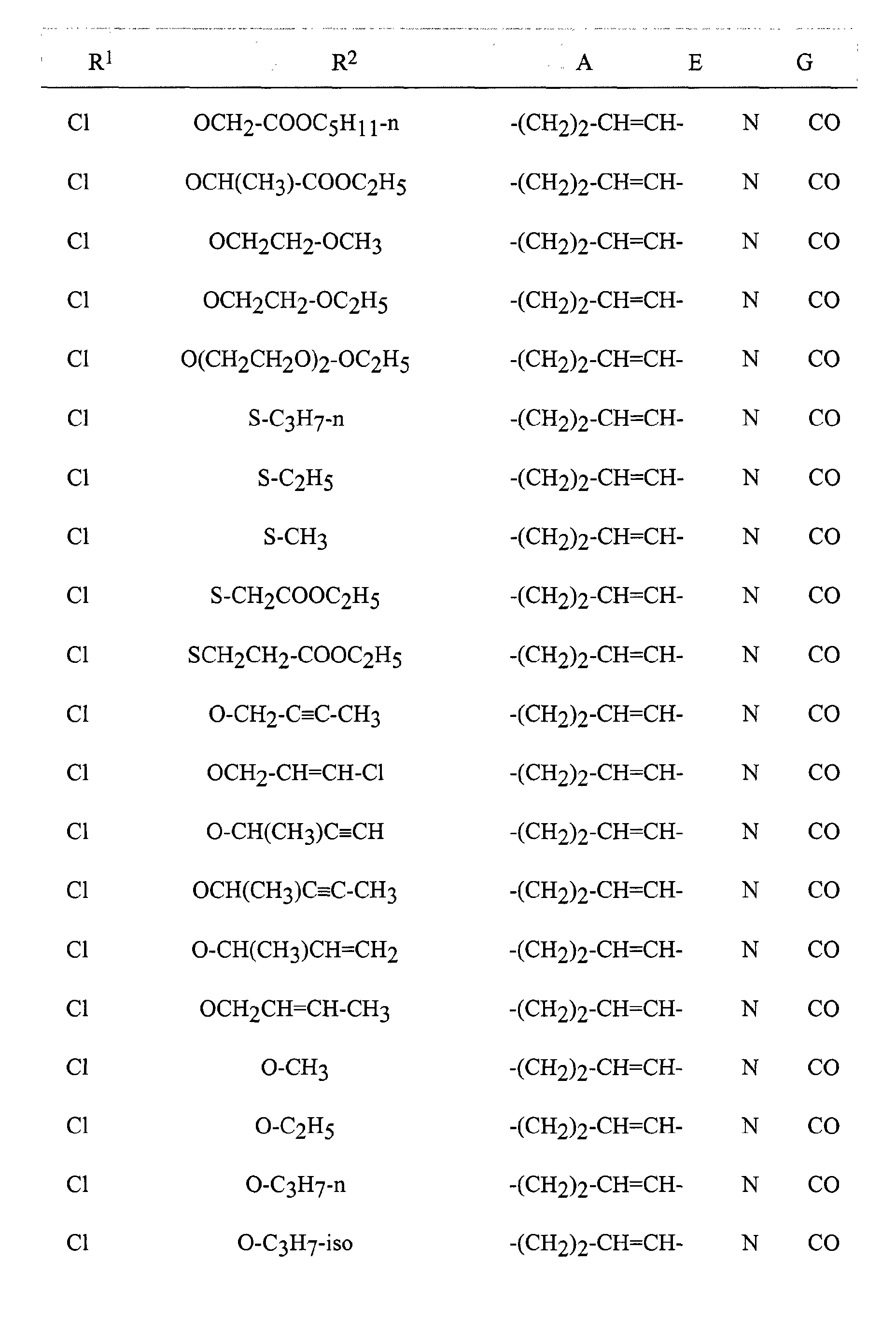

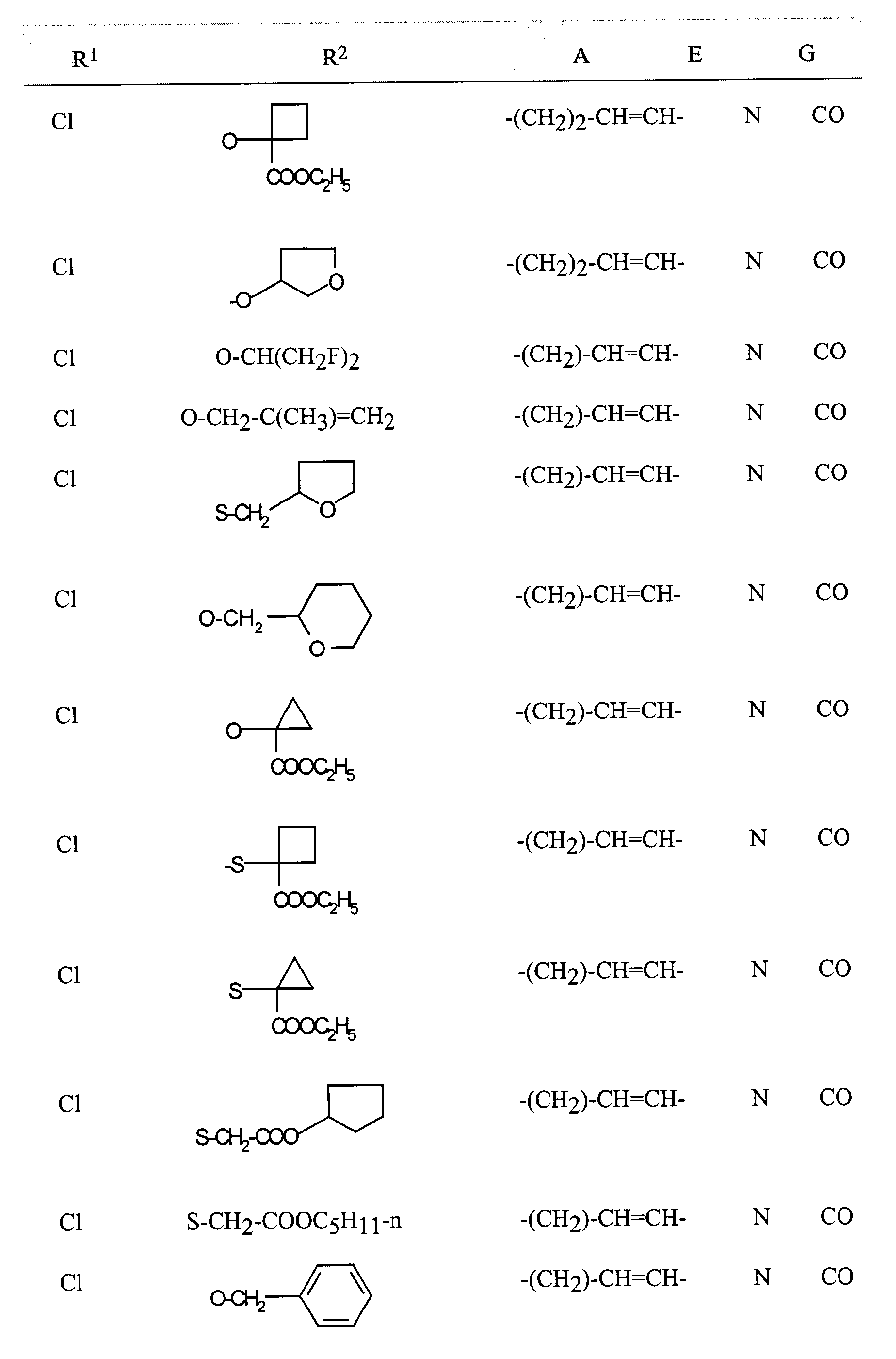

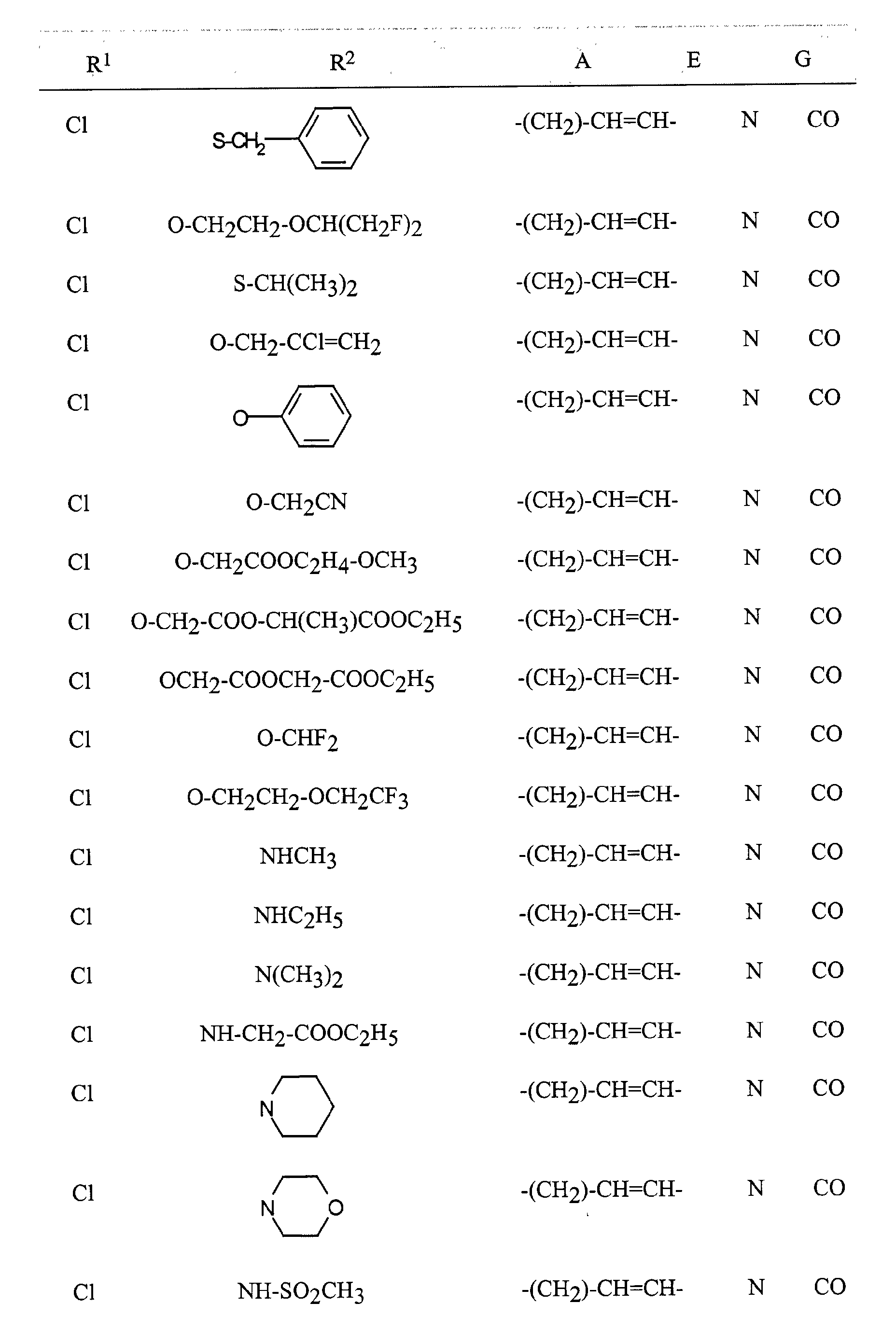

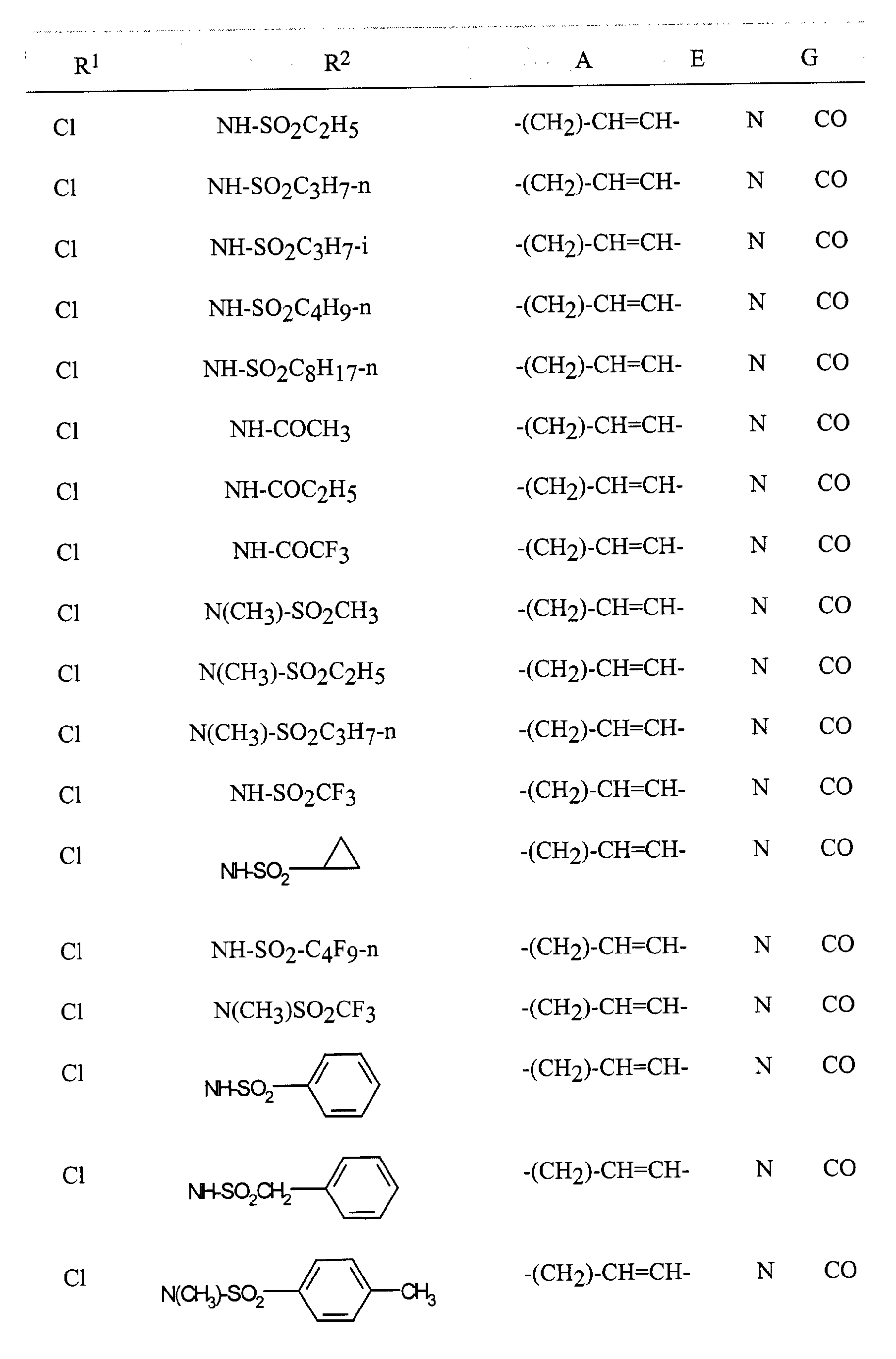

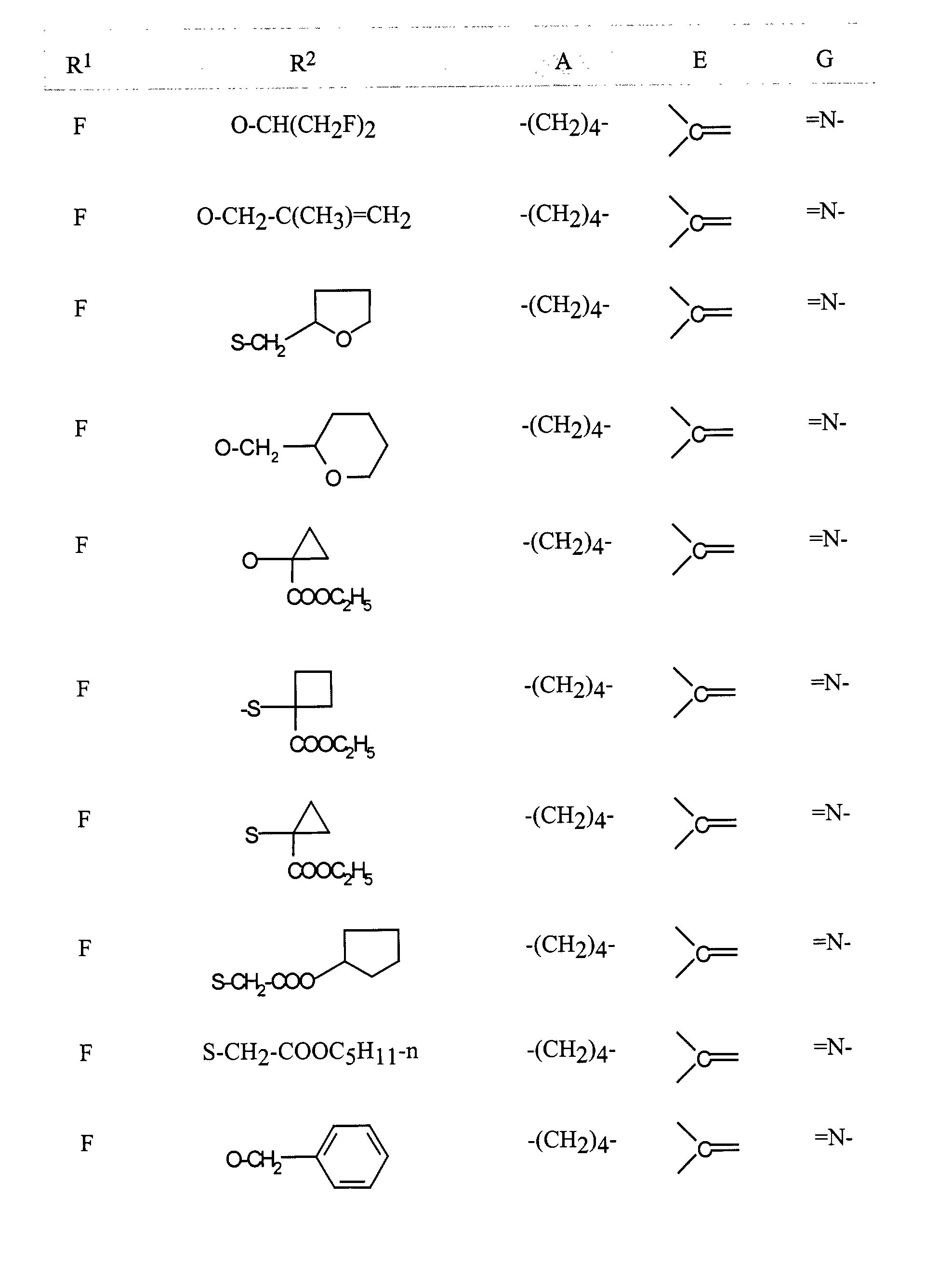

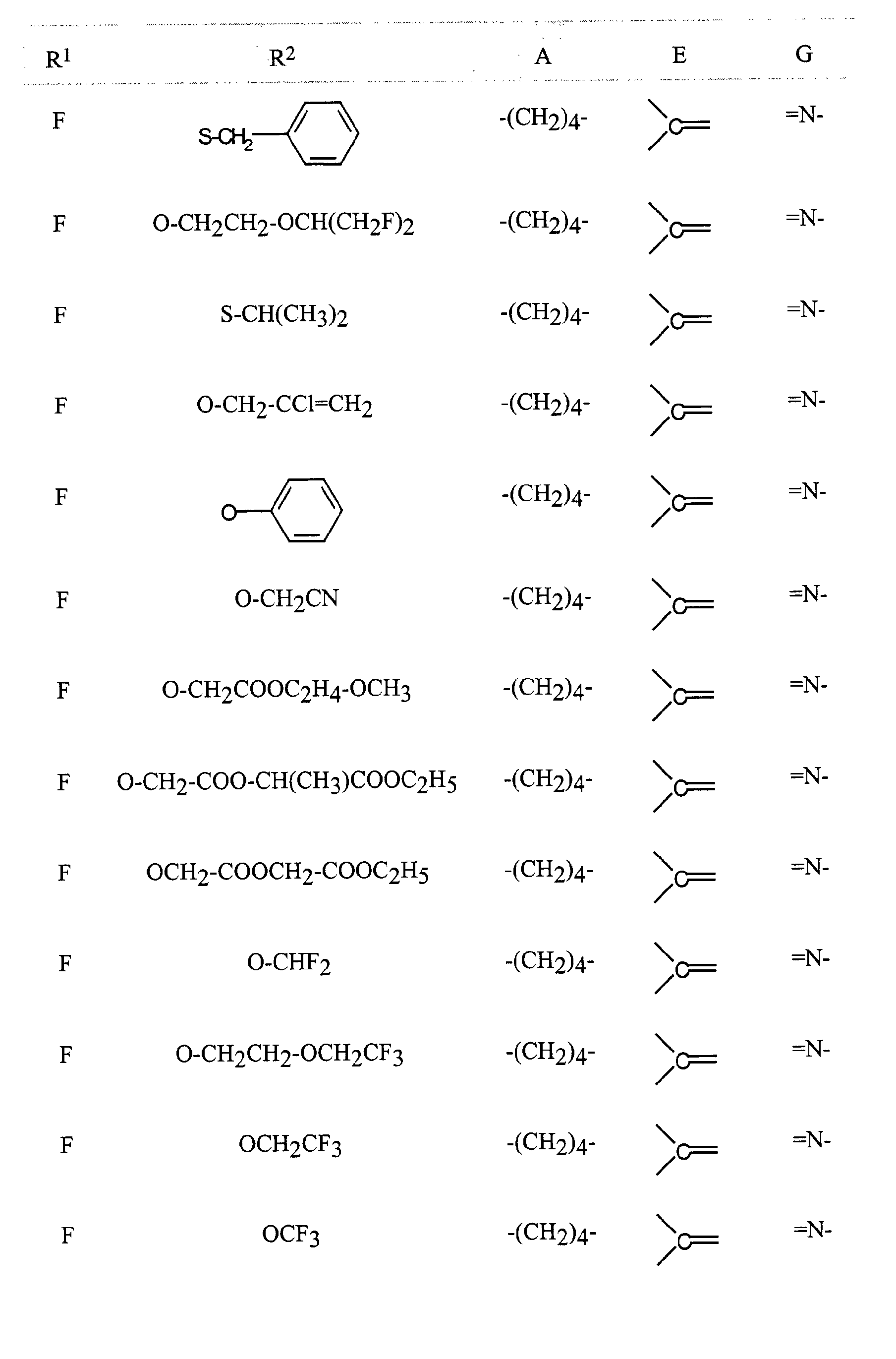

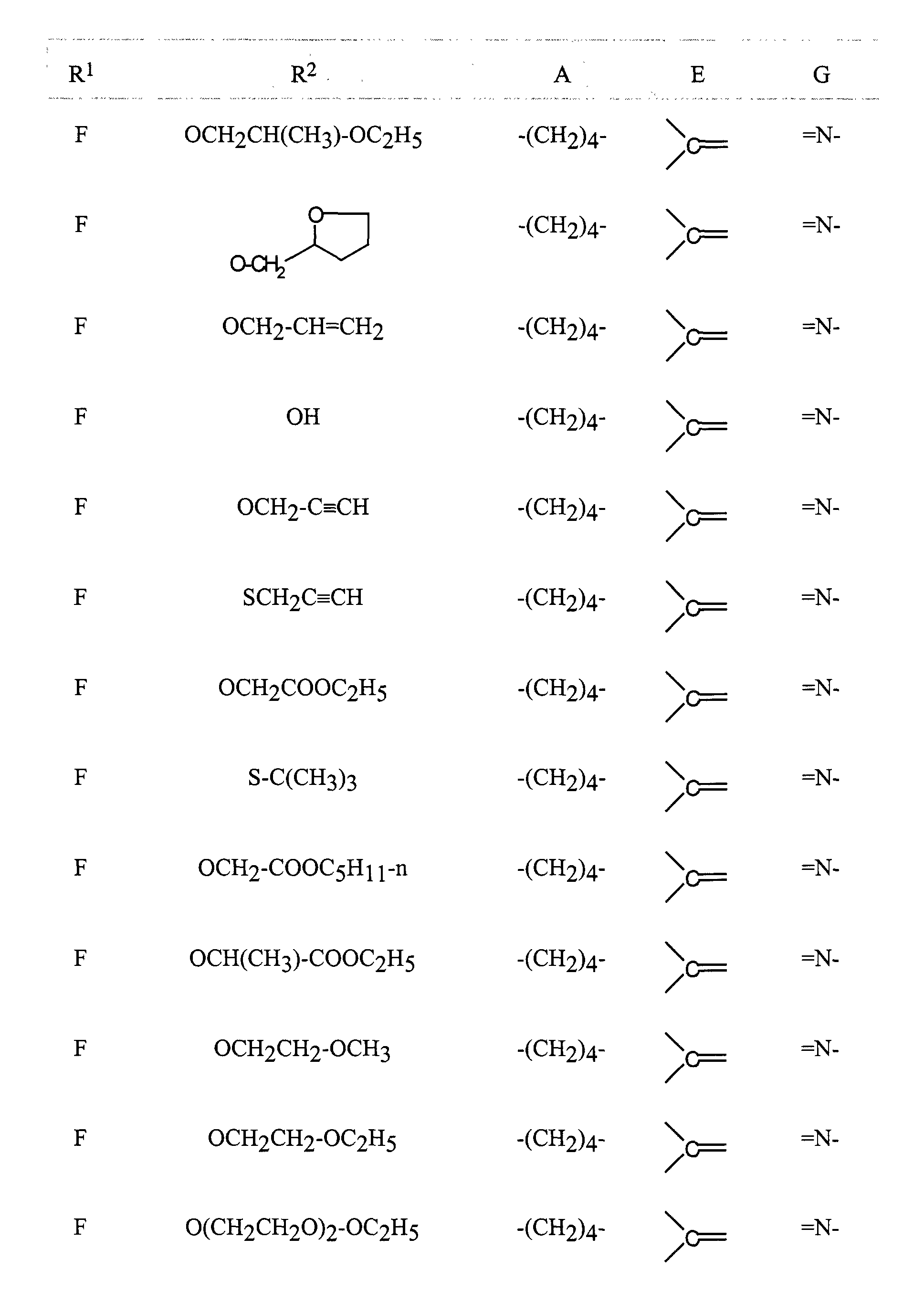

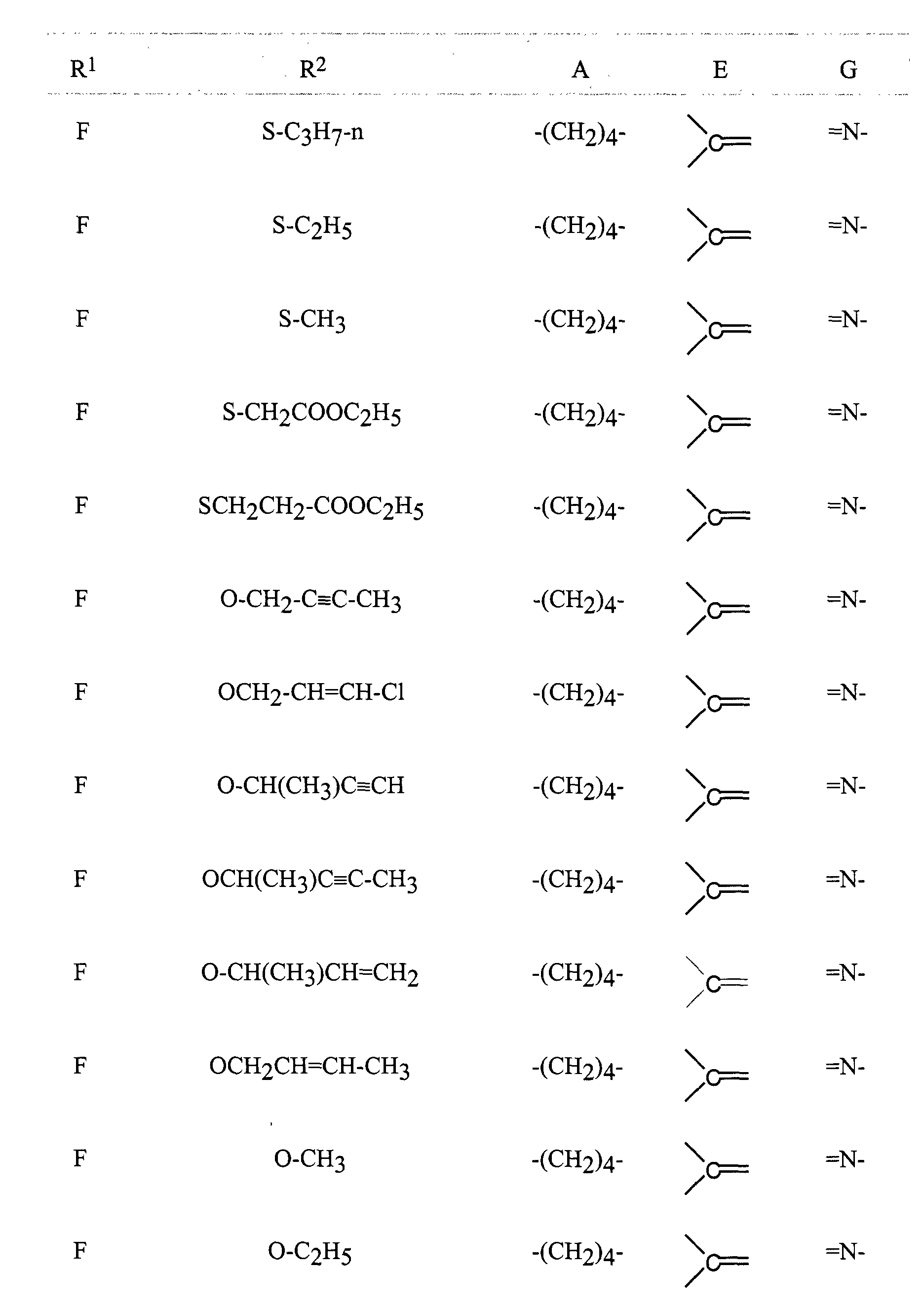

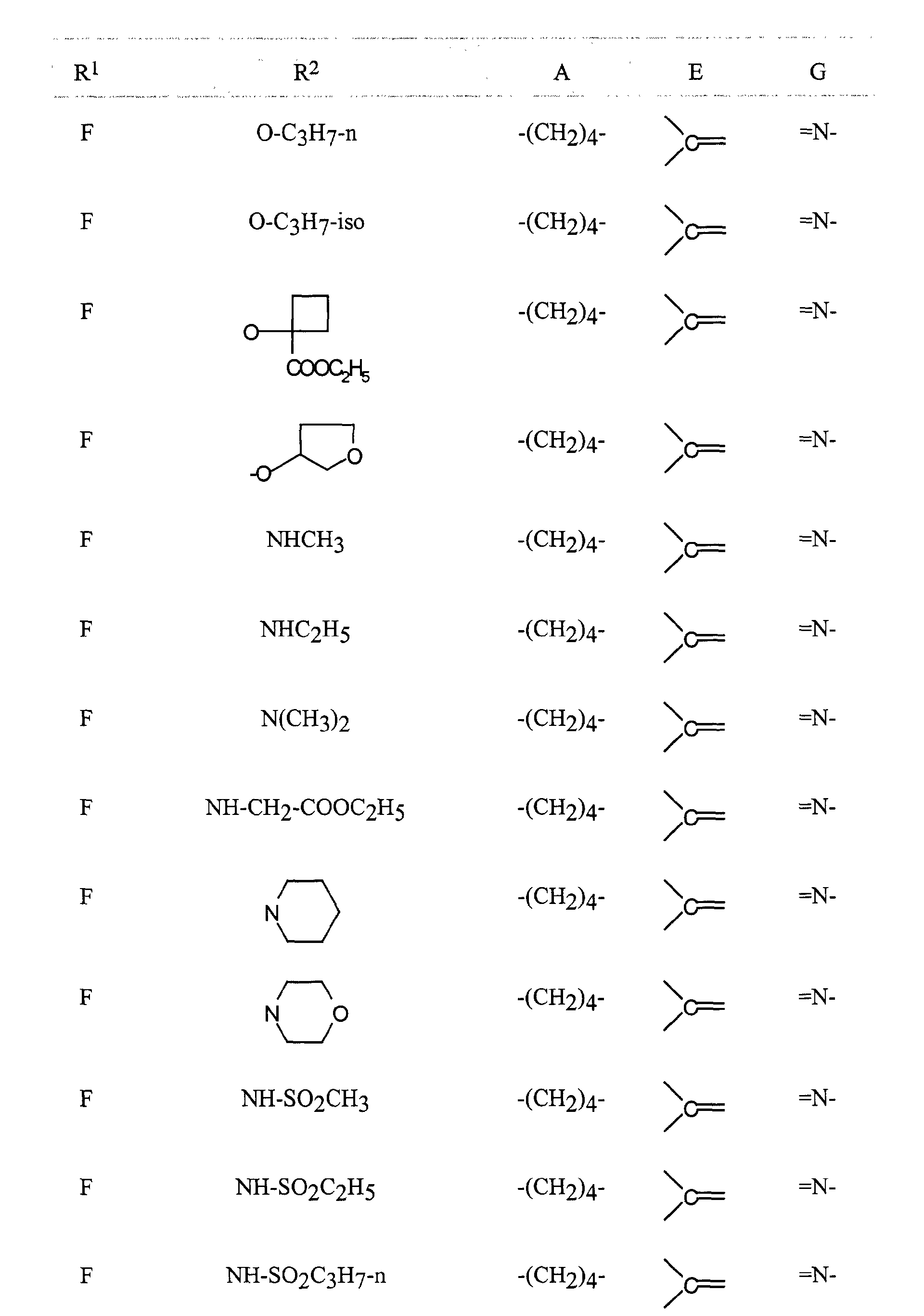

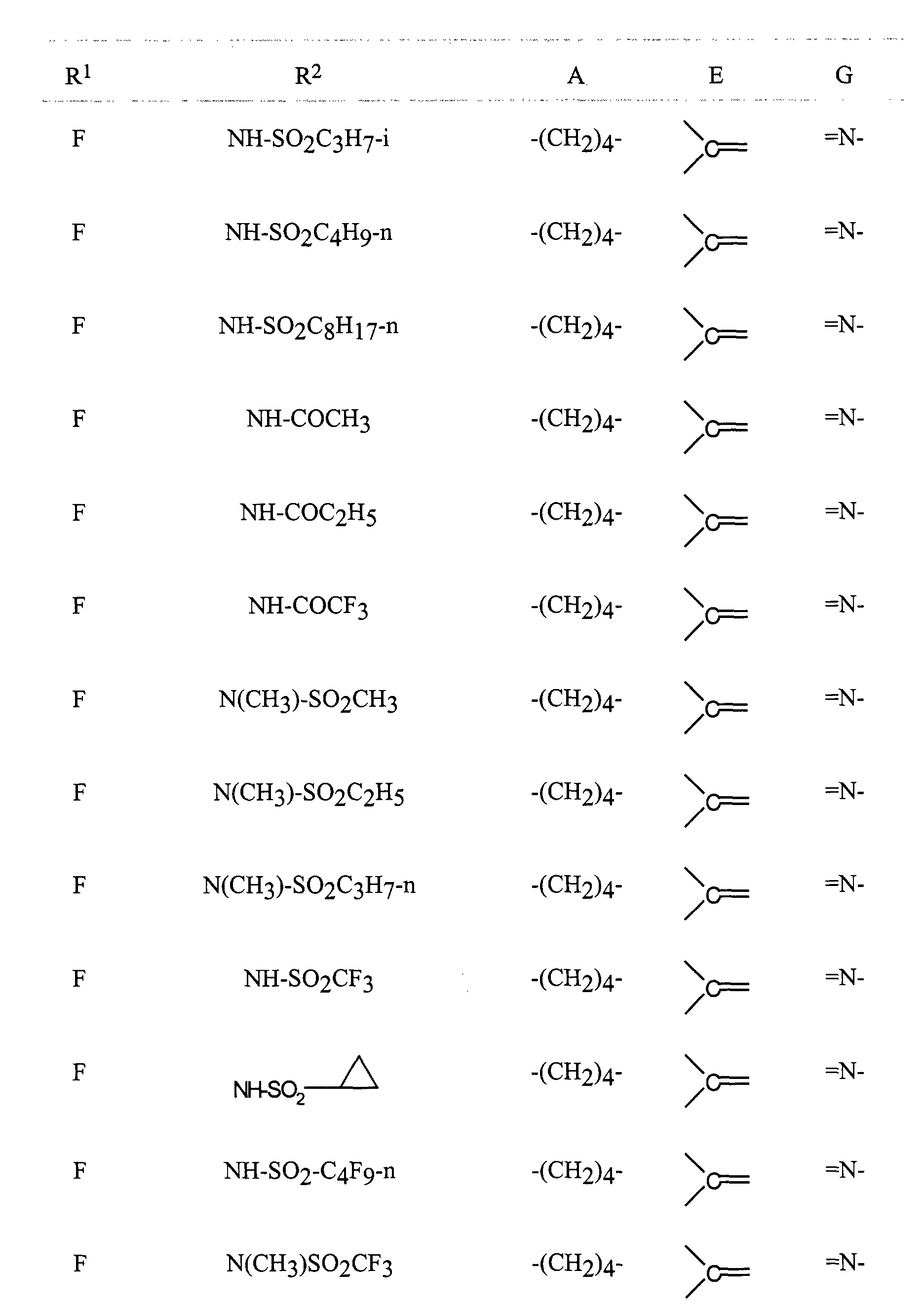

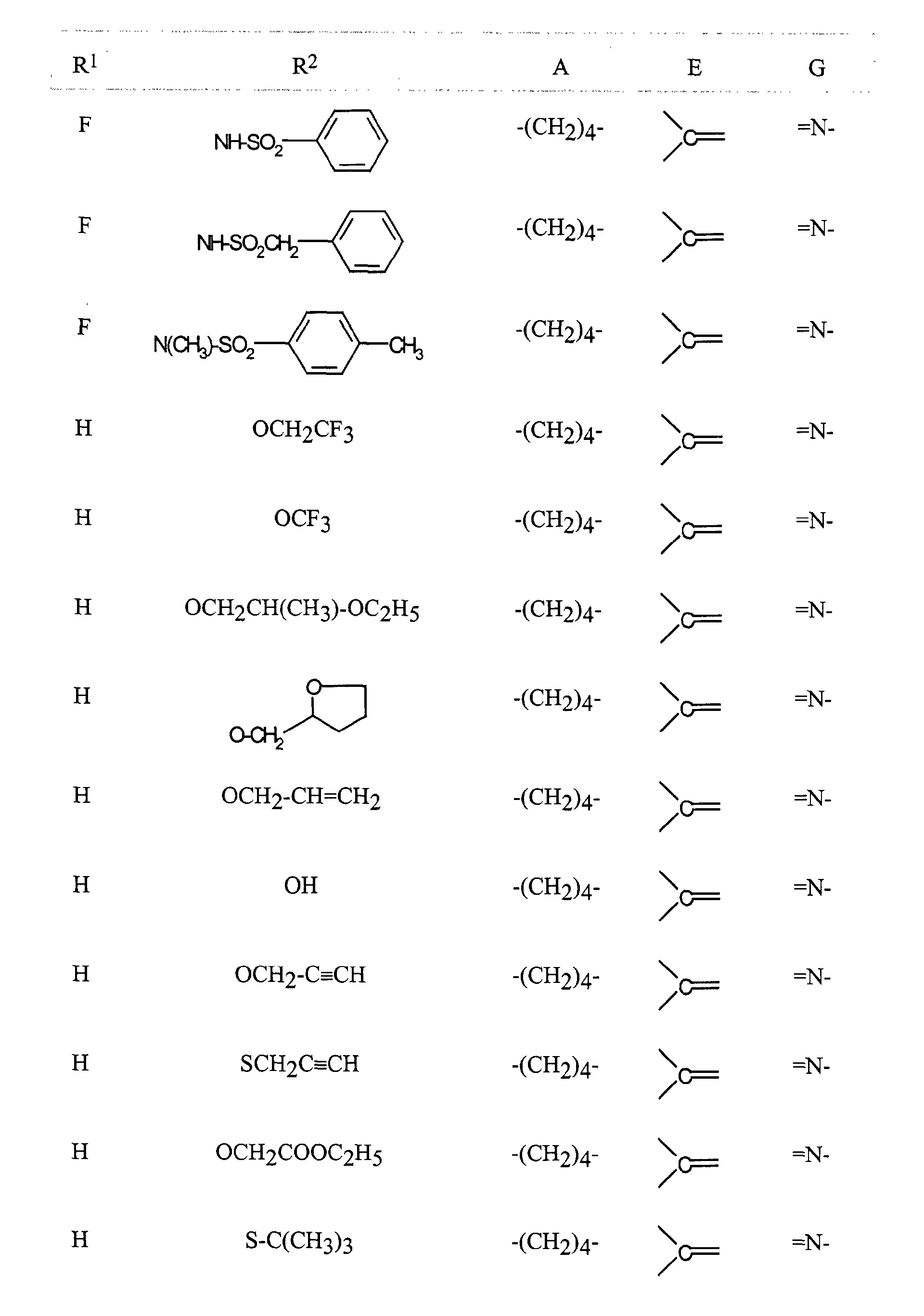

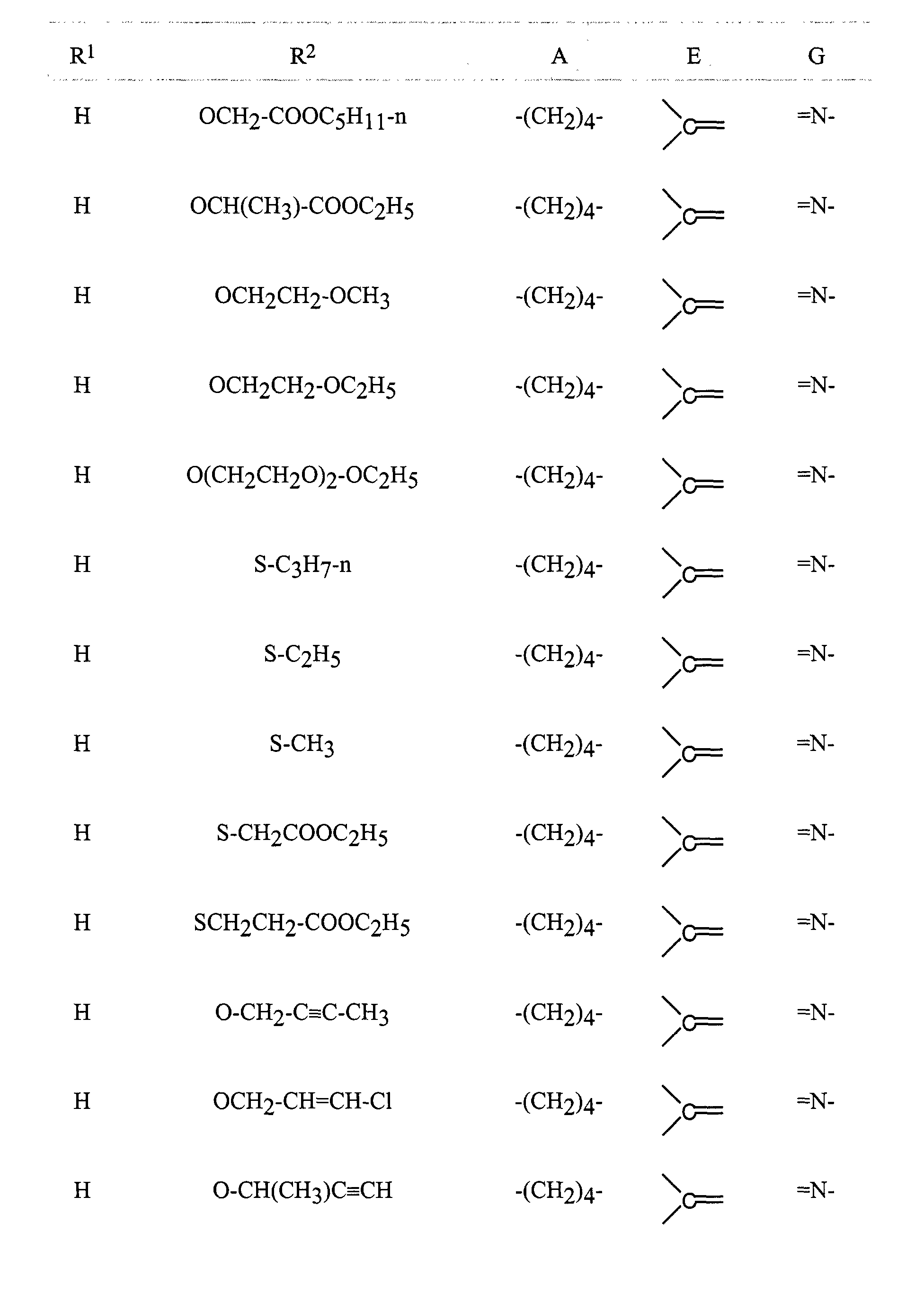

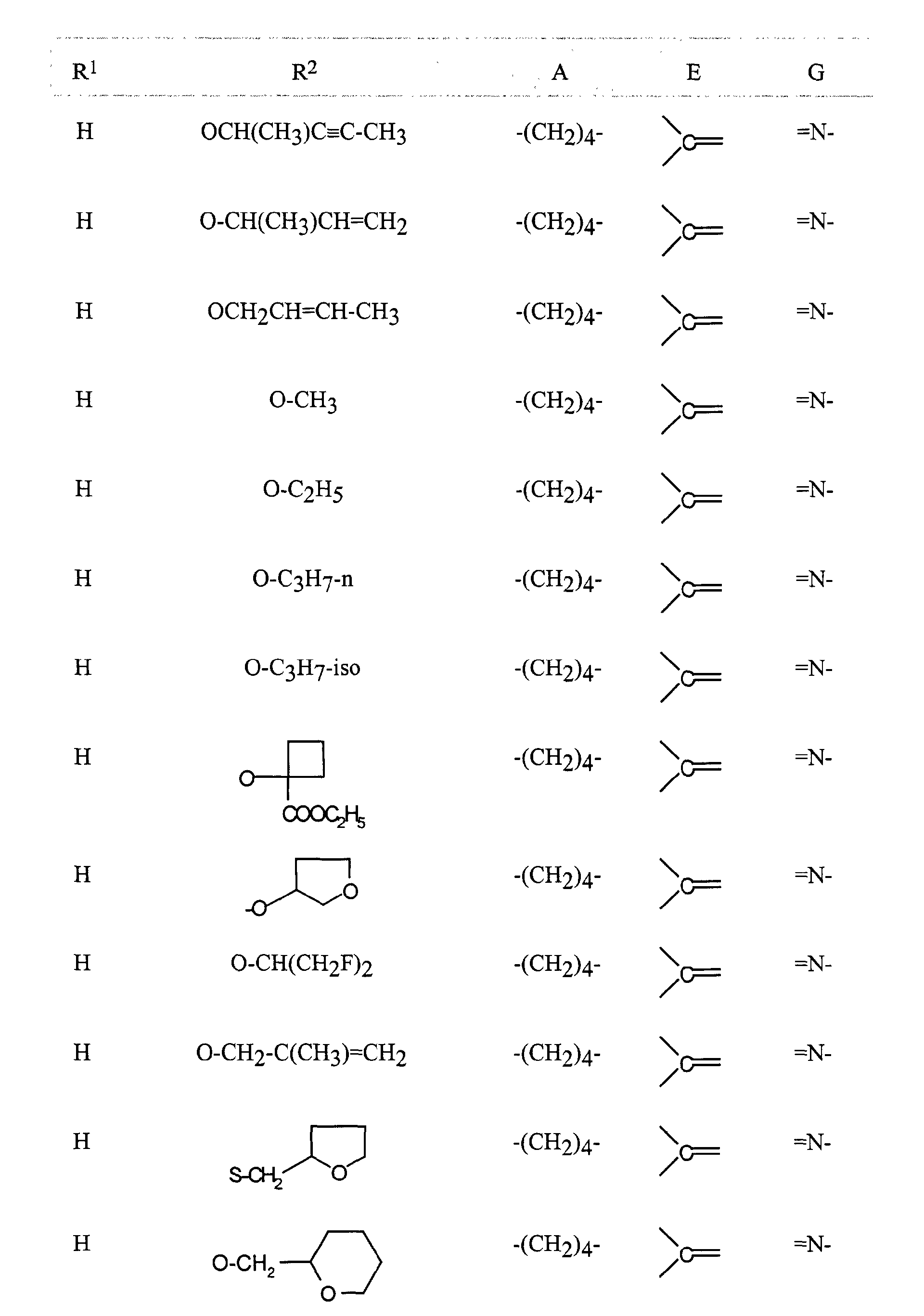

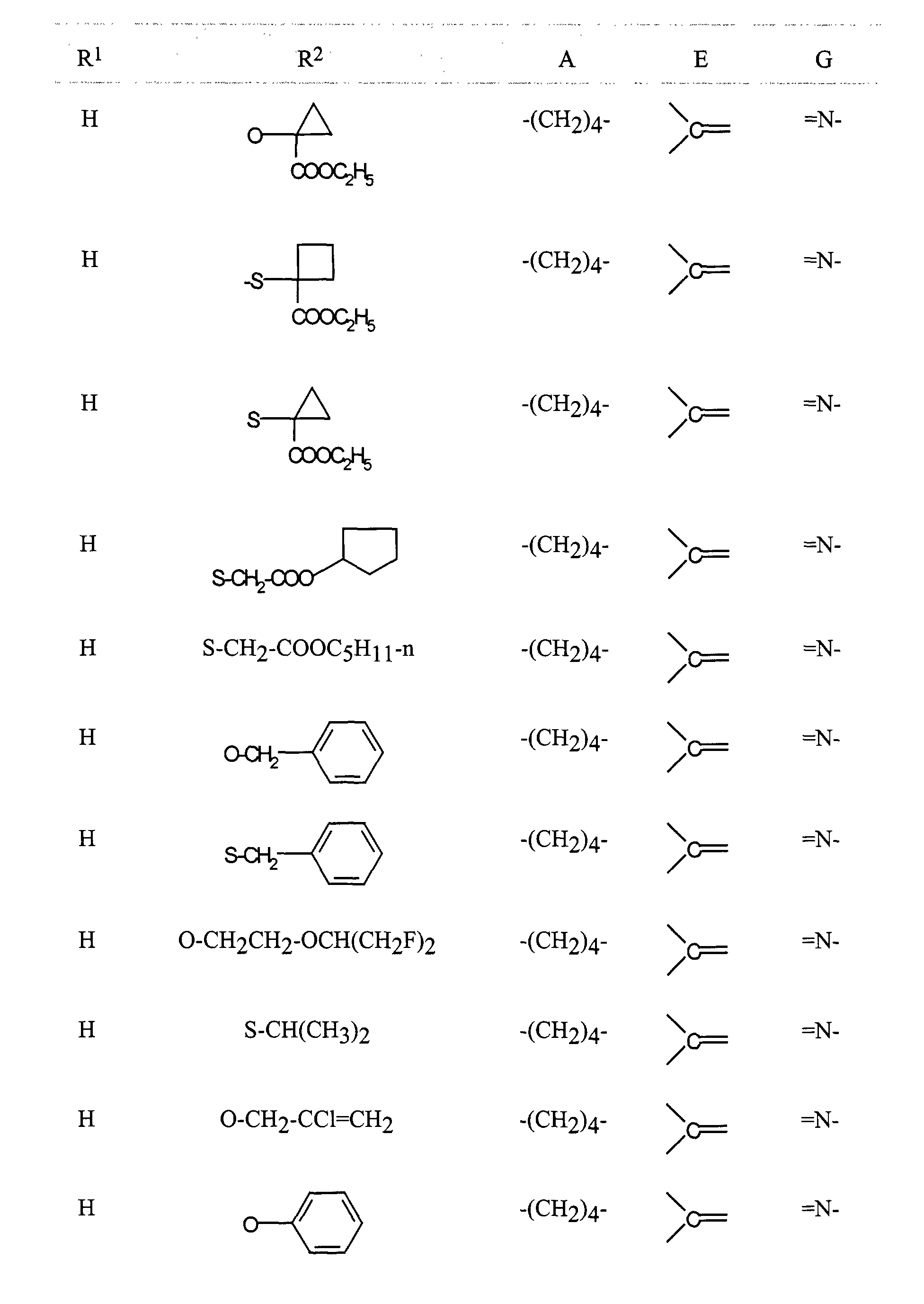

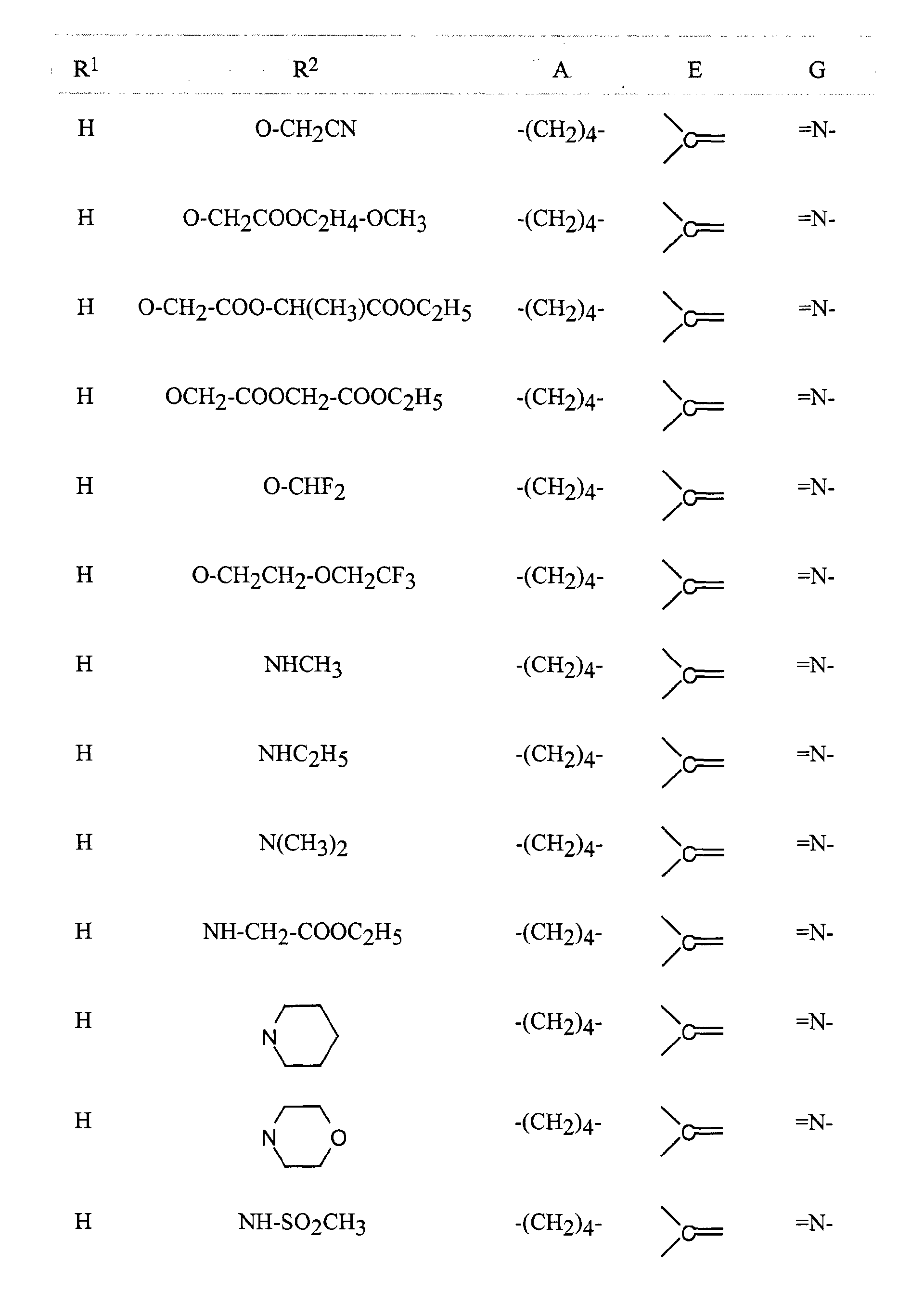

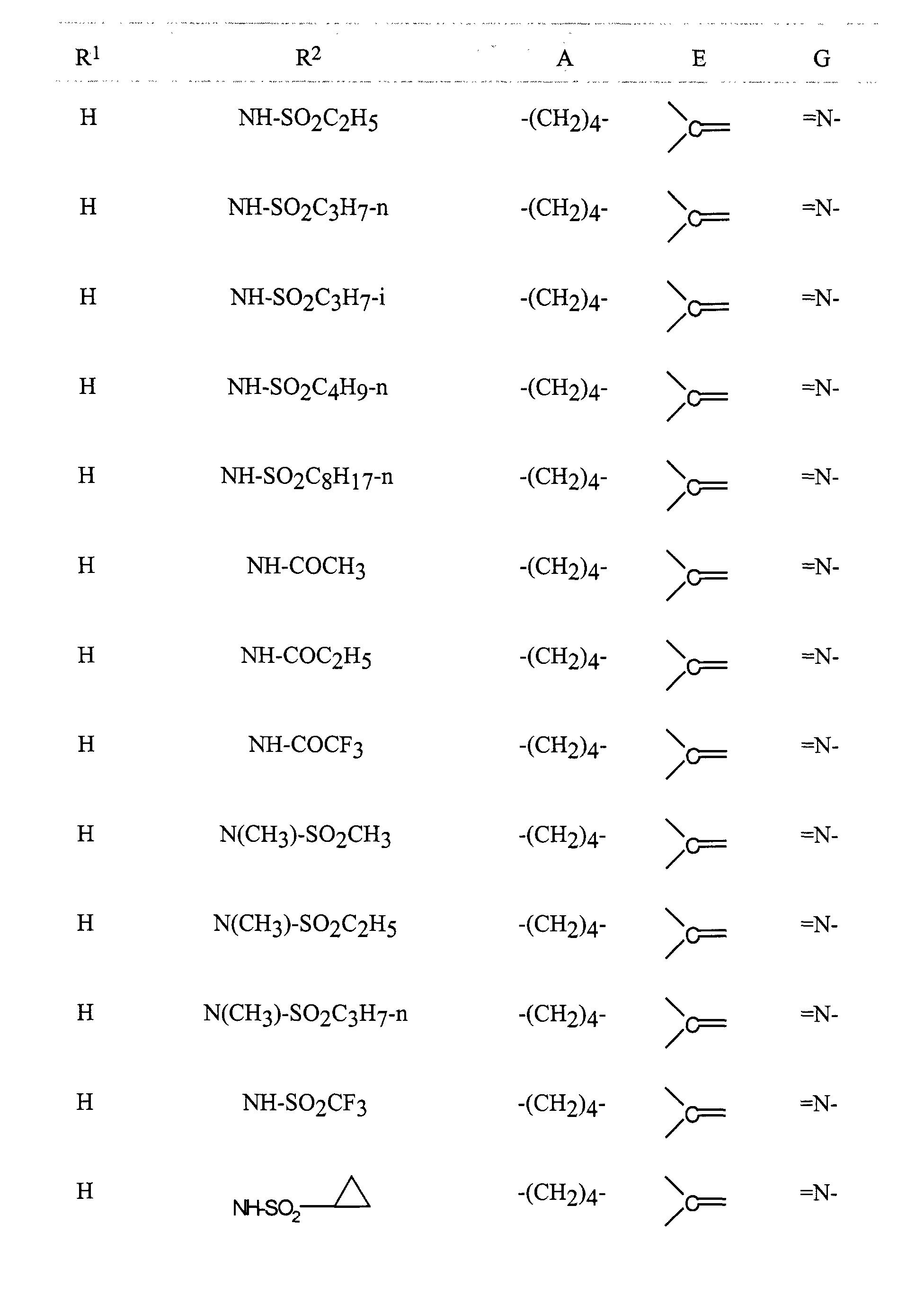

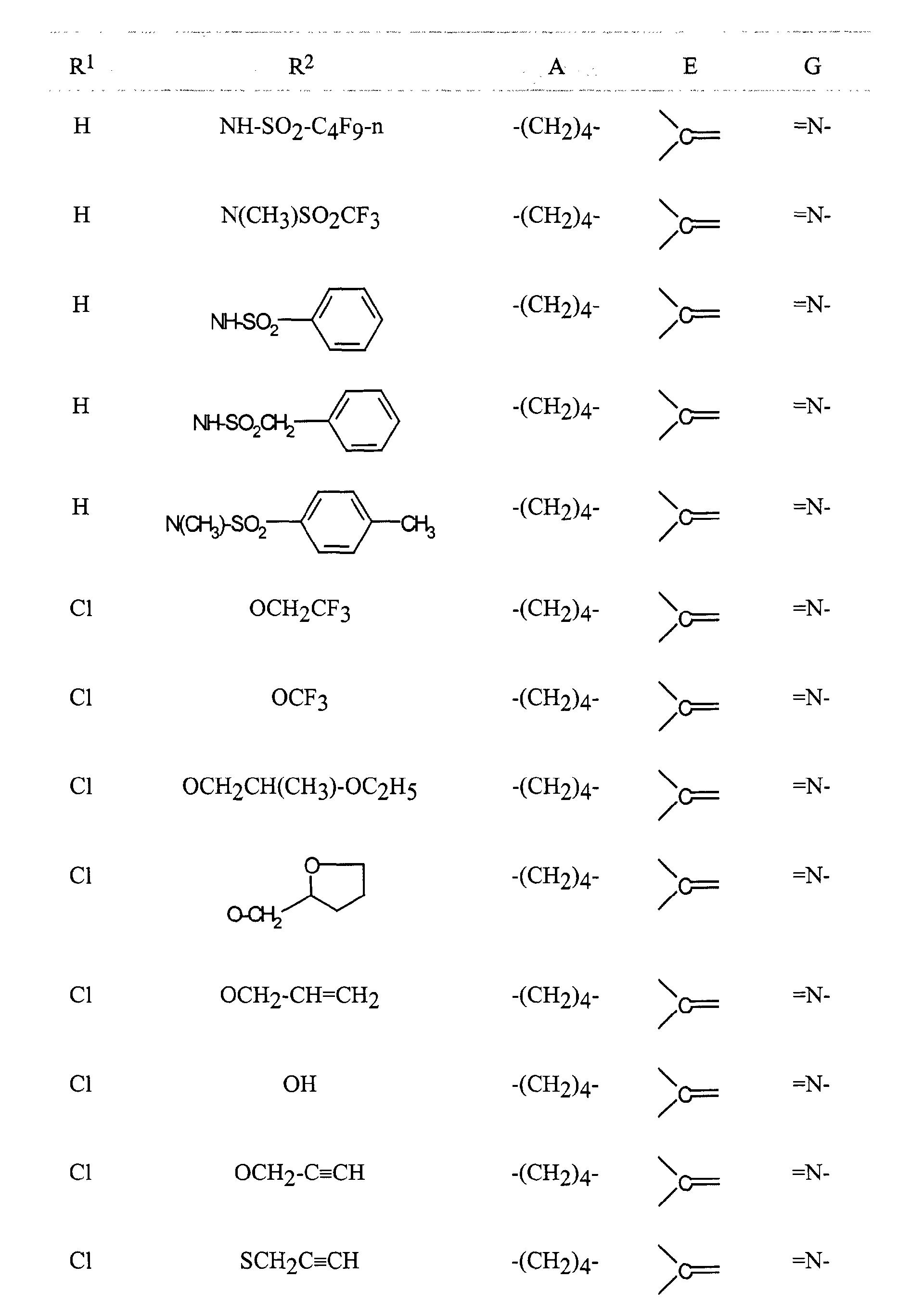

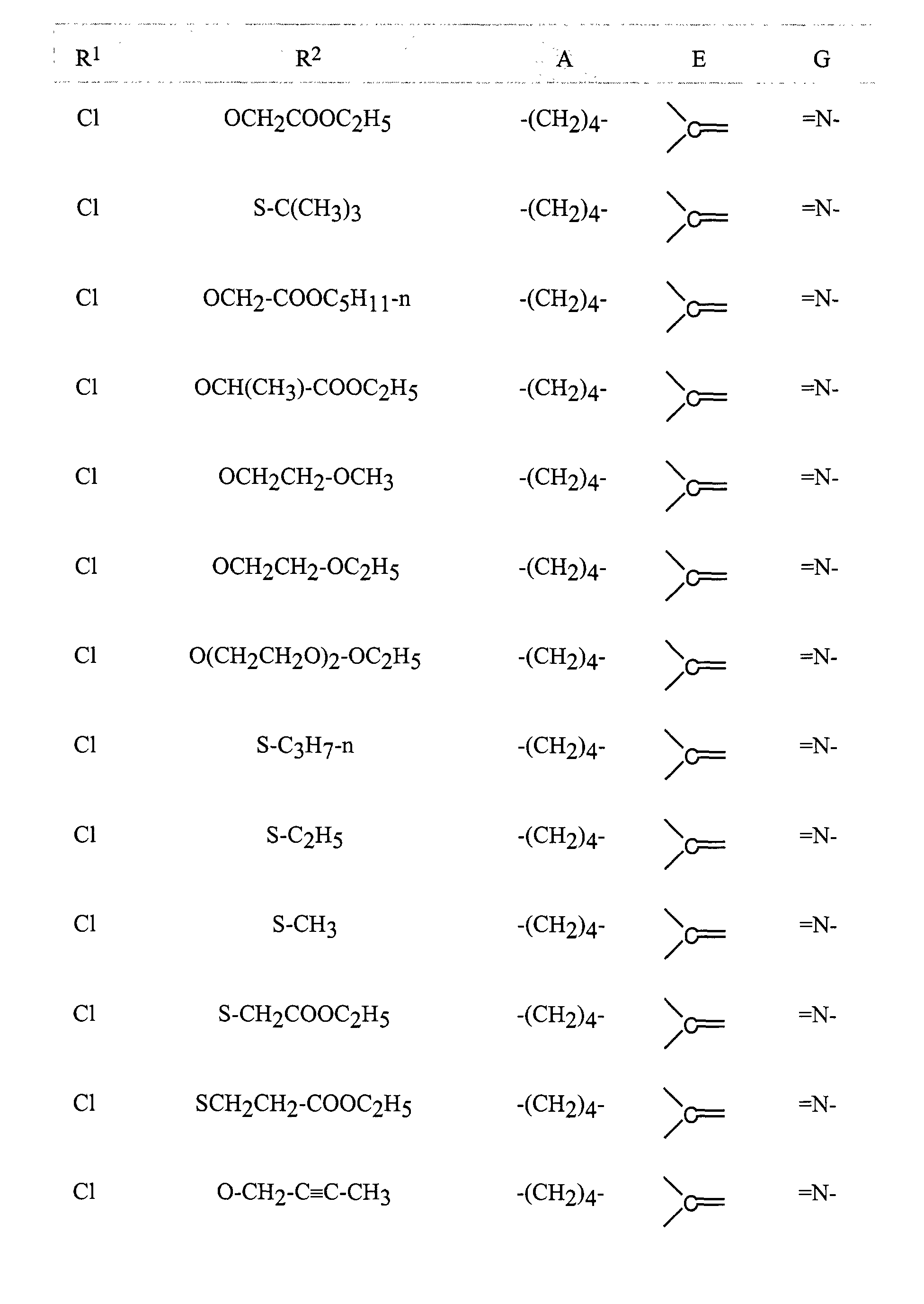

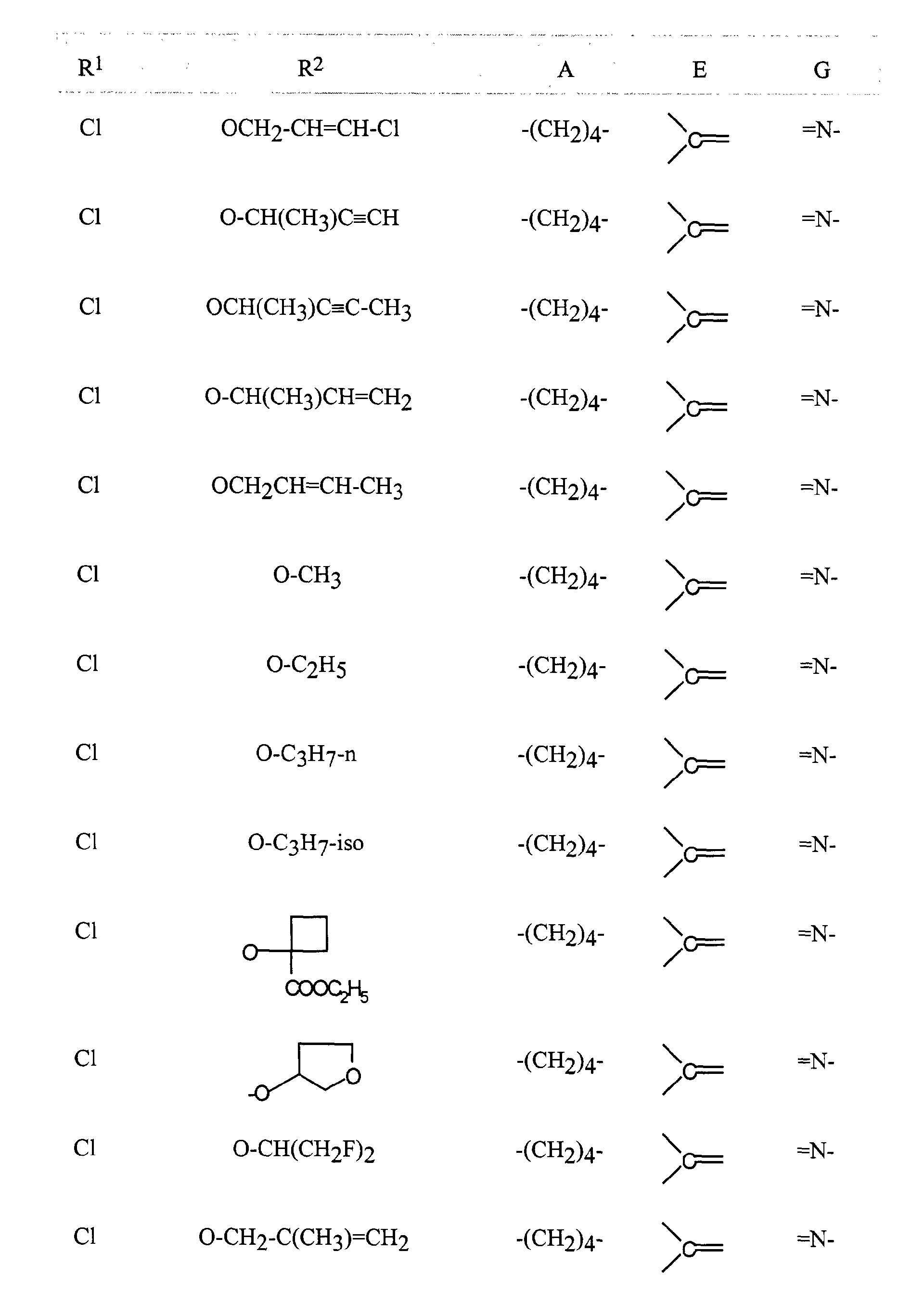

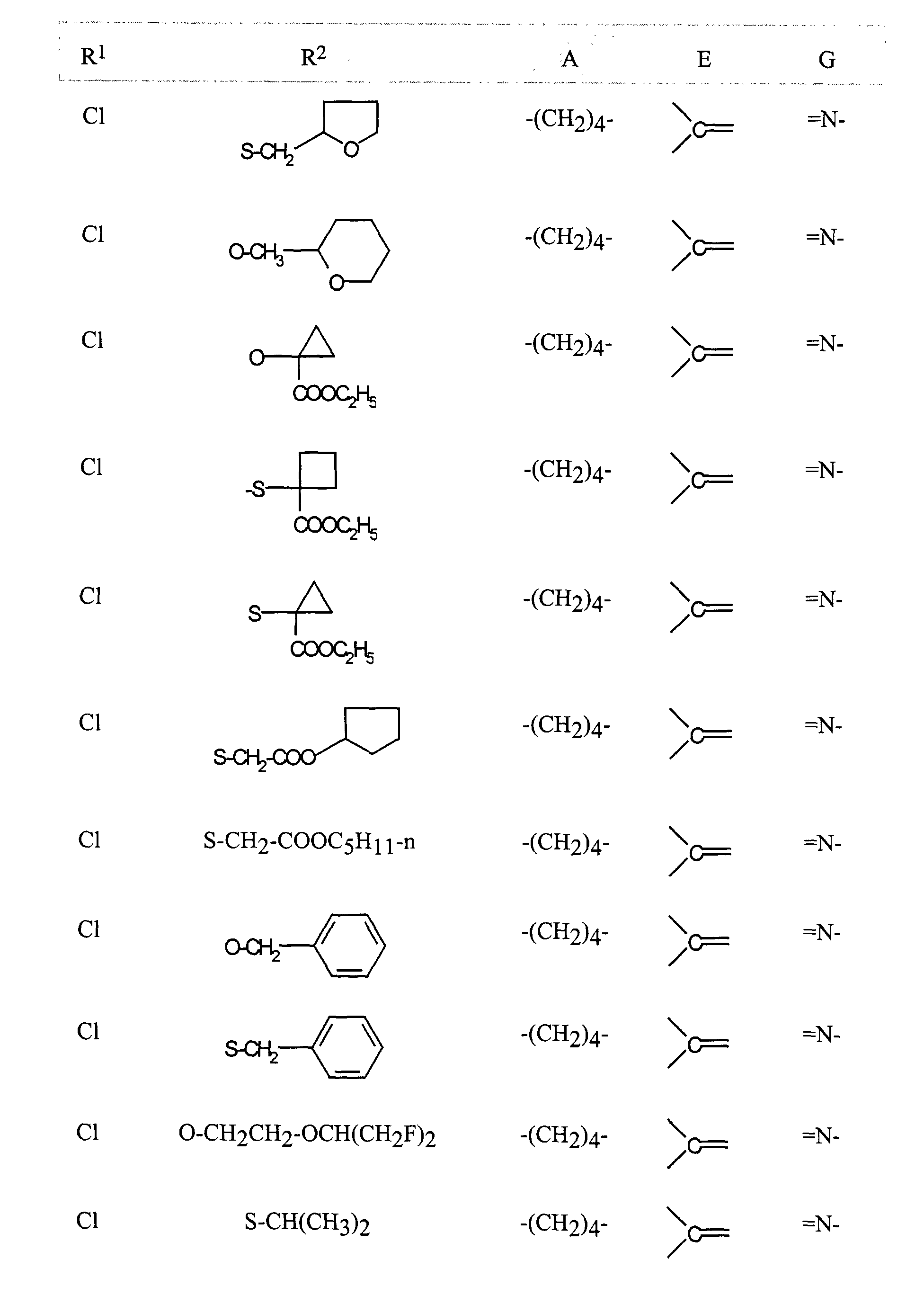

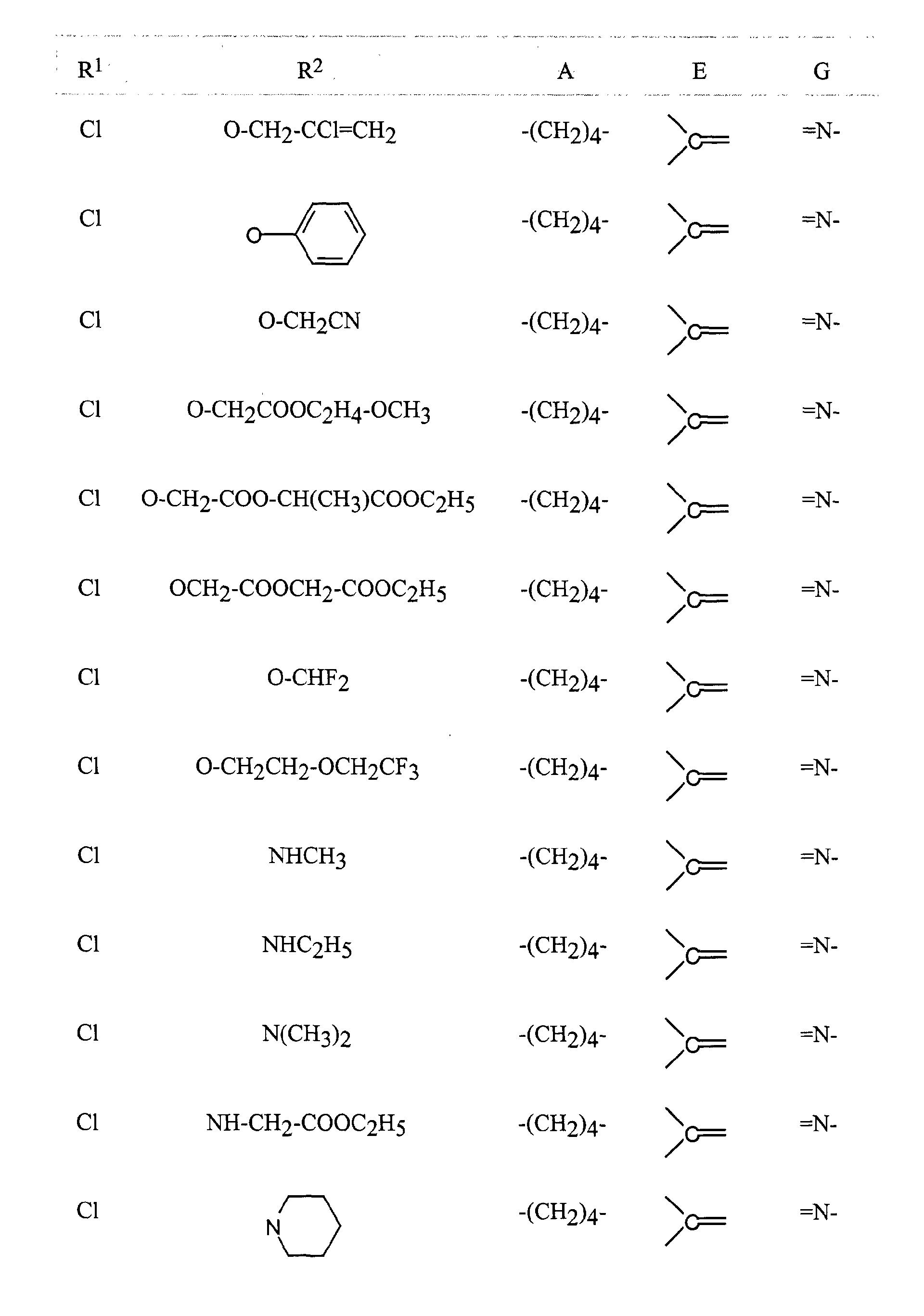

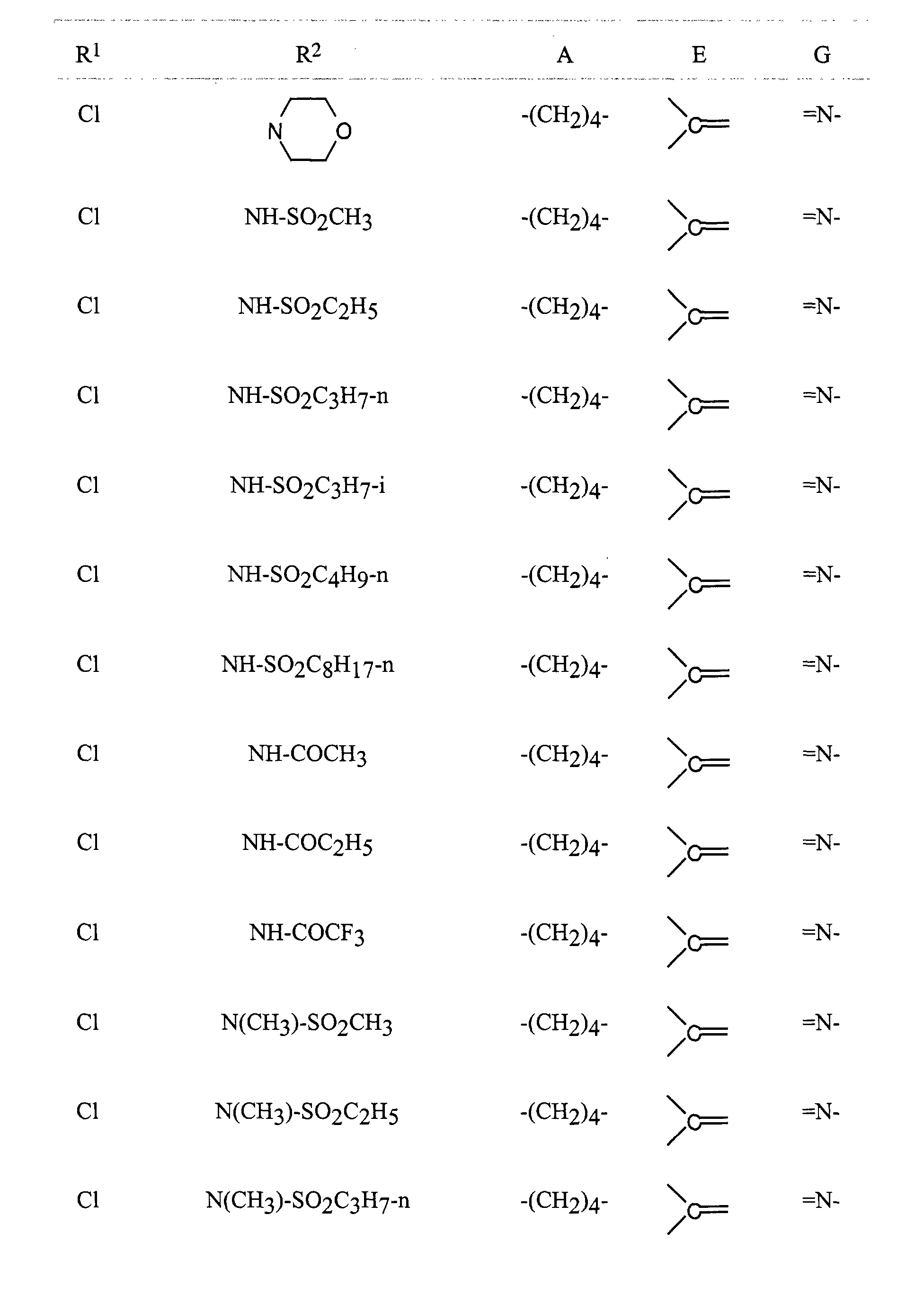

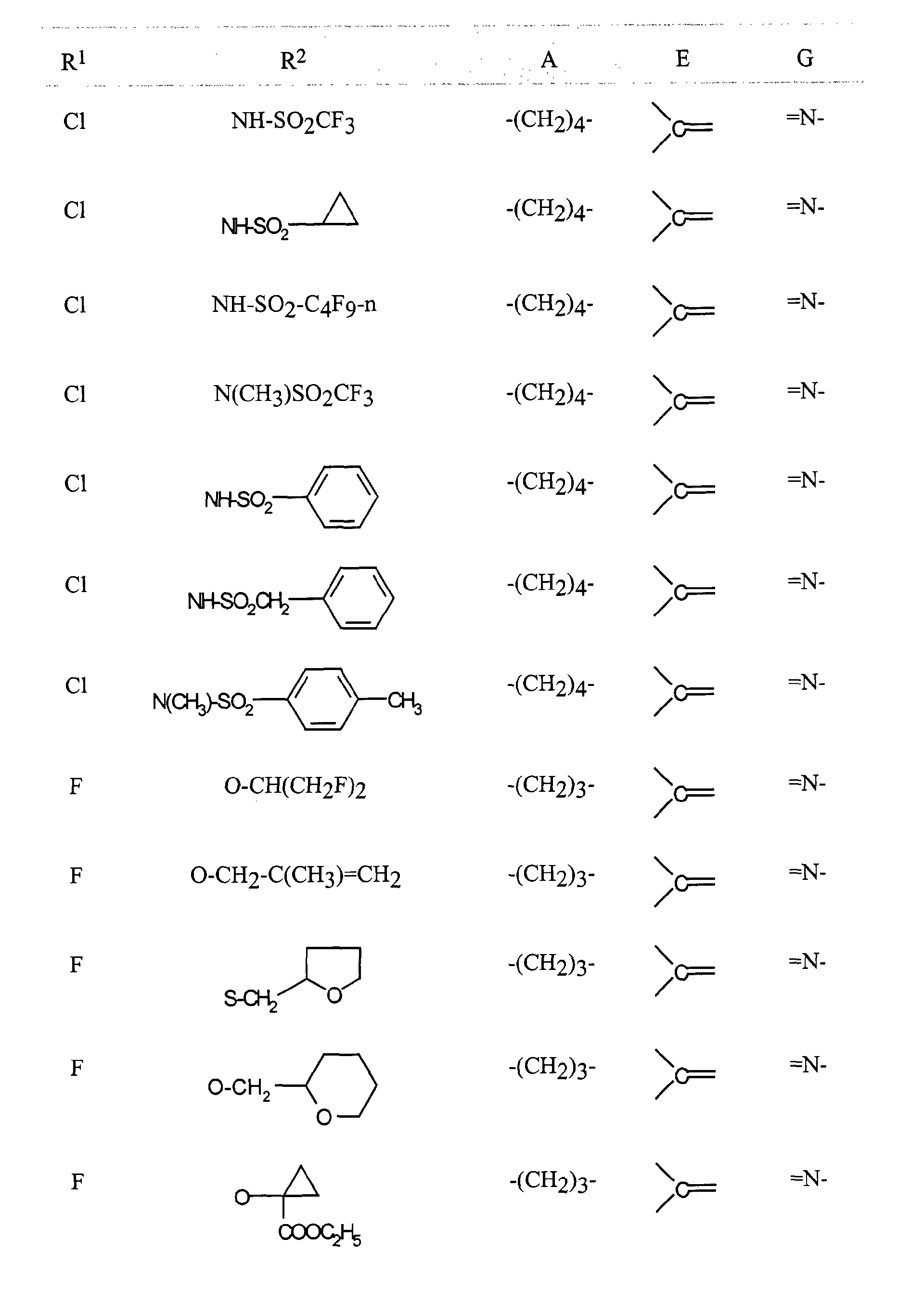

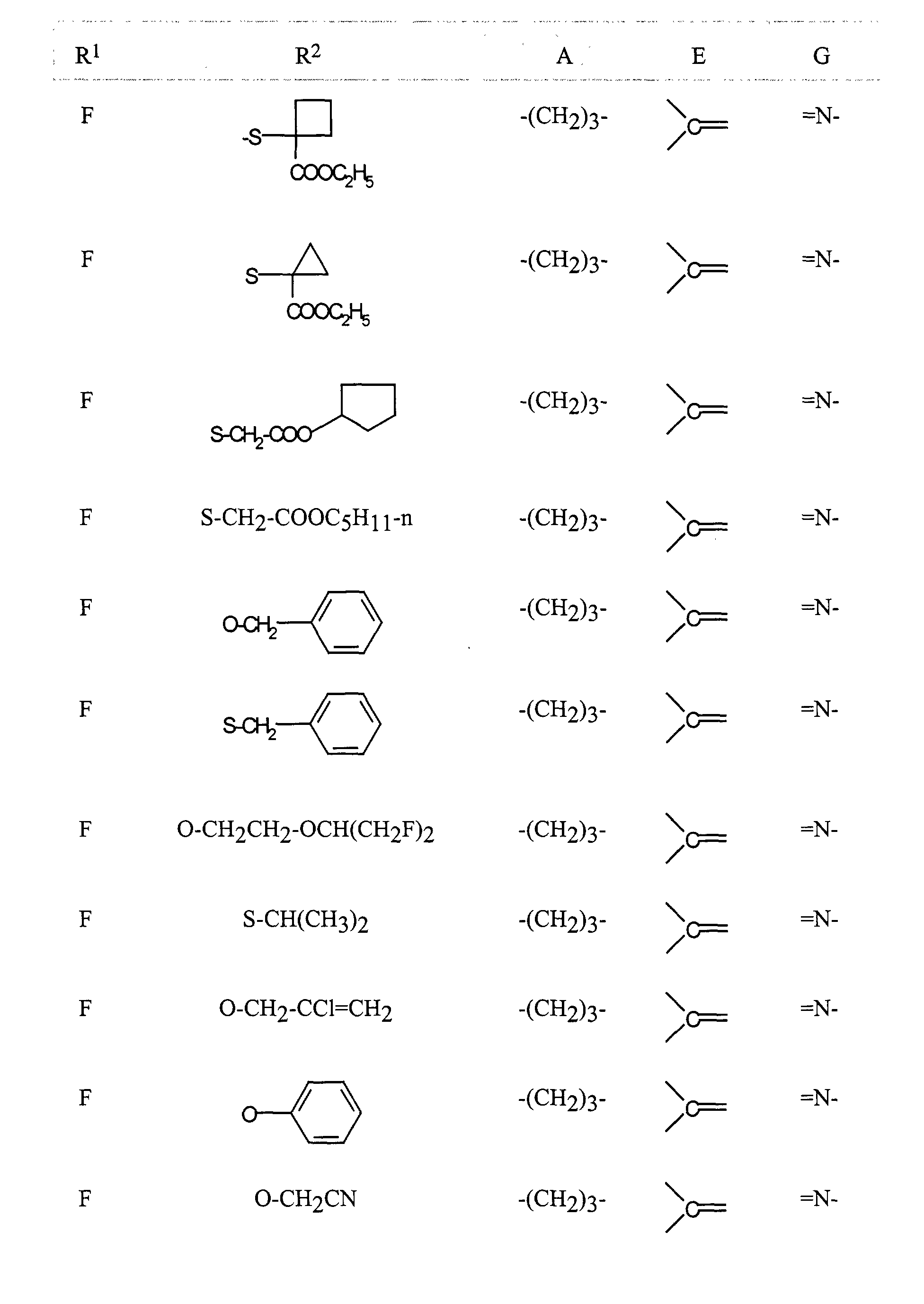

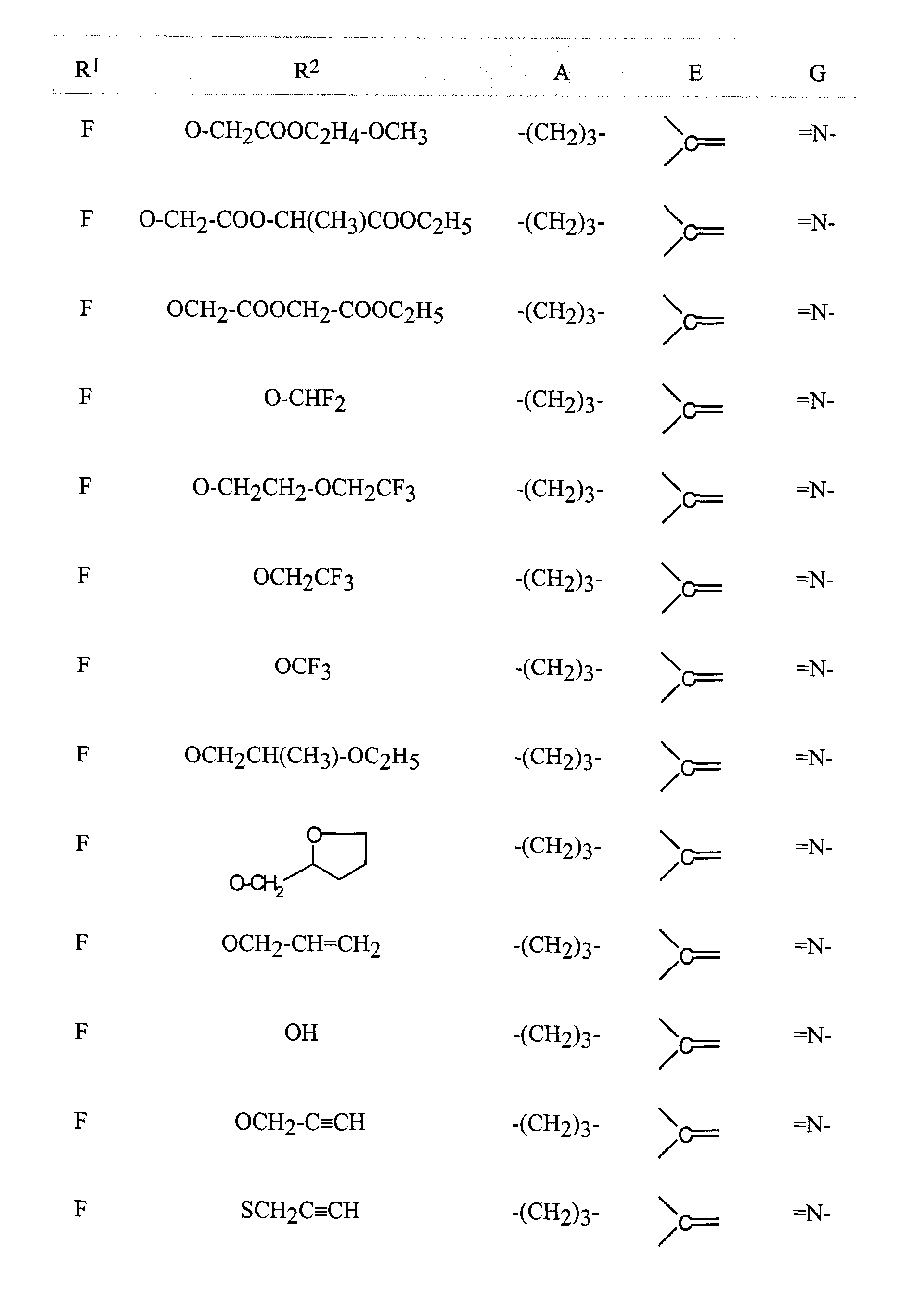

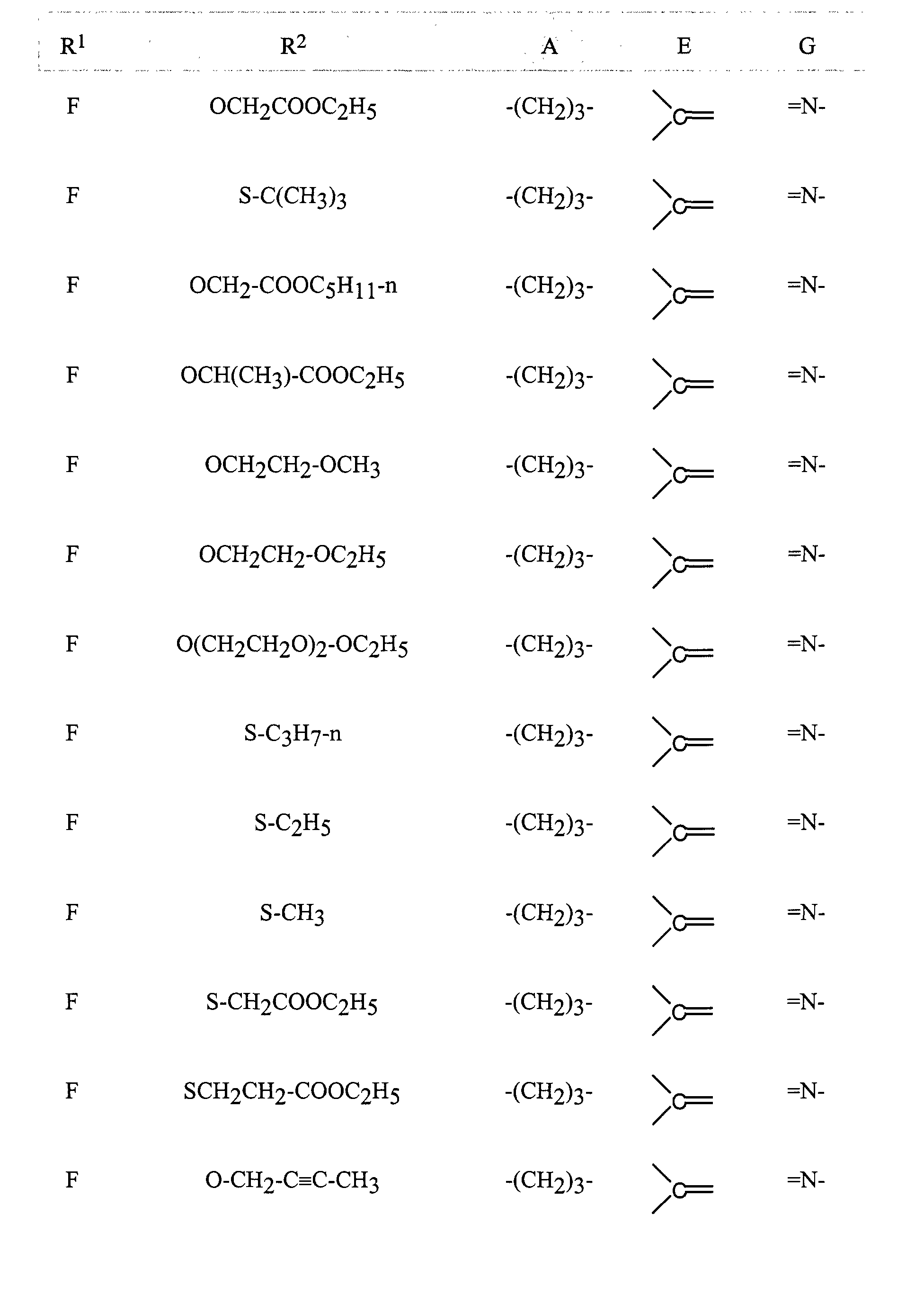

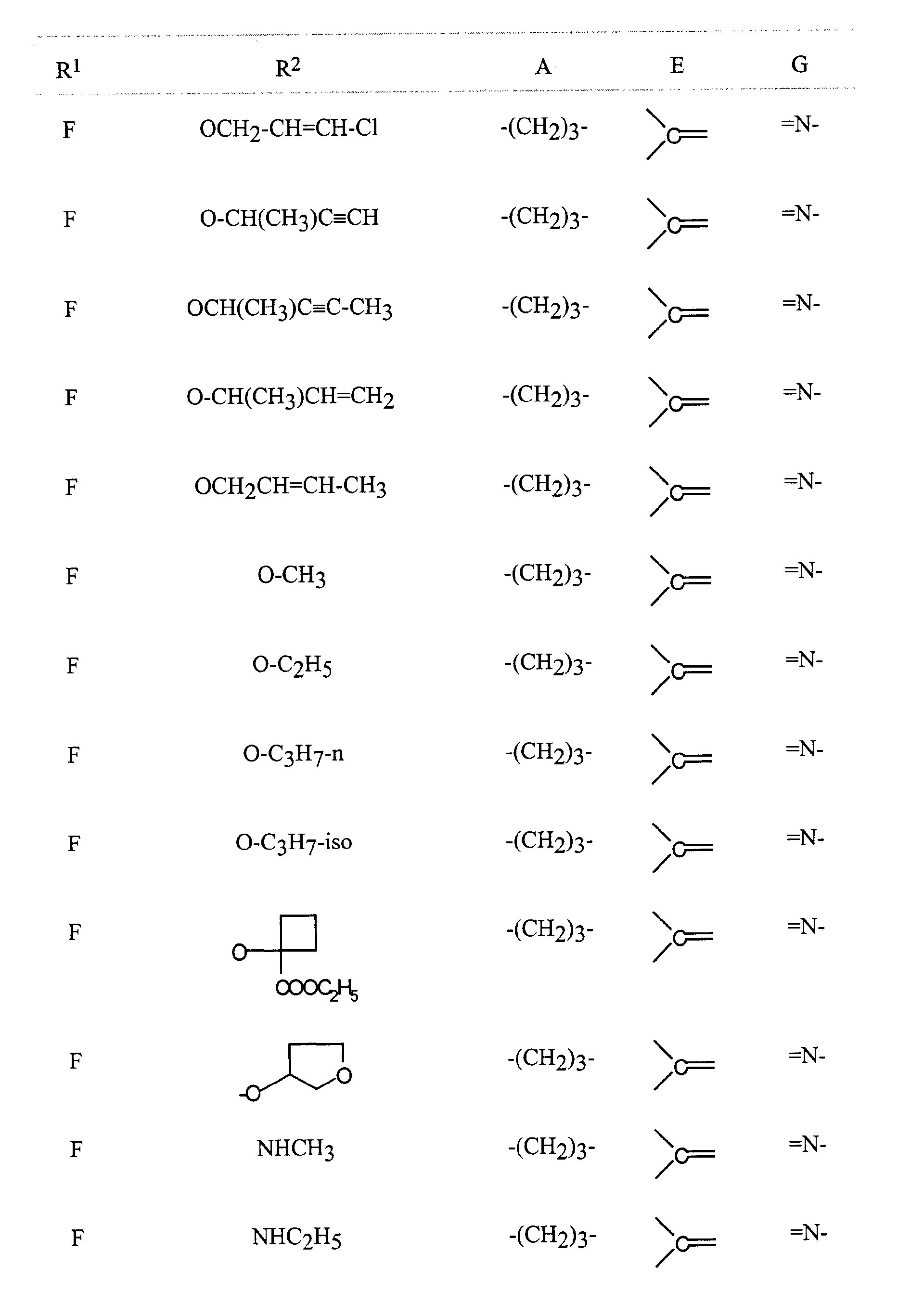

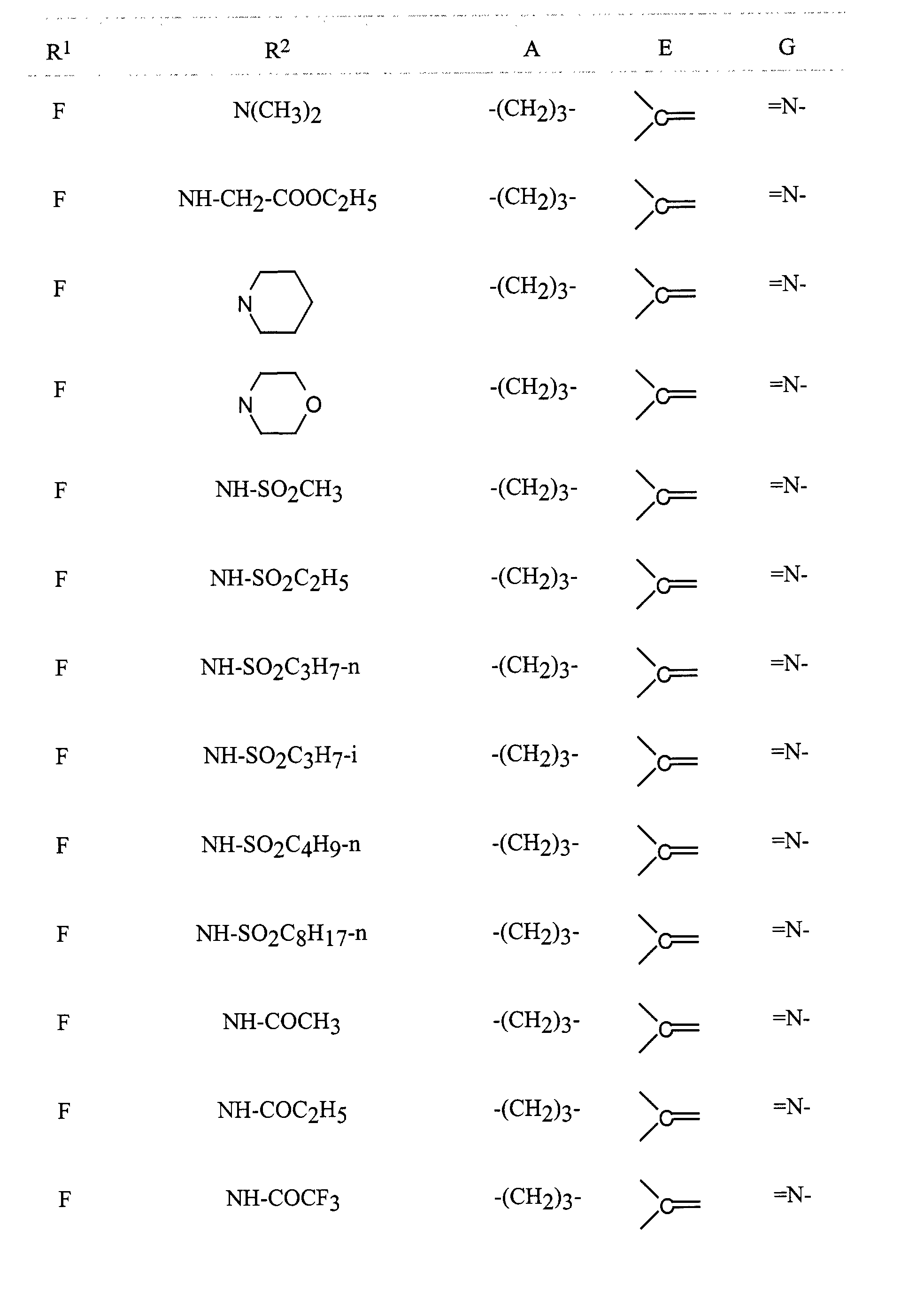

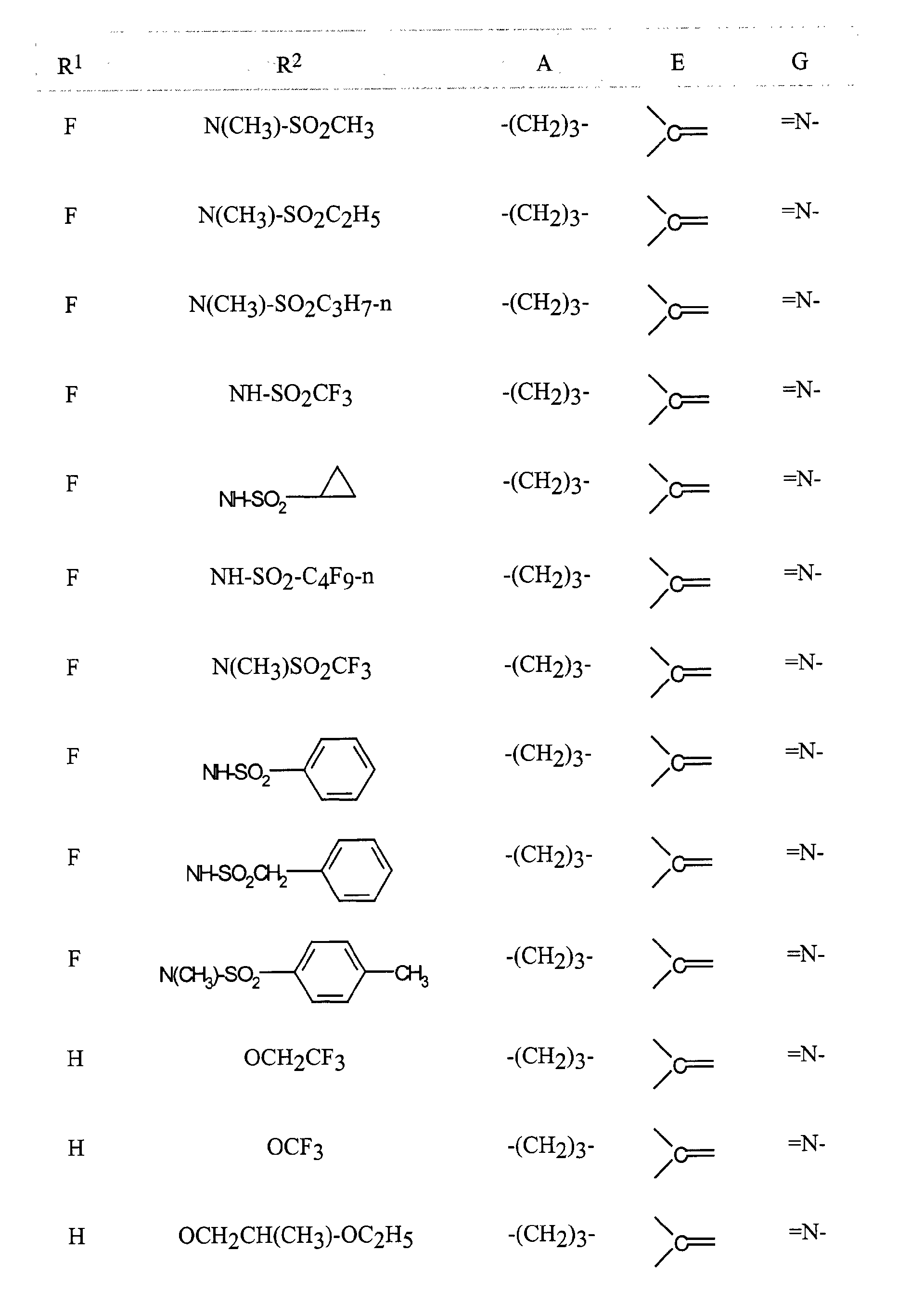

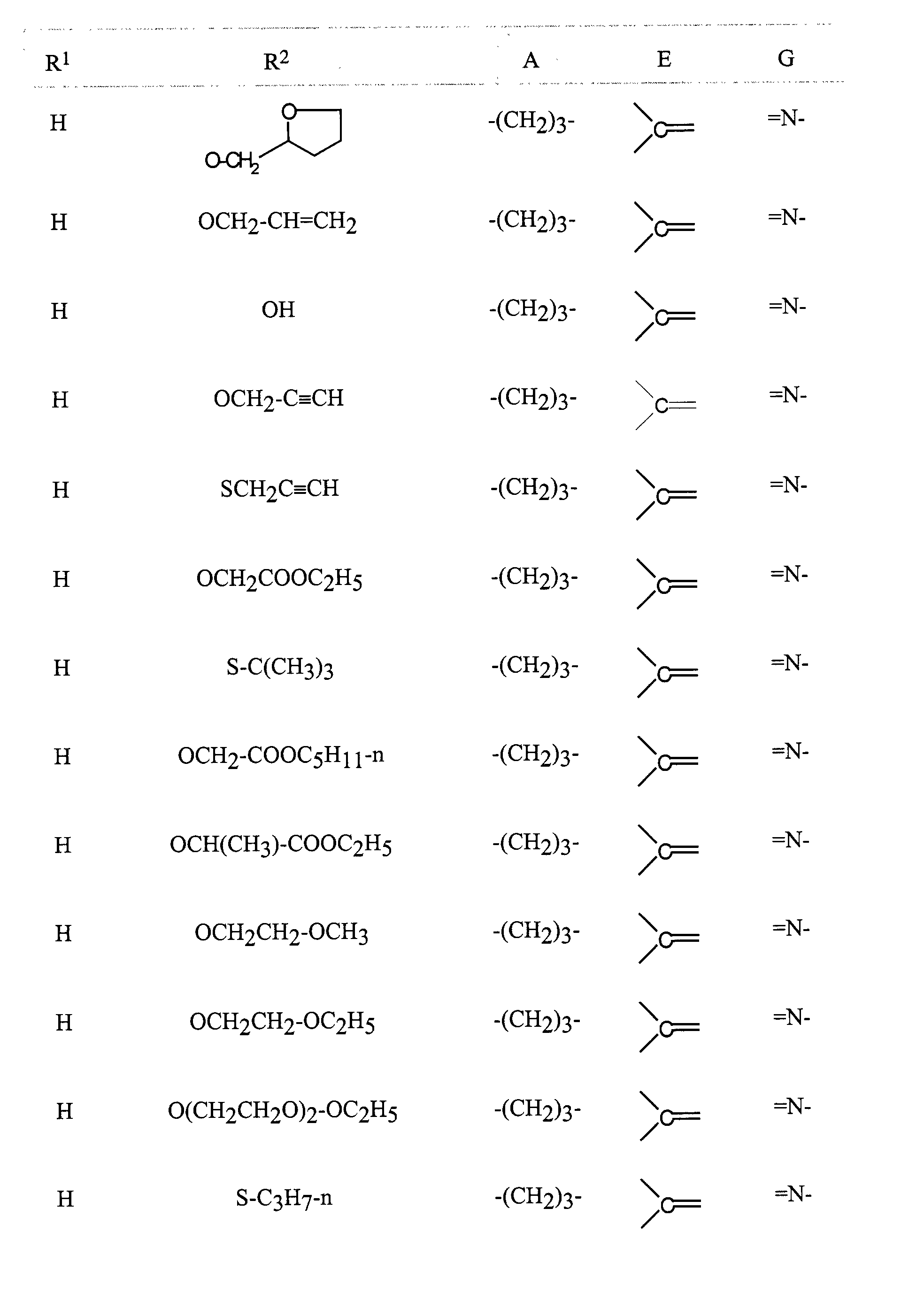

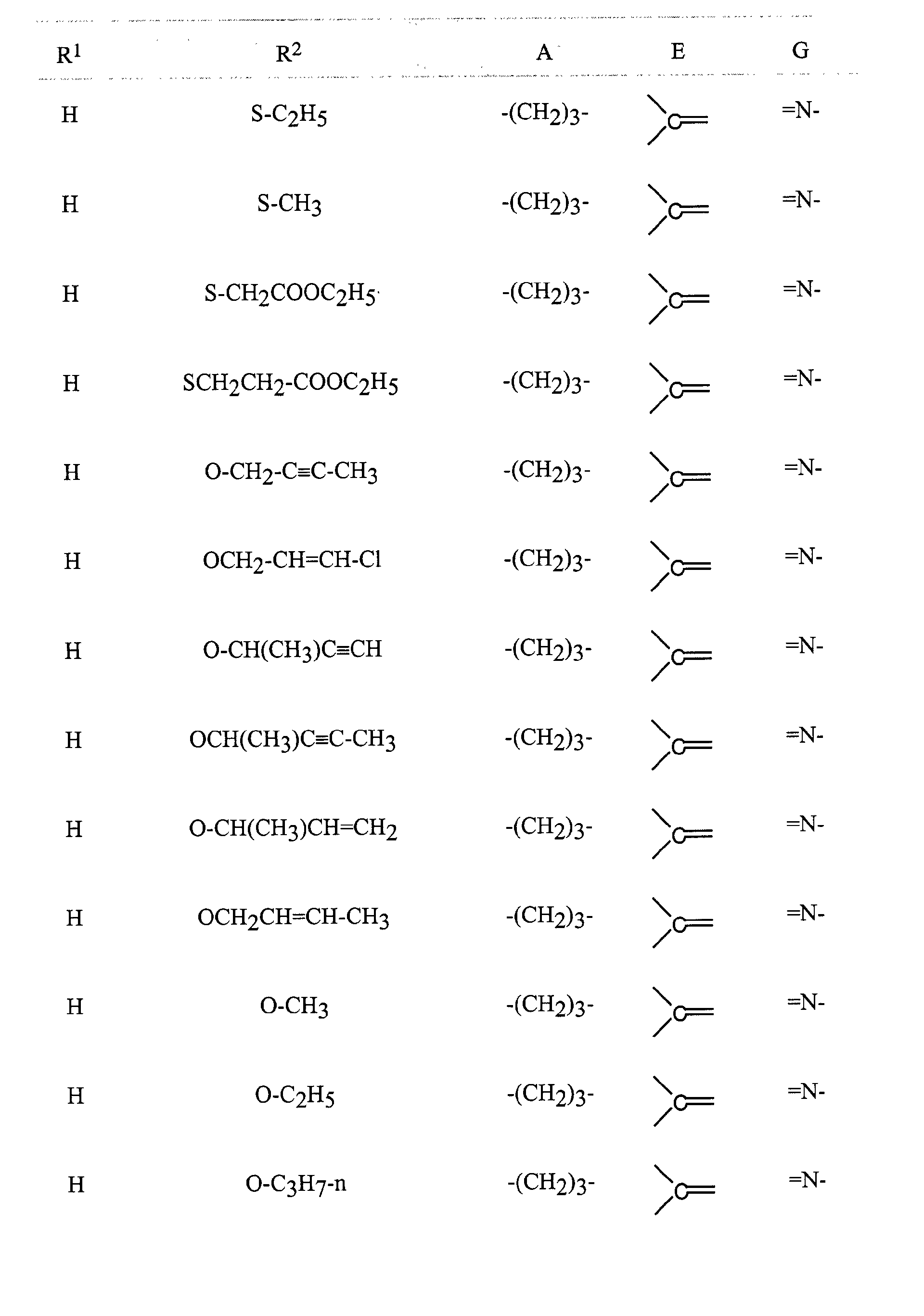

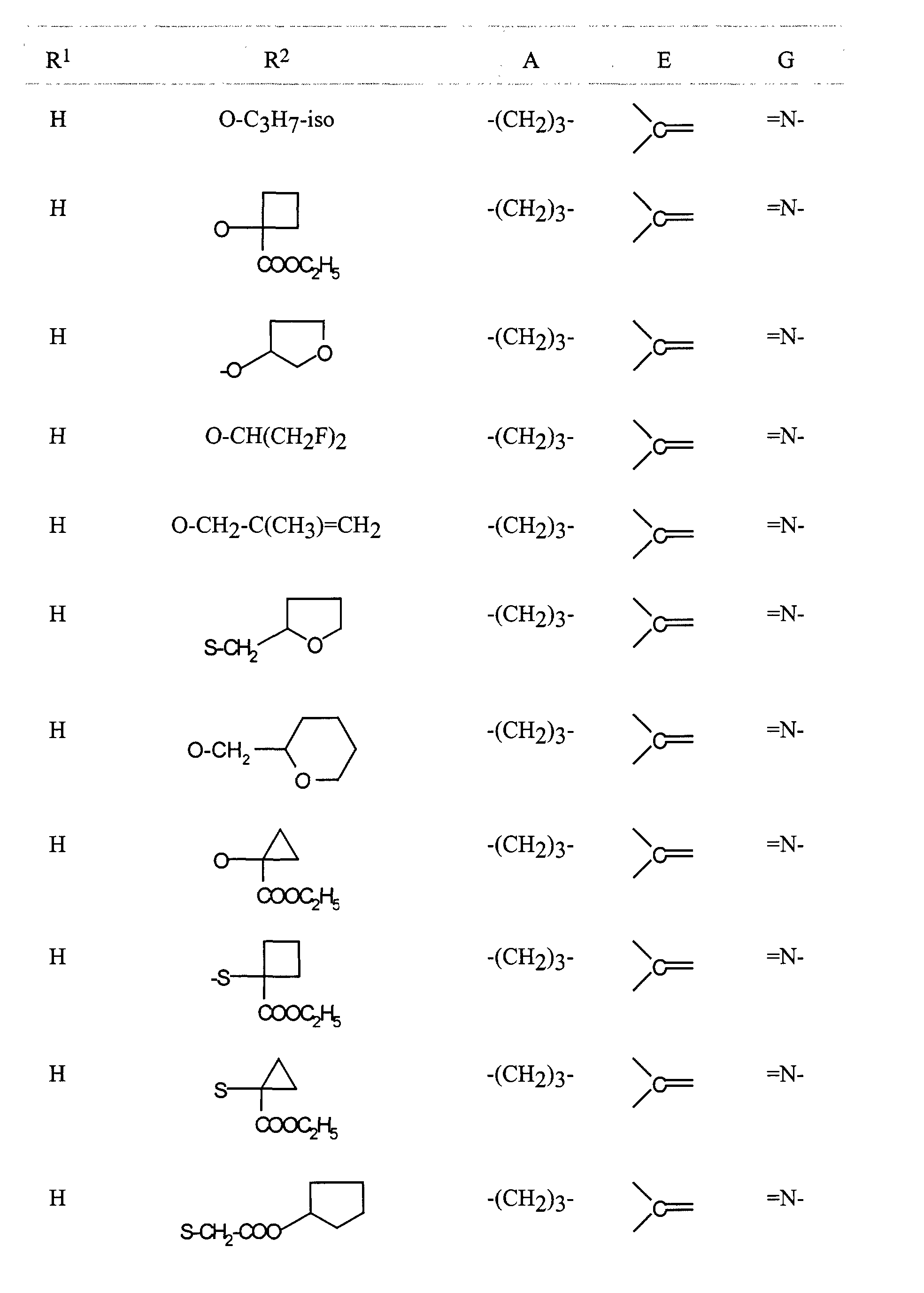

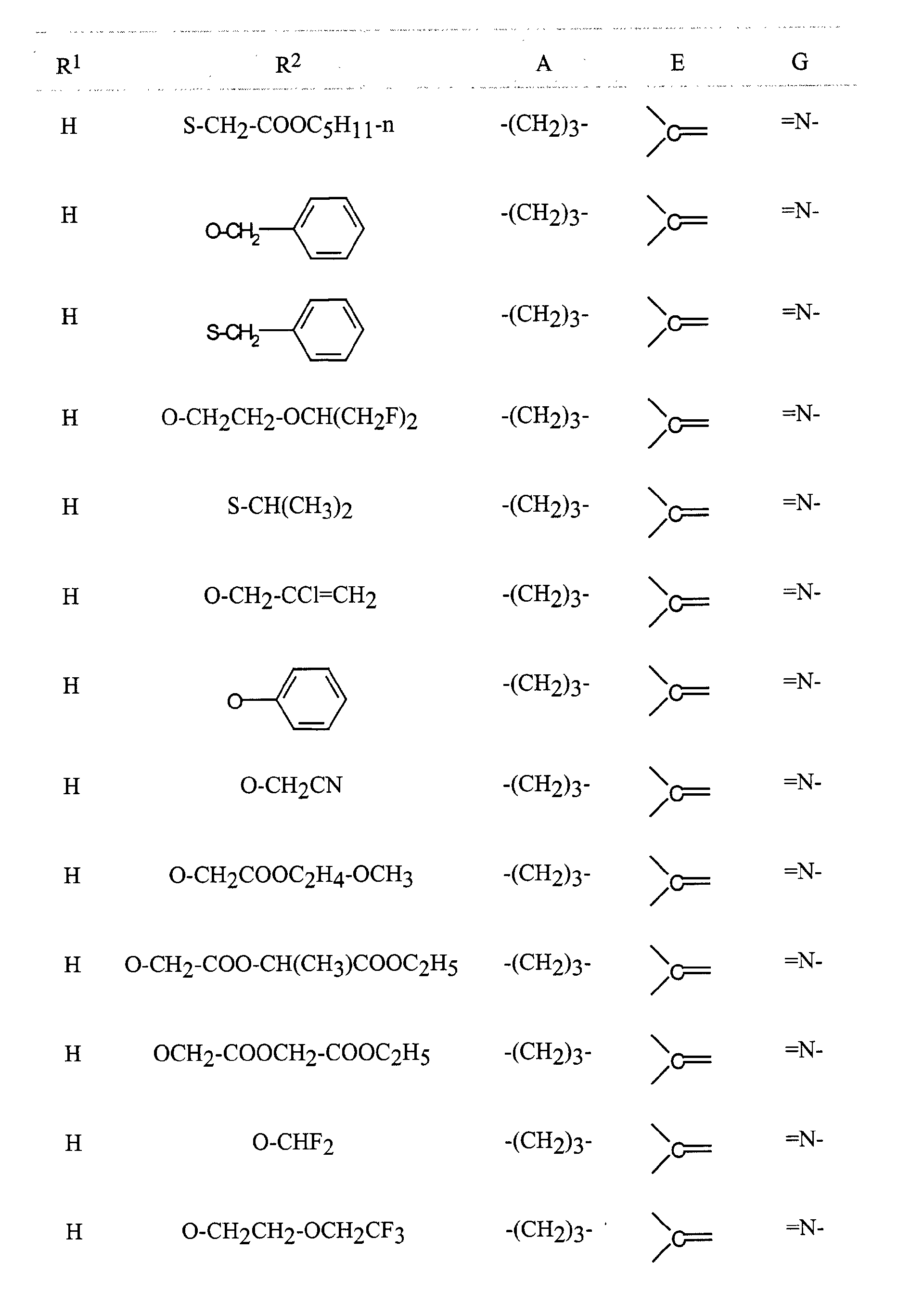

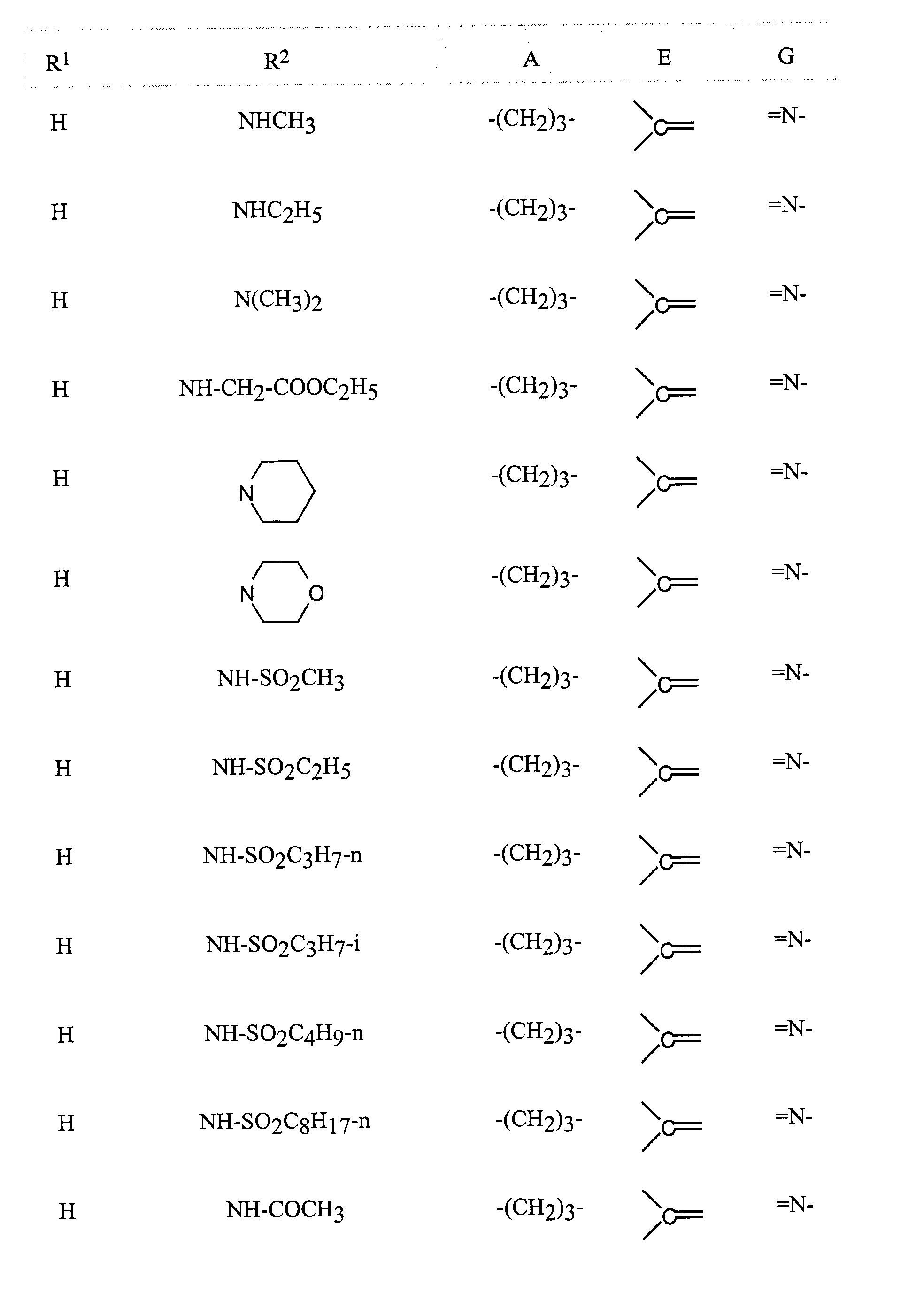

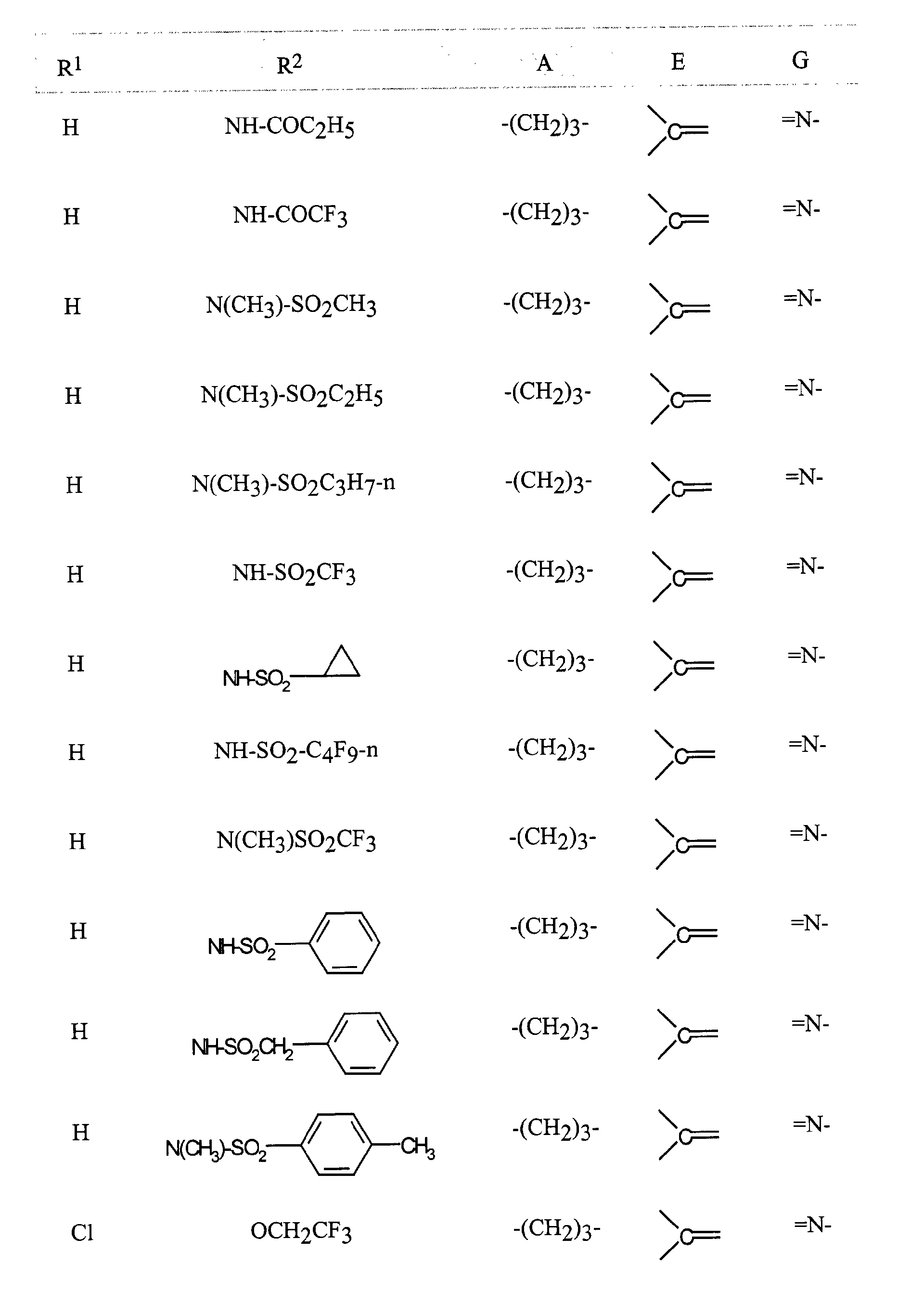

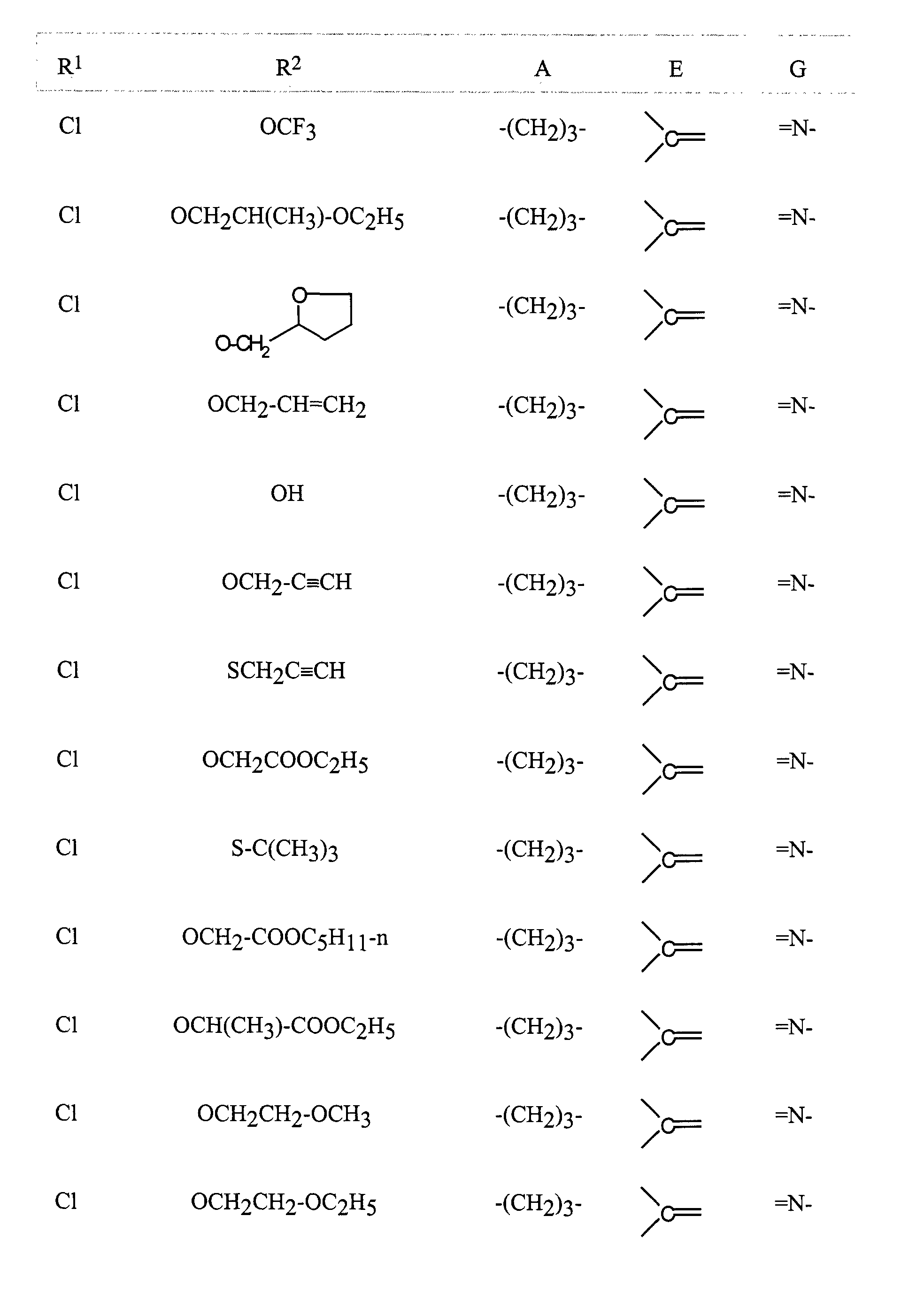

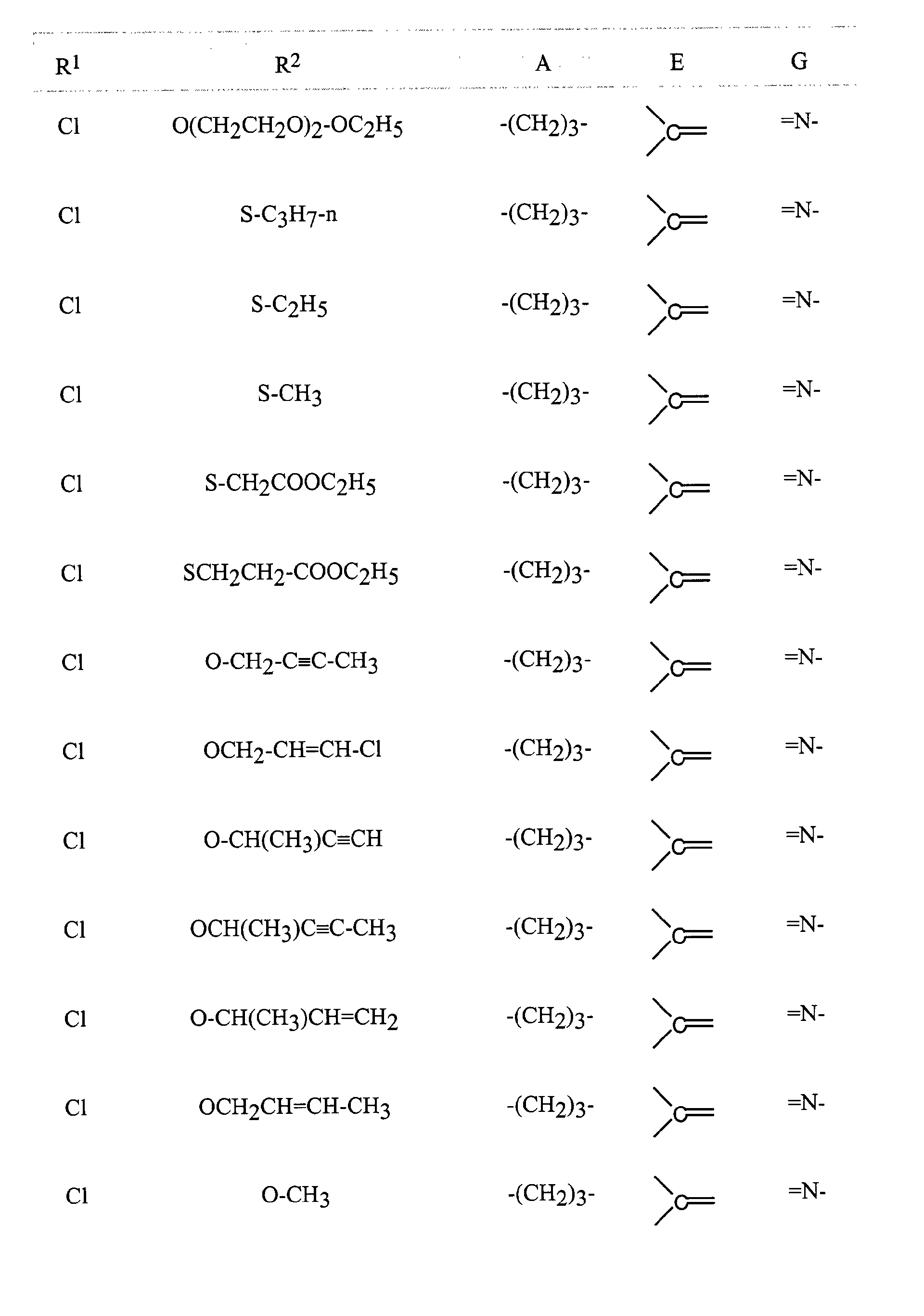

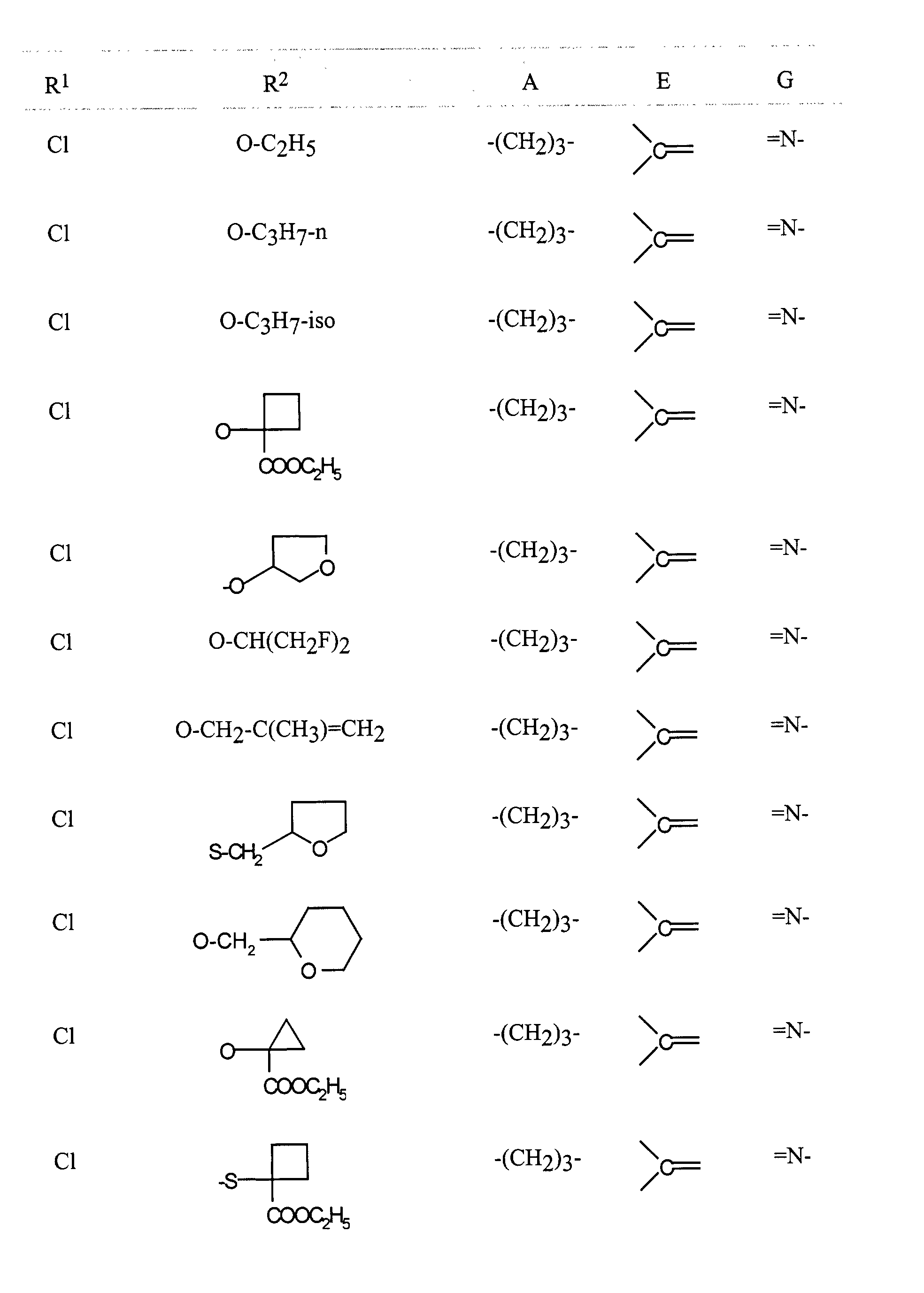

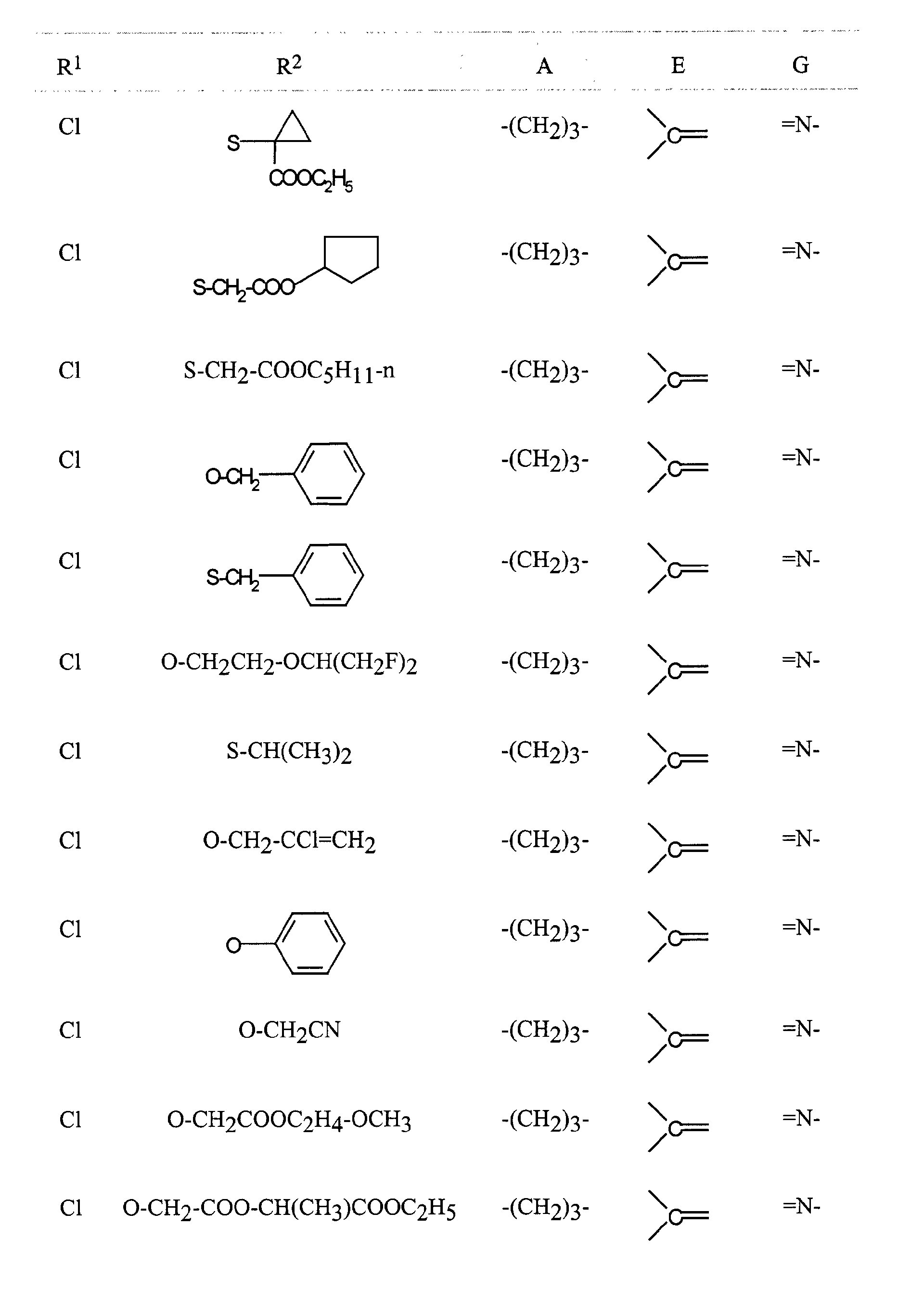

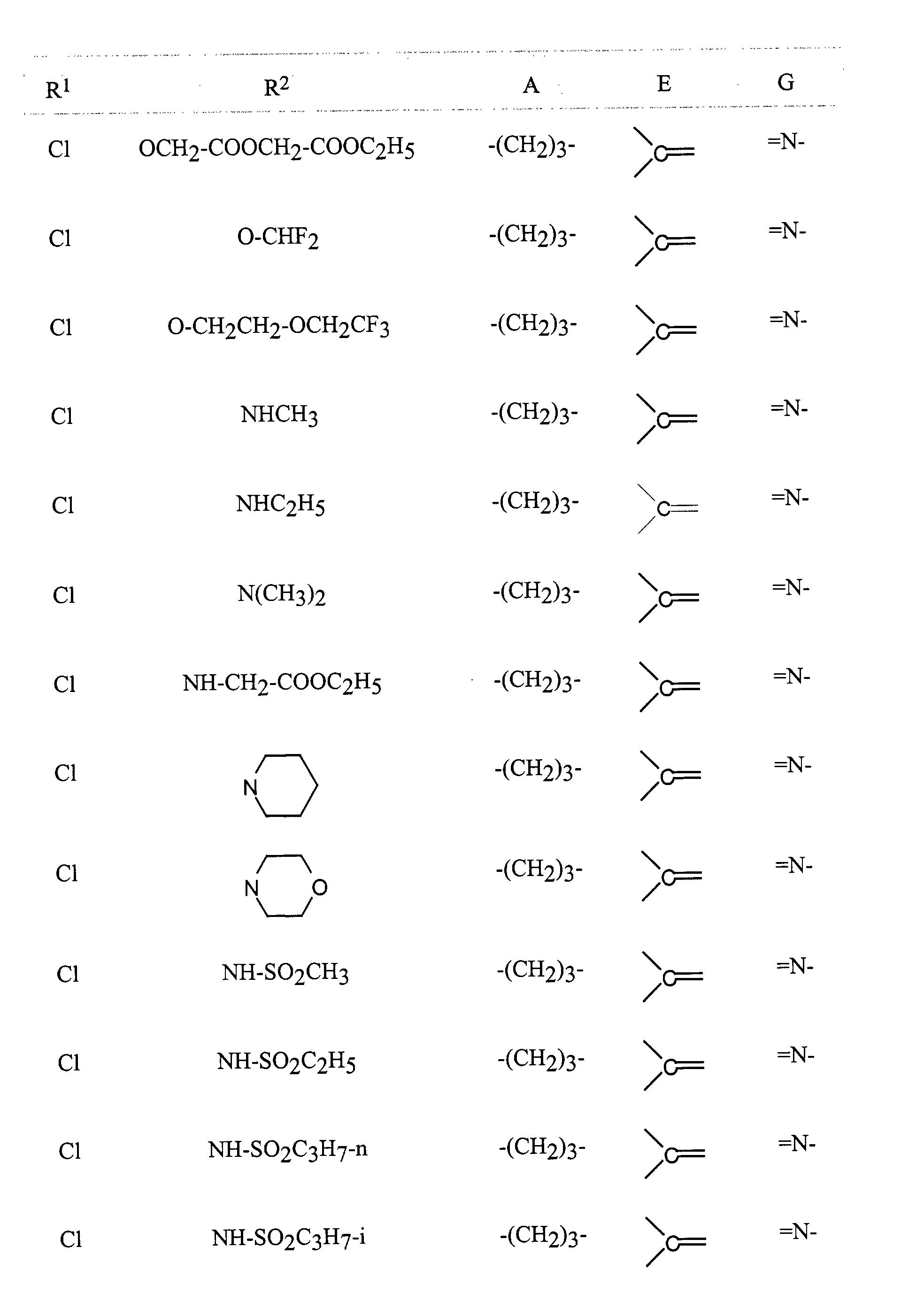

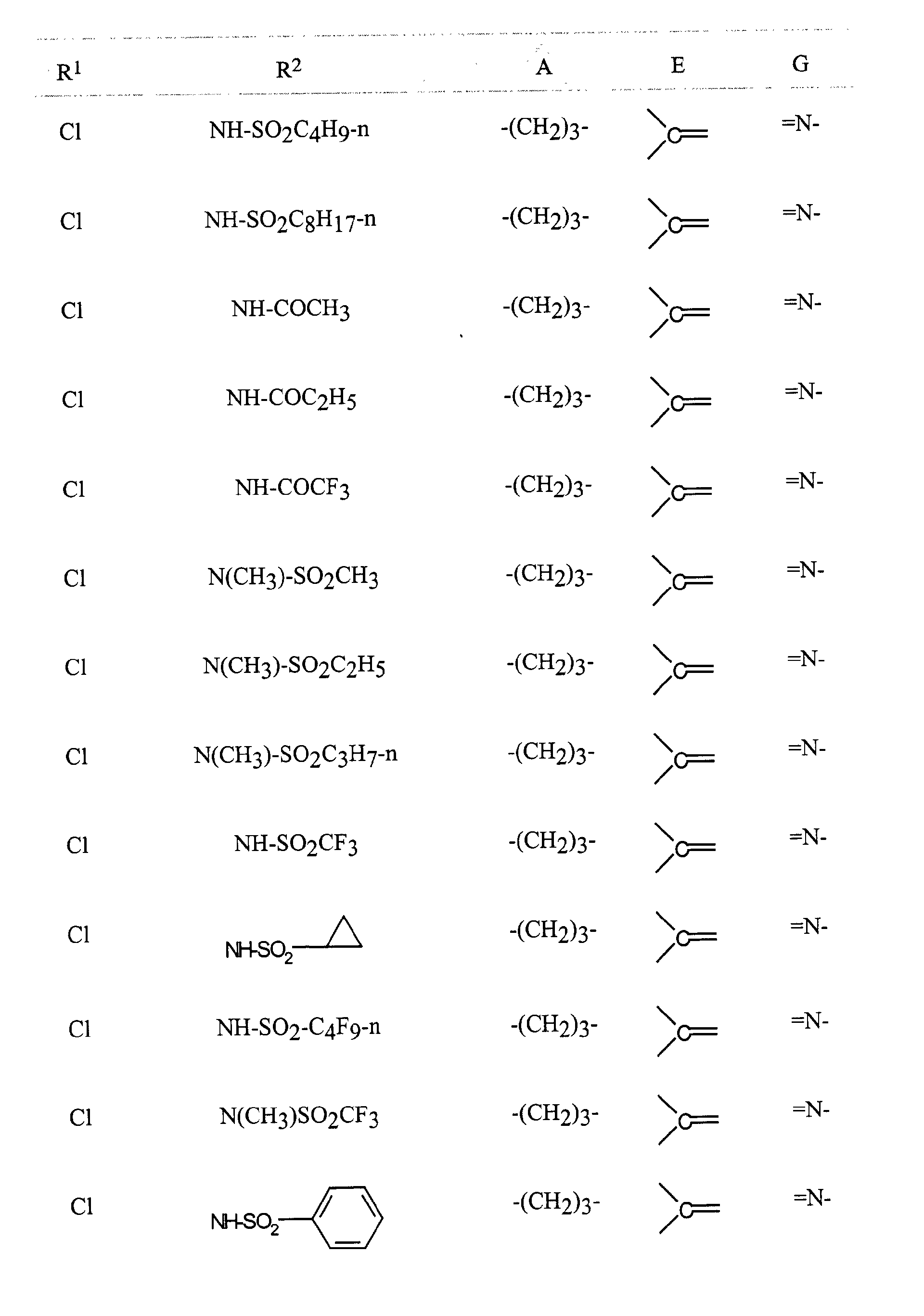

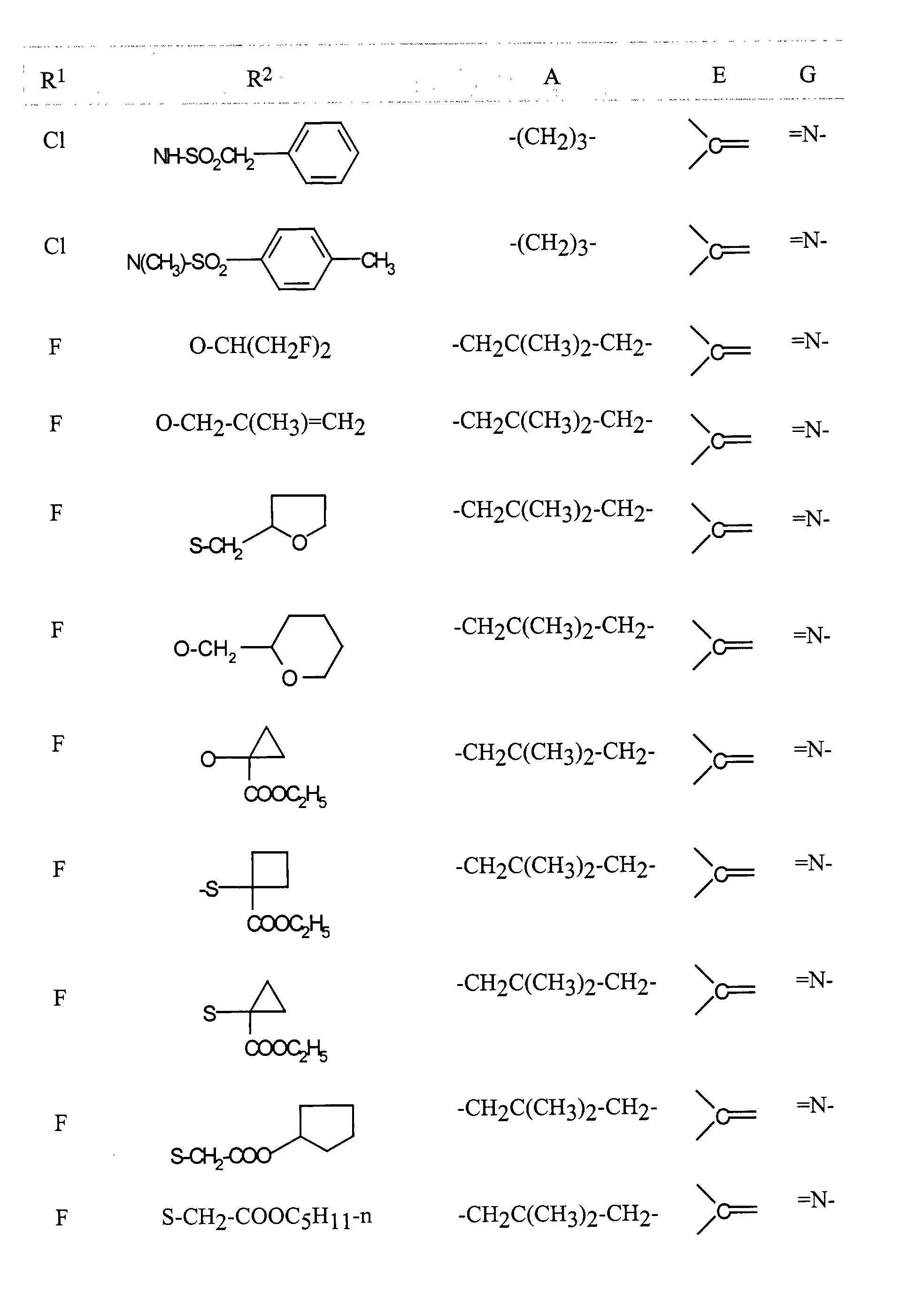

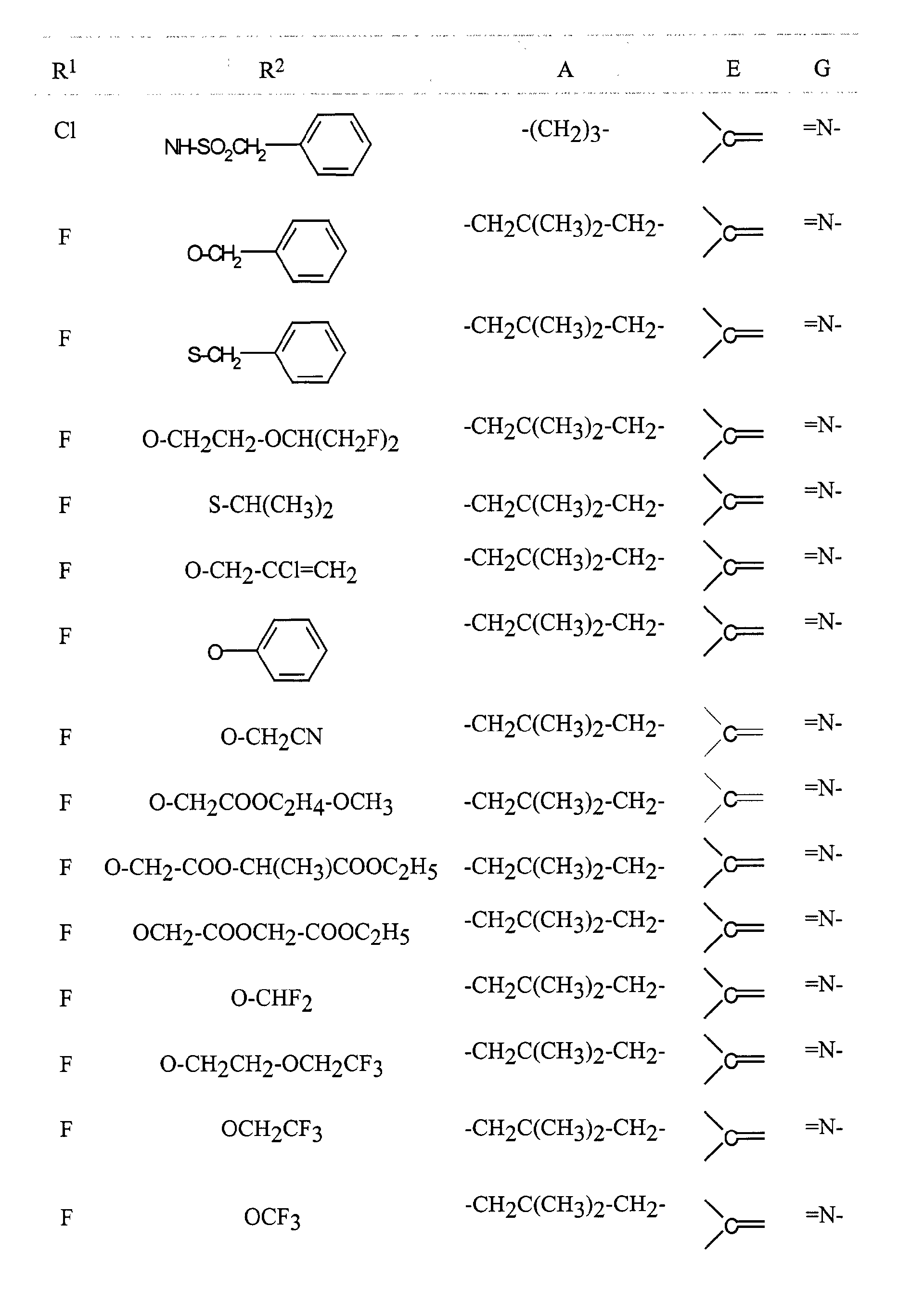

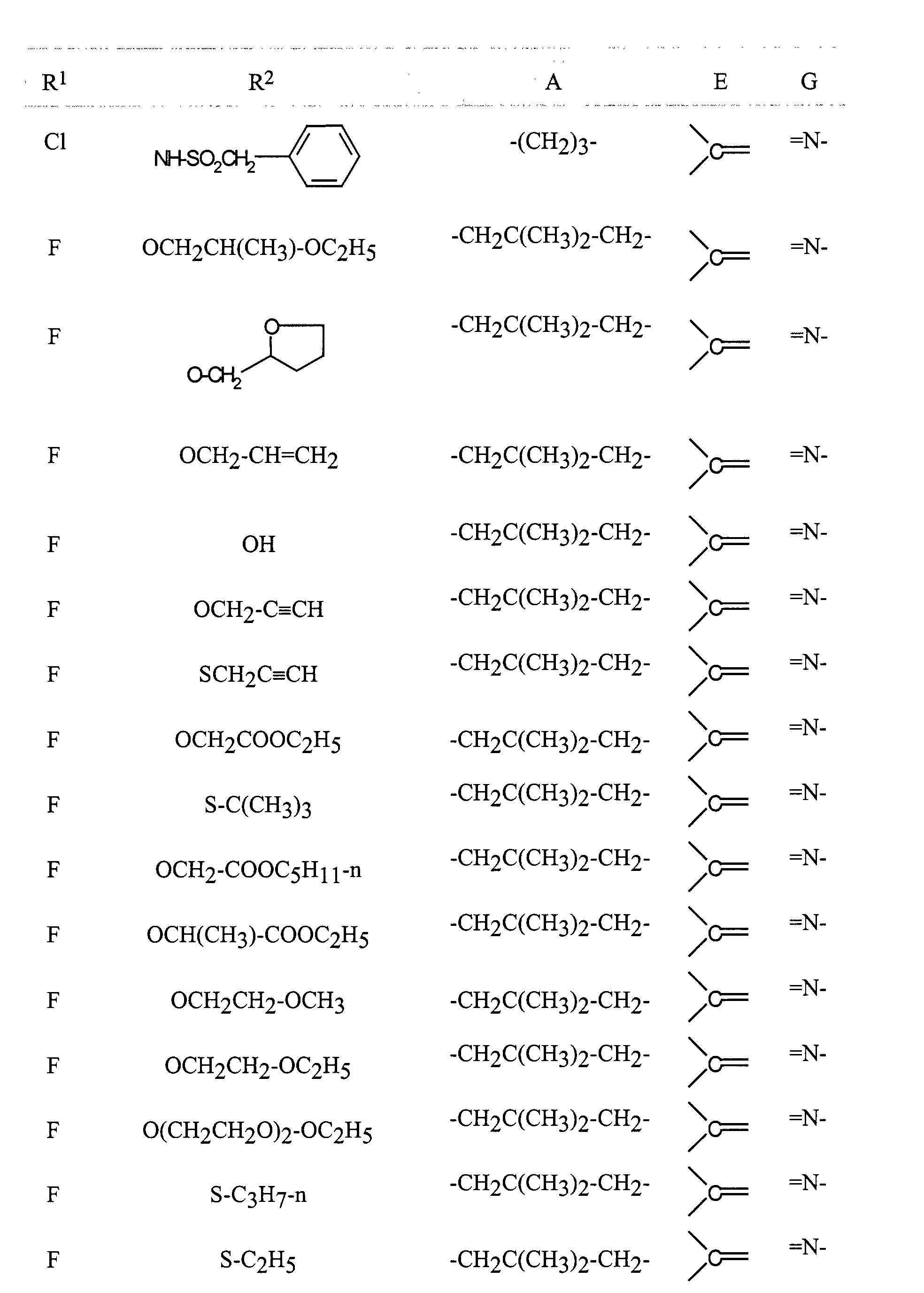

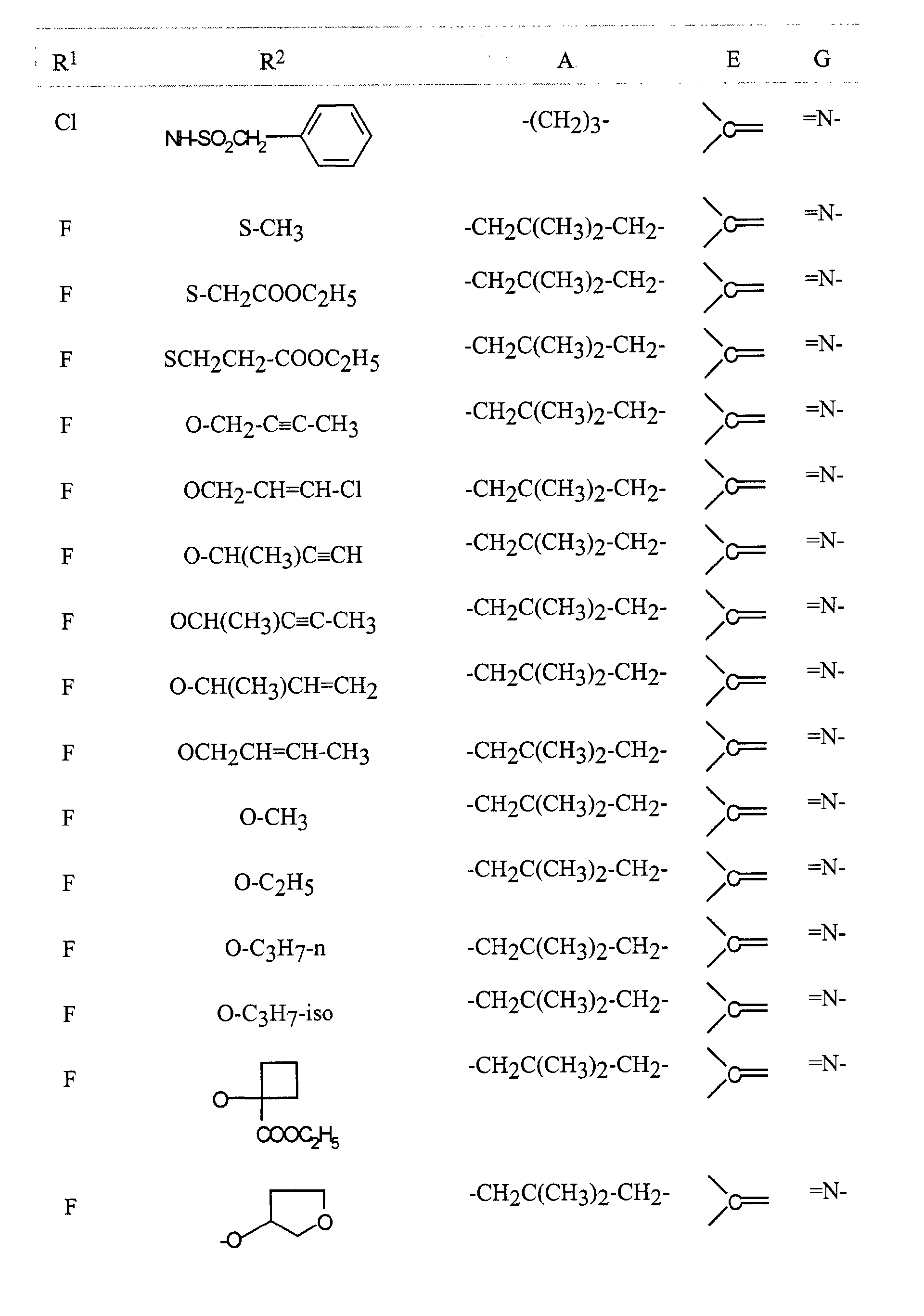

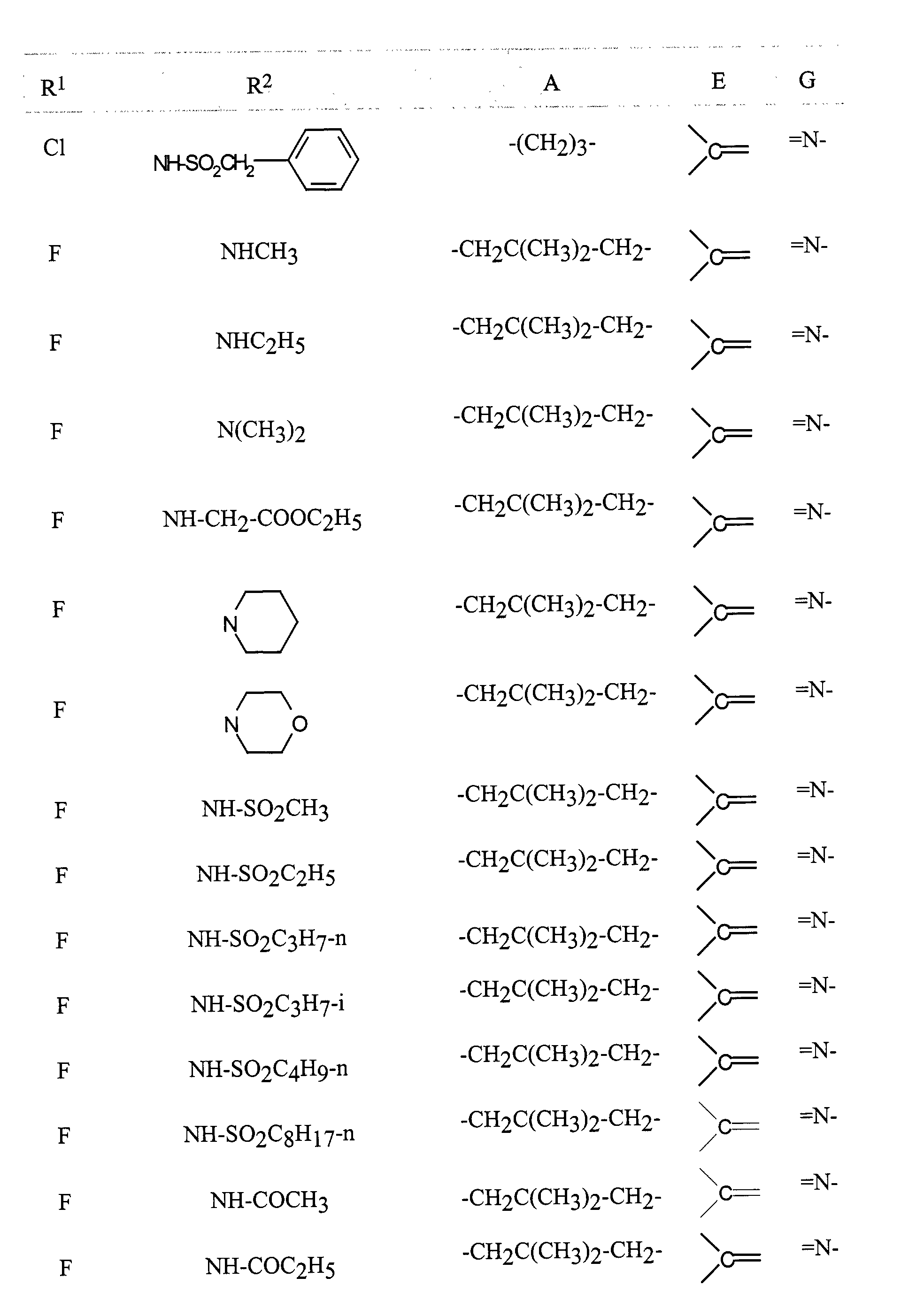

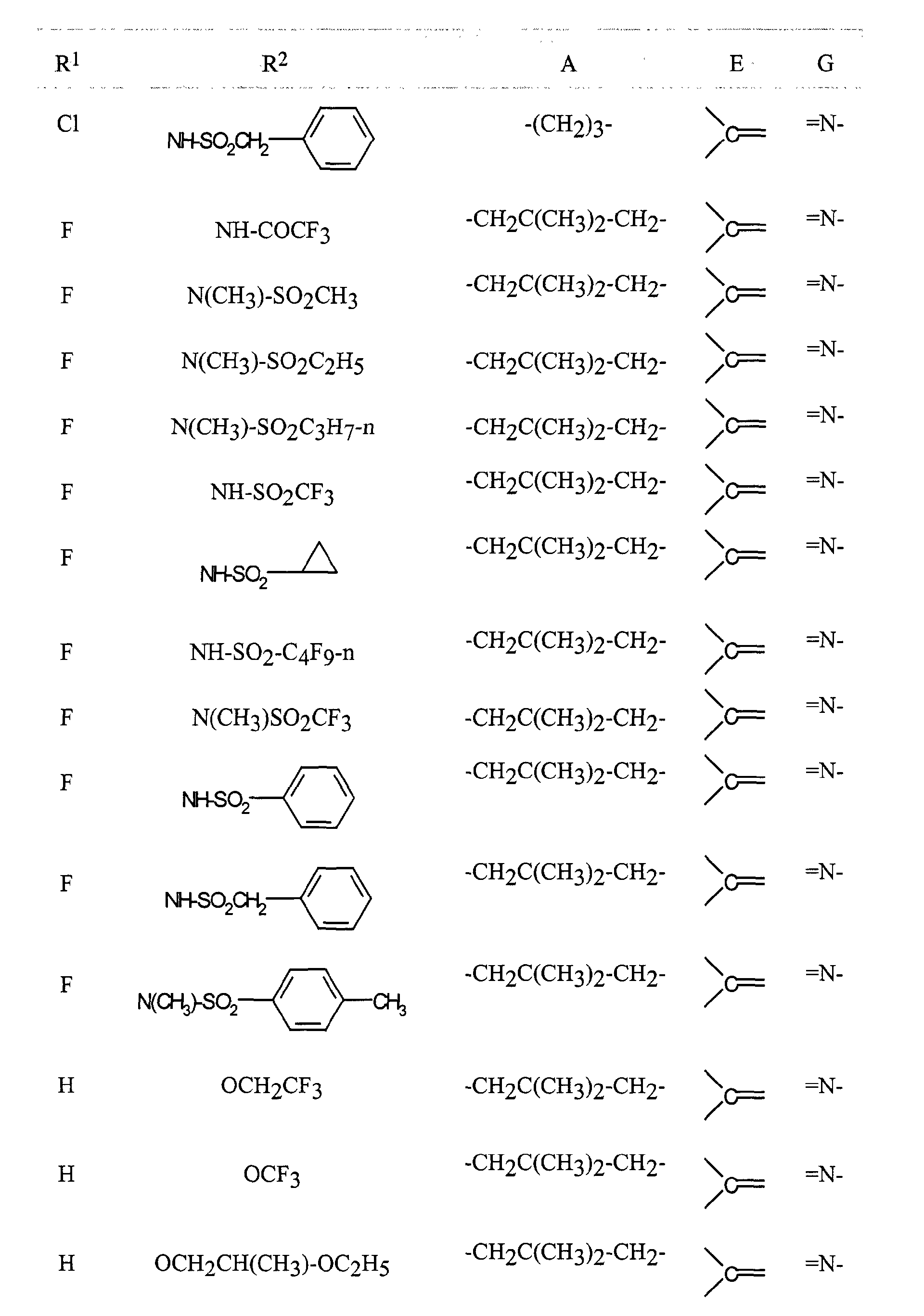

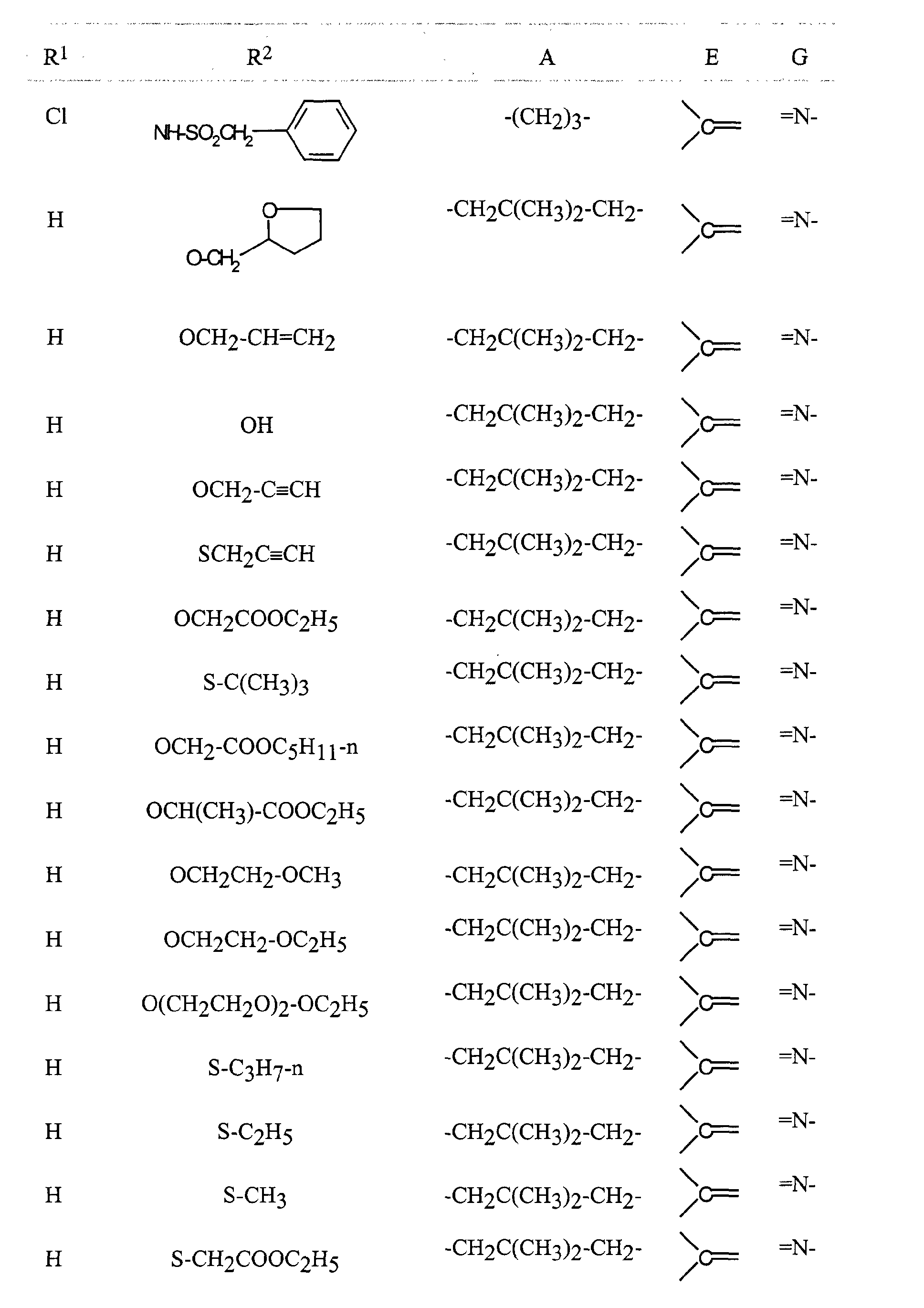

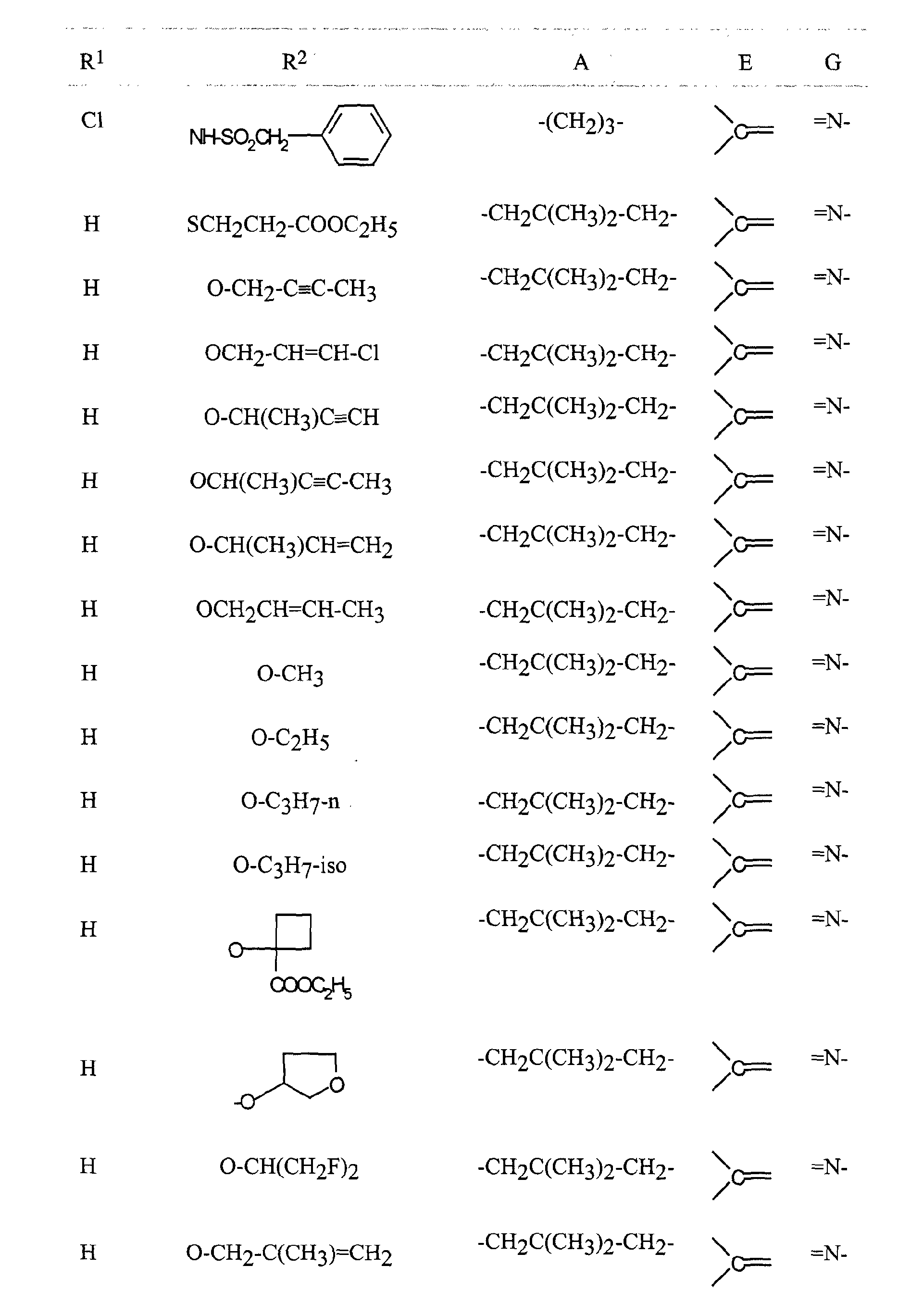

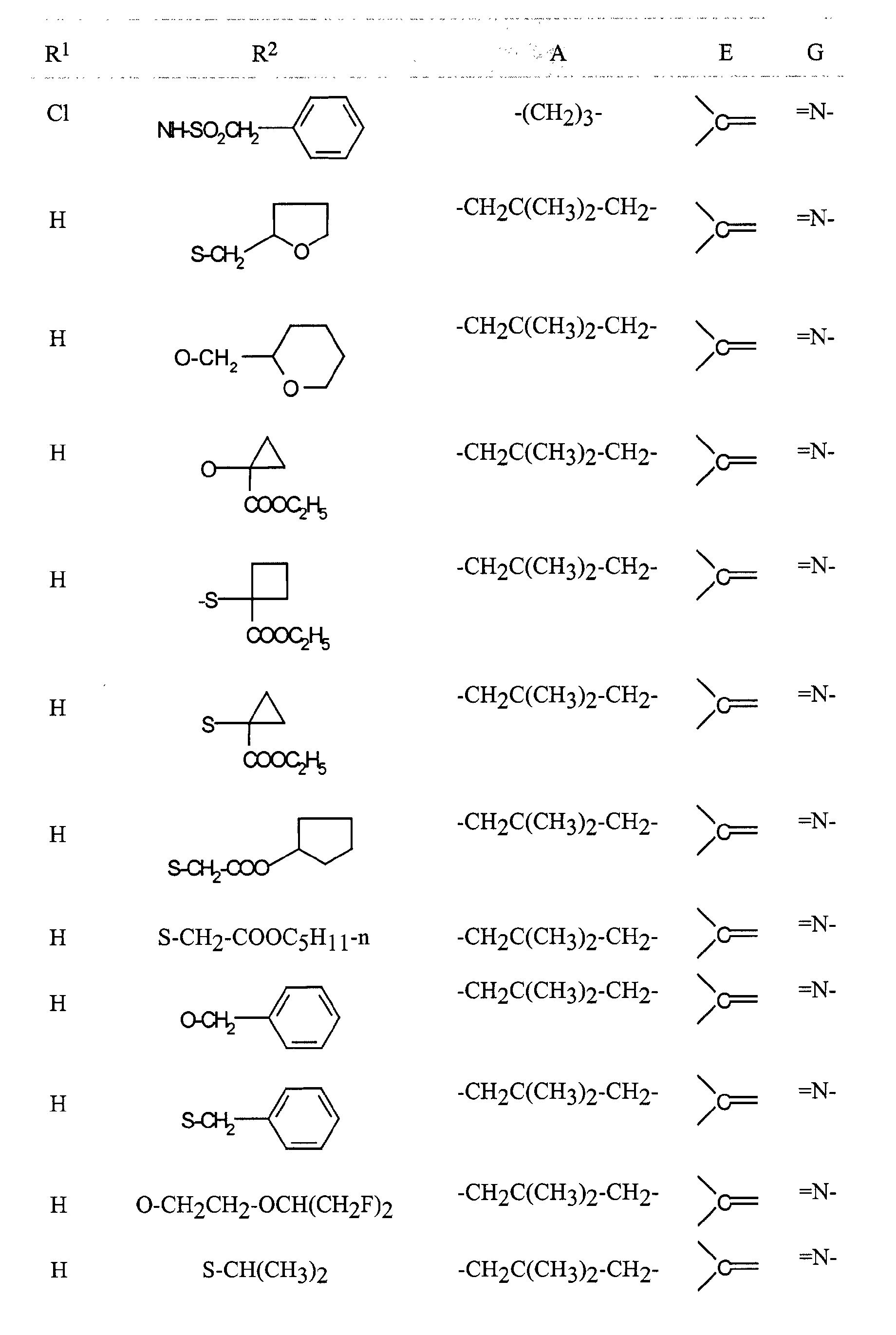

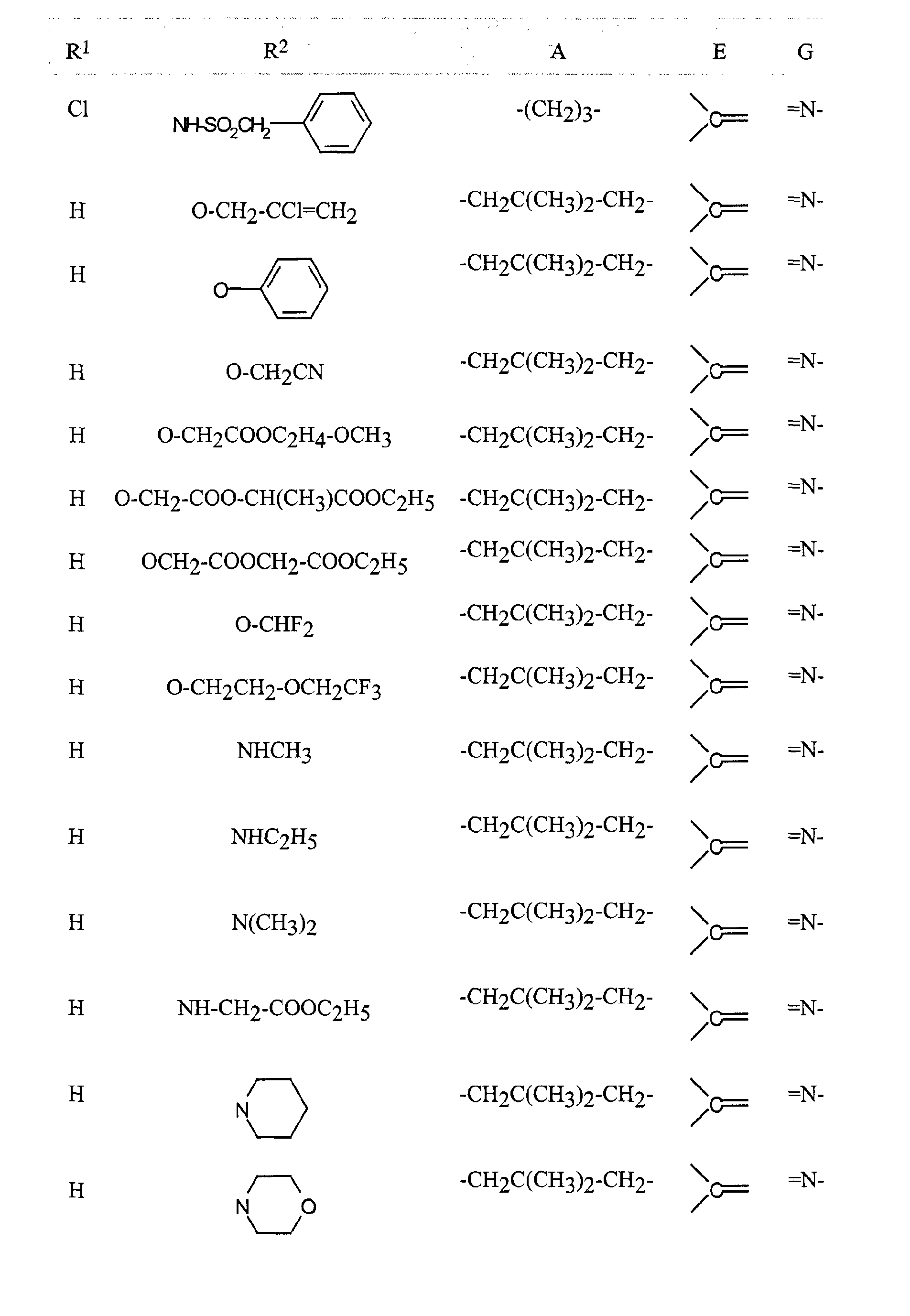

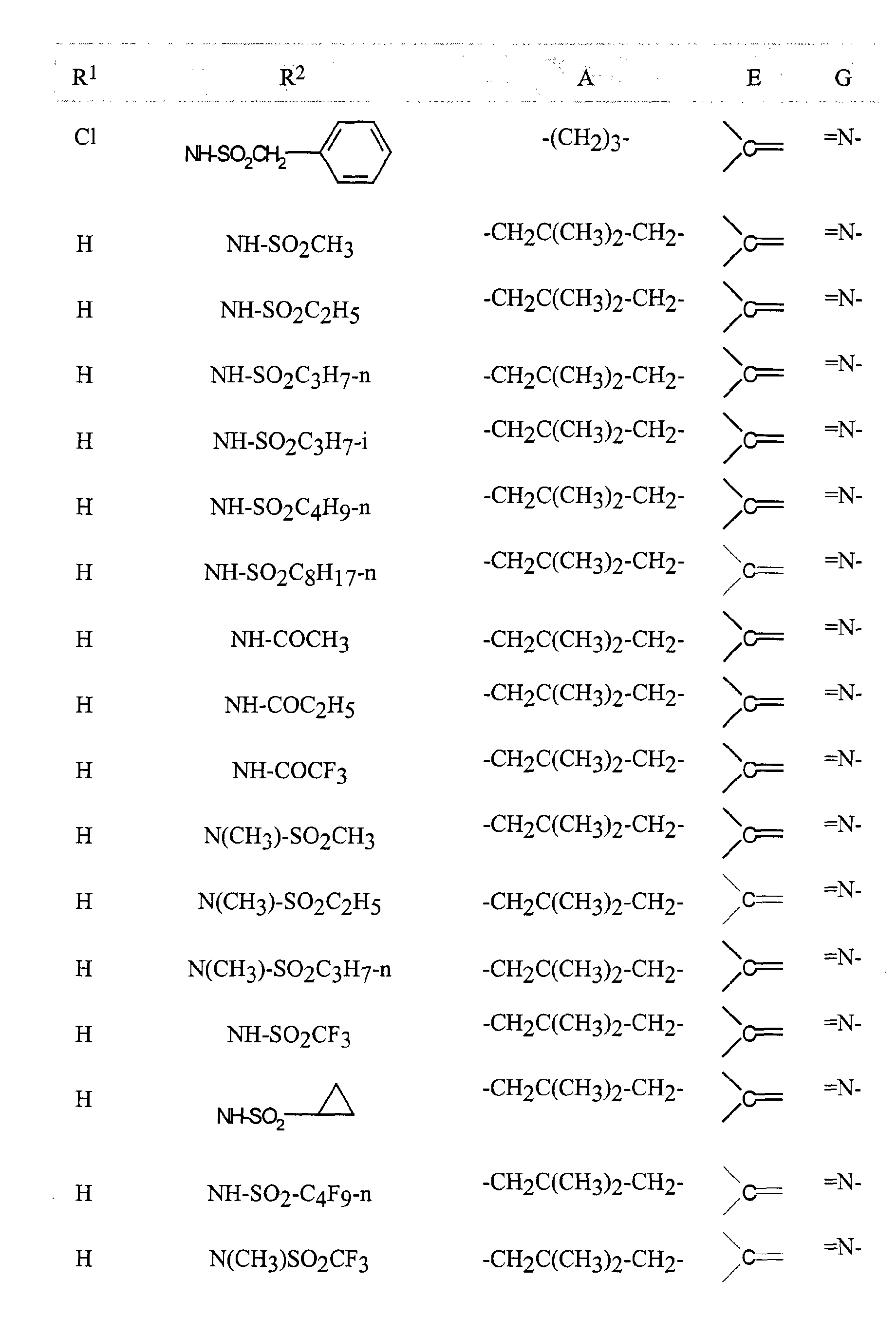

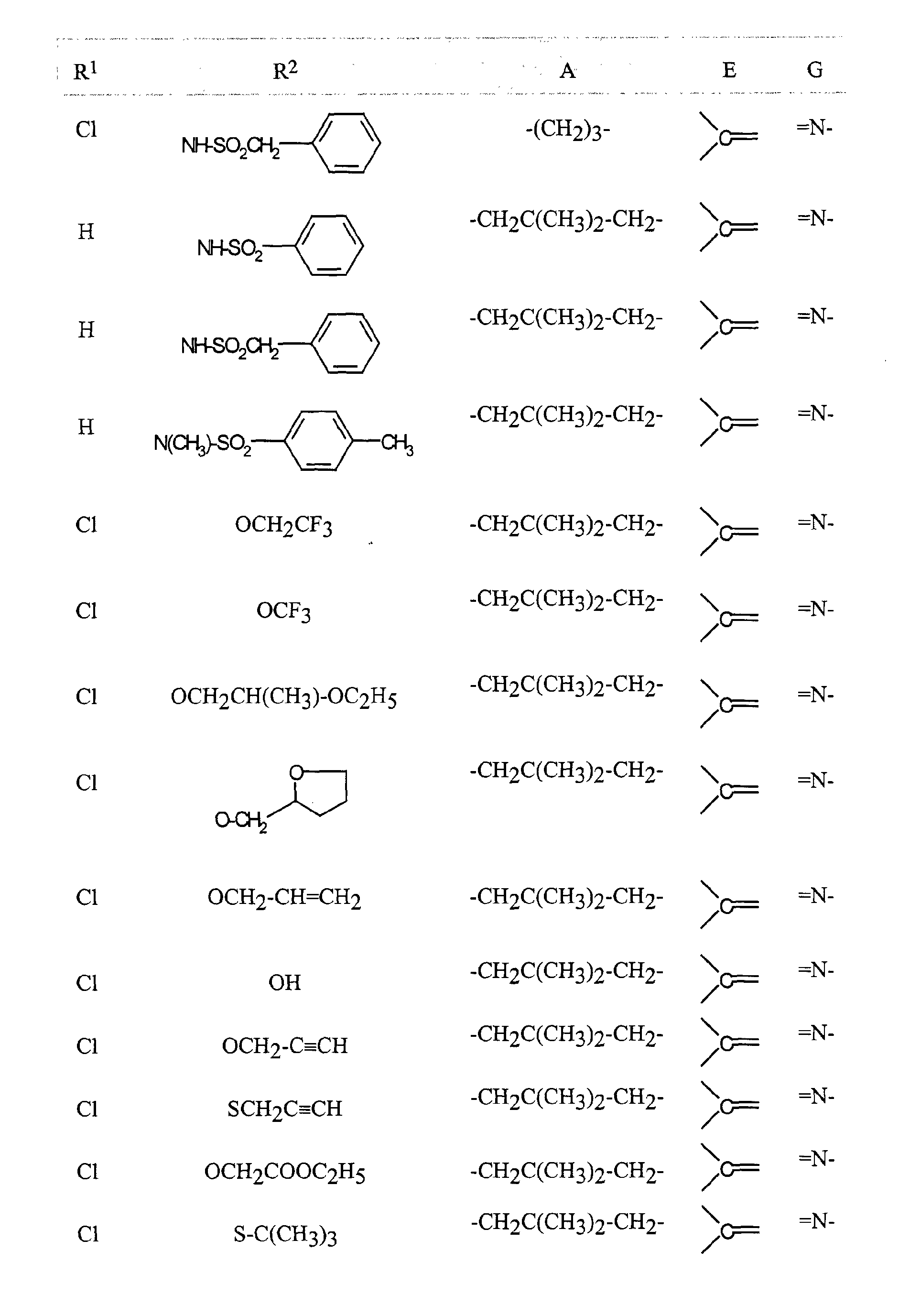

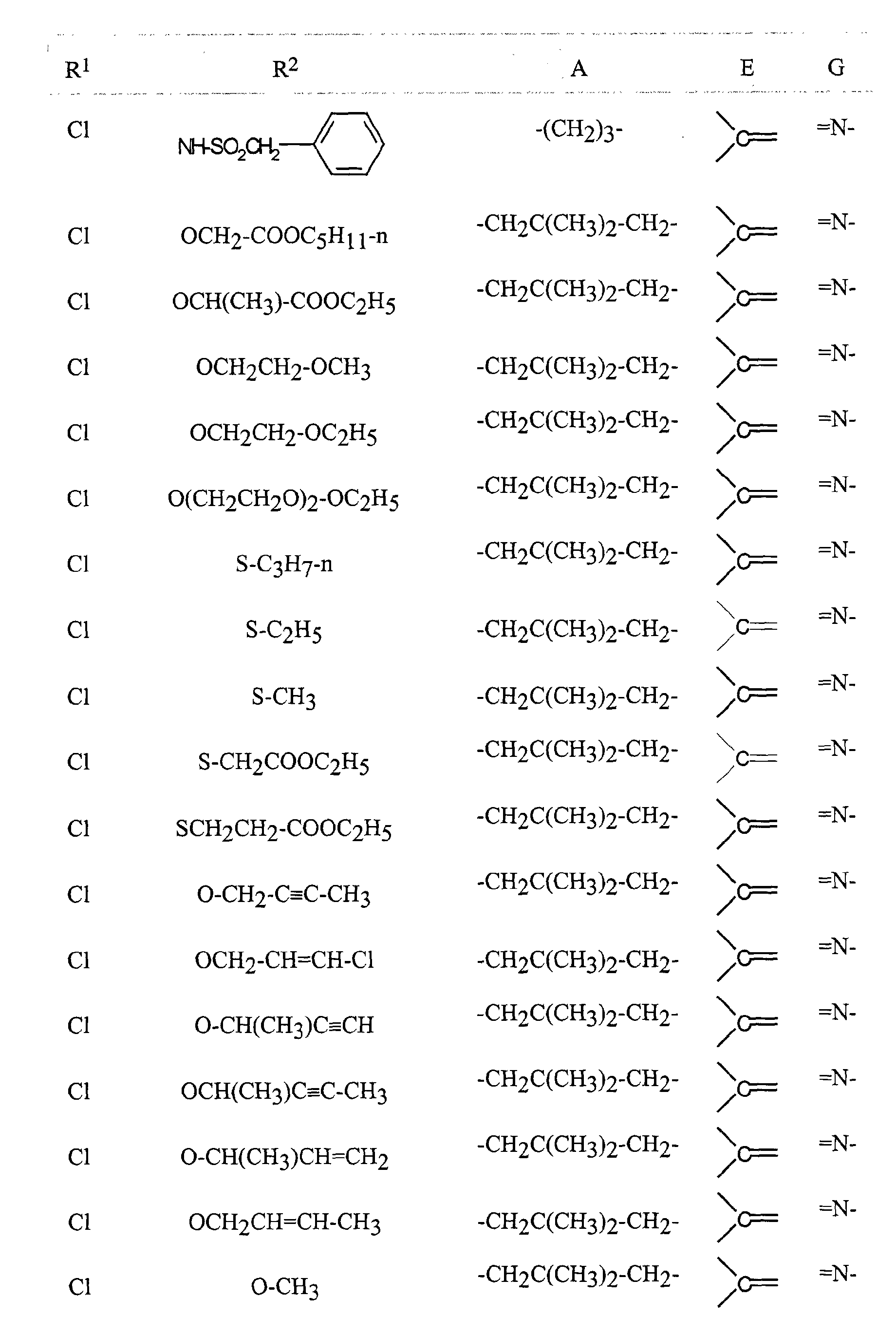

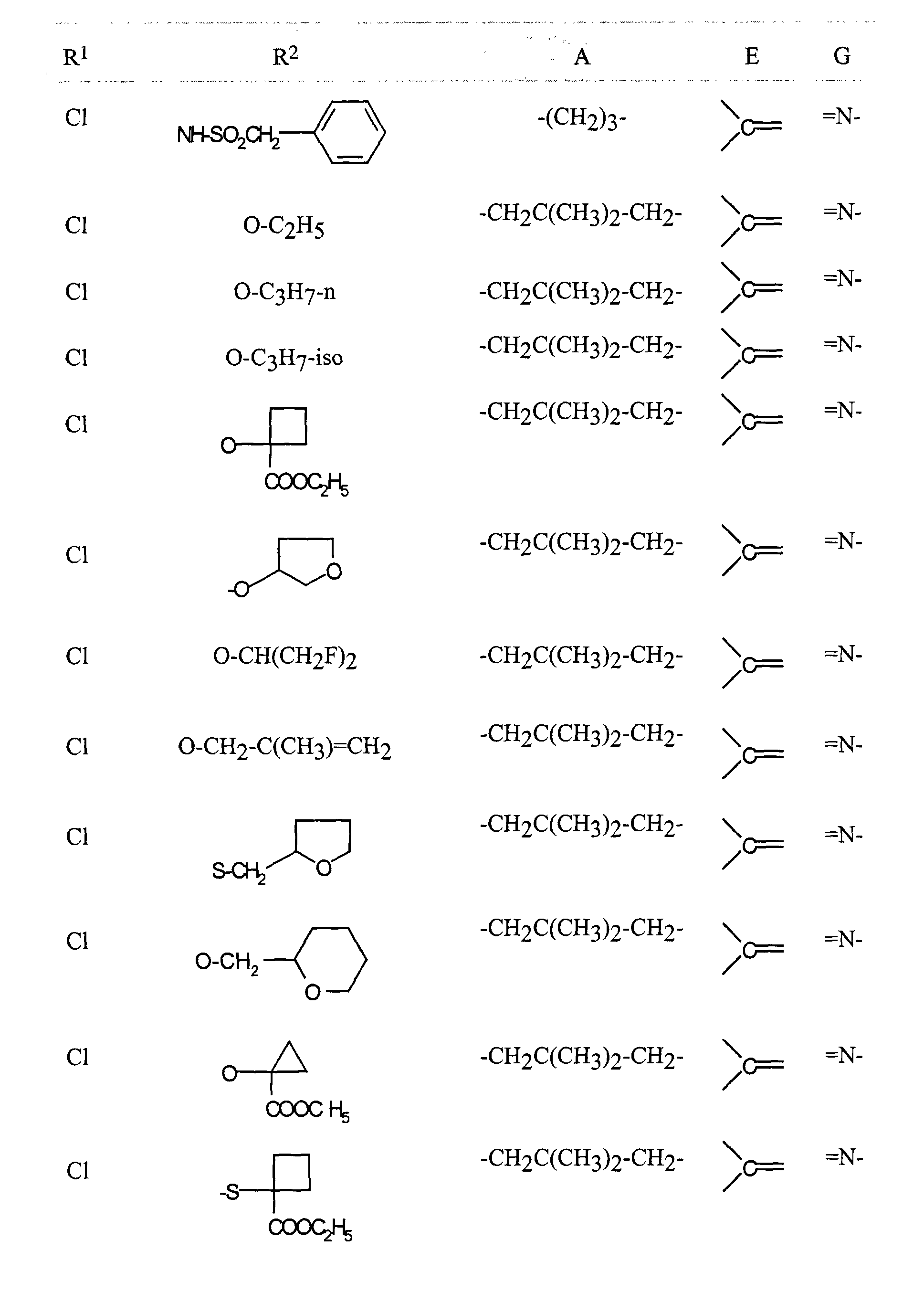

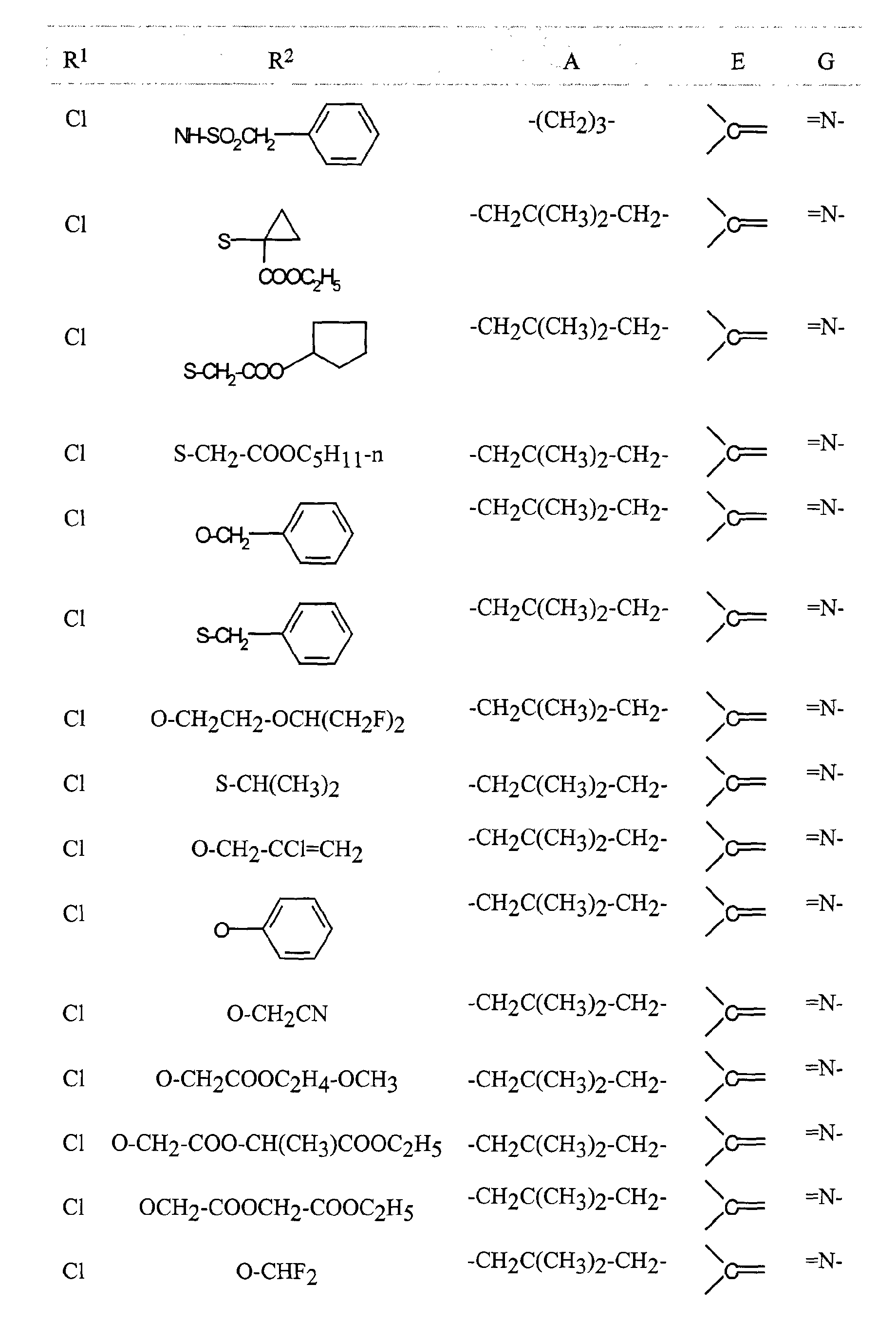

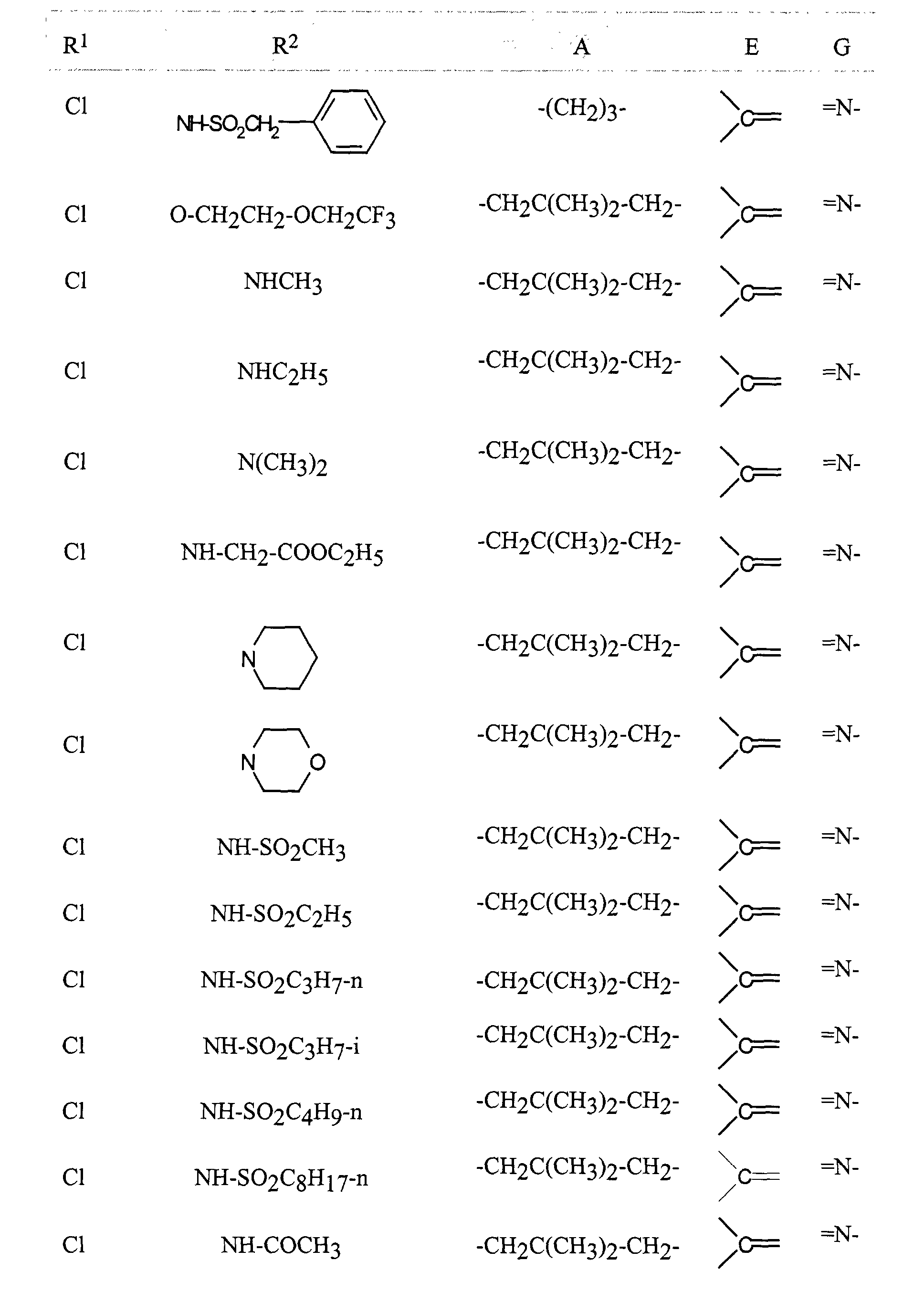

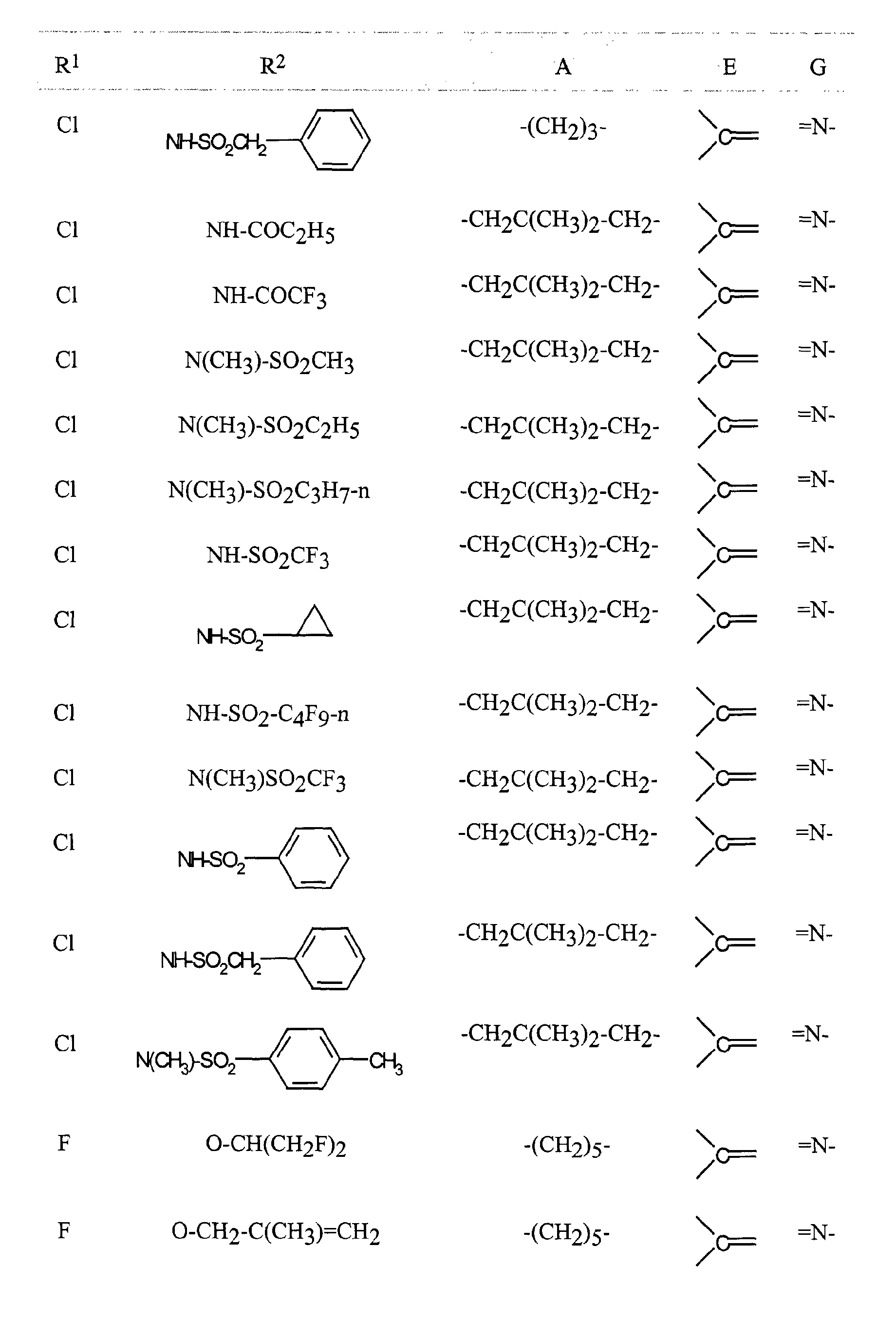

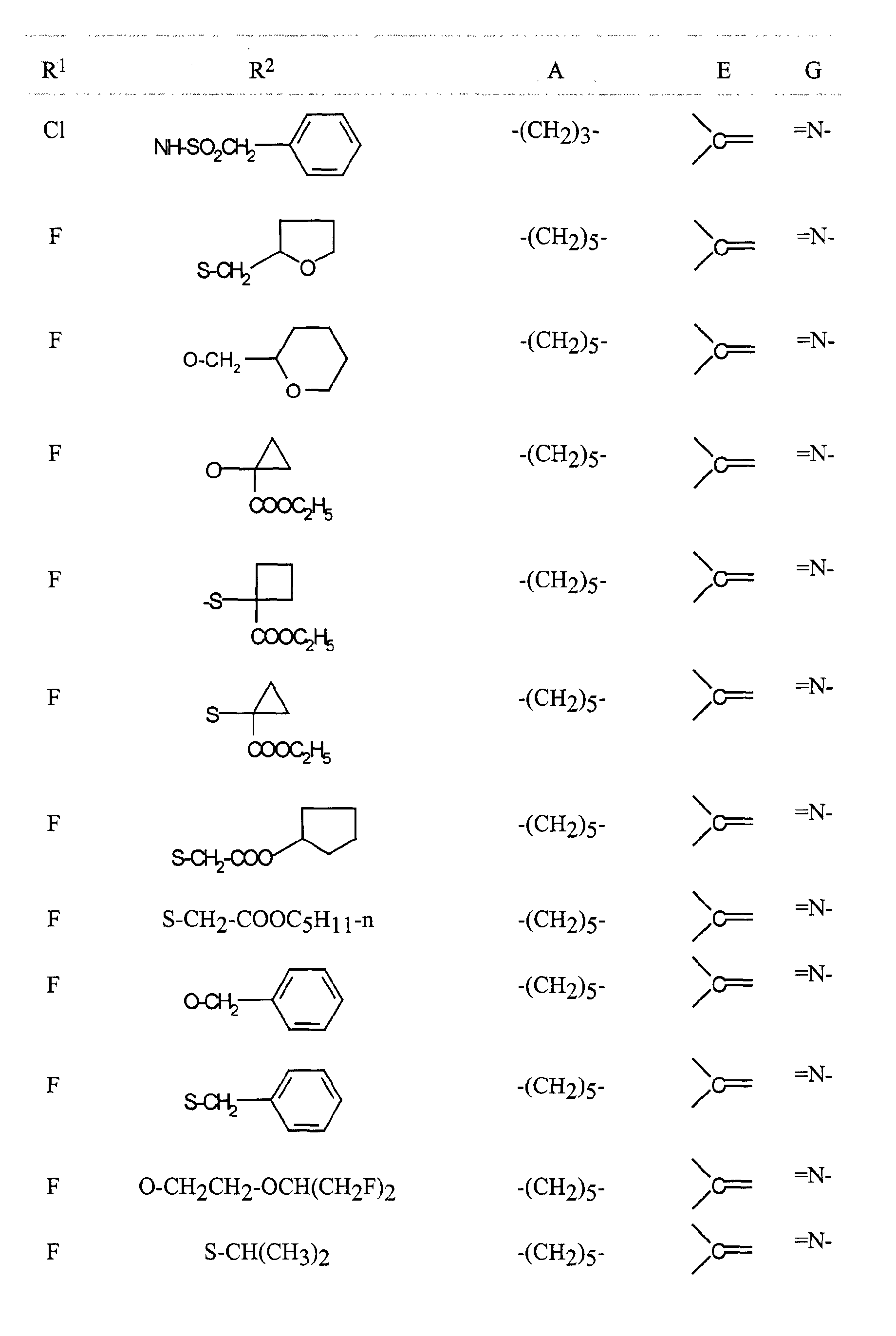

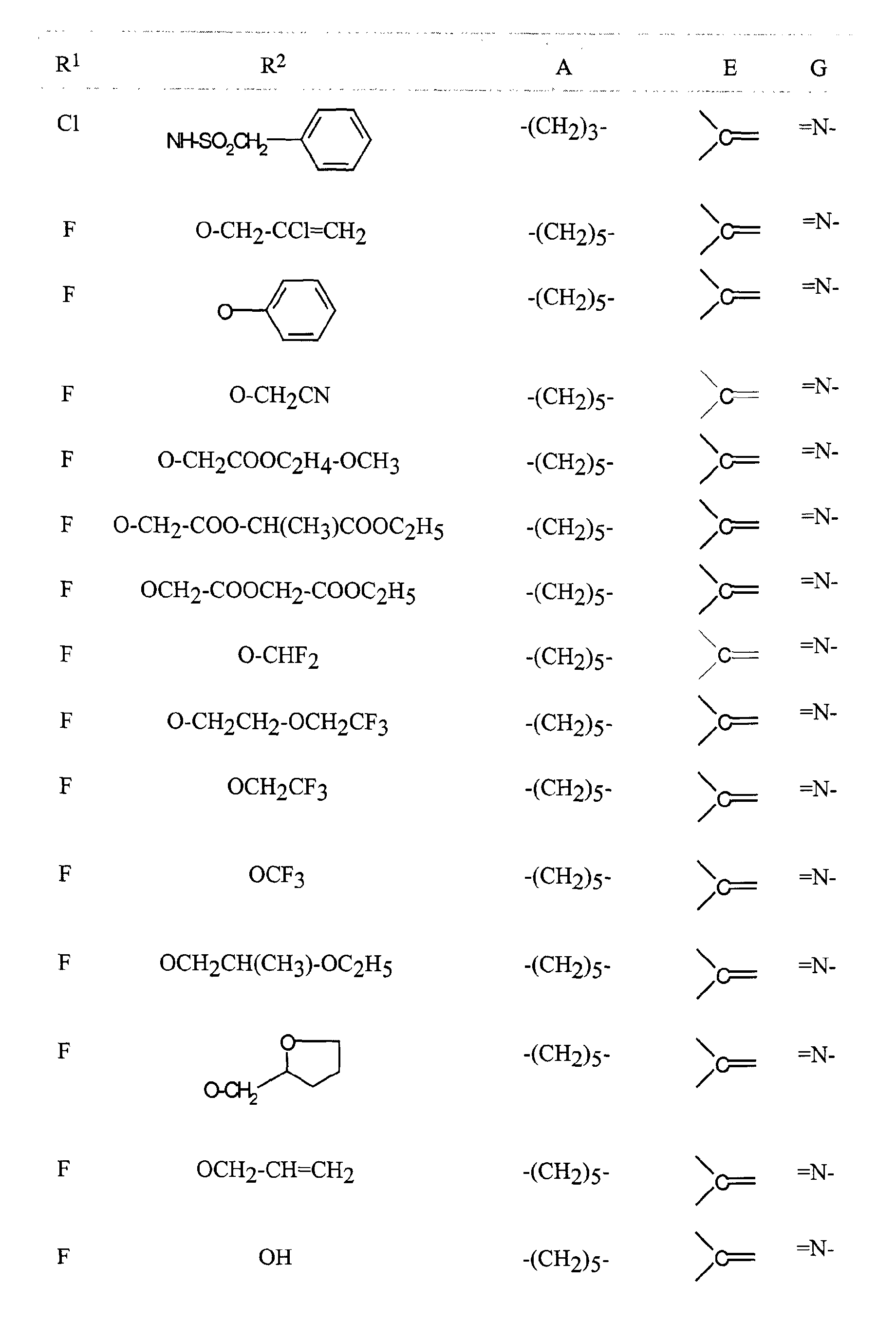

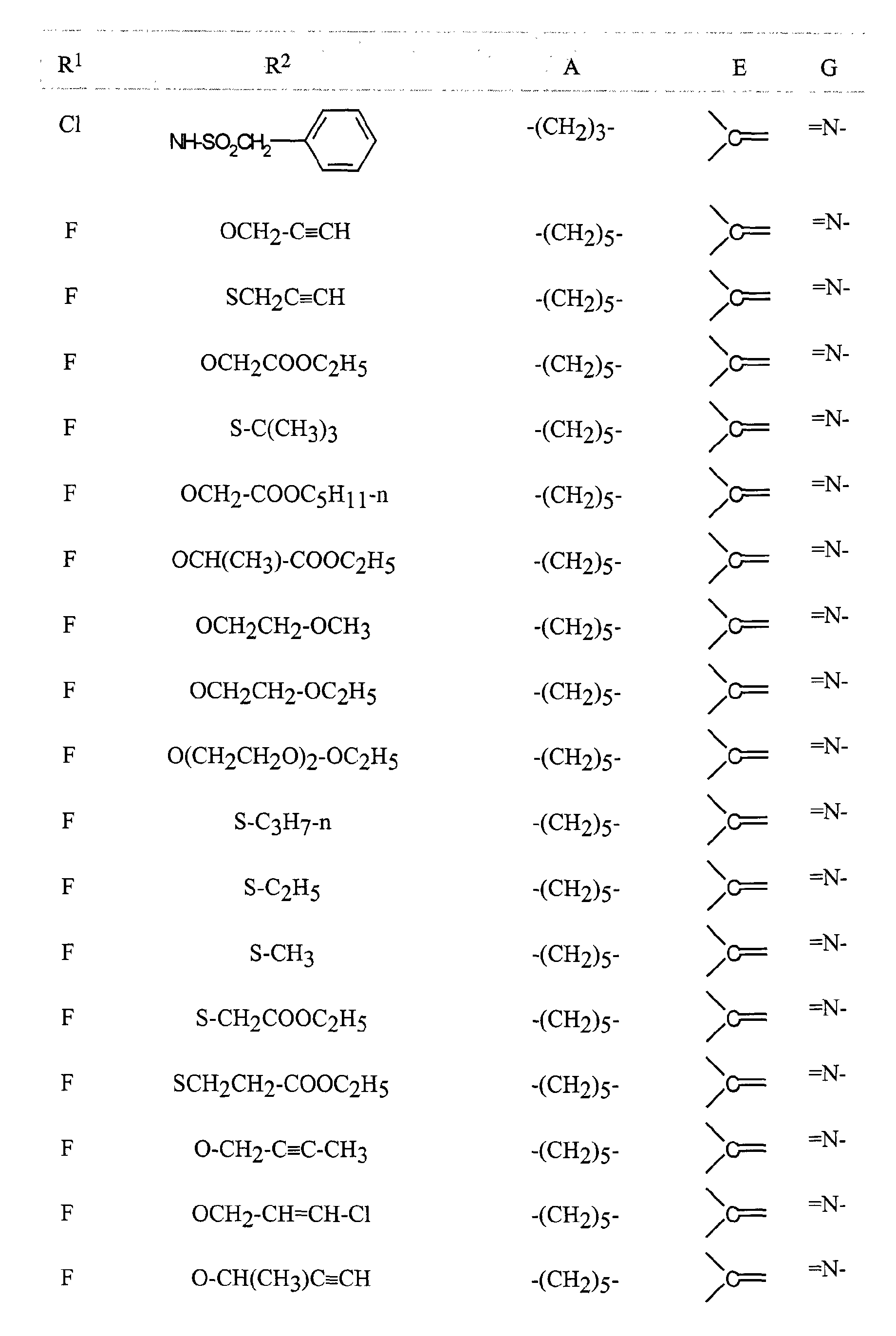

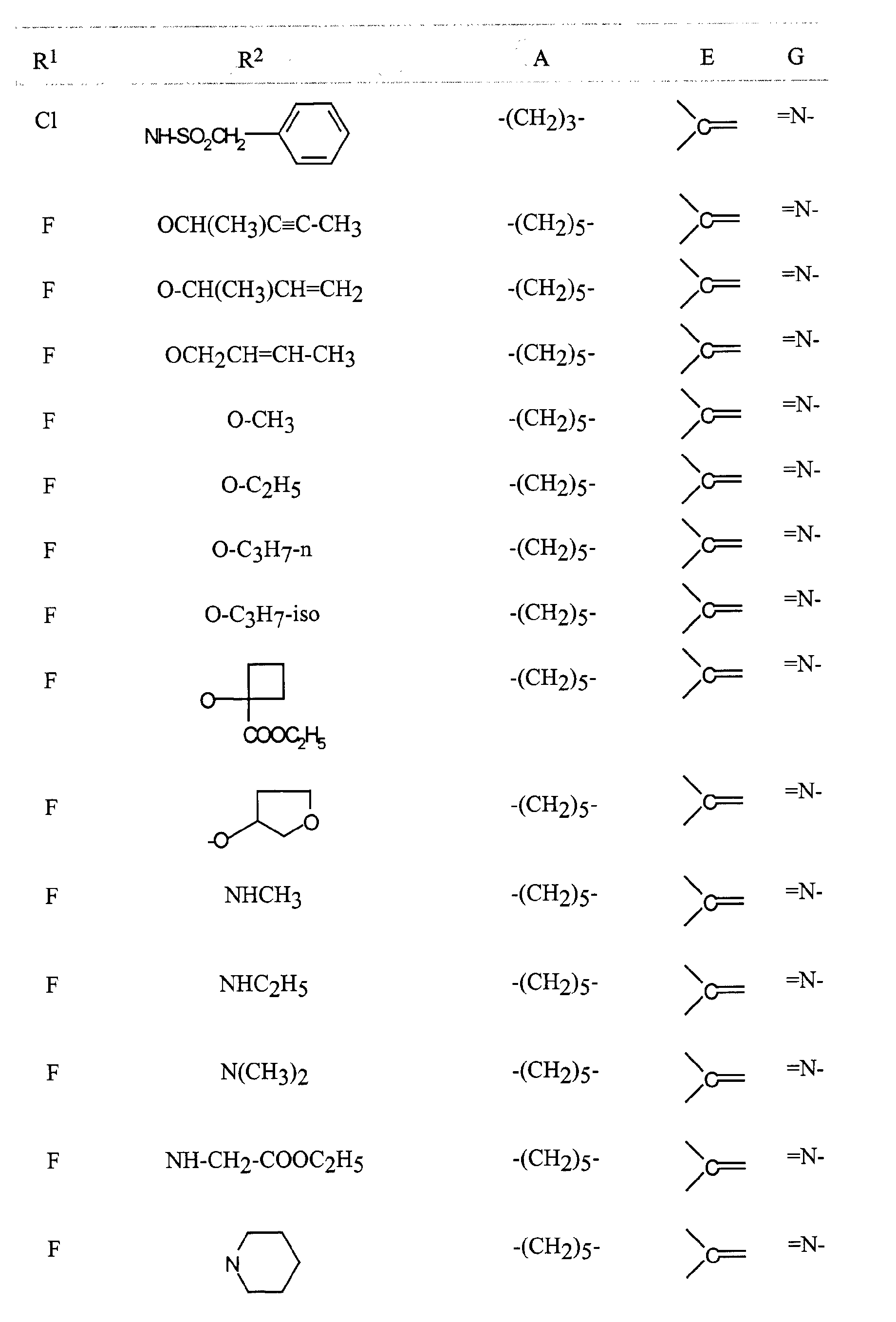

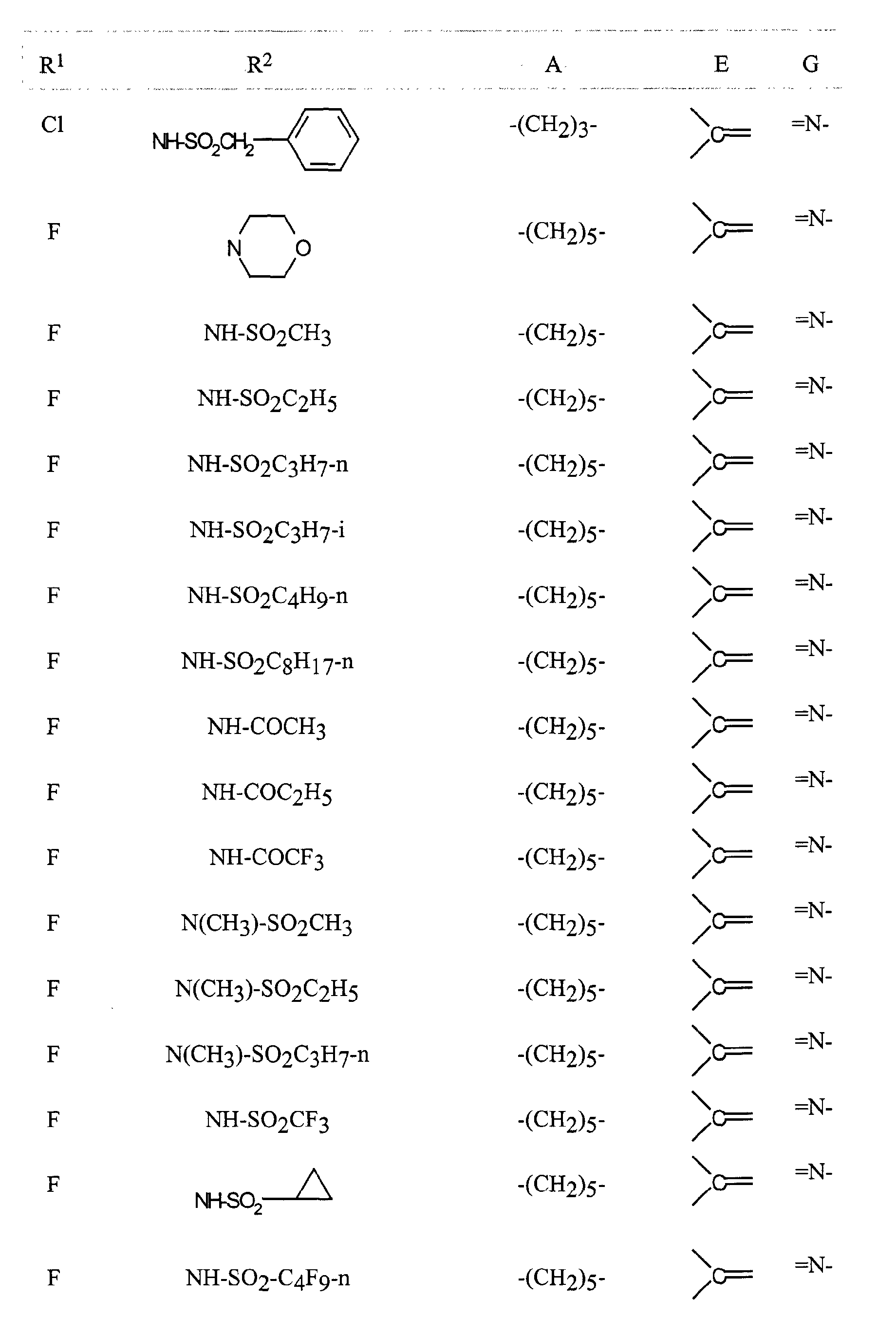

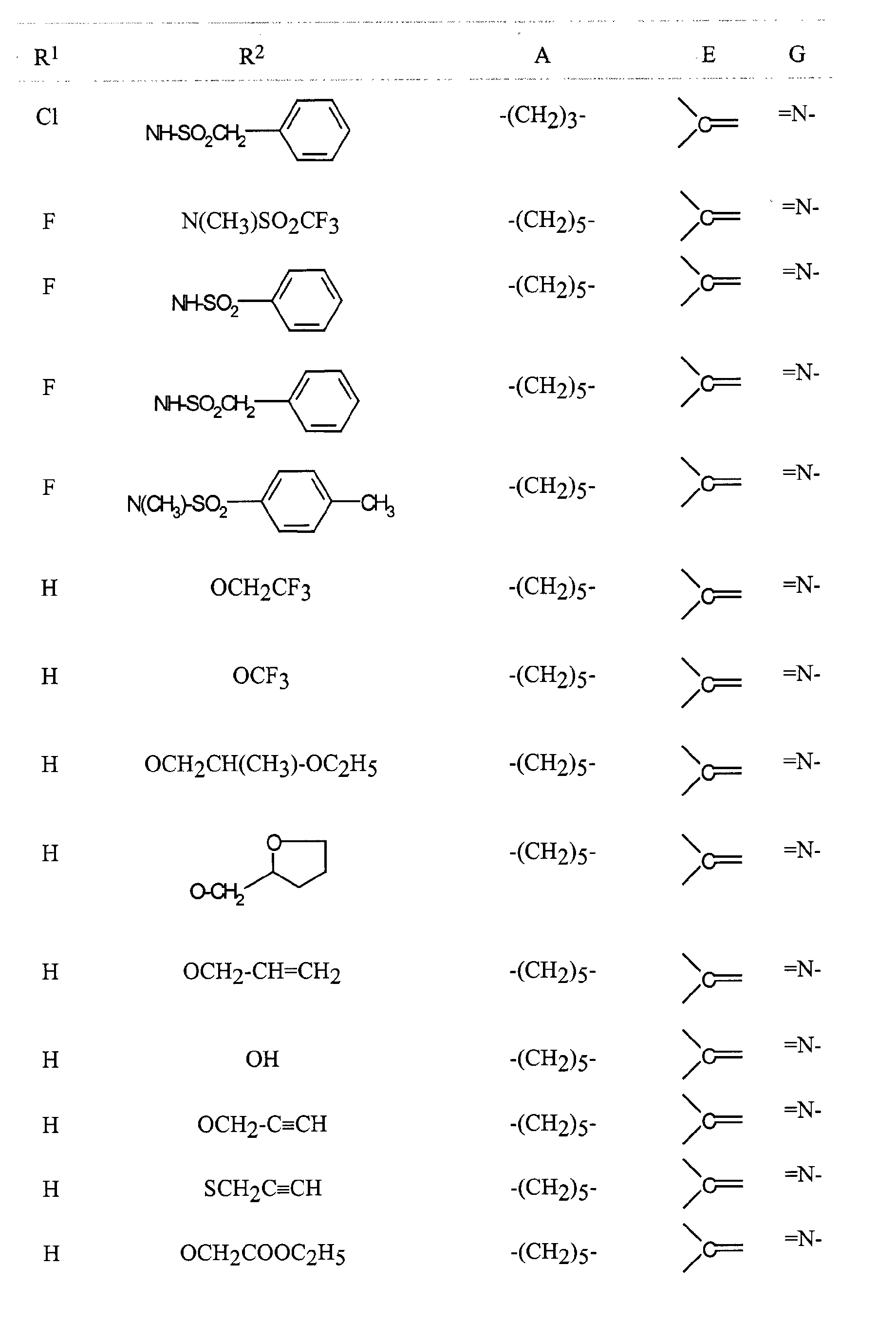

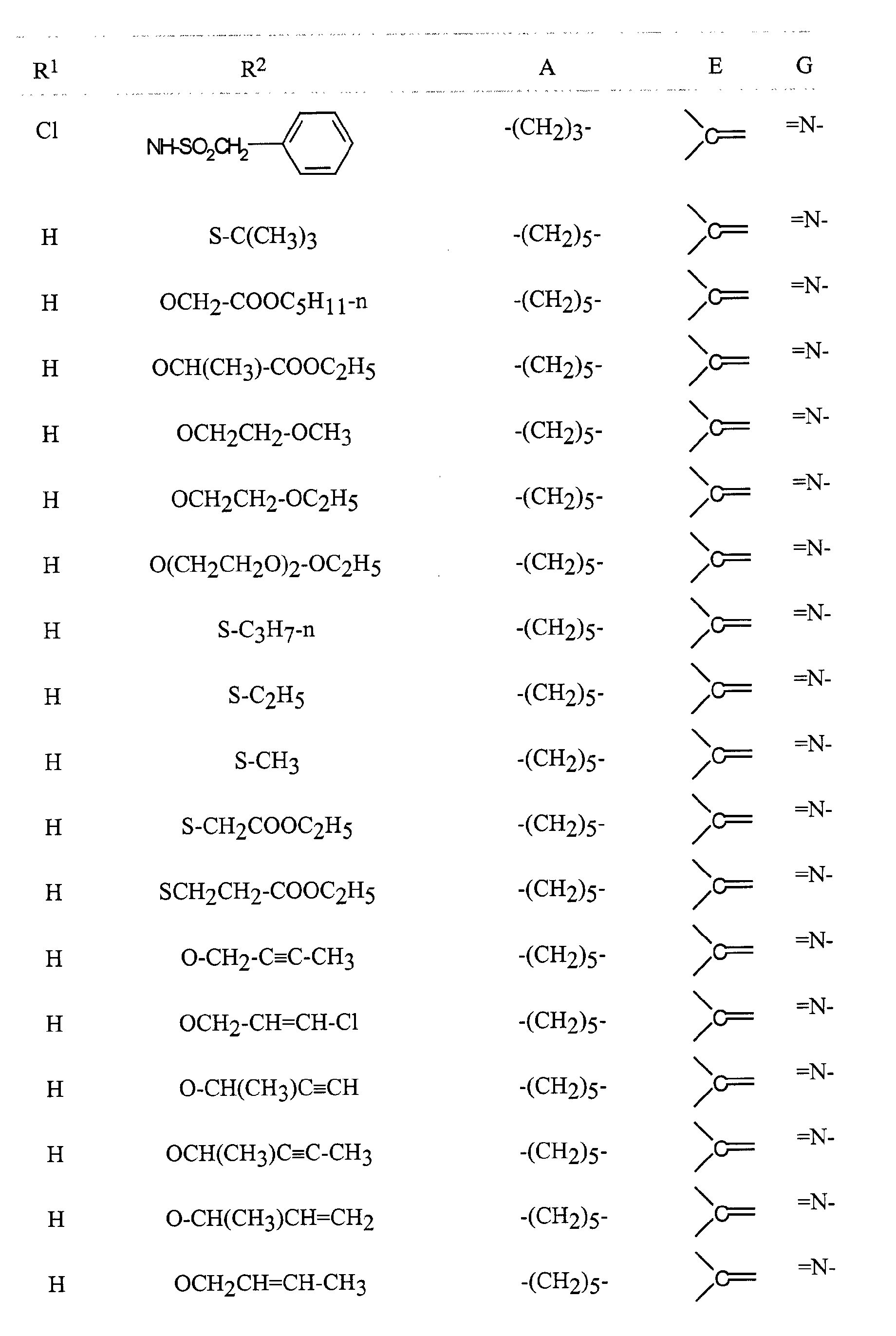

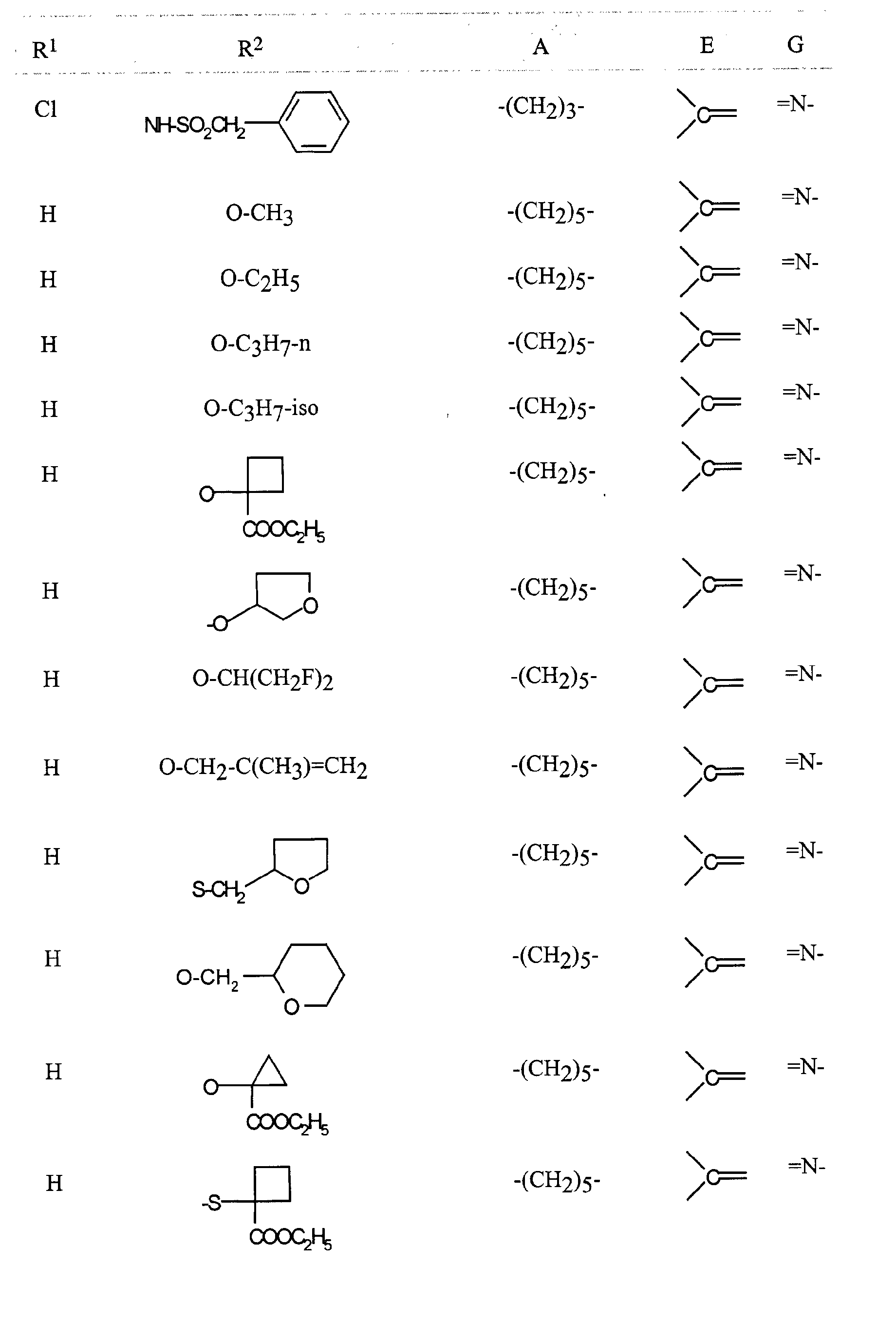

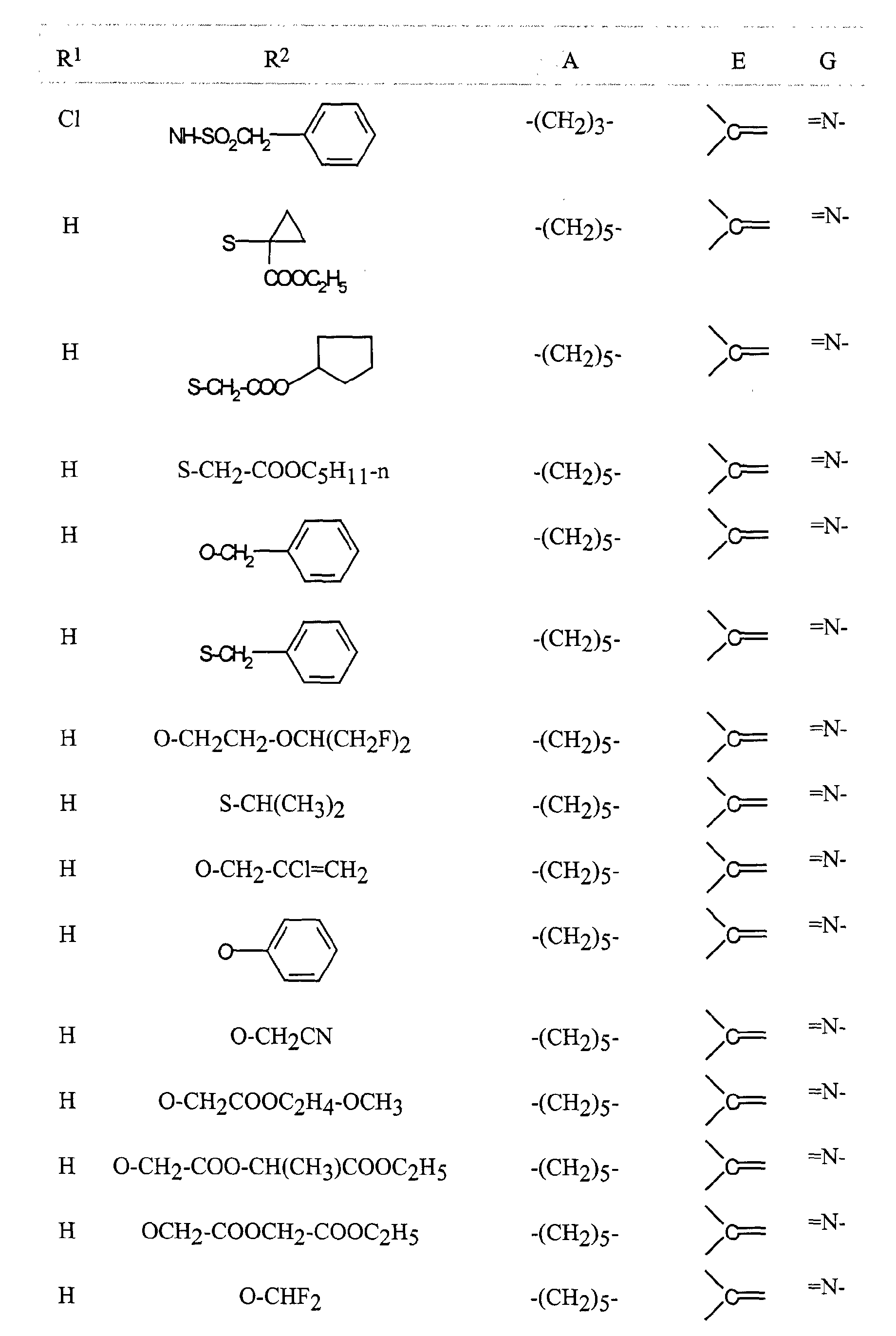

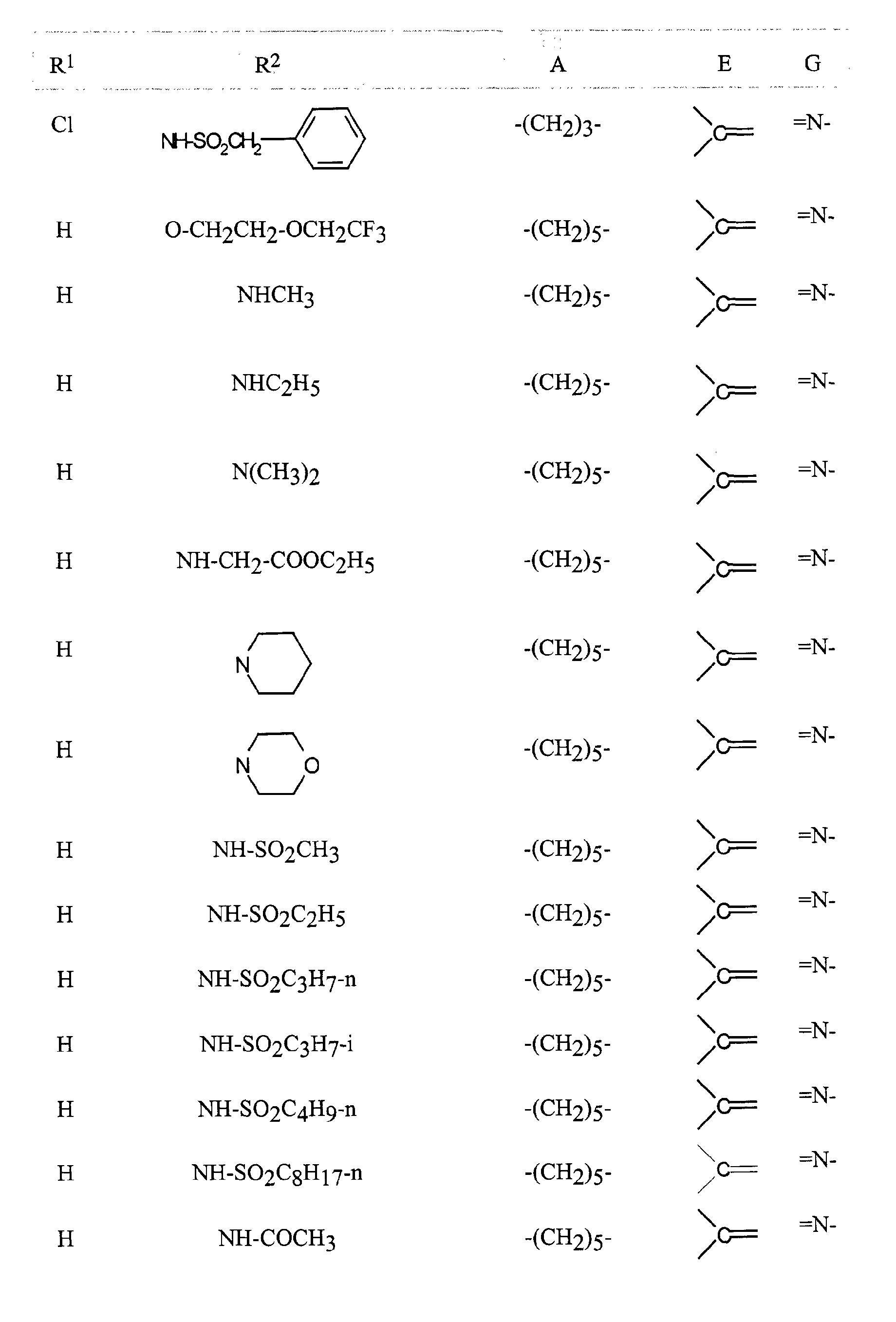

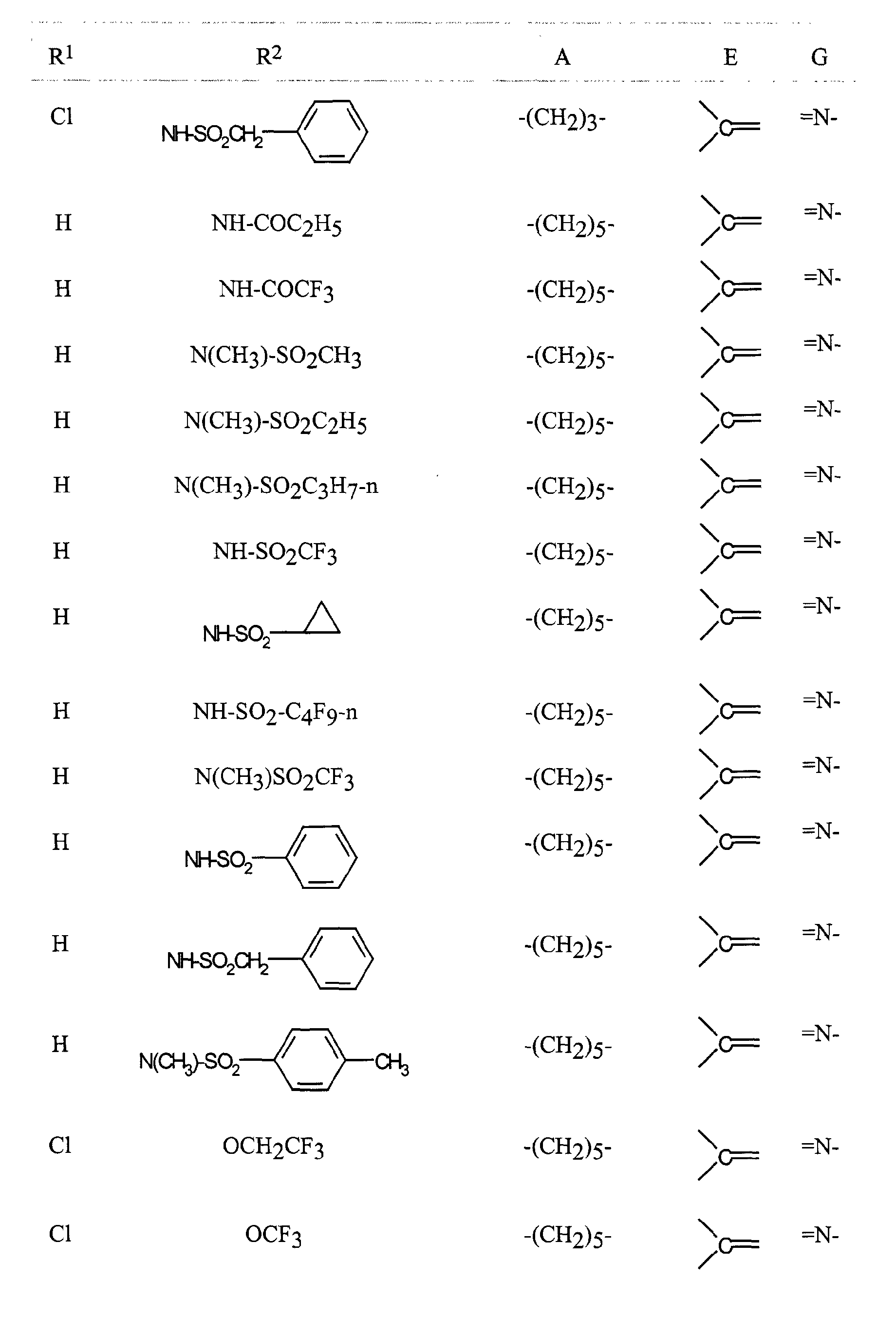

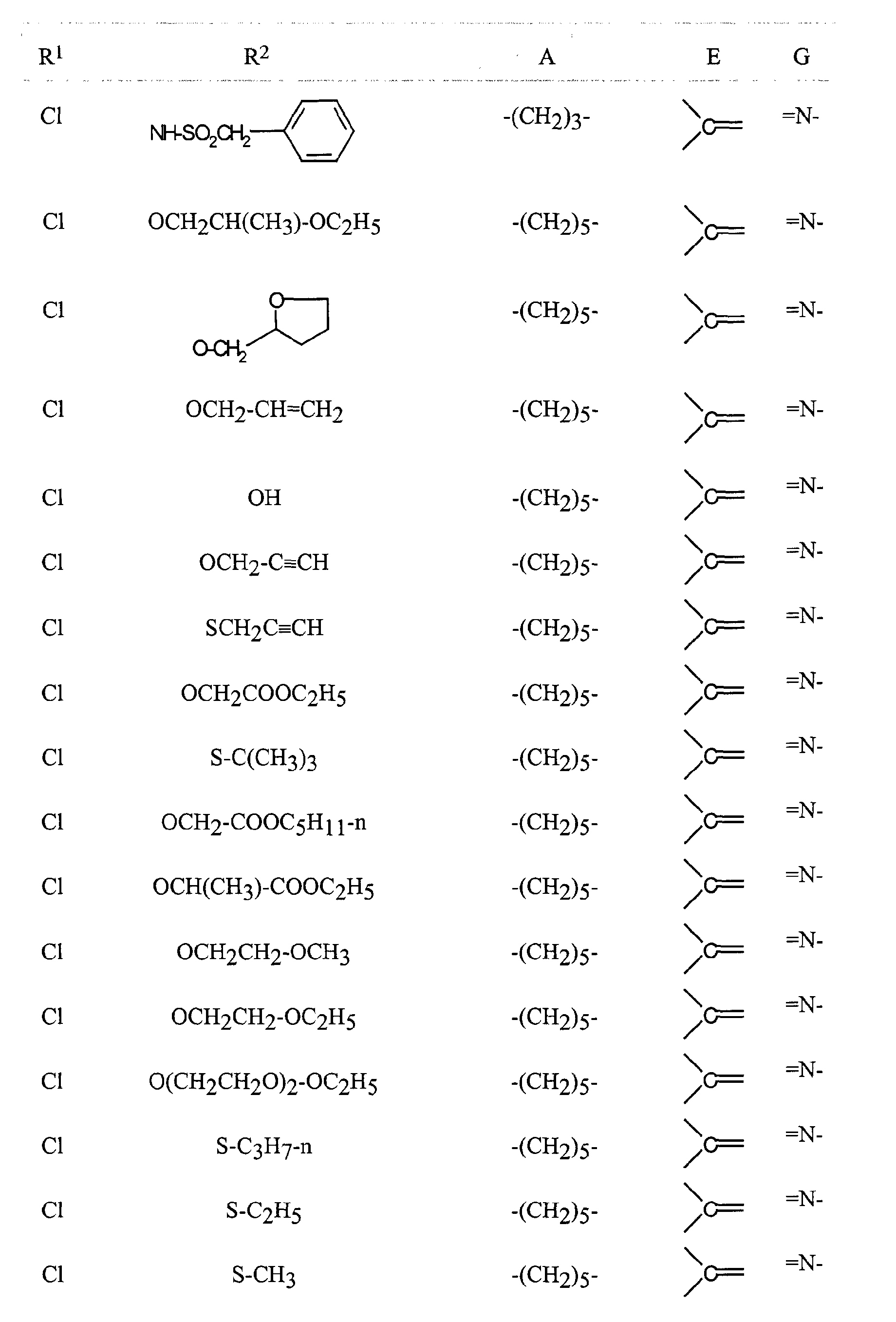

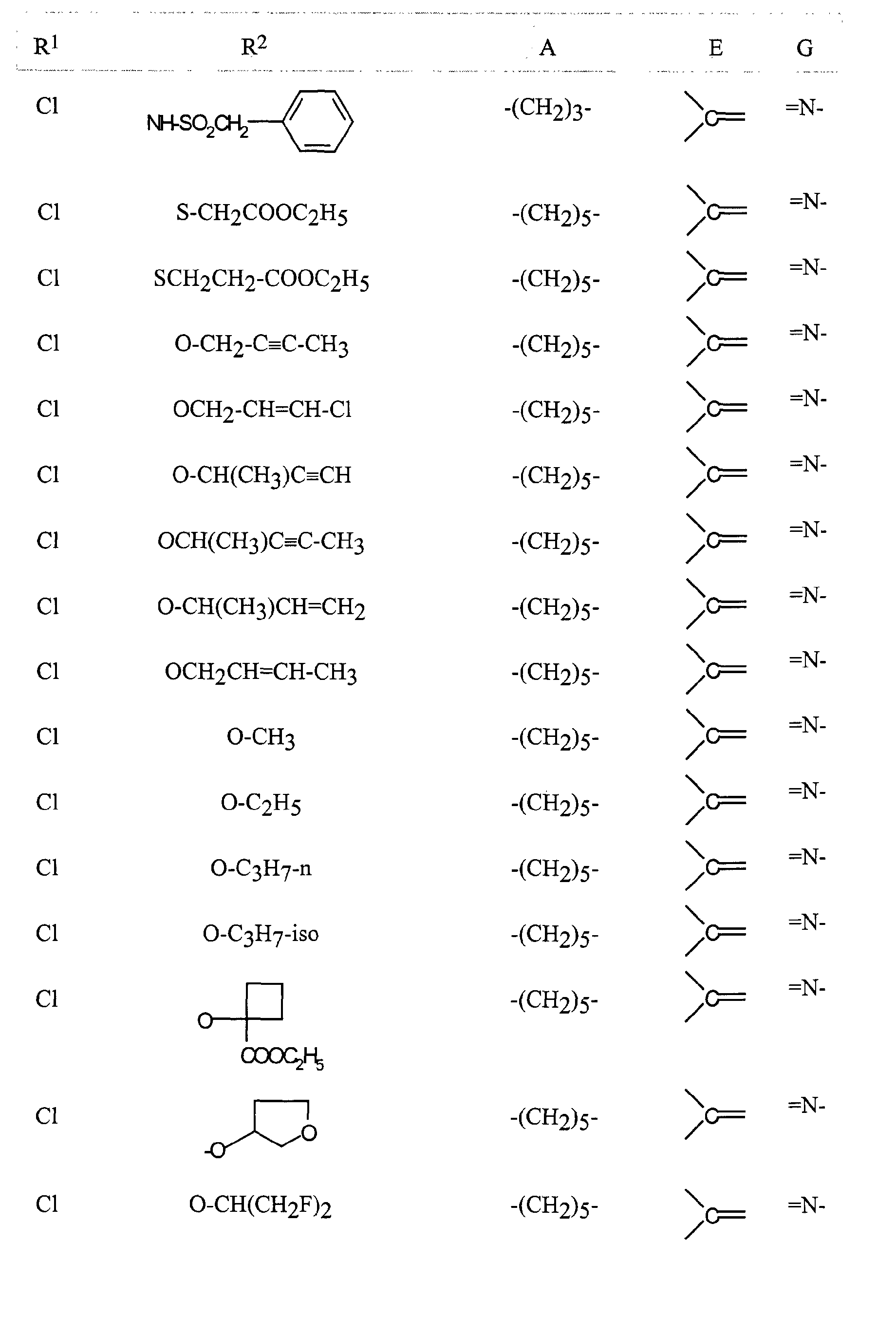

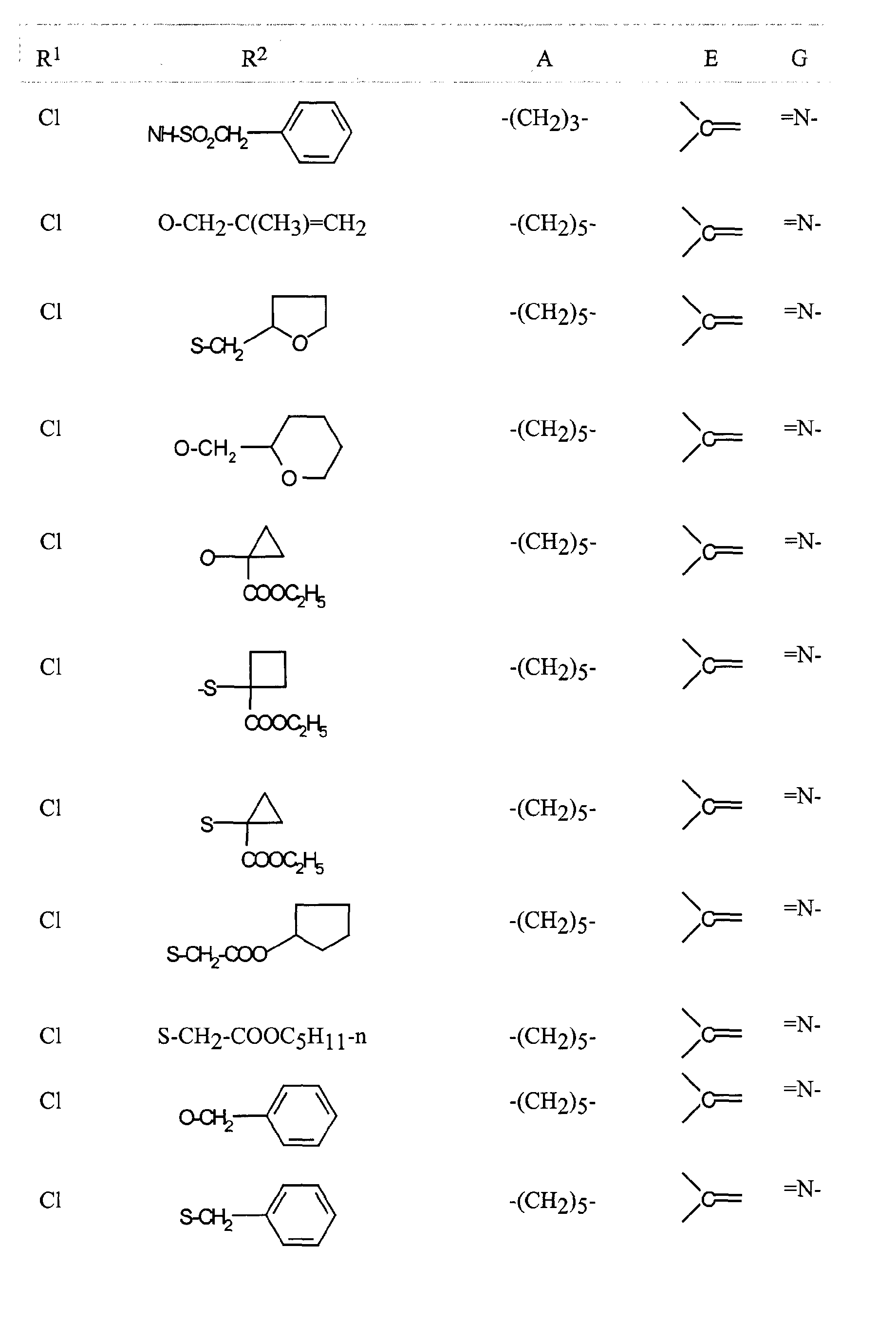

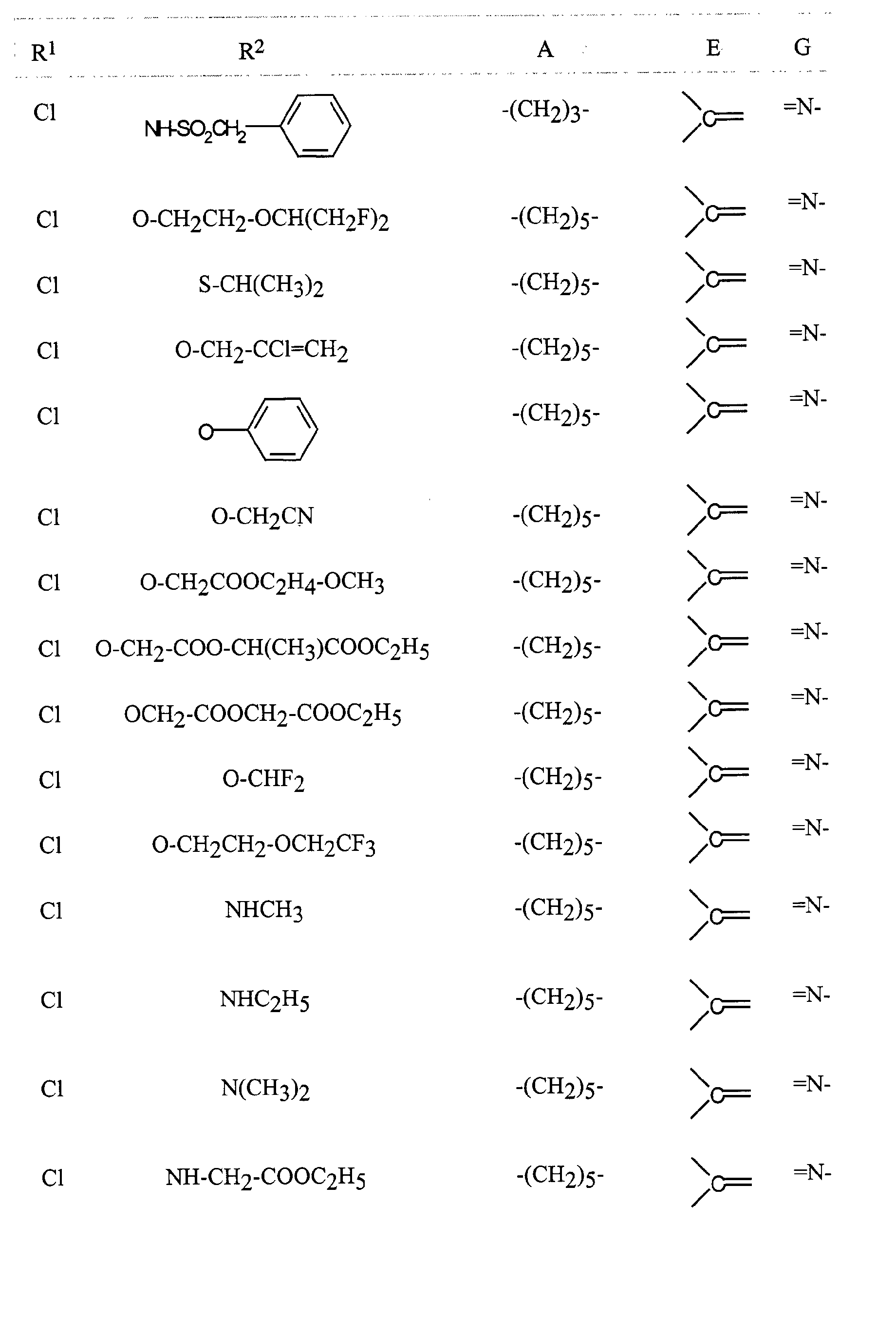

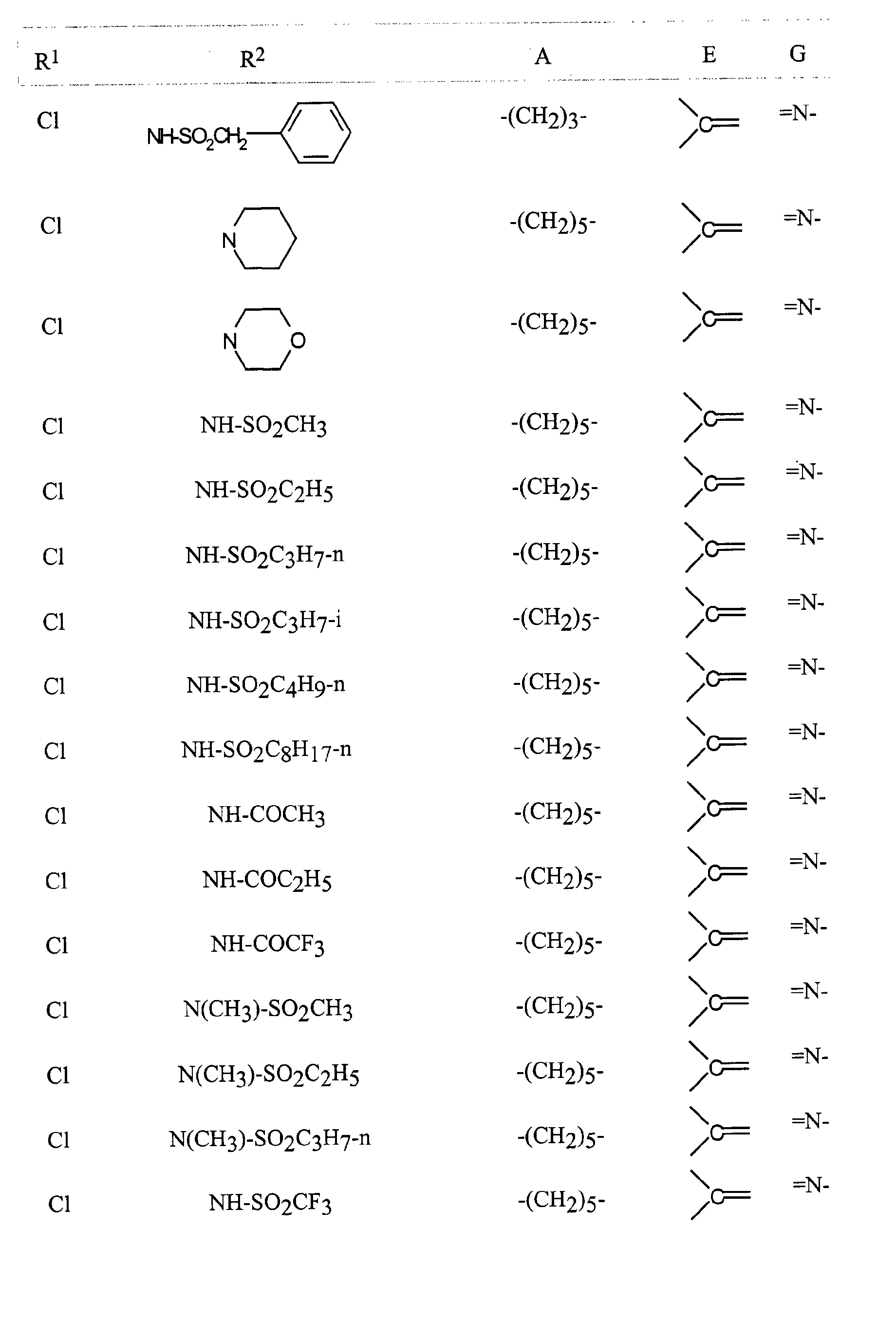

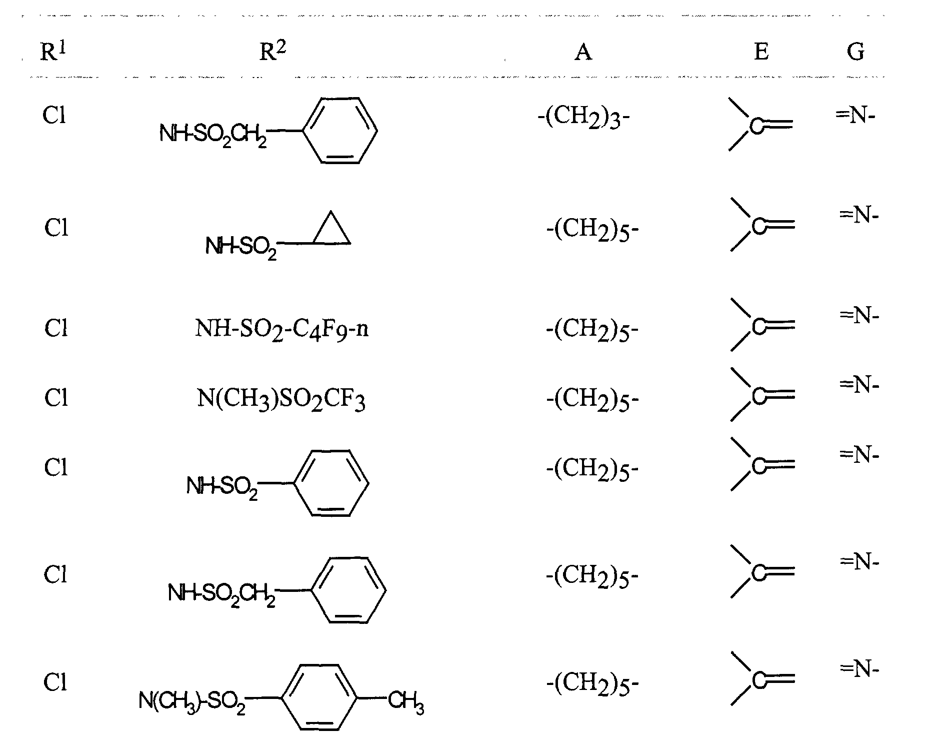

Die oben aufgeführten allgemeinen oder in Vorzugsbereichen angegebenen Restedefinitionen gelten sowohl für die Endprodukte der Formel (I), als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise 1-[N-(4-Cyano-2,5-difluor-phenyl)]-2-imino-piperidinthiocarboxamid als Ausgangsstoff und Schwefel als Oxidationsmittel, so kann der Reaktionsablauf beim erfindungsgemäßen Verfahren (a) durch das folgende Formelschema skizziert werden:

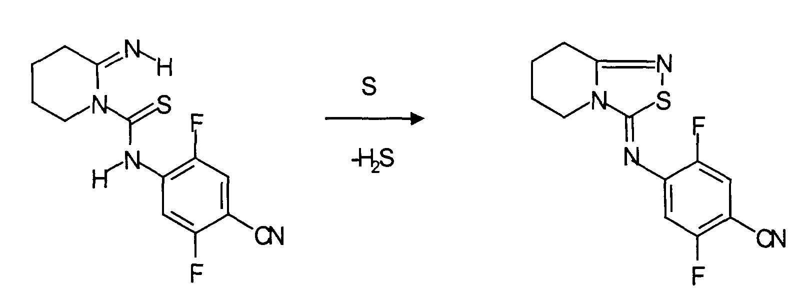

Verwendet man beispielsweise 1-[N-(4-Cyano-2-chlor-5 -ethoxy-phenyl)]-tetrahydro-(2H)-pyridazin-thiocarboxamid und Phosgen als Ausgangsstoffe, so kann der Reaktionsablauf beim erfindungsgemäßen Verfahren (b) durch das folgende Formelschema skizziert werden:

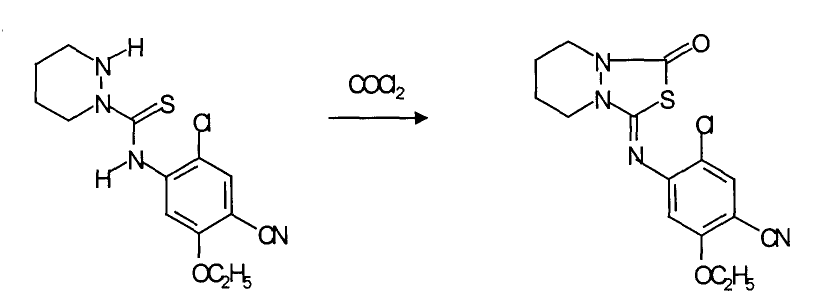

Verwendet man beispielsweise 9-(4-Cyano-2,5-difluor-phenyl-imino)-8-thia-1,6-diazabicyclo[4.3.0]nonan-7-on und Methansulfonamid als Ausgangsstoffe, so kann der Reaktionsablauf beim erfindungsgemäßen Verfahren (c) durch das folgende Formelschema skizziert werden:

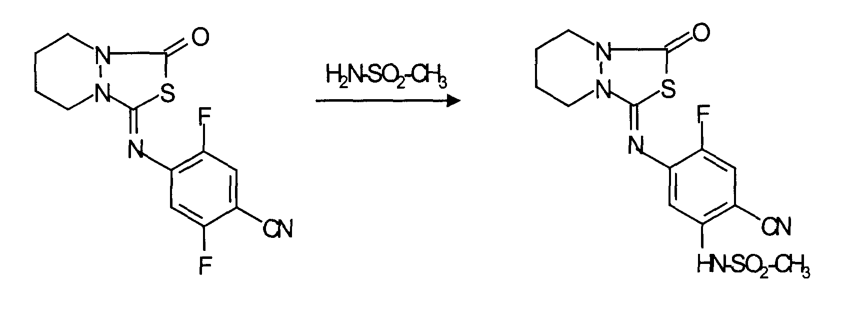

Die beim erfindungsgemäßen Verfahren (a) zur Herstellung der Verbindungen der Formel (I) als Ausgangsstoffe zu verwendenden substituierten Thiocarbonylaminoverbindungen sind durch die Formel (II) allgemein definiert.

In der Formel (II) haben R¹, R² und A vorzugsweise bzw. insbesondere diejenigen Bedeutungen, die bereits oben im Zusammenhang mit der Beschreibung der erfindungsgemäßen Verbindungen der Formel (I) als bevorzugt bzw. als insbesondere bevorzugt für R¹, R² und A angegeben wurden.

Die als Ausgangsstoffe benötigten substituierten Thiocarbonylaminoverbindungen der Formel (II) sind noch nicht aus der Literatur bekannt; sie sind als neue Stoffe Gegenstand der vorliegenden Anmeldung.

Man erhält die neuen Verbindungen der Formel (II), wenn man Cyanoarylisothiocyanate der allgemeinen Formel (VIII) 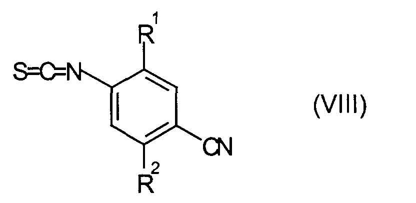 in welcher
R¹ und R² die oben angegebenen Bedeutungen haben,
mit Iminoverbindungen der allgemeinen Formel (IX) 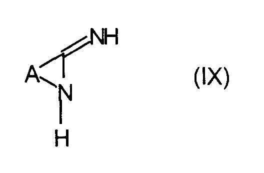
in welcher
A die oben angegebene Bedeutung hat,
gegebenenfalls in Gegenwart eines Verdünnungsmittels, wie z.B. Toluol, bei Temperaturen zwischen 0°C und 100°C umsetzt (vgl. die Herstellungsbeispiele).

Die als Vorprodukte benötigten Cyanoarylisothiocyanate der Formel (VIII) sind mit Ausnahme von 3-Chlor-4-cyano-phenyl-isothiocyanat und 3-Trifluormethyl-4-cyano-phenyl-isothiocyanat noch nicht aus der Literatur bekannt; sie sind als neue Stoffe Gegenstand der vorliegenden Anmeldung.

Man erhält die neuen Cyanoarylisothiocyanate der Formel (VIII), wenn man entsprechende Cyanoarylamine der allgemeinen Formel (X) 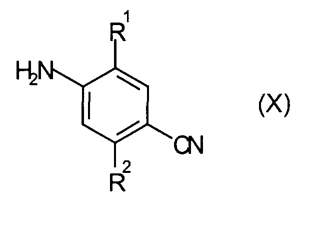 in welcher
R¹ und R² die oben angegebenen Bedeutungen haben,
mit Thiophosgen, gegebenenfalls in Gegenwart eines Reaktionshilfsmittels, wie z.B. Calciumcarbonat, und gegebenenfalls in Gegenwart von Verdünnungsmitteln, wie z.B. Methylenchlorid und Wasser, bei Temperaturen zwischen 0°C und 100°C umsetzt (vgl. die Herstellungsbeispiele).

Die Cyanoarylamine der allgemeinen Formel (X) sind bekannt und/oder können nach an sich bekannten Verfahren hergestellt werden (vgl. EP-A 224001).

Noch nicht aus der Literatur bekannt und als neue Verbindungen Gegenstand der vorliegenden Anmeldung sind die Cyanoarylamine der allgemeinen Formel (Xa) 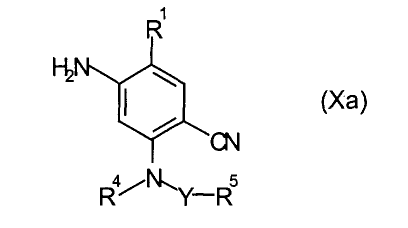 in welcher
R¹, R⁴ und R⁵ die oben angegebenen Bedeutungen haben und
Y für CO oder SO₂ steht.

Man erhält die neuen Cyanoarylamine der Formel (Xa), wenn man entsprechende Halogenarylamine der allgemeinen Formel (XI) 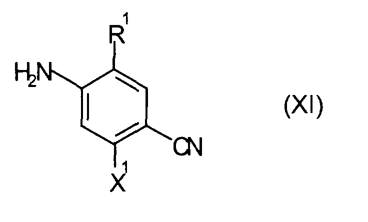 in welcher
R¹ und X¹ die oben angegebenen Bedeutungen haben,
mit Amiden der allgemeinen Formel (XII)

   R⁴-NH-Y-R⁵ (XII)
in welcher
R⁴, R⁵ und Y die oben angegebenen Bedeutungen haben,
gegebenenfalls in Gegenwart eines Säureakzeptors, wie z.B. Kaliumcarbonat, und gegebenenfalls in Gegenwart eines Verdünnungsmittels, wie z.B. N-Methyl-pyrrolidon, bei Temperaturen zwischen 100°C und 200°C umsetzt (vgl. die Herstellungsbeispiele).

Die weiter als Vorprodukte benötigten Iminoverbindungen der Formel (IX) sind bekannt und/oder können nach an sich bekannten Verfahren hergestellt werden (vgl. Angew. Chem. 81 (1969), 431-432; loc. cit. Int. Ed. Engl. 8 (1969), 457-458; J. Org. Chem. 33 (1968), 2109-2111).

Das erfindungsgemäße Verfahren (a) wird unter Verwendung von Oxidationsmitteln bzw. Dehydrierungsmitteln durchgeführt. Es kommen hierbei praktisch alle zur Oxidation bzw. Dehydrierung organisch chemischer Verbindungen üblichen Reagentien in Betracht. Beispielhaft seien Sauerstoff, Schwefel, Wasserstoffperoxid (Hydrogenperoxid), Natriumperoxodisulfat, Natriumthiosulfat, Chlor, Brom und Iod genannt.

Das erfindungsgemäße Verfahren (a) wird vorzugsweise unter Verwendung eines Verdünnungsmittels durchgeführt. Als Verdünnungsmittel kommen alle üblichen organischen oder anorganischen Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Alkohole, wie Methanol, Ethanol, n-oder i-Propanol oder n-, i-, s- oder t-Butanol sowie Wasser.

Das erfindungsgemäße Verfahren (a) wird gegebenenfalls in Gegenwart eines geeigneten Reaktionshilfsmittels durchgeführt. Als solche kommen vor allem die üblichen anorganischen oder organischen Basen in Betracht. Hierzu gehören beispielsweise Erdalkali- oder Alkalimetallhydroxide, wie Natriumhydroxid, Kaliumhydroxid, Calciumhydroxid oder auch Ammoniumhydroxid, Alkalimetall(hydrogen)carbonate, wie Natrium(hydrogen)carbonat, Kalium(hydrogen)carbonat oder Ammoniumcarbonat, Alkali- oder Erdalkalimetallacetate, wie Natriumacetat, Kaliumacetat, Calciumacetat oder Ammoniumacetat, Alkalimetall-alkoholate, wie Natrium- oder Kalium-methylat, Natrium- oder Kalium-ethylat, Natrium- oder Kalium-tert-butylat, sowie tertiäre Amine, wie Trimethylamin, Triethylamin, Tributylamin, N,N-Dimethylanilin, Pyridin, N-Methylpiperidin, 4-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 150°C, vorzugsweise bei Temperaturen zwischen 20°C und 120°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren bzw. äquivalenten Mengen eingesetzt. Es ist jedoch auch möglich, eine der jeweils eingesetzten Komponenten in einem größeren Überschuß zu verwenden. Die Umsetzung wird im allgemeinen in einem geeigneten Verdünnungsmittel durchgeführt und das Reaktionsgemisch wird bis zum Ende der Umsetzung bei der jeweils erforderlichen Temperatur gerührt. Die Aufarbeitung erfolgt nach üblichen Methoden.

Die beim erfindungsgemäßen Verfahren (b) zur Herstellung der Verbindungen der Formel (I) als Ausgangsstoffe zu verwendenden substituierten Thiocarbonylaminoverbindungen sind durch die Formel (III) allgemein definiert.

In der Formel (III) haben R¹, R² und A vorzugsweise bzw. insbesondere diejenigen Bedeutungen, die bereits oben im Zusammenhang mit der Beschreibung der erfindungsgemäßen Verbindungen der Formel (I) als bevorzugt bzw. als insbesondere bevorzugt für R¹, R² und A angegeben wurden.

Die als Ausgangsstoffe benötigten substituierten Thiocarbonylaminoverbindungen der Formel (III) sind noch nicht aus der Literatur bekannt; sie sind als neue Stoffe Gegenstand der vorliegenden Anmeldung.

Man erhält die neuen Verbindungen der Formel (III), wenn man Diazacycloalkane bzw. Diazacycloalkene der allgemeinen Formel (XI) 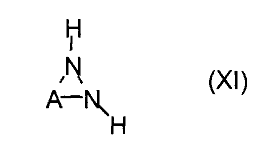 in welcher
A die oben angegebenen Bedeutung hat,
mit Cyanoarylisothiocyanaten der allgemeinen Formel (VIII) - oben -
gegebenenfalls in Gegenwart eines Verdünnungsmittels, wie z.B. Toluol, bei Temperaturen zwischen 0°C und 100°C umsetzt (vgl. die Herstellungsbeispiele).

Die als Vorprodukt benötigten Diazacycloalkane bzw. Diazacycloalkene der Formel (XI) sind bekannt und/oder können nach an sich bekannten Verfahren hergestellt werden (vgl. J. Org. Chem. 35 (1970), 1468-1471; Tetrahedron 31 (1975), 165-170).

Das erfindungsgemäße Verfahren (b) wird vorzugsweise unter Verwendung eines Verdünnungsmittels durchgeführt. Als Verdünnungsmittel kommen alle üblichen inerten organischen Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und 150°C, vorzugsweise bei Temperaturen zwischen 10°C und 100°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren bzw. äquivalenten Mengen eingesetzt. Es ist jedoch auch möglich, eine der jeweils eingesetzten Komponenten in einem größeren Überschuß zu verwenden. Die Umsetzung wird im allgemeinen in einem geeigneten Verdünnungsmittel durchgeführt und das Reaktionsgemisch wird bis zum Ende der Umsetzung bei der jeweils erforderlichen Temperatur gerührt. Die Aufarbeitung erfolgt nach üblichen Methoden (vgl. die Herstellungsbeispiele).

Die beim erfindungsgemäßen Verfahren (c) als Ausgangsstoffe zu verwendenden substituierten Cyanoaryliminoheterocyclen sind durch die Formel (Ia) allgemein definiert.

In der Formel (Ia) haben R¹, A, E und G vorzugsweise bzw. insbesondere diejenigen Bedeutungen, die bereits oben im Zusammenhang mit der Beschreibung der erfindungsgemäßen Verbindungen der Formel (I) als bevorzugt bzw. als insbesondere bevorzugt für R¹, A, E und G angegeben wurden;

X¹ steht vorzugsweise für Fluor oder Chlor, insbesondere für Fluor.

Die Ausgangsstoffe der Formel (Ia) sind erfindungsgemäße, neue Verbindungen; sie können nach den erfindungsgemäßen Verfahren (a) oder (b) hergestellt werden.

Das erfindungsgemäße Verfahren (c) wird vorzugsweise unter Verwendung eines Verdünnungsmittels durchgeführt. Als Verdünnungsmittel kommen alle üblichen organischen oder anorganischen Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Alkohole, wie Methanol, Ethanol, n-oder i-Propanol oder n-, i-, s- oder t-Butanol sowie Wasser.

Das erfindungsgemäße Verfahren (c) wird gegebenenfalls in Gegenwart eines Säureakzeptors durchgeführt. Als solche kommen vor allem die üblichen anorganischen oder organischen Basen in Betracht. Hierzu gehören beispielsweise Erdalkali- oder Alkalimetallhydroxide, wie Natriumhydroxid, Kaliumhydroxid, Calciumhydroxid oder auch Ammoniumhydroxid, Alkalimetall(hydrogen)carbonate, wie Natrium(hydrogen)carbonat, Kalium(hydrogen)carbonat oder Ammoniumcarbonat, Alkalioder Erdalkalimetallacetate, wie Natriumacetat, Kaliumacetat, Calciumacetat oder Ammoniumacetat, Alkalimetall-alkoholate, wie Natrium- oder Kalium-methylat, Natrium- oder Kalium-ethylat, Natrium- oder Kalium-tert-butylat, sowie tertiäre Amine, wie Trimethylamin, Triethylamin, Tributylamin, N,N-Dimethylanilin, Pyridin, N-Methylpiperidin, 4-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0°C und 150°C, vorzugsweise bei Temperaturen zwischen 10°C und 120°C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren bzw. äquivalenten Mengen eingesetzt. Es ist jedoch auch möglich, eine der jeweils eingesetzten Komponenten in einem größeren Überschuß zu verwenden. Die Umsetzung wird im allgemeinen in einem geeigneten Verdünnungsmittel durchgeführt und das Reaktionsgemisch wird bis zum Ende der Umsetzung bei der jeweils erforderlichen Temperatur gerührt. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuronethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuronmethyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Die Herstellung und die Verwendung der erfindungsgemäßen Wirkstoffe geht aus den nachfolgenden Beispielen hervor.

### Herstellungsbeispiele:

### Beispiel 1

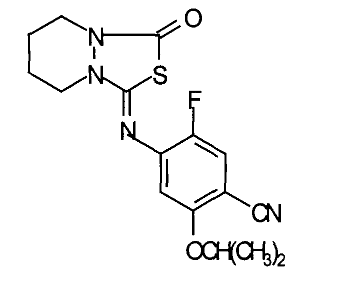

Zu einer Lösung von 9,7 g (0,03 Mol) 1-[N-(4-Cyano-2-fluor-5-i-propoxy-phenyl)]-tetrahydro-(2H)-pyridazin-thiocarboxamid in 150 ml Dichlormethan gibt man tropfenweise bei 20°C 26 ml (0,05 Mol) einer 20%igen Lösung von Phosgen in Toluol. Die Reaktionsmischung wird 3 Stunden bei 20°C gerührt und dann auf etwa die gleiche Menge Eiswasser gegeben. Die organische Phase wird abgetrennt, mit Magnesiumsulfat getrocknet und filtriert. Das Filtrat wird im Wasserstrahlvakuum eingeengt und der Rückstand durch Säulenchromatographie (Kieselgel, Dichlormethan) gereinigt.

Man erhält 1,4 g (13 % der Theorie) 9-(4-Cyano-2-fluor-5-isopropoxy-phenylimino)-8-thia-1,6-diazabicyclo[4.3.0]nonan-7-on vom Schmelzpunkt 108°C.

### Beispiel 2

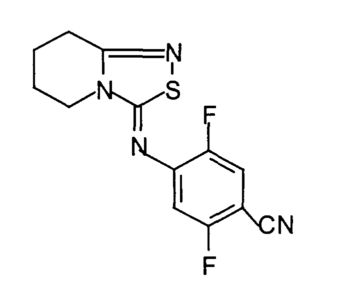

Zu einer Mischung aus 10,8 g (0,08 Mol) 2-Iminopiperidin-hydrochlorid und 100 ml Dichlormethan gibt man bei 0°C eine Lösung aus 3,2 g (0,08 Mol) Natriumhydroxid in 10 ml Wasser. Es wird eine Stunde nachgerührt, danach gibt man bei 0°C bis 5°C eine Lösung aus 15,7 g (0,08 Mol) 4-Cyano-2,5-difluorphenylisothiocyanat in 150 ml Dichlormethan tropfenweise hinzu. Es wird drei Stunden bei Raumtemperatur nachgerührt. Anschließend wird die Reaktionsmischung auf -20°C abgekühlt und mit 12,8 g (0,08 Mol) Brom in 40 ml Dichlormethan versetzt. Es wird drei Stunden nachgerührt, danach der Feststoff abfiltriert und das Filtrat nacheinander mit Natriumhydrogencarbonatlösung und Wasser gewaschen. Die organische Phase wird über Magnesiumsulfat getrocknet und im Vakuum vom Lösungsmittel befreit. Der verbleibende Feststoff wird aus wenig Acetonitril umkristallisiert.

Man erhält 3,1 g (13% der Theorie) 9-(4-Cyano-2,5-difluor-phenyl-imino)-8-thia-1,7-diazabicyclo[4.3.0]non-6-en vom Schmelzpunkt 137°C.

Analog Beispiel 1 und 2 sowie entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

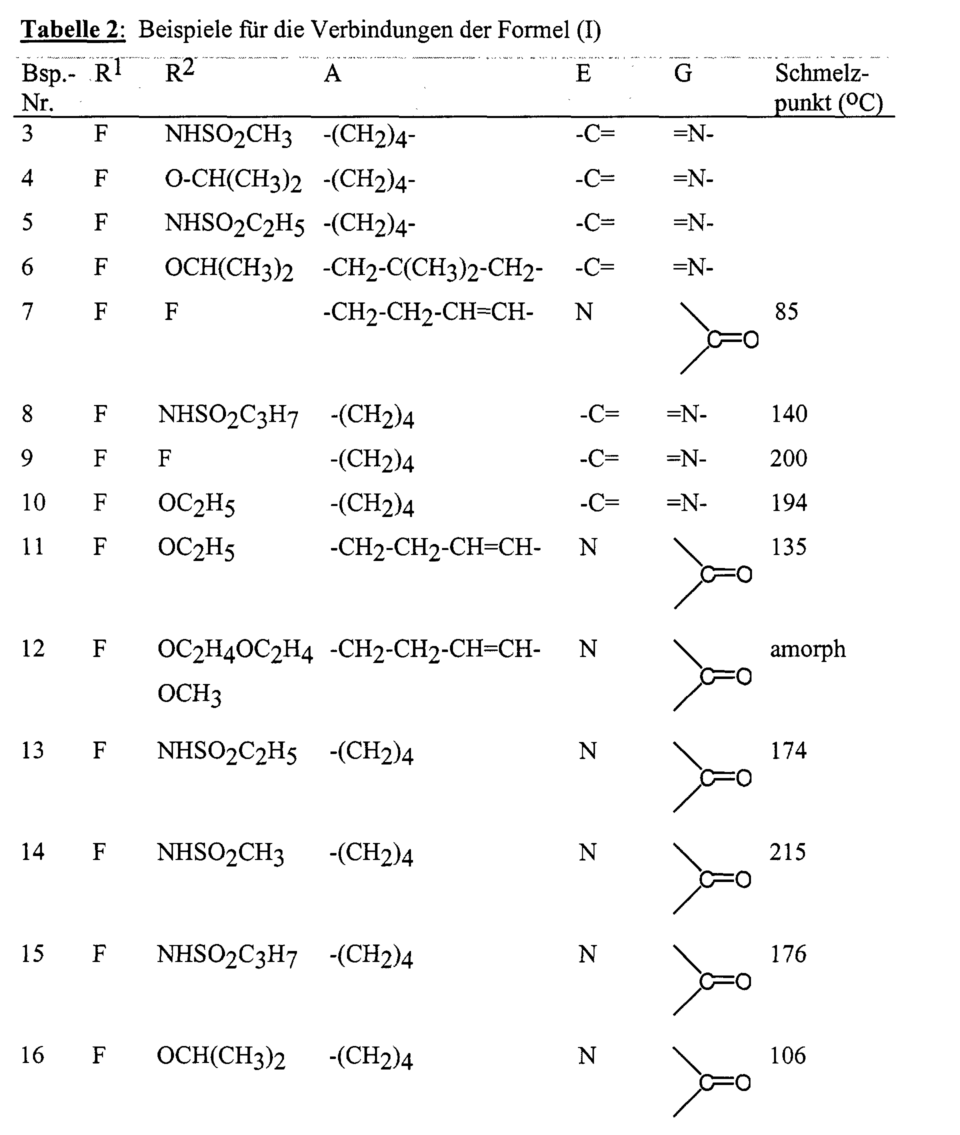

### Ausgangsstoffe der Formel (III):

### Beispiel (III-1)

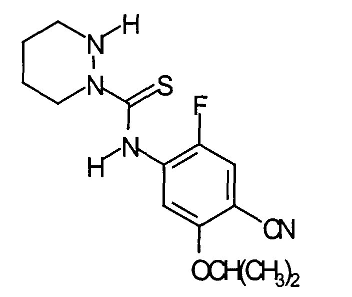

Zu einer Lösung von 2,9 g (34 mMol) Hexahydropyridazin in 40 ml Toluol gibt man unter Rühren bei 20°C 8,0 g (34 mMol) 4-Cyano-2-fluor-5-isopropoxy-phenylisothiocyanat. Das Reaktionsgemisch wird 16 Stunden bei 20°C gerührt und anschließend im Wasserstrahlvakuum eingeengt.

Man erhält 10,3 g (94 % der Theorie) 1-[N-(4-Cyano-2-fluor-5-isopropoxy-phenyl)]-tetrahydro-(2H)-pyridazin-thiocarboxamid als öligen Rückstand, welcher allmählich kristallin erstarrt.

Schmelzpunkt: 42°C.

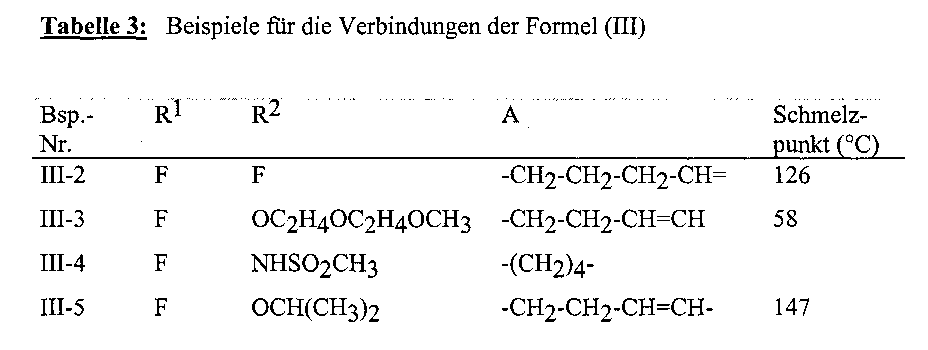

### Ausgangsstoffe der Formel (VIII):

### Beispiel (VIII-1)

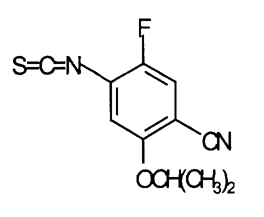

Zu einer Mischung aus 6 g (0,06 Mol) Calciumcarbonat, 30 ml Wasser, 6,9 g (0,06 Mol) Thiophosgen und 30 ml Dichlormethan gibt man unter Rühren bei ca. 30°C 7,8 g (0,04 Mol) 4-Cyano-2-fluor-5-isopropoxy-anilin in 25 ml Dichlormethan. Das Reaktionsgemisch wird ca. 18 Stunden bei 30°C bis 35°C gerührt. Dann wird nach Filtration die organische Phase abgetrennt, mit Magnesiumsulfat getrocknet und filtriert. Vom Filtrat wird das Lösungsmittel im Wasserstrahlvakuum sorgfältig abdestilliert.

Man erhält 8,4 g (89 % der Theorie) 4-Cyano-2-fluor-5-isopropoxy-phenyl-isothiocyanat als öligen Rückstand, welcher allmählich kristallin erstarrt.

Schmelzpunkt: 73°C.

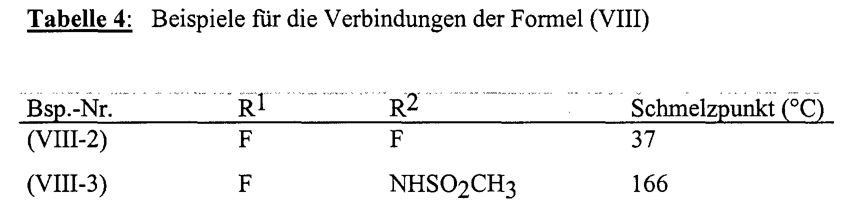

### Ausgangsstoffe der Formel (X):

### Beispiel X-1:

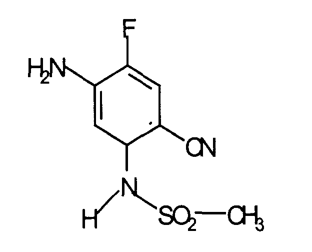

Eine Mischung aus 92,4 g (0,6 Mol) 4-Cyano-2,5-difluor-anilin, 60 g (0,60 Mol) Methansulfonamid, 166 g Kaliumcarbonat und 80 ml N-Methyl-pyrrolidon wird 10 Stunden auf 180°C erhitzt. Nach dem Erkalten wird die Mischung in 5 Liter Wasser eingerührt und die erhaltene Lösung zweimal mit je 400 ml Essigsäureethylester gewaschen. Die wäßrige Phase wird dann mit 300 ml Essigsäureethylester überschichtet und mit 10%iger Salzsäure angesäuert. Das kristallin anfallende Produkt wird dann durch Absaugen isoliert.

Man erhält 70 g (51% der Theorie) N-(5-Amino-2-cyano-4-fluor-phenyl)-methansul-fonamid vom Schmelzpunkt 238°C.

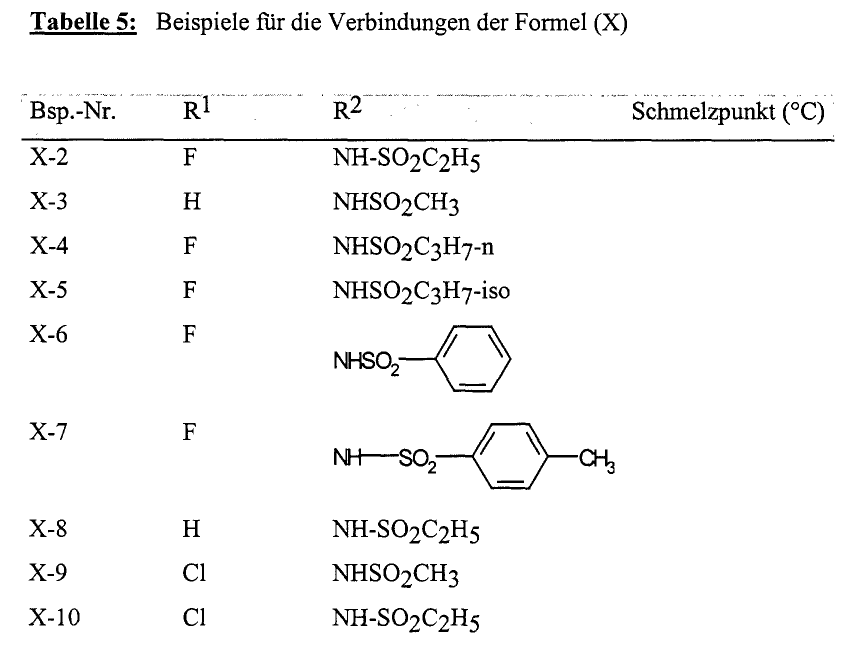

### Anwendungsbeispiele:

### Beispiel A

Post-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

### Es bedeuten:

- O % =: keine Wirkung (wie unbehandelte Kontrolle)
- 100 % =: totale Vernichtung

In diesem Test zeigt beispielsweise die Verbindung gemäß Herstellungsbeispiel 1 bei einer Aufwandmenge von 15 g/ha und sehr guter Verträglichkeit gegenüber Kulturpflanzen, wie z.B. Gerste (0 %), starke Wirkung gegen Unkräuter wie Abutilon (100 %), Amaranthus (80 %), Chenopodium (95 %), Ipomoea (100 %) und Veronica (85 %).

### Beispiel B

Pre-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle; entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

### Es bedeuten:

- O % =: keine Wirkung (wie unbehandelte Kontrolle)
- 100 % =: totale Vernichtung

In diesem Test zeigt beispielsweise die Verbindung gemäß Herstellungsbeispiel 1 bei einer Aufwandmenge von 60 g/ha und sehr guter Verträglichkeit gegenüber Kulturpflanzen, wie z.B. Gerste (0 %), starke Wirkung gegen Unkräuter wie Aeopecurus (90 %), Digiteria (95 %), Abutilon 100 %), Chenopodium 100 %), Matricaria (100 %) und Dinapis (80 %).

## Patentansprüche

1. Verbindungen der Formel (Xa) 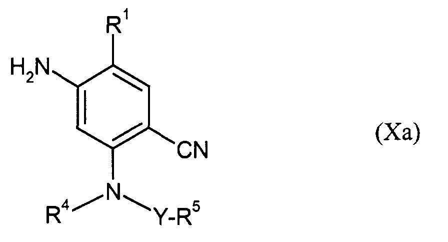 in welcher
R¹ für Wasserstoff oder Halogen steht,
R⁴ für Wasserstoff oder Alkyl mit 1 bis 8 Kohlenstoffatomen steht,
R⁵ für jeweils gegebenenfalls durch Fluor, Chlor, Brom, Cyano, Carboxy, C₁-C₄-Alkoxy, C₁-C₄-Alkoxy-C₁-C₄-alkoxy, C₁-C₄-Alkylthio, C₁-C₄-Alkylcarbonyl oder C₁-C₄-Alkoxy-carbonyl substituiertes Alkyl mit 1 bis 8 Kohlenstoffatomen steht,
R⁵ weiterhin für jeweils gegebenenfalls durch Fluor, Chlor oder Brom substituiertes Alkenyl oder Alkinyl mit jeweils 2 bis 8 Kohlenstoffatomen steht,
R⁵ weiterhin für gegebenenfalls durch Fluor, Chlor, Brom, Cyano, Carboxy, C₁-C₄-Alkyl oder C₁-C₄-Alkoxy-carbonyl substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im Alkylteil oder für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxycarbonyl (welches gegebenenfalls durch Fluor, Chlor, Brom, Methoxy oder Ethoxy substituiert ist), durch Phenyl, Phenylmethyl, Phenoxy, Phenylthio, Phenylsulfinyl oder Phenylsulfonyl (welche jeweils gegebenenfalls durch Fluor, Chlor, Brom, Cyano, Methyl, Methoxy, Trifluormethyl, Difluormethoxy und/oder Trifluormethoxy substituiert sind) substituiertes Aryl oder Arylalkyl mit 6 oder 10 Kohlenstoffatomen im Arylteil und gegebenenfalls 1 bis 4 Kohlenstoffatomen im Alkylteil steht, und
Y für CO oder SO₂ steht.

2. Verbindungen der Formel (Xa) gemäß Anspruch 1, **dadurch gekennzeichnet, dass**
R¹ für Wasserstoff, Fluor oder Chlor steht,
R⁴ für Wasserstoff, Methyl, Ethyl, n- oder i-Propyl, n-, i- oder s-Butyl steht,
R⁵ für jeweils gegebenenfalls durch Fluor, Chlor, Brom, Cyano, Carboxy, Methoxy, Ethoxy, Methoxyethoxy, Methylthio, Ethylthio, Acetyl, Propionyl, Methoxycarbonyl oder Ethoxycarbonyl substituiertes Methyl, Ethyl, n- oder i-Propyl, n-, i-, s oder t-Butyl steht,
R⁵ weiterhin für jeweils gegebenenfalls durch Fluor, Chlor substituiertes Allyl Crotonyl, 1-Methyl-allyl, Propargyl oder 1-Methyl-propargyl steht,
R⁵ weiterhin für jeweils gegebenenfalls durch Fluor, Chlor, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,

3. Verfahren zum Herstellen von Verbindungen der Formel (Xa) gemäß Anspruch 1 oder 2, **dadurch gekennzeichnet, dass** man Halogenarylamine der Formel (XI) 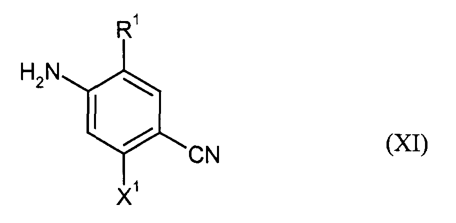 in welcher
R¹ und X¹ die in Anspruch 1 oder 2 angegebene Bedeutung haben,
mit Amiden der Formel (XII)
R⁴-NH-Y-R⁵ (XII)
in welcher
R⁴, R⁵ und Y die in Anspruch 1 oder 2 angegebene Bedeutung haben,
umsetzt.
